# EUROPÄISCHE PATENTSCHRIFT

(11) **EP 2 402 329 B1**
(45) Veröffentlichungstag und Bekanntmachung des Hinweises auf die Patenterteilung: **22.05.2013**
(21) Anmeldenummer: 11176839.6
(22) Anmeldetag: 14.08.2007
(51) Int. Cl.: C07D 401/02, C07D 205/04, C07D 207/06, C07D 223/04, C07D 403/04, C07D 403/06, C07C 311/16, C07C 311/29

(54) **Analgetische Phenylsulfonamide**
Analgetic phenylsulphonamides
Phénylsulfonamides analgesiques

(30) Priorität: 19.08.2006 DE 102006039003
(43) Veröffentlichungstag der Anmeldung: 04.01.2012
(62) Teilanmeldung aus: 07788420.3
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: Kauffmann-Hefner, Iris, 88448 Attenweiler (DE); Hauel, Norbert, 88433 Schemmerhofen (DE); Walter, Rainer, 88400 Biberach (DE); Ebel, Heiner, 88400 Mettenberg (DE); Doods, Henri, 88447 Warthausen (DE); Ceci, Angelo, 88441 Mittelbiberach (DE); Schuler-Metz, Annette, 89081 Ulm (DE); Konetzki, Ingo, 52076 Aachen-Oberforstbach (DE)
(74) Vertreter: Simon, Elke Anna Maria

(56) Entgegenhaltungen:
- WO-A-02/053516
- WO-A-03/106428
- WO-A-2006/036664

## Beschreibung

Gegenstand der vorliegenden Erfindung sind spezifische Verbindungen der allgemeinen Formel I 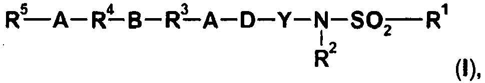 deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweise, deren Herstellung, die die pharmakologisch wirksamen Verbindungen enthaltenden Arzneimittel, deren Herstellung und deren Verwendung.

### DETAILLIERTE BESCHREIBUNG DER ERFINDUNG

Verbindungen der allgemeinen Formel **I,** nämlich

| **Beispiel** | **Struktur** |
|---|---|
| (1) | 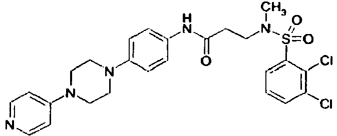 |
| (2) | 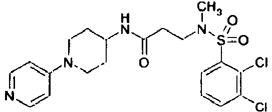 |
| (3) | 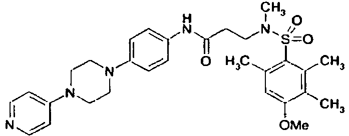 |
| (4) | 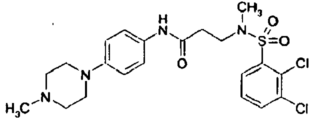 |
| (5) | 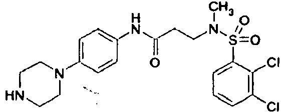 |
| (6) | 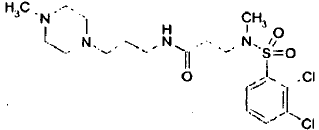 |
| (7) | 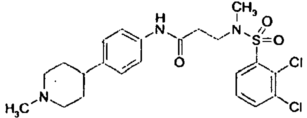 |
| (8) | 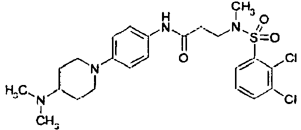 |
| (9) | 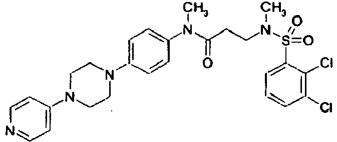 |
| (10) | 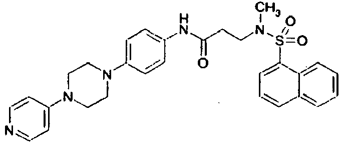 |
| (11) | 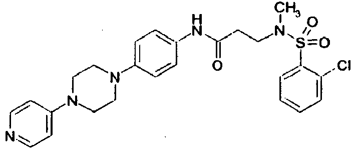 |
| (12) | 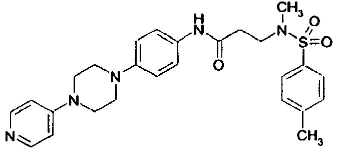 |
| (13) | 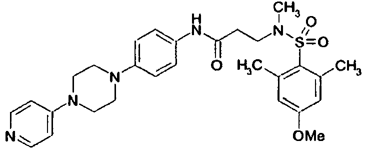 |
| (14) | 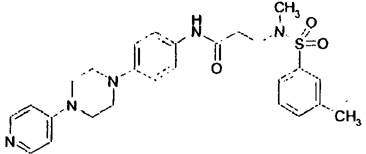 |
| (15) | 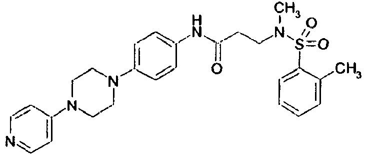 |
| (16) | 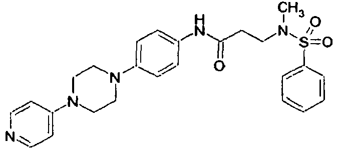 |
| (17) | 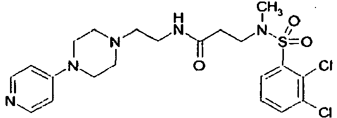 |
| (18) | 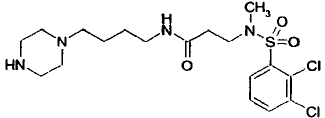 |
| (19) | 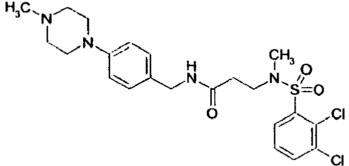 |
| (20) | 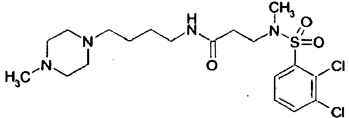 |
| (21) | 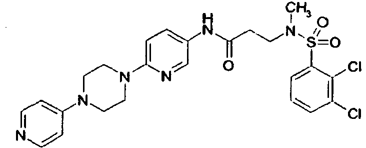 |
| (22) | 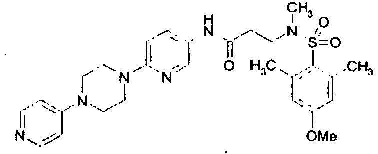 |
| (23) | 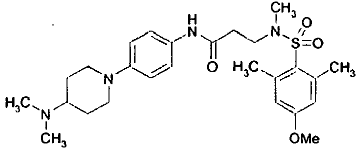 |
| (24) | 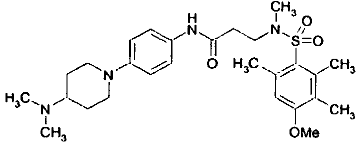 |
| (25) | 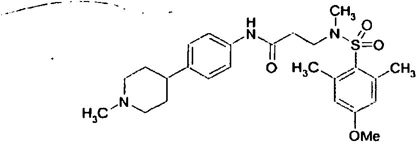 |
| (26) | 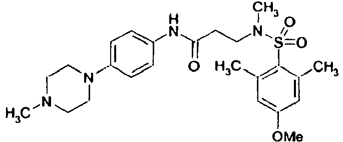 |
| (27) | 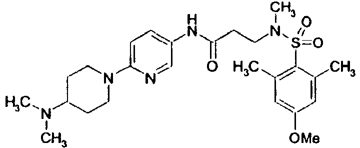 |
| (28) | 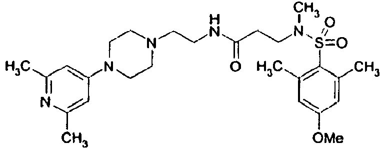 |
| (29) | 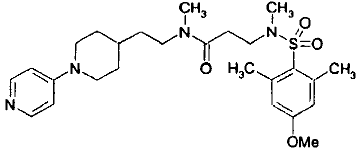 |
| (30) | 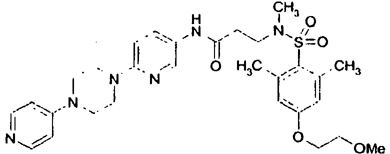 |
| (31) | 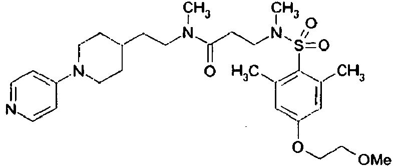 |
| (32) | 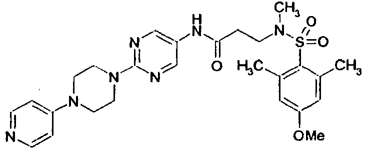 |
| (33) | 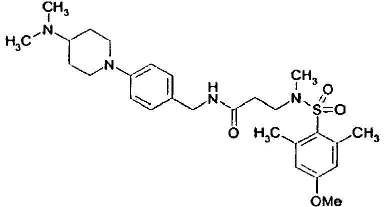 |
| (34) | 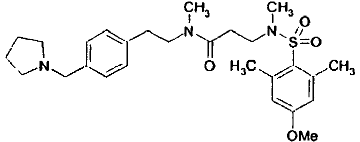 |
| (35) | 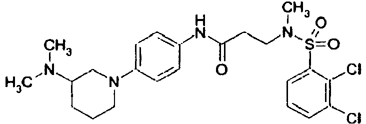 |
| (36) | 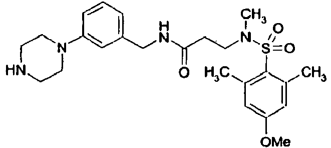 |
| (37) | 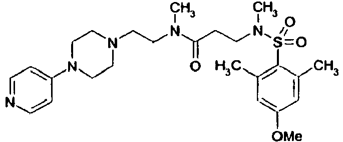 |
| (38) | 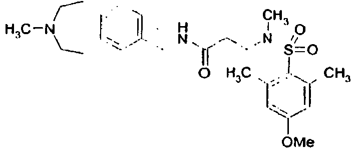 |
| (39) | 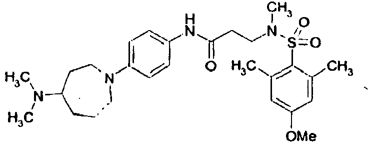 |
| (40) | 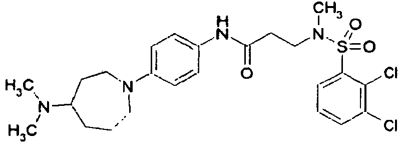 |
| (41) | 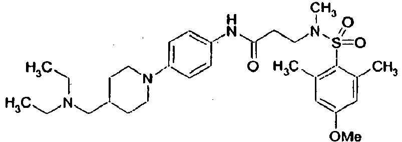 |
| (42) | 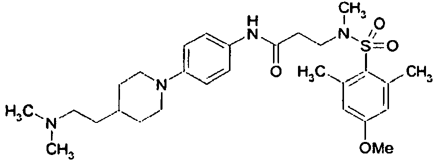 |
| (43) | 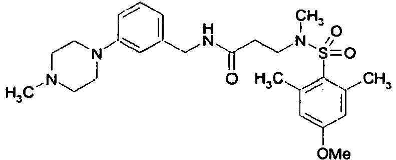 |
| (44) | 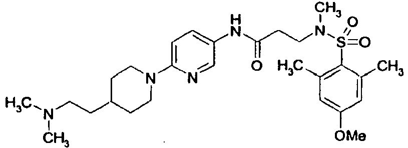 |
| (45) | 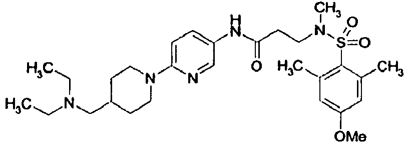 |
| (46) | 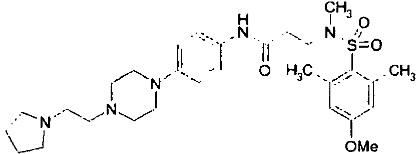 |
| (47) | 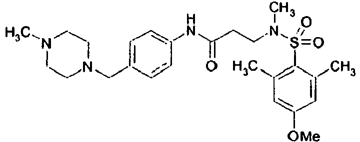 |
| (48) | 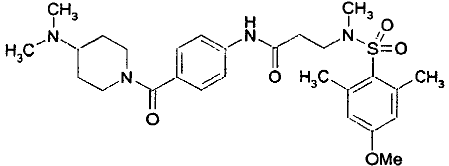 |
| (49) | 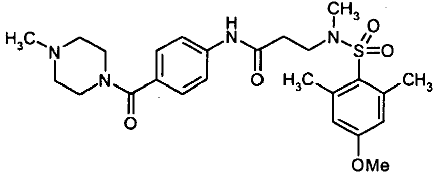 |
| (50) | 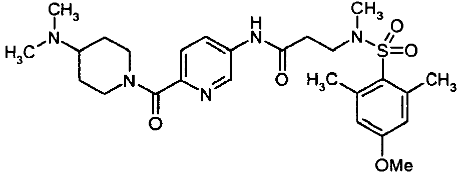 |
| (51) | 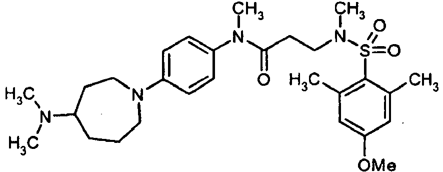 |
| (52) | 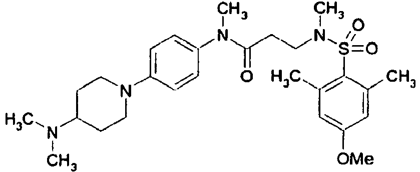 |
| (83) | 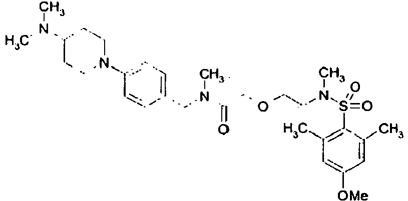 |
| (84) | 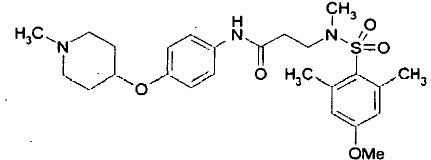 |
| (85) | 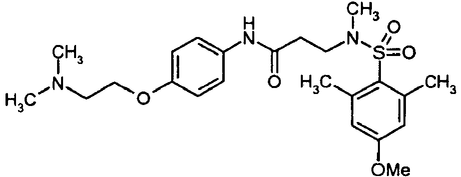 |
| (86) | 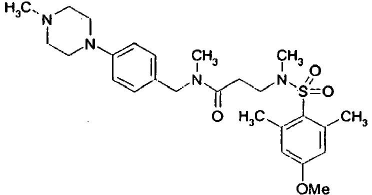 |
| (87) | 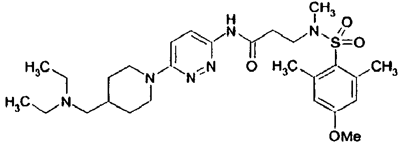 |
| (88) | 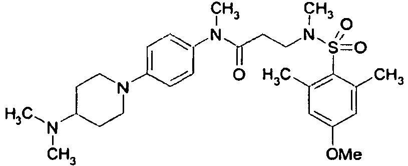 |
| (89) | 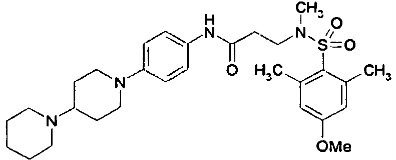 |
| (90) | 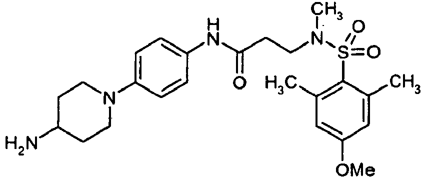 |
| (91) | 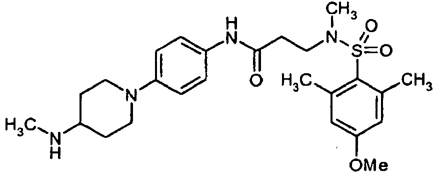 |
| (92) | 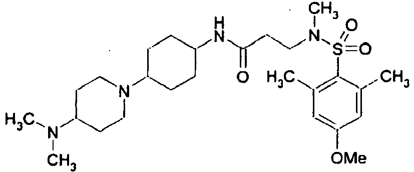 |
| (93) | 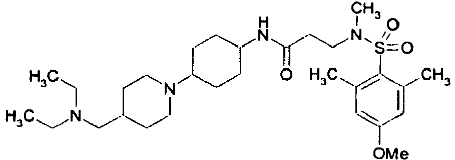 |
| (94) | 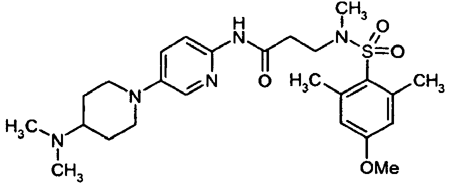 |
| (95) | 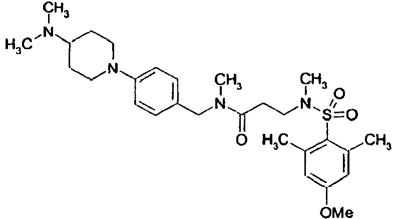 |
| (96) | 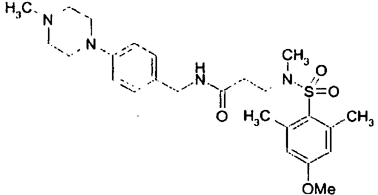 |
| (97) | 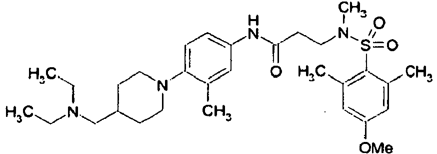 |
| (98) | 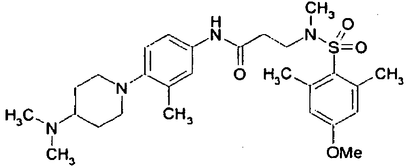 |
| (99) | 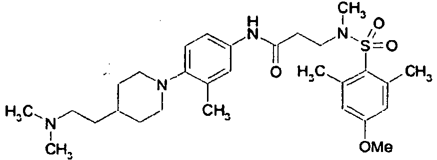 |
| (100) | 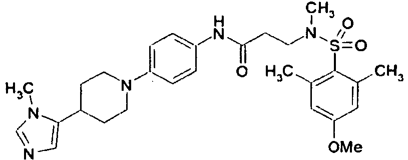 |
| (101) | 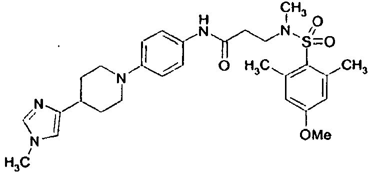 |
| (140) | 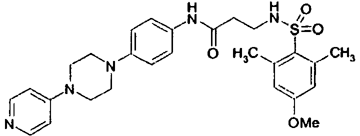 |
| (141) | 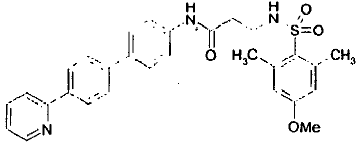 |
| (142) | 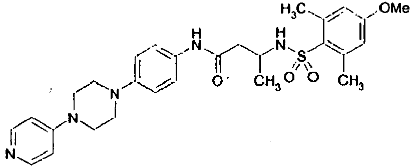 |
| (143) | 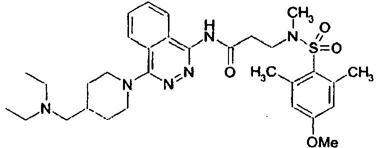 |
| (144) | 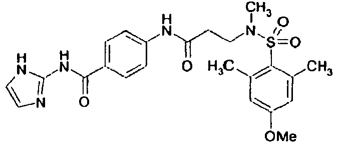 |
| (145) | 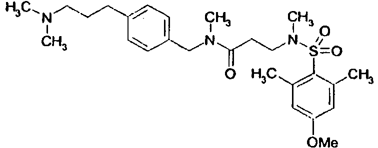 |
| (146) | 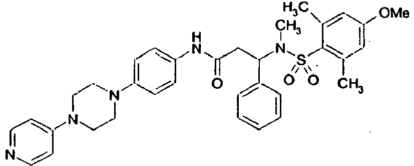 |
| (147) | 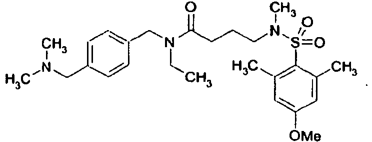 |
| (148) | 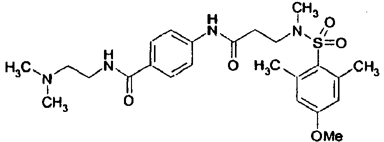 |
| (149) | 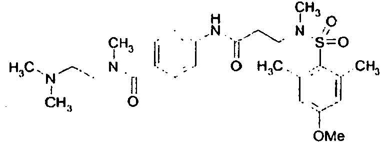 |
| (150) | 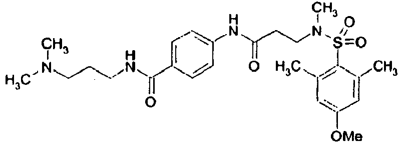 |
| (151) | 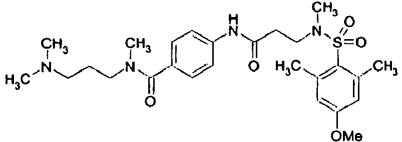 |
| (152) | 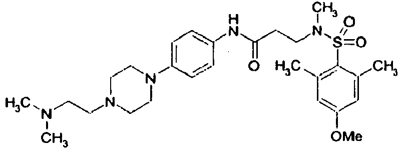 |
| (153) | 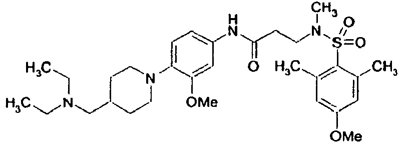 |
| (154) | 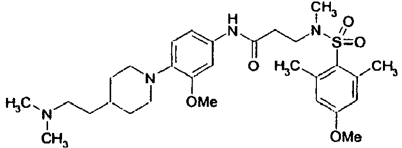 |
| (155) | 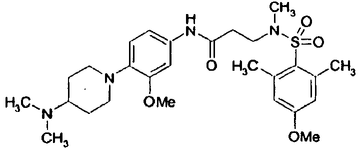 |
| (156) | 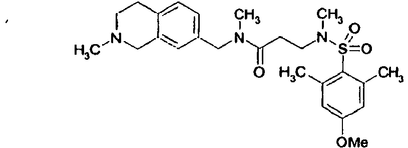 |
| (157) | 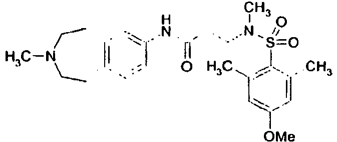 |
| (158) | 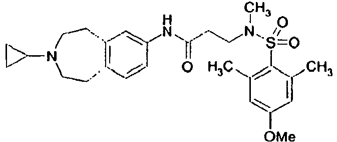 |
| (159) | 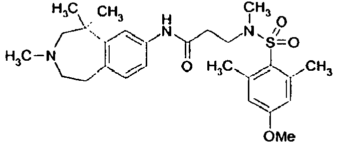 |
| (160) | 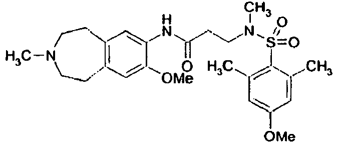 |
| (161) | 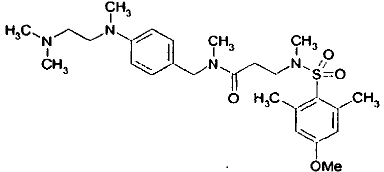 |
| (162) | 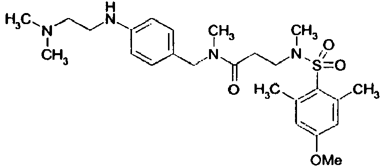 |
| (163) | 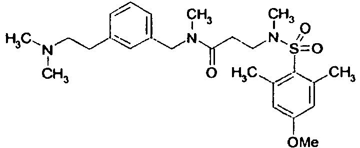 |
| (164) | 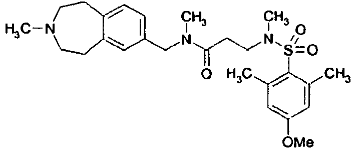 |
| (165) | 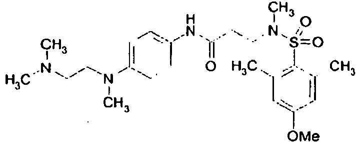 |
| (166) | 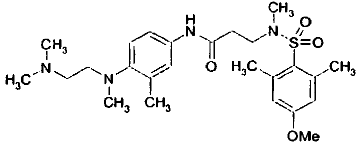 |
| (167) | 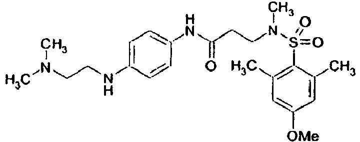 |
| (168) | 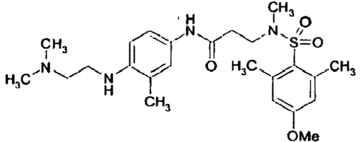 |
| (169) | 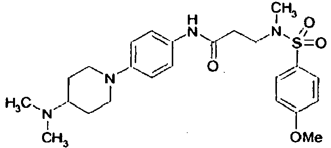 |
| (170) | 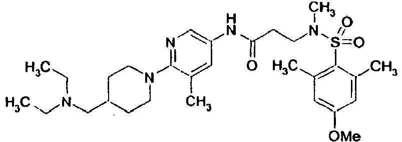 |
| (171) | 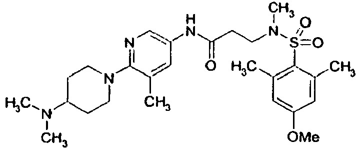 |
| (172) | 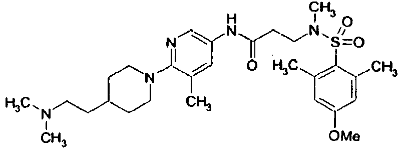 |
| (173) | 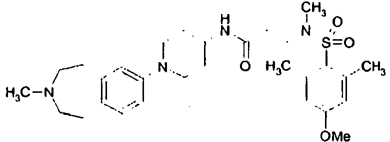 |
| (174) | 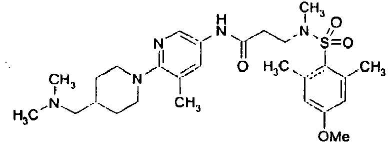 |
| (175) | 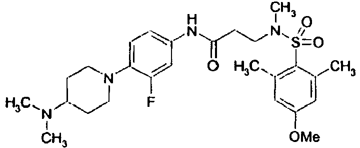 |
| (176) | 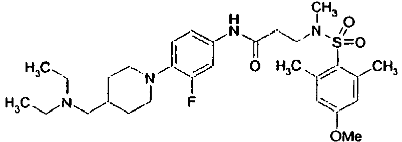 |
| (177) | 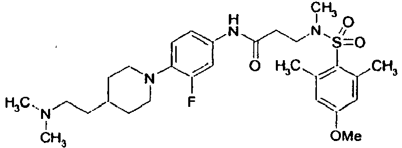 |
| (178) | 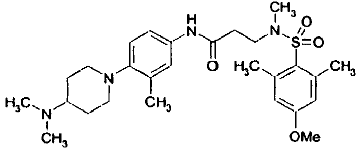 |
| (179) | 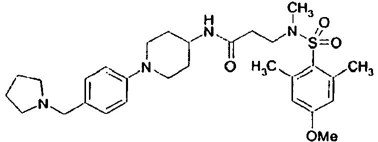 |
| (180) | 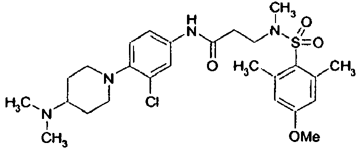 |
| (181) | 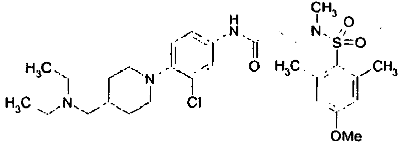 |
| (182) | 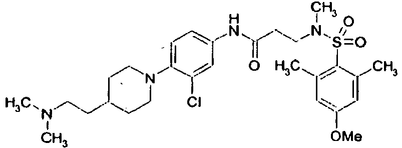 |
| (183) | 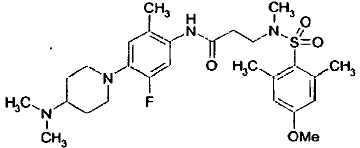 |
| (184) | 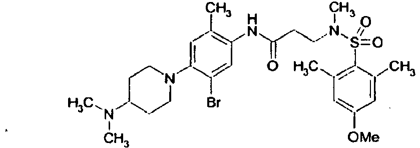 |
| (185) | 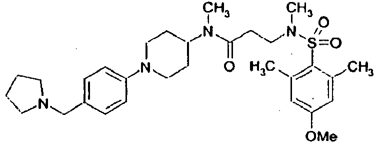 |
| (186) | 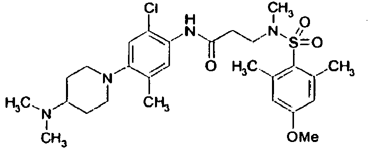 |
| (187) | 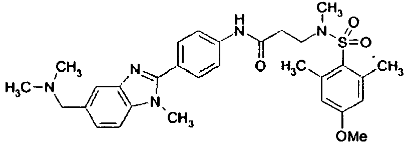 |
| (188) | 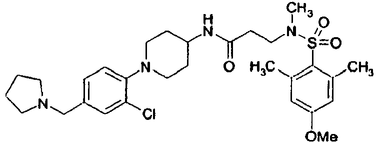 |
| (189) | 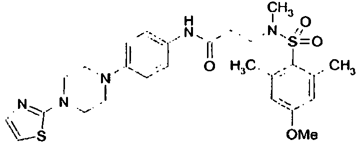 |
| (190) | 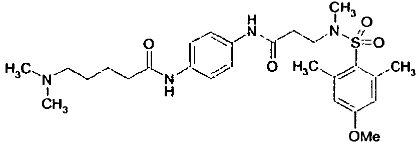 |
| (191) | 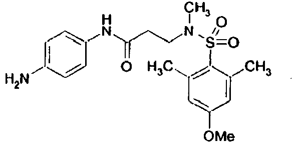 |
| (192) | 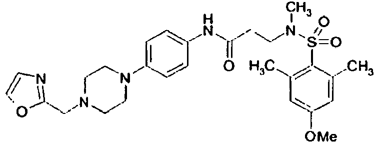 |
| (193) | 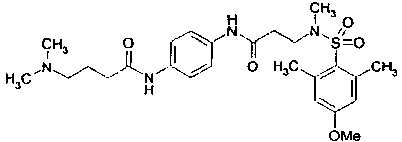 |
| (194) | 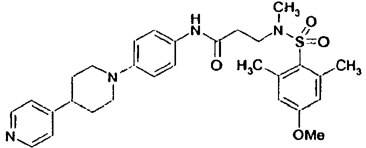 |
| (195) | 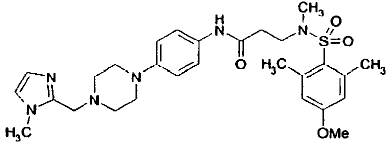 |
| (196) | 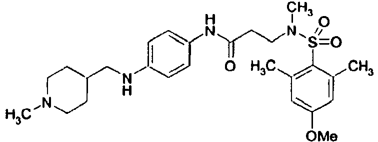 |
| (197) | 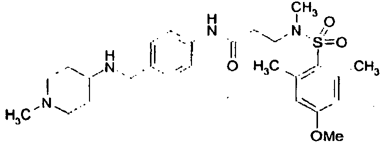 |
| (198) | 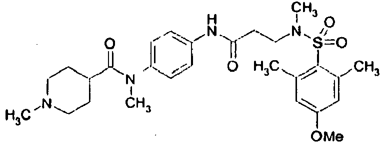 |
| (199) | 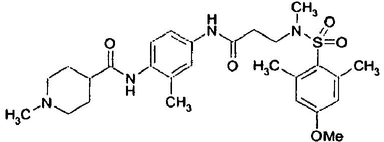 |
| (200) | 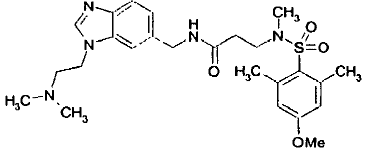 |
| (201) | 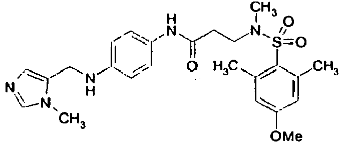 |
| (202) | 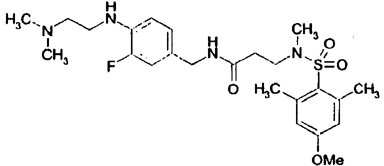 |
| (203) | 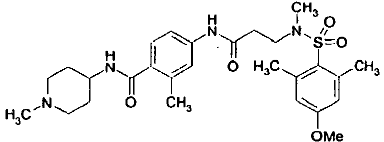 |
| (204) | 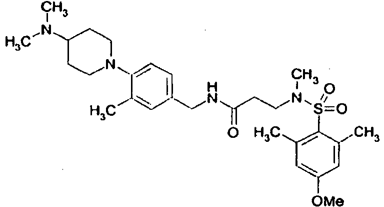 |
| (205) | 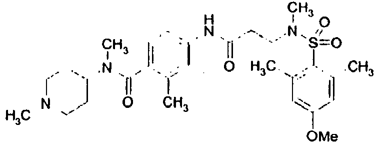 |
| (206) | 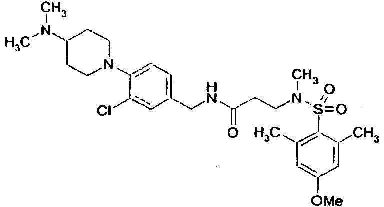 |
| (207) | 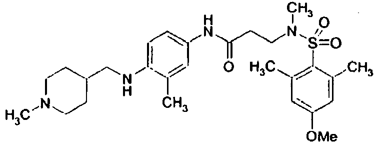 |
| (208) | 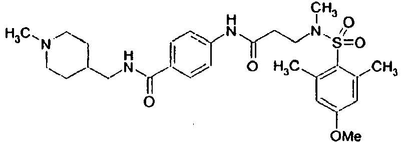 |
| (209) | 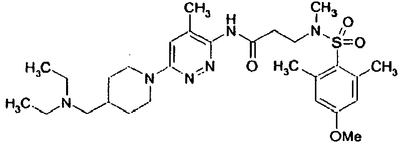 |
| (210) | 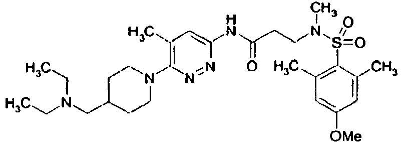 |
| (211) | 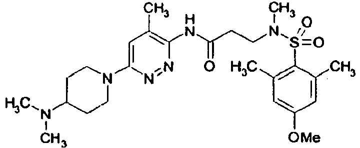 |
| (212) | 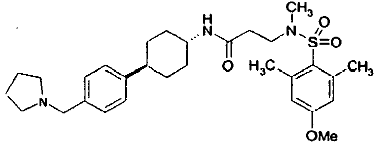 |
| (213) | 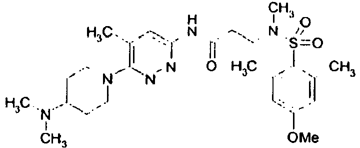 |
| (214) | 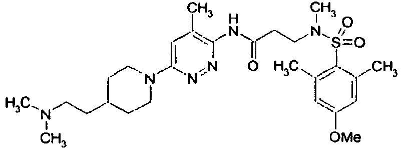 |
| (215) | 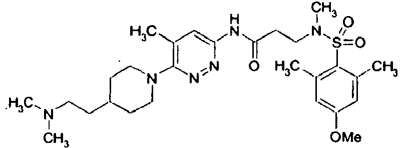 |
| (216) | 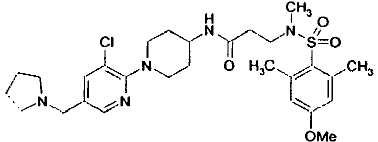 |
| (217) | 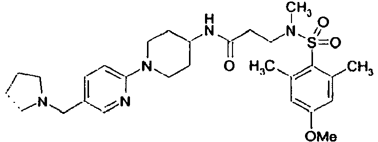 |
| (218) | 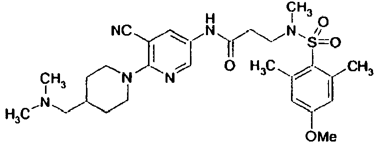 |
| (219) | 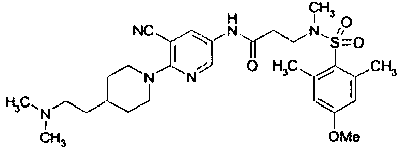 |
| (220) | 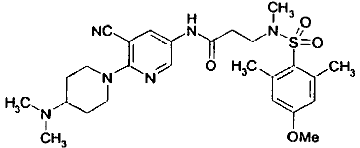 |
| (221) | 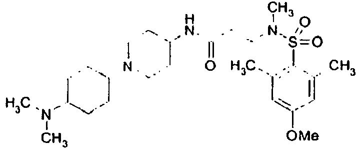 |
| (222) | 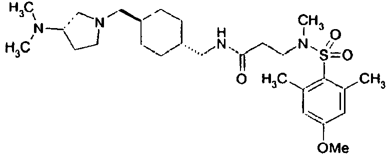 |
| (223) | 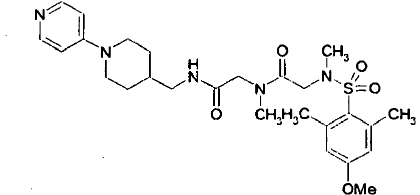 |
| (224) | 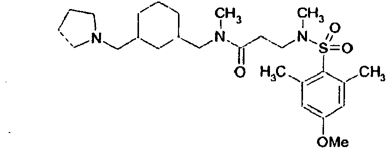 |
| (225) | 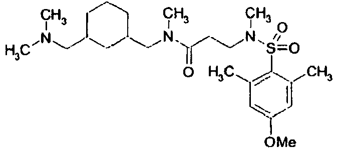 |
| (226) | 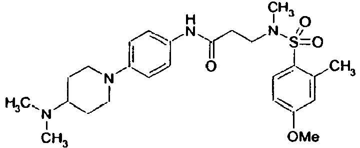 |
| (227) | 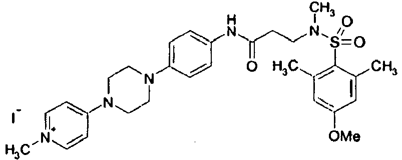 |
| (228) | 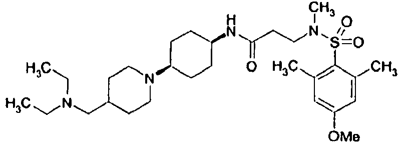 |
| (229) | 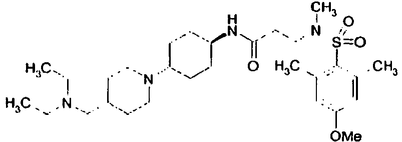 |
| (230) | 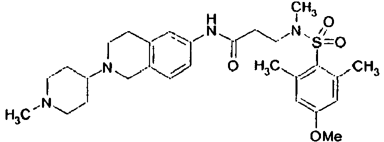 |
| (231) | 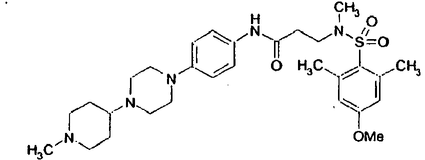 |
| (232) | 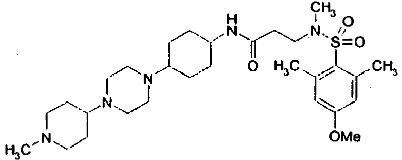 |
| (233) | 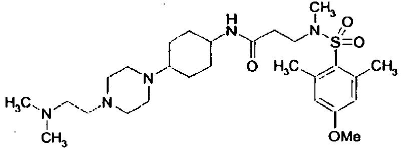 |
| (234) | 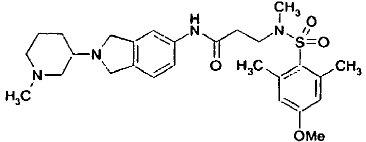 |
| (235) | 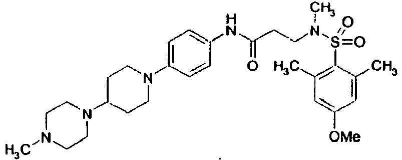 |
| (236) | 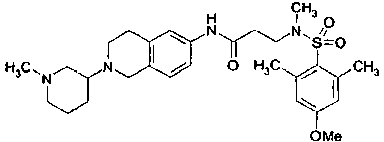 |
| (237) | 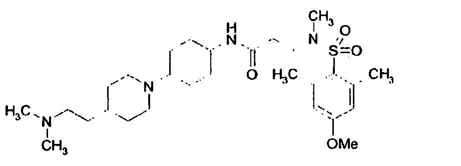 |
| (238) | 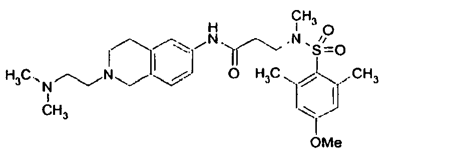 |
| (239) | 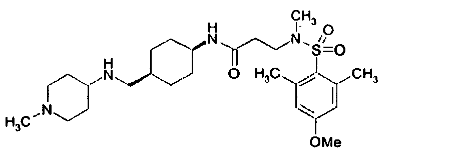 |
| (240) | 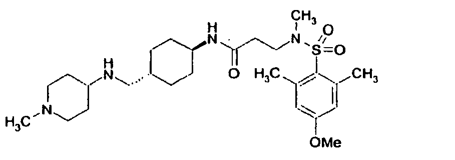 |
| (241) | 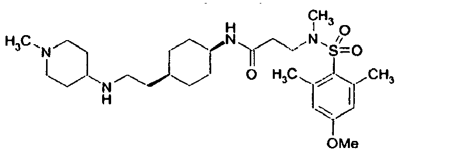 |
| (242) | 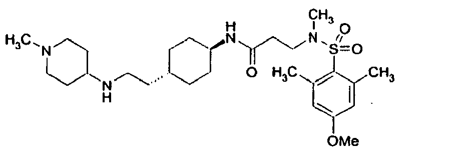 |
| (243) | 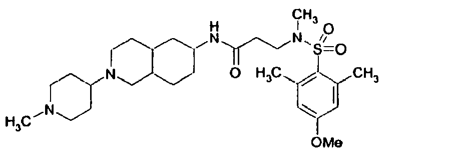 |
| (244) | 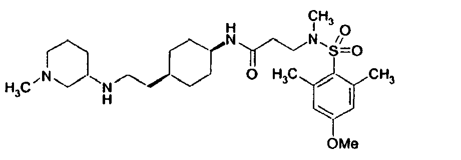 |
| (245) | 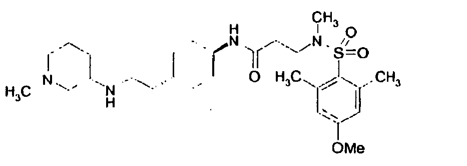 |
| (246) | 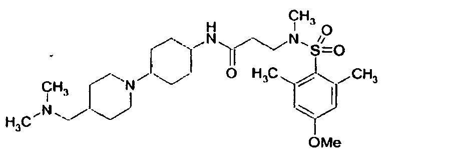 |
| (247) | 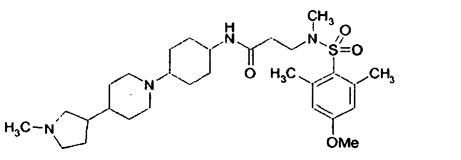 |
| (248) | 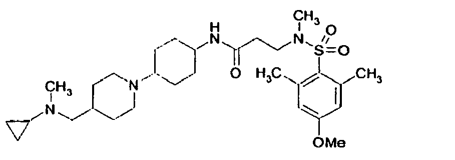 |
| (249) | 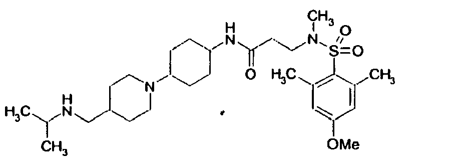 |
| (250) | 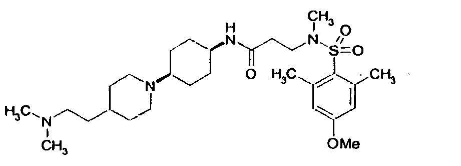 |
| (251) | 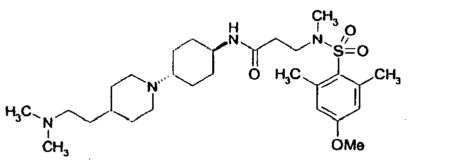 |
| (252) | 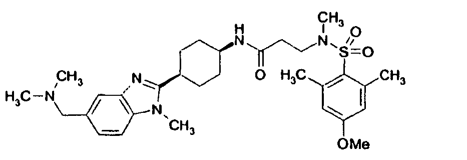 |
| (253) | 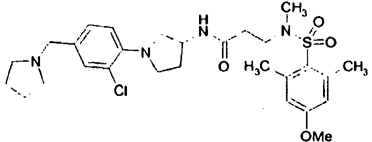 |
| (254) | 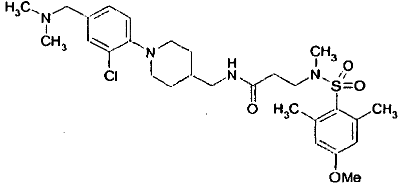 |
| (255) | 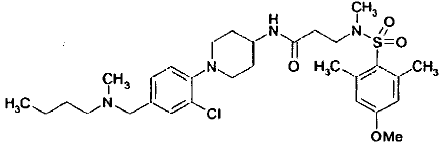 |
| (256) | 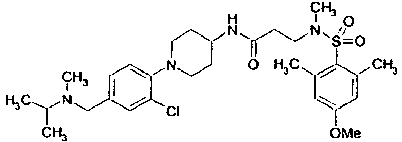 |
| (257) | 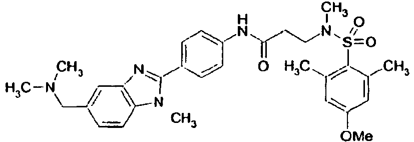 |
| (258) | 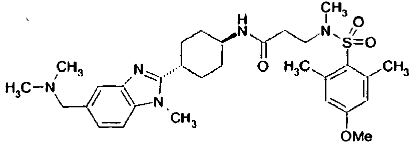 |
| (259) | 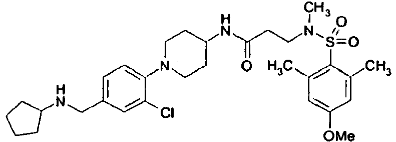 |
| (260) | 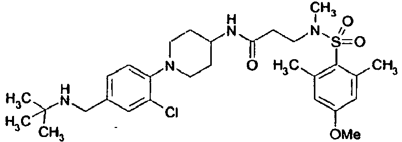 |
| (261) | 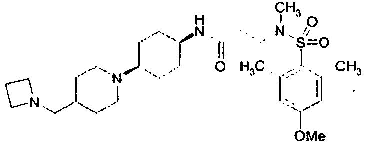 |
| (262) | 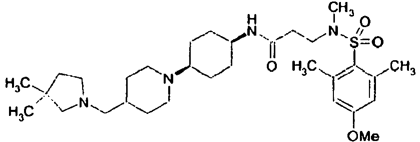 |
| (263) | 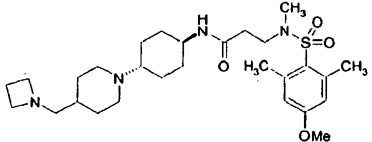 |
| (264) | 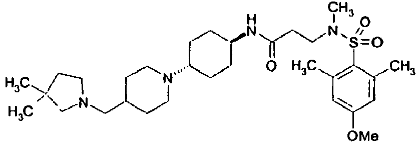 |
| (265) | 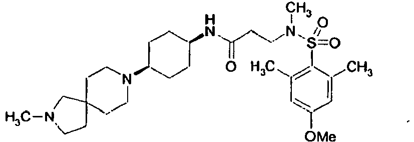 |
| (266) | 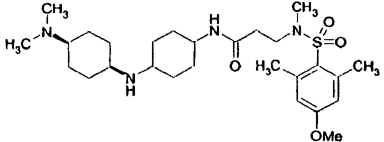 |
| (267) | 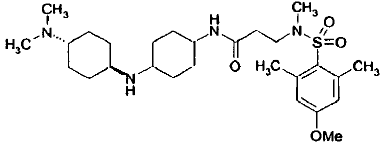 |
| (268) | 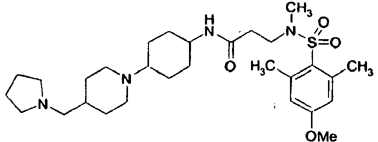 |
| (269) | 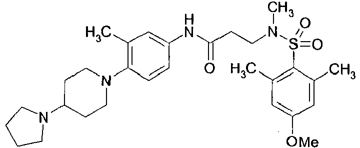 |
| (270) | 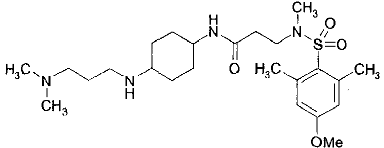 |
| (271) | 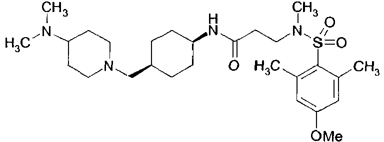 |
| (272) | 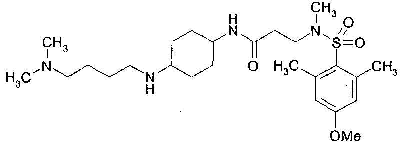 |
| (273) | 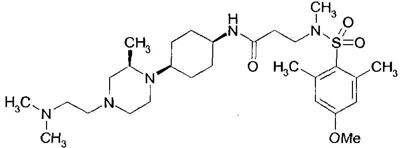 |
| (274) | 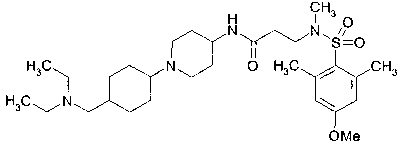 |
| (275) | 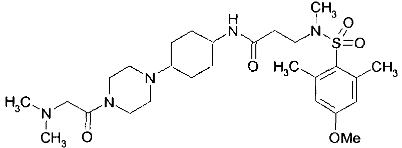 |
| (276) | 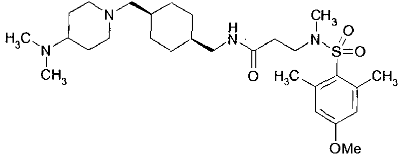 |
| (277) | 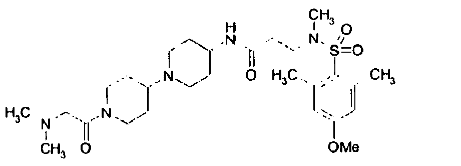 |
| (278) | 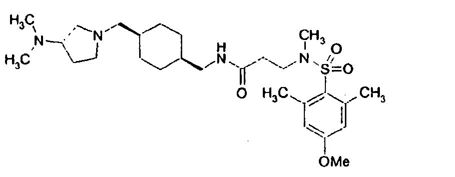 |
| (279) | 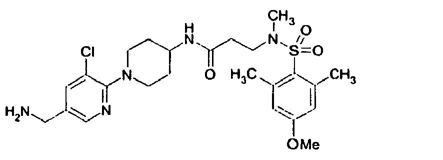 |
| (280) | 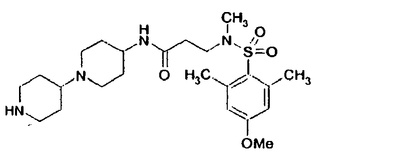 |
| (281) | 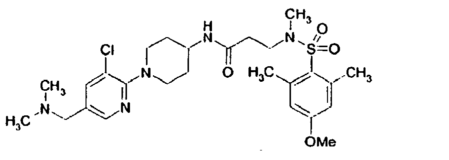 |
| (282) | 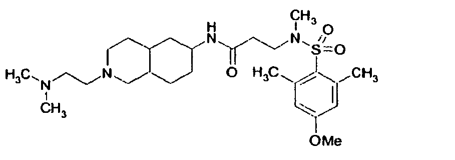 |
| (283) | 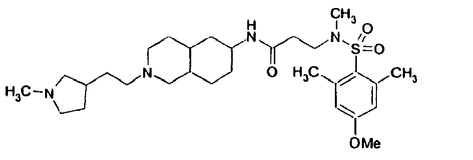 |
| (284) | 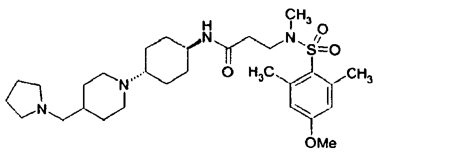 |
| (285) | 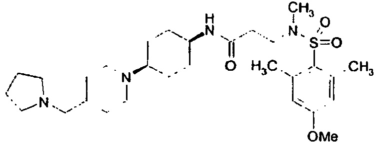 |
| (286) | 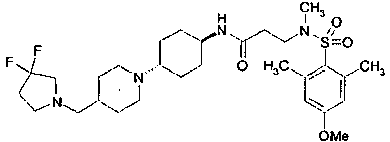 |
| (287) | 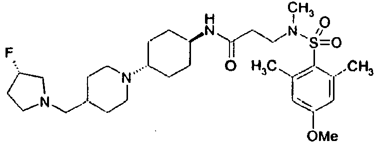 |
| (288) | 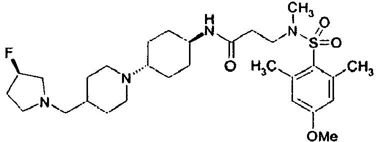 |
| (289) | 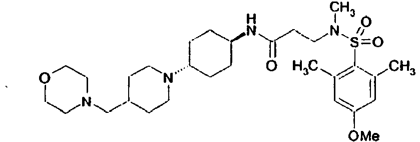 |
| (290) | 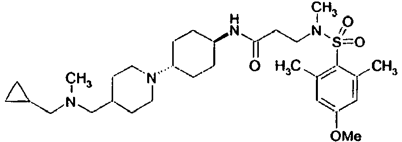 |
| (291) | 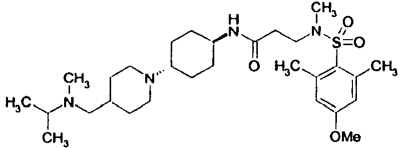 |
| (292) | 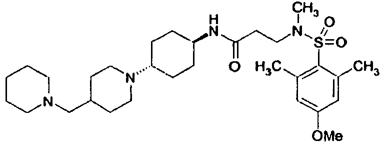 |
| (293) | 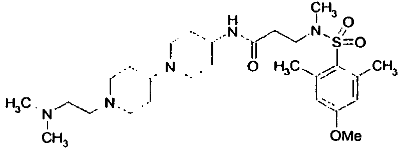 |
| (294) | 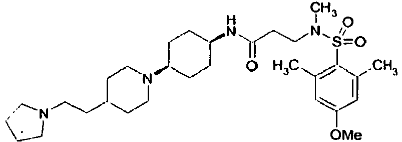 |
| (295) | 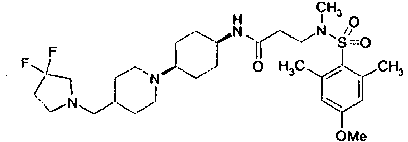 |
| (296) | 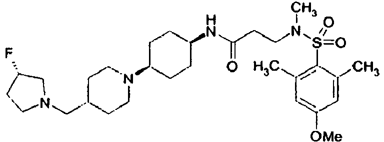 |
| (297) | 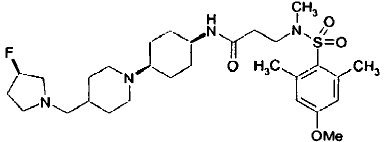 |
| (298) | 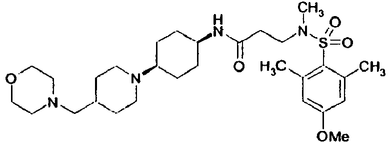 |
| (299) | 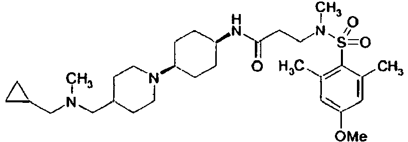 |
| (300) | 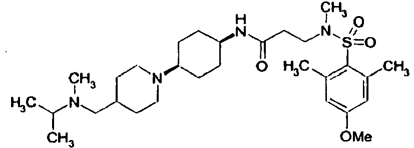 |
| (301) | 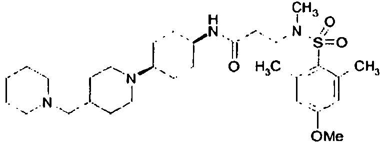 |
| (302) | 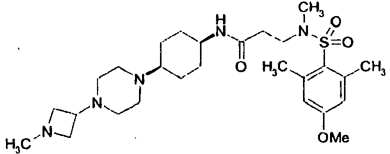 |
| (303) | 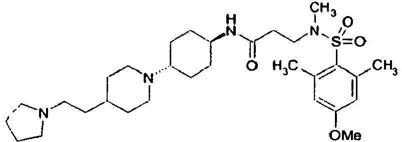 |
| (304) | 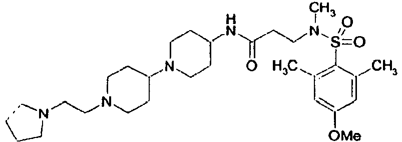 |
| (305) | 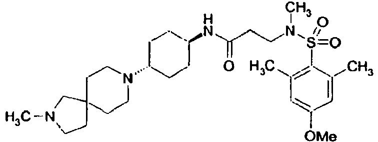 |
| (306) | 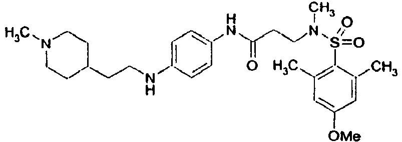 |
| (307) | 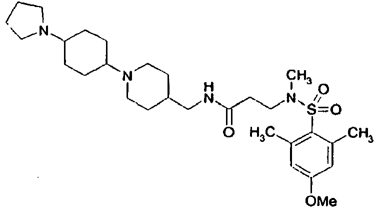 |
| (308) | 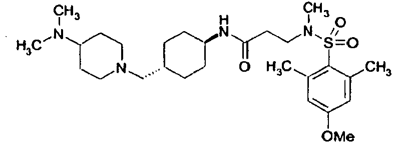 |
| (309) | 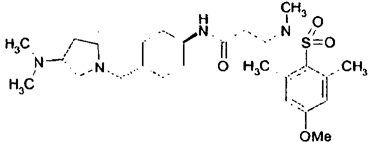 |
| (310) | 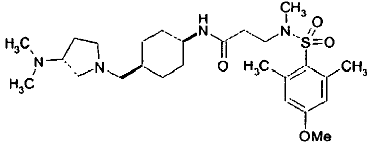 |
| (311) | 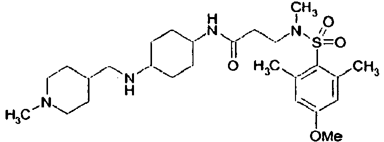 |
| (312) | 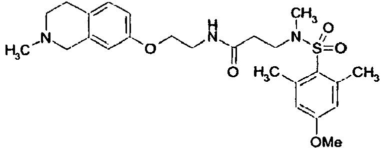 |
| (313) | 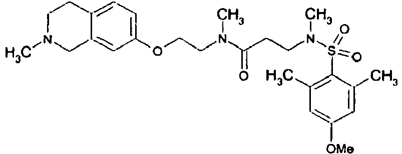 |
| (314) | 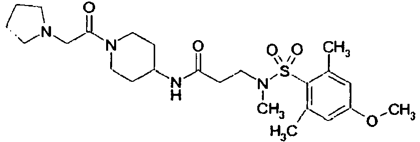 |
| (315) | 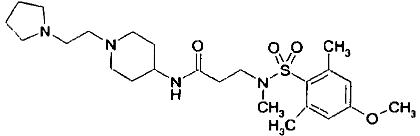 |
| (316) | 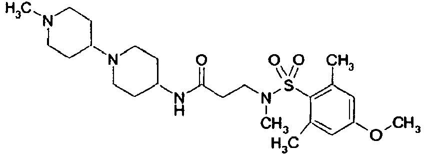 |
| (317) | 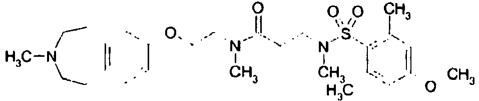 |
| (549) | 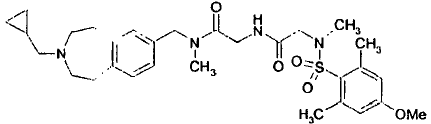 |
| (551) | 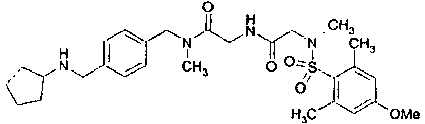 |
| (563) | 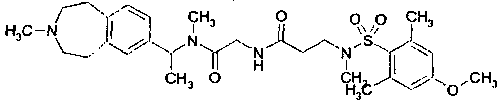 |
| (593) | 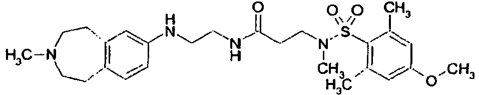 |
| (594) | 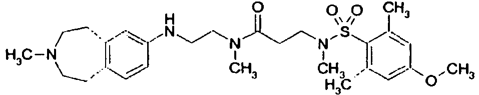 |
| (595) | 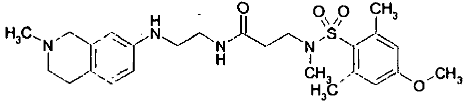 |
| (596) | 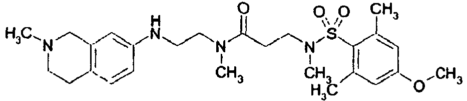 |
| (597) | 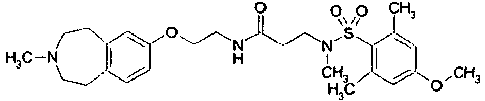 |
| (598) | 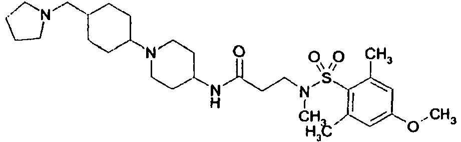 |
| (599) | 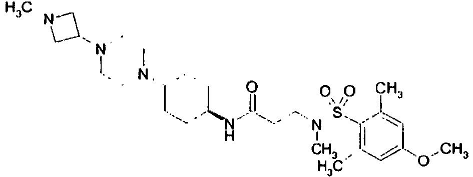 |
| (600) | 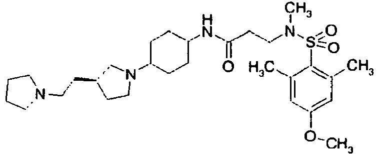 |
| (601) | 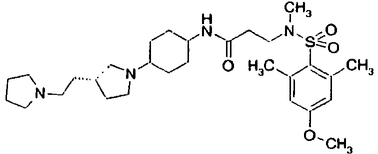 |
| (602) | 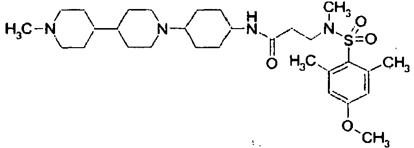 |
| (606) | 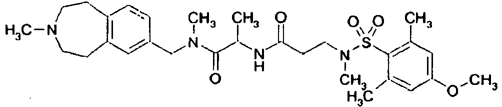 |
| (607) | 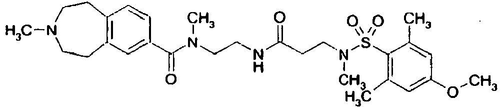 |
| (608) | 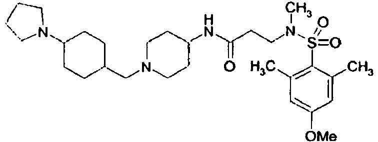 |
| (609) | 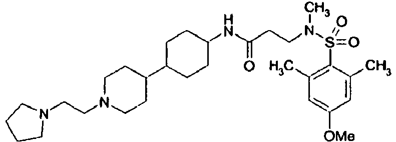 |
| (610) | 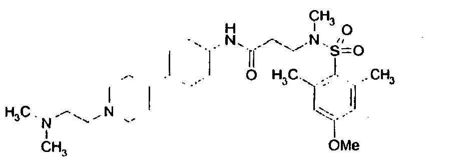 |
| (611) | 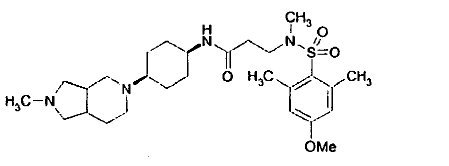 |
| (636) | 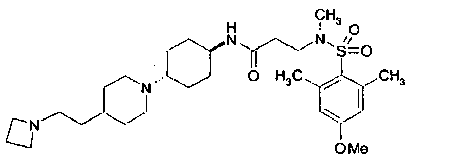 |
| (637) | 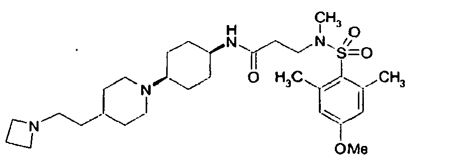 |

deren Enantiomere, deren Diastereomere, deren Gemische und deren Salze, insbesondere deren physiologisch verträgliche Salze mit organischen oder anorganischen Säuren oder Basen.

### VERWENDETE BEGRIFFE UND DEFINITIONEN

Verbindungen der allgemeinen Formel I können, sofern sie geeignete basische Funktionen enthalten, beispielsweise Aminogruppen, insbesondere für pharmazeutische Anwendungen in ihre physiologisch verträglichen Salze mit anorganischen oder organischen Säuren übergeführt werden. Als anorganische Säuren kommen hierfür beispielsweise Bromwasserstoffsäure, Phosphorsäure, Salpetersäure, Salzsäure, Schwefelsäure, Methansulfonsäure, Ethansulfonsäure, Benzolsulfonsäure oder p-Toluolsulfonsäure in Frage, als organische Säuren kommen beispielsweise Äpfelsäure, Bernsteinsäure, Essigsäure, Fumarsäure, Maleinsäure, Mandelsäure, Milchsäure, Weinsäure oder Zitronensäure in Betracht. Weiterhin können gegebenenfalls im Molekül vorhandene tertiäre Aminogruppen quarternisiert werden. Zur Umsetzung werden dabei Alkylhalogenide eingesetzt. Erfindungsgemäß bevorzugt wird für die Quartenisierung Methyliodid verwendet.

Weiterhin lassen sich die Verbindungen der allgemeinen Formel I, sofern sie geeignete Carbonsäurefunktionen enthalten, gewünschtenfalls in ihre Additionssalze mit anorganischen oder organischen Basen überführen. Als anorganische Basen kommen hierfür beispielsweise Alkali- oder Erdalkalimetallhydroxide, beispielsweise Natriumhydroxid oder Kaliumhydroxid, oder Carbonate, Ammoniak, Zink- oder Ammoniumhydroxide in Frage; als organische Amine kommen beispielsweise Diethylamin, Triethylamin, Ethanolamin, Diethanolamin, Triethanolamin, Cyclohexylamin oder Dicyclohexylamin in Betracht.

Die erfindungsgemäßen Verbindungen können als Racemate vorliegen, sofern sie nur ein Chiralitätselement besitzen, sie können aber auch als reine Enantiomere, d.h. in (*R*)- oder (*S*)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel I vorhanden ist, sowie die einzelnen optisch aktiven Enatiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

### Methodenbeschreibuna zur hBK1-Rezeptorbindung

CHO-Zellen, die den hBK1-Rezeptor exprimieren, werden in "Dulbecco's modified Medium" kultiviert. Von konfluenten Kulturen wird das Medium entfernt, die Zellen werden mit PBS-Puffer gewaschen, abgeschabt und durch Zentrifugieren isoliert. Anschließend werden die Zellen in Suspension homogenisiert, das Homogenat zentrifugiert und resuspendiert. Nach Bestimmung des Proteingehalts wird die so erhaltene Membranpräparation bei -80°C eingefroren.

Nach dem Auftauen werden 200 µl des Homogenats (50 bis100 µg Protein/Assay) für 60 Minuten bei Raumtemperatur mit 0.5 bis 1.0 nM Kallidin (DesArg10,Leu9), [3,4-Prolyl-3,43H(N)] und ansteigenden Konzentrationen der Testsubstanz in einem Gesamtvolumen von 250 µl inkubiert. Die Inkubation wird durch rasche Filtration durch GF/B-Glasfaserfilter, die mit Polyethyleneimine (0.3%) vorbehandelt wurden, beendet. Die an das Protein gebundene Radioaktivität wird mit einem TopCount NXT gemessen. Als nichtspezifische Bindung wird die gebundene Radioaktivität in Gegenwart von 1.0 µM Kallidin (DesArg10,Leu9), [3,4-Prolyl-3,43H(N)] definiert. Die Analyse der Konzentrations-Bindungskurve erfolgt mit Hilfe einer computergestützten nichtlinearen Kurvenanpassung. Aus den so erhaltenen Daten wird für die Testsubstanz der entsprechende Kᵢ - Wert ermittelt.

Um zu zeigen, dass die Verbindungen der allgemeinen Formel **I** mit unterschiedlichen Strukturelementen gute bis sehr gute Bradikinin-B1-Rezeptor antagonistische Aktivitäten zeigen, werden in der folgenden Tabelle die Kᵢ-Werte angegeben, die gemäß dem voranstehenden Versuchsprotokoll erhalten wurden. Dazu wird angemerkt, dass die Verbindungen im Hinblick auf ihre unterschiedlichen strukturellen Elemente ausgewählt wurden und nicht um spezifische Verbindungen hervorzuheben:

| **Beispiel** | **Kᵢ [nM]** |
|---|---|
| (1) | 6.2 |
| (13) | 2.1 |
| (22) | 7 |
| (97) | 6.7 |
| (180) | 5.2 |
| (181) | 7.1 |
| (182) | 4.8 |
| (183) | 6.6 |
| (184) | 1.3 |
| (186) | 3.4 |
| (188) | 9.4 |
| (216) | 4.9 |
| (227) | 4.8 |
| (269) | 7.8 |
| (303) | 6.32 |

### INDIKATIONEN

Aufgrund ihrer pharmakologischen Eigenschaften eignen sich die neuen Verbindungen und deren physiologisch verträglichen Salze zur Behandlung von Krankheiten und Krankheitssymptomen, die wenigstens teilweise durch die Stimulierung von Bradykinin-B1-Rezeptoren hervorgerufen werden.

Aufgrund ihrer pharmakologischen Wirkung eignen sich die Substanzen zur Behandlung
(a) von **akuten Schmerzen** wie z.B. Zahnschmerzen, peri- und postoperativen Schmerzen, traumatischen Schmerzen, Muskelschmerzen, Verbrennungsschmerzen, Schmerzen bei Sonnenbrand, Trigeminusneuralgie, Schmerzen bei Koliken, sowie bei Spasmen des Magen-Darm-Trakts oder des Uterus;
(b) von **Eingeweideschmerzen** wie z.B. chronischen Beckenschmerzen, gynäkologischen Schmerzen, Schmerzen vor und während der Menstruation, Schmerzen bei Pankreatitis, bei peptischen Ulzera, bei interstitieller Zystitis, bei Nierenkolik, bei Angina pectoris, Schmerzen bei Kolon irritabile, bei nicht-ulzeröser Dyspepsie und bei Gastritis, nicht-kardialen Thoraxschmerzen und Schmerzen bei myokardialer Ischämie und Herzinfarkt;
(c) von **neuropathischen Schmerzen** wie z.B. schmerzhaften Polyneuropathien, Schmerzen bei diabetischer Neuropathie, AIDS-assoziierten neuropathischen Schmerzen, Schmerzen bei Lumbago, bei nicht-herpesassoziierter Neuralgie, bei Post-zoster Neuralgie, bei Nervenverletzungen, bei Schädel-Hirn-Trauma, Schmerzen bei Nervenchädigungen infolge von Toxinen oder Chemotherapie, Phantomschmerzen, Schmerzen bei multipler Sklerose, bei Nervenwurzelabriß und schmerzhaften traumatisch bedingten Einzelnervenschädigungen;
(d) von **entzündlichen / Schmerzrezeptor-vermittelten Schmerzen** im Zusammenhang mit Erkrankungen wie Osteoarthritis, rheumatoider Arthritis, rheumatischem Fieber, Tendo-Synovitis, Sehnenentzündungen, Gicht, Vulvodynie, Schädigungen und Erkrankungen der Muskeln und Faszien (muskuläre Verletzungen, Fibromyalgie), Osteoarthritis, juveniler Arthritis, Spondylitis, Gicht-Arthritis, Psoriasis-Arthritis, Fibromyalgie, Myositis, Migräne, Zahnerkrankungen, Influenza und anderen Virusinfektionen wie Erkältungen, systemischer Lupus erythematodes,
(e) von **Tumorschmerzen** im Zusammenhang mit Krebserkrankungen wie lymphatischer oder myeloischer Leukämie, Hodgkin Erkrankung, Non-Hodgkin Lymphomen, Lymphogranulomatose, Lymphosarkomen, soliden malignen Tumoren und ausgedehnten Metastasen;
(f) von **Kopfschmerz-Erkrankungen** wie z.B. Kopfschmerzen unterschiedlicher Ursache, Cluster-Kopfschmerz, Migräne (mit oder ohne Aura) und Spannungskopfschmerz. Weiterhin eigenen sich die Verbindungen zur Behandlung
(g) von entzündlichen Veränderungen im Zusammenhang mit Erkrankungen der Atemwege wie Asthma bronchiale, einschließlich allergischem Asthma (atopisch und nicht-atopisch) sowie Bronchospasmen bei Anstrengung, beruflich-bedingtem Asthma, viraler oder bakterieller Exazerbation einer bestehenden Asthma-Erkrankung und anderen nicht-allergisch bedingten asthmatischen Erkrankungen;
   chronisch obstruktiver Lungen-Erkrankung (COPD) einschließlich Lungenemphysem, akutem Atemnotsyndrom des Erwachsenen (ARDS), Bronchitis, Lungenentzündung, allergischer Rhinitis (saisonal und ganzjährig), vasomotorischer Rhinitis und Staublungen-Erkrankungen wie Aluminose, Anthrakose, Asbestose, Chalikose, Siderose, Silikose, Tabakose und Byssinose;
(h) von Entzündungserscheinungen bei Sonnenbrand und Verbrennungen, Ödemen nach Traumata durch Verbrennungen, Hirnödemen und Angioödemen, Darmerkrankungen einschließlich Morbus Crohn und Kolitis ulzerosa, Reizdarmsyndrom, Pankreatitis, Nephritis, Zystitis (interstitielle Zystitis), Uveitis; entzündlichen Erkrankungen der Haut (wie z.B. Psoriasis und Ekzeme), vaskulären Bindegewebserkrankungen, Lupus, Zerrungen und Frakturen;
(i) von Diabetes Mellitus und dessen Folgen (wie z.B. diabetische Vaskulopathie, diabetische Neuropathie, diabetische Retinopathie) und von diabetischen Symptomen bei Insulitis (z.B. Hyperglycämie, Diurese, Proteinurie und erhöhte renale Nitrit- und Kallikrein-Exkretion);
(j) von neurodegenerativen Erkrankungen wie der Parkinson'schen Erkrankung und der Alzheimer Erkrankung;
(k) von Sepsis und septischem Schock nach bakteriellen Infektionen oder nach Trauma;
(l) von Juckreiz verursachenden Syndromen und allergischen Hautreaktionen;
(m) von Osteoporose;
(n) von Epilepsie;
(o) von Verletzungen des Zentralnervensystems;
(p) von Wunden und Gewebeschädigungen;
(q) von Zahnfleischentzündungen;
(r) von benigner Prostata-Hyperplasie und hyperaktiver Blase;
(s) von Pruritus;
(t) von Vitiligo;
(u) von Störungen der Motilität von respiratorischen, genito-urinalen, gastro-intestinalen oder vaskulären Regionen und
(v) von post-operativem Fieber.

Neben der Eignung als Humantherapeutika, sind diese Substanzen auch nützlich in der veterinärmedizinischen Therapie von Haustieren, exotischen Tieren und Nutztieren.

Zur Behandlung von Schmerzen kann es vorteilhaft sein, die erfindungsgemässen Verbindungen mit belebenden Stoffen wie Koffein oder anderen schmerzlindernden Wirkstoffen zu kombinieren. Stehen zur Behandlung der Ursache der Schmerzen geeignete Wirkstoffe zur Verfügung, so können diese mit den erfindungsgemässen Verbindungen kombiniert werden. Sind unabhängig von der Schmerzbehandlung auch noch weitere medizinische Behandlungen angezeigt, zum Beispiel gegen Bluthochdruck oder Diabetes, so können auch die dafür nötigen Wirkstoffe mit den erfindungsgemässen Verbindungen kombiniert werden.

Für eine Kombinationstherapie kommen beispielsweise die folgenden Verbindungen in Frage:
Nicht-steroidale Antirheumatika (NSAR): COX-2 Hemmer wie Propionsäure-Derivate (Alminoprofen, Benoxaprofen, Bucloxinsäure, Carprofen, Fenhufen, Fenoprofen, Fiuprofen, Fiulbiprofen, Ibuprofen, Indoprofen, Ketoprofen, Miroprofen, Naproxen, Oxaprozin, Pirprofen, Pranoprofen, Suprofen, Tiaprofensäure, Tioxaprofen), Essigsäure-Derivate (Indomethacin, Acemetacin, Alcofenac, Isoxepac, Oxpinax, Sulindac, Tiopinac, Tolmetin, Zidometacin, Zomepirac) Fenaminsäure-Derivate (Meclofenaminsäure, Mefenaminsäure, Tolfenaminsäure), Biphenyl-Carboxylsäure-Derivate, Oxicame (Isoxicam, Meloxicam, Piroxicam, Sudoxicam und Tenoxicam), Salicylsäure-Derivate (Acetylsalicylsäure, Sulfasalazin, warum nicht auch Mesalazin, Olsalazin, und Pyrazolone (Apazon, Bezpiperylon, Feprazon, Mofebutazon, Oxyphenbutazon, Phenylbutazon, warum nicht auch Propyphenazon und Metamizol, und Coxibe (Celecoxib, Valecoxib, Rofecoxib, Etoricoxib).
Opiat Rezeptor Agonisten wie z. B. Morphin, Propoxyphen (Darvon), Tramadol, Buprenorphin. Cannabinoid Agonisten wie z.B. GW-1000, KDS-2000, SAB-378, SP-104, NVP001-GW-843166, GW-842166X, PRS-211375.
Natriumkanalblocker wie z.B. Carbamazepin, Mexiletin, Lamotrigin, Pregabalin, Tectin, NW-1029, CGX-1002.
N-Typ Calciumkanalblocker wie z.B. Ziconitid, NMED-160, SP1-860.
Serotonerge und noradrenerge Modulatoren wie z.B. SR-57746, Paroxetin, Duloxetin, Clonidin, Amitriptylin, Citalopram.
Corticosteroide wie z.B. Betamethason, Budesonid, Cortison, Dexamethason, Hydrocortison, Methylprednisolon, Prednisolon, Prednison und Triamcinolon.
Histamin H1-Rezeptorantagonisten wie z.B. Bromophtniramint, Chlorpheniramin, Dexchlorpheniramin, Triprolidin, Clemastin, Diphenhydramin, Diphenylpyralin, Tripelennamin, Hydroxyzin, Methdijazin, Promethazin, Trimeprazin Azatadin, Cyproheptadin, Antazolin, Pheniramin, Pyrilamin, Astemizol, Terfenadin, Loratadin, Cetirizin, Desloratadin, fexofenadin, Levocetirizin.
Histamin H2-Rezeptor Antagonisten wie z.B. Cimetidin, Famotidin, und Ranitidin. Protonenpumpenhemmer wie z.B. Omeprazol, Pantoprazol, Esomeprazol.
Leukotrien Antagonisten und 5-Lipoxygenasehemmer wie z.B. Zafirfukast, Montelukast, Pranlukast und Zileuton.
Lokalanästhetika wie z.B. Ambroxol, Lidocain.
VR1 Agonisten und Antagonisten wie z.B. NGX-4010, WL-1002, ALGRX-4975, WL-10001, AMG-517.
Nikotinrezeptor Agonisten wie z.B. ABT-202, A-366833, ABT-594, BTG-102, A-85380, CGX1204.
P2X3-Rezeptor Antagonisten wie z.B. A-317491, ISIS-13920, AZD-9056.
NGF Agonisten und Antagonisten wie z.B. RI-724, RI-1024, AMG-819, AMG-403, PPH 207. NK1 und NK2 Antagonisten wie z.B. DA-5018, R-116301, CP-728663, ZD-2249.
NMDA Antagonisten wie z.B. NER-MD-11, CNS-5161, EAA-090, AZ-756, CNP-3381. Kaliumkanal Modulatoren wie z.B. CL-888, ICA-69673, Retigabin.
GABA Modulatoren wie z.B. Lacosamid.
Serotonerge und noradrenerge Modulatoren wie z.B. SR-57746, Paroxetin, Duloxetin, Clonidin, Amitriptylin, Citalopram, Flibanserin.
Anti-Migräne Therapeutika wie z.B. Sumatriptan, Zolmitriptan, Naratriptan, Eletriptan.

Die zur Erzielung einer schmerzstillenden Wirkung erforderliche Dosierung beträgt bei intravenöser Gabe zweckmäßigerweise 0.01 bis 3 mg/kg Körpergewicht, vorzugsweise 0.1 bis 1 mg/kg, und bei oraler Gabe 0.1 bis 8 mg/kg Körpergewicht, vorzugsweise 0.5 bis 3 mg/kg, jeweils 1 bis 3 x täglich. Die erfindungsgemäß hergestellten Verbindungen können intravenös, subkutan, intramuskulär, intrarektal, intranasal, durch Inhalation, transdermal oder oral verabreicht werden, wobei zur Inhalation insbesondere Aerosolformulierungen geeignet sind. Sie können gegebenenfalls zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden.

### EXPERIMENTELLER TEIL

Für die hergestellten Verbindungen liegen in der Regel IR-, ¹H-NMR und/oder Massenspektren vor. Die bei den Fliessmitteln angegebenen Verhältnisse beziehen sich auf Volumeneinheiten der jeweiligen Lösungsmittel. Die angegebenen Volumeneinheiten bei Ammoniak beziehen sich auf eine konzentrierte Lösung von Ammoniak in Wasser.

Soweit nicht anders vermerkt sind die bei den Aufarbeitungen der Reaktionslösungen verwendeten Säure-, Basen- und Salzlösungen wässrige Systeme der angegebenen Konzentrationen.

Zu chromatographischen Reinigungen wird Kieselgel der Firma Millipore (MATREX^{™}, 35-70 µm) oder Alox (E. Merck, Darmstadt, Aluminiumoxid 90 standardisiert, 63-200 µm, Artikel-Nr: 1.01097.9050) verwendet.

In den Versuchsbeschreibungen werden die folgenden Abkürzungen verwendet:
- CDI: 1,1'-Carbonyldiimidazol
- DC: Dünnschichtchromatogramm
- DIPEA: Diisopropylethylamin
- DMAP: 4-Dimethylaminopyridin
- DMF: Dimethylformamid
- DMSO: Dimethylsulfoxid
- HATU: O-(7-Azabenzotriazol-1-yl)-*N,N,N',N'*-tetramethyluroniumhexafluorphosphat
- *tert*: tertiär
- TBTU: 2-(1*H*-Benzotriazol-1-yl)-1,1,3,3-tetramethyluronium-tetrafluorborat
- THF: Tetrahydrofuran

Folgende analytische HPLC-Methoden wurden verwendet:

| | | | | |
|---|---|---|---|---|
| Methode 1: | Säule: | XTerra^{™} MS C18, 2.5 µM, 4.6 x 30 mm | | |
| | Detektion: | 210 - 420 nm | | |
| | Fließmittel A: | Wasser / 0.1 % Ameisensäure | | |
| | Fließmittel B: | Acetonitril / 0.1 % Ameisensäure | | |
| | Gradient: | | | |

| | Zeit in min | %A | %B | Flussrate in mL/min |
|---|---|---|---|---|
| | 0.0 | 95.0 | 5.0 | 1.0 |
| | 0.1 | 95.0 | 5.0 | 1.0 |
| | 3.1 | 2.0 | 98.0 | 1.0 |
| | 4.5 | 2.0 | 98.0 | 1.0 |
| | 5.0 | 95.0 | 5.0 | 1.0 |

| | | | | |
|---|---|---|---|---|
| Methode 2: | Säule: | Microsorb C18, 3 µM, 4.6 x 50 mm | | |
| | Detektion: | 220 - 320 nm | | |
| | Fließmittel A: | Wasser / 0.1 % TFA | | |
| | Fließmittel B: | Acetonitril / 0.1 % TFA | | |
| | Gradient: | | | |

| | Zeit in min | %A | %B | Flussrate in mL/min |
|---|---|---|---|---|
| | 0.0 | 95.0 | 5.0 | 1.5 |
| | 0.5 | 95.0 | 5.0 | 1.5 |
| | 3.8 | 2.0 | 98.0 | 1.5 |
| | 4.3 | 2.0 | 98.0 | 1.5 |
| | 4.35 | 95.0 | 5.0 | 1.5 |
| | 4.6 | 95.0 | 5.0 | 1.5 |

| | | | | |
|---|---|---|---|---|
| Methode 3: | Säule: | XTerra^{™} MS C18, 3.5 µM, 4.6 x 50 mm | | |
| | Detektion: | 210 - 420 nm | | |
| | Fließmittel A: | Wasser / 0.1 % Ameisensäure | | |
| | Fließmittel B: | Acetonitril / 0.1 % Ameisensäure | | |
| | Gradient: | | | |

| | Zeit in min | %A | %B | Flussrate in mL/min |
|---|---|---|---|---|
| | 0.0 | 95.0 | 5.0 | 1.0 |
| | 0.1 | 95.0 | 5.0 | 1.0 |
| | 7.1 | 2.0 | 98.0 | 1.0 |
| | 7.9 | 2.0 | 98.0 | 1.0 |
| | 8.0 | 95.0 | 5.0 | 1.0 |

| | | | | |
|---|---|---|---|---|
| Methode 4: | Säule: | Zorbax Stable Bond C18, 3.5 µM, 4.6 x 75 mm | | |
| | Detektion: | 230 - 360 nm | | |
| | Fließmittel A: | Wasser / 0.1 % Ameisensäure | | |
| | Fließmittel B: | Acetonitril / 0.1 % Ameisensäure | | |
| | Gradient: | | | |

| | Zeit in min | %A | %B | Flussrate in mL/min |
|---|---|---|---|---|
| | 0.0 | 95.0 | 5.0 | 1.6 |
| | 0.1 | 95.0 | 5.0 | 1.6 |
| | 4.5 | 10.0 | 90.0 | 1.6 |
| | 5.09 | 10.0 | 90.0 | 1.6 |

| Zeit in min | | %A | %B | Flussrate in mL/min |
|---|---|---|---|---|
| 5.5 | | 90.0 | 10.0 | 1.6 |

| | | | | |
|---|---|---|---|---|
| Methode 5: | Säule: | Interchim Strategy C18, 5 µM, 4.6 x 50 mm | | |
| | Detektion: | 220 - 320 nm | | |
| | Fließmittel A: | Wasser / 0.1 % TFA | | |
| | Fließmittel B: | Acetonitril | | |
| | Gradient: | | | |

| | Zeit in min | %A | %B | Flussrate in mL/min |
|---|---|---|---|---|
| | 0.0 | 95.0 | 5.0 | 3.0 |
| | 0.3 | 95.0 | 5.0 | 3.0 |
| | 2.0 | 2.0 | 98.0 | 3.0 |
| | 2.4 | 2.0 | 98.0 | 3.0 |
| | 2.45 | 95.0 | 5.0 | 3.0 |
| | 2.8 | 95.0 | 5.0 | 3.0 |

| | | | | |
|---|---|---|---|---|
| Methode 6: | Säule: | Merck Cromolith Speed ROD RP18e, 4.6 x 50 mm | | |
| | Detektion: | 190 - 400 nm | | |
| | Fließmittel A: | Wasser / 0.1 % Ameisensäure | | |
| | Fließmittel B: | Acetonitril / 0.1 % Ameisensäure | | |
| | Gradient: | | | |

| | Zeit in min | %A | %B | Flussrate in mL/min |
|---|---|---|---|---|
| | 0.0 | 90.0 | 10.0 | 1.5 |
| | 4.5 | 10.0 | 90.0 | 1.5 |
| | 5.0 | 10.0 | 90.0 | 1.5 |
| | 5.5 | 90.0 | 10.0 | 1.5 |

| | | | | |
|---|---|---|---|---|
| Methode 7: | Säule: | Waters SunFire C18, 3.5 µM, 4.6 x 50 mm | | |
| | Detektion: | 210 - 500 nm | | |
| | Fließmittel A: | Wasser / 0.1 % TFA | | |
| | Fließmittel B: | Acetonitril / 0.1 % TFA | | |
| | Gradient: | | | |

| | Zeit in min | %A | %B | Flussrate in mL/min |
|---|---|---|---|---|
| | 0.0 | 95.0 | 5.0 | 1.5 |
| | 2.0 | 2.0 | 98.0 | 1.5 |
| | 3.0 | 2.0 | 98.0 | 1.5 |

| Zeit in min | | %A | %B | Flussrate in mL/min |
|---|---|---|---|---|
| 3.4 | | 95.0 | 5.0 | 1.5 |

| | | | | |
|---|---|---|---|---|
| Methode 8: | Säule: | Waters XBridge C18, 3.5 µM, 4.6 x 50 mm | | |
| | Detektion: | 210 - 500 nm | | |
| | Fließmittel A: | Wasser / 0.1 % TFA | | |
| | Fließmittel B: | Acetonitril / 0.1 % TFA | | |
| | Gradient: | | | |

| | Zeit in min | %A | %B | Flussrate in mL/min |
|---|---|---|---|---|
| | 0.0 | 95.0 | 5.0 | 1.0 |
| | 0.1 | 95.0 | 5.0 | 1.0 |
| | 5.1 | 2.0 | 98.0 | 1.0 |
| | 6.5 | 2.0 | 98.0 | 1.0 |
| | 7.0 | 95.0 | 5.0 | 1.0 |

| | | | | |
|---|---|---|---|---|
| Methode 9: | Säule: | Merck Chromolith^{™} Flash RP18e, 4.6 x 25 mm | | |
| | Detektion: | 190 - 400 nm | | |
| | Fließmittel A: | Wasser / 0.1 % Ameisensäure | | |
| | Fließmittel B: | Acetonitril / 0.1 % Ameisensäure | | |
| | Gradient: | | | |

| | Zeit in min | %A | %B | Flussrate in mL/min |
|---|---|---|---|---|
| | 0.0 | 90.0 | 10.0 | 1.6 |
| | 2.7 | 10.0 | 90.0 | 1.6 |
| | 3.0 | 10.0 | 90.0 | 1.6 |
| | 3.3 | 90.0 | 10.0 | 1.6 |

| | | | | |
|---|---|---|---|---|
| Methode 10: | Säule: | Merck Chromolith^{™} Flash RP18e, 4.6 x 25 mm | | |
| | Detektion: | 210 - 400 nm | | |
| | Fließmittel A: | Wasser / 0.1 % TFA | | |
| | Fließmittel B: | Acetonitril / 0.1 % TFA | | |
| | Gradient: | | | |

| | Zeit in min | %A | %B | Flussrate in mL/min |
|---|---|---|---|---|
| | 0.0 | 95.0 | 5.0 | 2.5 |

| Zeit in min | | %A | %B | Flussrate in mL/min |
|---|---|---|---|---|
| 0.2 | | 95.0 | 5.0 | 2.5 |
| 1.5 | | 2.0 | 98.0 | 2.5 |
| 1.7 | | 2.0 | 98.0 | 2.5 |
| 1.9 | | 95.0 | 5.0 | 2.5 |
| 2.2 | | 95.0 | 5.0 | 2.5 |

| | | | | |
|---|---|---|---|---|
| Methode 11: | Säule: | Waters XBridge C18, 3.5 µM, 4.6 x 50 mm | | |
| | Detektion: | 210 - 500 nm | | |
| | Fließmittel A: | Wasser / 0.1 % TFA | | |
| | Fließmittel B: | Acetonitril / 0.1 % TFA | | |
| | Gradient: | | | |

| | Zeit in min | %A | %B | Flussrate in mL/min |
|---|---|---|---|---|
| | 0.0 | 95.0 | 5.0 | 1.5 |
| | 2.0 | 0.0 | 100.0 | 1.5 |
| | 3.0 | 0.0 | 100.0 | 1.5 |
| | 3.4 | 95.0 | 5.0 | 1.5 |

| | | | | |
|---|---|---|---|---|
| Methode 12: | Säule: | YMC-Pack ODS-AQ, 3.0 µM, 4.6 x 75 mm | | |
| | Detektion: | 230 - 360 nm | | |
| | Fließmittel A: | Wasser / 0.1 % Ameisensäure | | |
| | Fließmittel B: | Acetonitril / 0.1 % Ameisensäure | | |
| | Gradient: | | | |

| | Zeit in min | %A | %B | Flussrate in mL/min |
|---|---|---|---|---|
| | 0.0 | 95.0 | 5.0 | 1.6 |
| | 4.5 | 10.0 | 90.0 | 1.6 |
| | 5.0 | 10.0 | 90.0 | 1.6 |
| | 5.5 | 90.0 | 10.0 | 1.6 |

Folgende Mikrowellen-Apparatur wurde verwendet: Biotage EmrysOptimizer^{™}

### Herstellung der Endverbindungen

### Beispiel 1

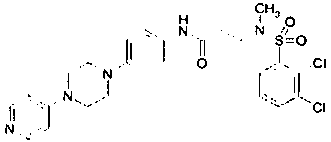

### 1a)

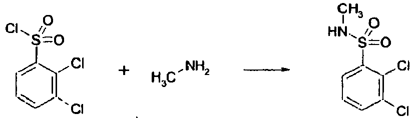

Eine Mischung aus 1.0 g (4.07 mMol) 2,3-Dichlorbenzolsulfonsäurechlorid, 0.33 g (4.89 mMol) Methylamin Hydrochlorid, 2.73 ml (19.55 mMol) Triethylamin und 20 ml Dichlormethan wird über Nacht bei Raumtemperatur gerührt. Die Reaktionsmischung wird anschließend je einmal mit 1 N HCl, gesättigter Natriumhydrogencarbonat-Lösung, Wasser und gesättigter Natriumchloridlösung gewaschen, über Natriumsulfat getrocknet und dann bis zur Trockne eingeengt.
C₇H₇Cl₂NO₂S (240.11)
[M+H]+ = 240/242/244
DC: Kieselgel, Petrolether / ethylacetat 2:1, Rf-Wert = 0.50

### 1 b)

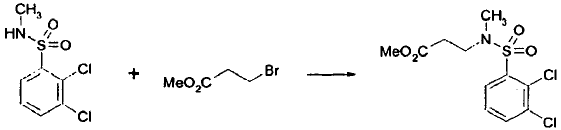

Eine Mischung aus 0.9 g (3.75 mMol) Produkt aus 1a und 20 ml DMF wird vorgelegt und mit 1.55 g (11.24 mMol) Kaliumcarbonat und 0.49 ml (4.50 mMol) 3-Brompropionsäureethylester versetzt. Die Reaktionsmischung wird über Nacht bei Raumtemperatur gerührt und dann mit Wasser versetzt. Man extrahiert zweimal mit Ethylacetat. Die organischen Extrakte werden dreimal mit Wasser und einmal mit gesättigter Natriumchloridlösung gewaschen, über Natriumsulfat getrocknet und zur Trockne eingeengt.
C₁₁H₁₃Cl₂NO₄S (326.20)
[M+H]+ = 326/328/330
DC: Kieselgel, Petrolether / Ethylacetat 2:1, Rf-Wert = 0.45

### 1c)

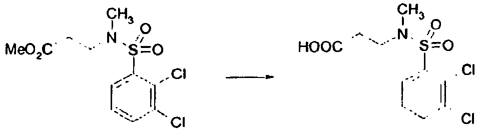

Eine Mischung aus 1.15 g (3.53 mMol) Produkt aus 1b, 0.74 g (17.63 mMol) Lithiumhydroxid Monohydrat, 15 ml THF und 15 ml Wasser wird eine Stunde bei Raumtemperatur gerührt. Danach wird das THF im Vakuum entfernt und der Rückstand mit konzentrierter HCl sauer gestellt. Die Reaktionsmischung wird anschließend dreimal mit Ethylacetat extrahiert. Die organischen Extrakte werden mit gesättigter Natriumchloridlösung gewaschen, über Natriumsulfat getrocknet und zur Trockne eingeengt. Das Rohprodukt wird mit Diethylether verrieben und abgesaugt.
C₁₀H₁₁Cl₂NO₄S (312.17)
[M+H]+ = 310/312/314
DC: Kieselgel, Petrolether / Ethylacetat 2:1, Rf-Wert = 0.03

### 1d)

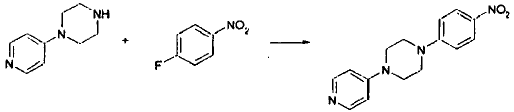

Eine Mischung aus 5.0 g (30.63 mMol) 1-Pyridin-4-yl-piperazin, 4.32 g (30.63 mMol) 1-Fluor-4-nitrobenzol (Aldrich), 10.62 ml (76.59 mMol) Triethylamin und 100 ml DMF wird 50 min unter Rückfluß erhitzt und anschließend bis zur Trockene eingeengt. Das so erhaltene Rohprodukt wird durch Säulenchromatographie über Kieselgel (Elutionsmittel: Dichlormethan / Ethanol /Ammoniak 12:1:0.1 bis 10:1:0.1) gereinigt.
C₁₅H₁₆N₄O₂ (284.31)
[M+H]+ = 285
DC: Kieselgel, Dichlormethan / Methanol / Ammoniak 9:1:0.1, Rf-Wert = 0.52

### 1e)

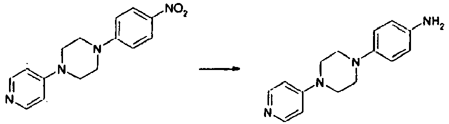

Eine Mischung aus 4.95 g (17.41 mMol) Produkt aus 1d, 0.6 g Palladium auf Kohle (10%), 120 ml Dichlormethan und 20 ml Methanol wird fünf Stunden im Autoklaven bei Raumtemperatur hydriert. Anschließend wird abgesaugt und der Filterkuchen noch sechsmal mit Dichlormethan / Methanol 1:1 aufgekocht und wieder abgesaugt. Die vereinigten Filtrate werden im Vakuum bis zur Trockene eingeengt.
C₁₅H₁₈N₄ (254.33)
[M+H]+ = 255

### 1f)

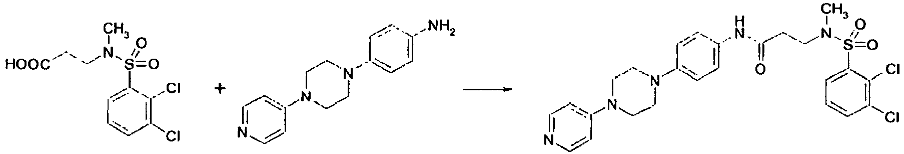

Eine Mischung aus 1.25 g (4.00 mMol) Produkt aus 1c, 2.0 ml (14.34 mMol) Triethylamin, 1.28 g (4.00 mMol) TBTU und 7 ml DMF wird 45 min bei Raumtemperatur gerührt. Danach wird 1.0 g (3.93 mMol) Produkt aus 1 e hinzugefügt und über Nacht weiter bei Raumtemperatur gerührt. Anschließend wird die Reaktionsmischung in Wasser geschüttet und mit Dichlormethan extrahiert. Die organischen Extrakte werden mit Wasser gewaschen, über Na₂SO₄ getrocknet und bis zur Trockene eingeengt. Das so erhaltene Rohprodukt wurde durch Säulenchromatographie über Kieselgel (Elutionsmittel: Dichlormethan mit 5-20 % Methanol) gereinigt.
C₂₅H₂₇Cl₂N₅O₃S (548.49)
[M+H]+ = 548/550/552
DC: Kieselgel, Dichlormethan / Methanol 4:1, Rf-Wert = 0.65

### Beispiel 2

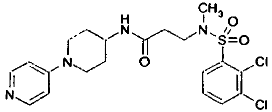

### 2a)

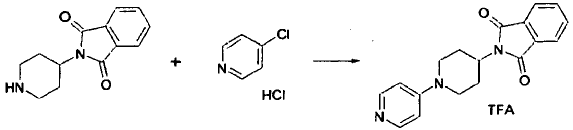

Eine Mischung aus 0.5 g (2.17 mMol) *N*-(1-Benzylpiperidin-4-yl)-phthalimid (Bioorg. Med. Chem. Lett. 11, 2001, 2325-2330), 0.33 g (2.17 mMol) 4-Chlorpyridin Hydrochlorid, 1.2 ml (8.69 mMol) Triethylamin und 2.4 ml Ethanol absolut wird eine Stunde in der Mikrowelle auf 150°C erhitzt. Die Reaktionsmischung wird anschließend mit Ethanol verdünnt, der entstandene Niederschlag wird abfiltriert. Man engt das Filtrat bis zur Trockene ein und reinigt das Rohprodukt durch präparative HPLC.
C₁₈H₁₇N₃O₂ x C₂HF₃O₂ (421.37)
[M+H]+ = 308
HPLC (Methode 1): Retentionszeit = 2.07 min

### 2b)

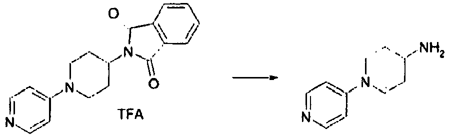

Eine Mischung aus 0.3 g (0.71 mMol) Produkt aus 2a, 0.09 g (1.42 mMol) Hydrazinhydrat 80%ig und 6 ml Ethanol absolut wird vier Stunden unter Rückfluß erhitzt. Die Reaktionsmischung wird anschließend auf 0°C abgekühlt, der entstandene Niederschlag wird abfiltriert. Man engt das Filtrat bis zur Trockene ein.
C₁₀H₁₅N₃ (177.25)
[M+H]+ = 178

### 2c)

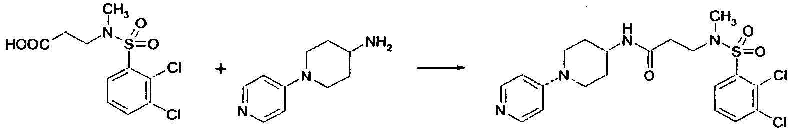

Beispiel 2 wird analog zu 1f aus 0.22 g (0.71 mMol) Produkt aus 1c, 0.24 g (1.35 mMol) Produkt aus 2b, 0.3 ml (2.13 mMol) Triethylamin und 0.23 g (0.71 mMol) TBTU in 5.5 ml DMF hergestellt.
C₂₀H₂₄Cl₂N₄O₃S (471.40)
[M+H]+ = 471/473/475
DC: Kieselgel, Dichlormethan / Methanol 9:1, Rf-Wert = 0.2

### Beispiel 3

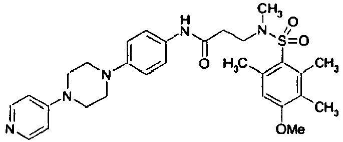

### 3a)

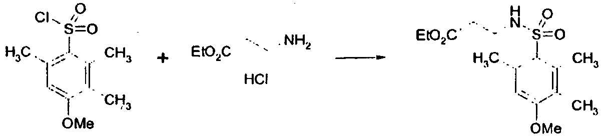

Eine Mischung aus 0.99 g (4.00 mMol) 4-Methoxy-2,3,6-trimethyl-benzolsulfonylchlorid, 0.69 g (4.51 mMol) β-Alaninethylester Hydrochlorid, 2.23 ml (15.98 mMol) Triethylamin und 20 ml Dichlormethan wird über Nacht bei Raumtemperatur gerührt. Die Reaktionsmischung wird anschließend mit 0.5 M HCl, gesättigter Natriumhydrogencarbonatlösung, Wasser und gesättigter Natrium-chloridlösung gewaschen, über Natriumsulfat getrocknet und im Vakuum bis zur Trockene eingeengt.
C₁₅H₂₃NO₅S (329.41)
[M+H]+ = 330
DC: Kieselgel, Petrolether / Ethylacetat 2:1, Rf-Wert = 0.43

### 3b)

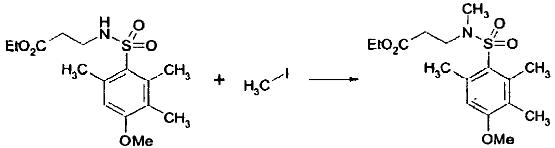

Eine Mischung aus 1.24 g (3.76 mMol) Produkt aus 3a, 0.84 ml (13.55 mMol) Methyliodid, 1.04 g (7.53 mMol) Kaliumcarbonat wasserfrei und 10 ml DMF wird fünf Stunden bei Raumtemperatur gerührt. Die Reaktionsmischung wird anschließend im Vakuum bis zur Trockene eingeengt, der Rückstand wird in Ethylacetat aufgenommen. Man wäscht mit Wasser, gesättigter Natriumhydrogencarbonatlösung und gesättigter Natriumchforidlösung, trocknet über Natriumsulfat und engt im Vakuum bis zur Trockene ein.
C₁₆H₂₅NO₅S (343.44)
[M+H]+ = 344
DC: Kieselgel, Petrolether / Ethylacetat 2:1, Rf-Wert = 0.52

### 3c)

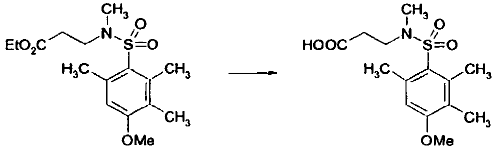

Die Säure wird analog zu 1c aus 1.29 g (3.76 mMol) Produkt aus 3b, 0.79 g (18.80 mMol) Lithiumhydroxid Monohydrat, 15 ml THF und 15 ml Wasser hergestellt.
C₁₄H₂₁NO₅S (315.39)
[M+H]+ = 316
DC: Kieselgel, Petrolether/ Ethylacetat 2:1, Rf-Wert = 0.07

### 3d)

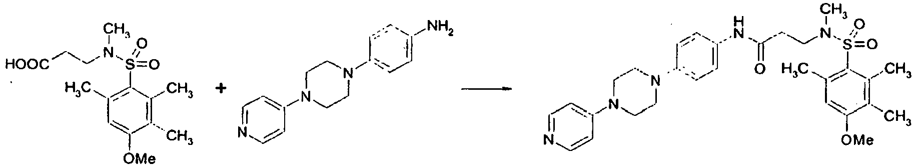

Beispiel 3 wird analog zu 1f aus 0.15 g (0.47 mMol) Produkt aus 3c, 0.12 g (0.47 mMol) Produkt aus 1e, 0.2 ml (1.43 mMol) Triethylamin und 0.15 g (0.48 mMol) TBTU in 8 ml DMF hergestellt.
C₂₉H₃₇N₅O₄S (551.70)
[M+H]+ = 552
DC: Kieselgel, Dichlormethan / Methanol 9:1, Rf-Wert = 0.38

### Beispiel 4

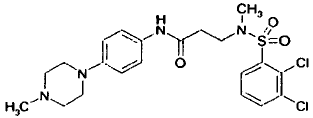

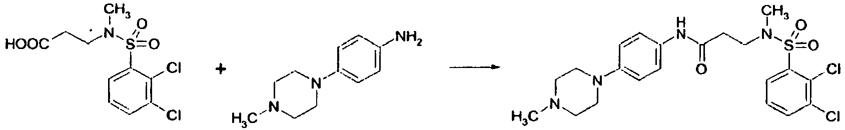

Beispiel 4 wird analog zu 1f aus 0.39 g (1.26 mMol) Produkt aus 1c, 0.24 g (1.26 mMol) 4-(4-Methylpiperazin-1-yl)-anilin (J. Med. Chem. SIR 48, 7, 2005, 2371-2387), 0.35 ml (2.51 mMol) Triethylamin und 0.50 g (1.32 mMol) HATU in 5 ml DMF hergestellt.
C₂₁H₂₆CL₂N₄O₃S (485.43)
[M+H]+ = 485/487/489
HPLC (Methode 2): Retentionszeit = 2.64 min

### Beispiel 5

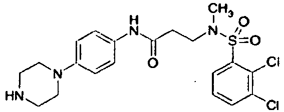

### 5a)

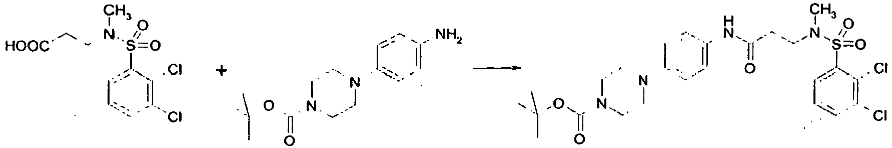

5a wird analog zu 1f aus 0.39 g (1.26 mMol) Produkt aus 1 c, 0.24 g (1.26 mMol) 4-(4-Methylpiperazin-1-yl)-anilin (J. Med. Chem. SIR 48, 7, 2005, 2371-2387), 0.35 ml (2.51 mMol) Triethylamin und 0.50 g (1.32 mMol) HATU in 5 ml DMF hergestellt.
C₂₅H₃₂CL₂N₄O₅S (571.52)

### 5b)

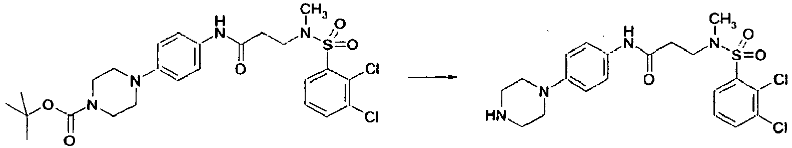

Eine Mischung aus 0.60 g (1.05 mMol) Produkt aus 5a, 3 ml TFA und 3 ml Dichlormethan wird zwei Stunden bei Raumtemperatur gerührt. Man engt die Reaktionsmischung bis zur Trockene ein und reinigt das Rohprodukt durch präparative HPLC.
C₂₀H2₄C₁₂N₄O₃S (471.40)
[M+H]+ = 471/473/475
HPLC (Methode 2): Retentionszeit = 2.58 min

### Beispiel 6

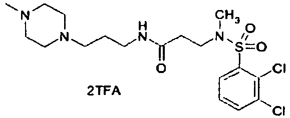

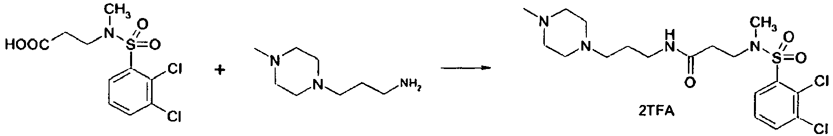

Beispiel 6 wird analog zu 1f aus 0.22 g (0.71 mMol) Produkt aus 1c, 0.12 g (0.78 mMol) 3-(4-Methylpiperazin-1-yl)-propylamin (Bioorg. Med. Chem. Lett. 13, 2003, 2131-2136), 0.30 ml (2.13 mMol) Triethylamin und 0.23 g (0.71 mMol) TBTU in 5.5 ml THF hergestellt.
C₁₈H₂₈C₁₂N₄O₃S x 2C₂HF₃O₂ (679.46)
[M+H]+ = 451/453/455
HPLC (Methode 5): Retentionszeit = 1.37 min

### Beispiel 7

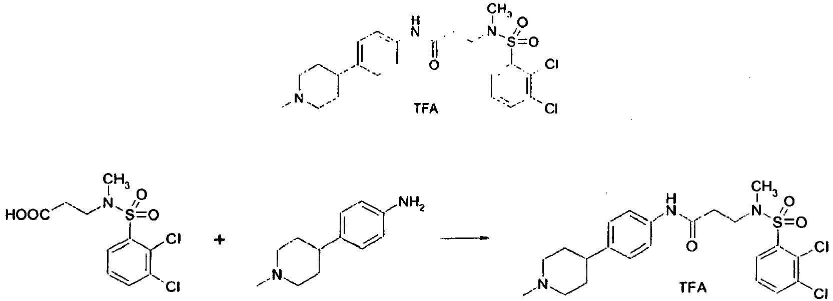

Beispiel 7 wird analog zu 1f aus 0.22 g (0.71 mMol) Produkt aus 1 c, 0.14 g (0.71 mMol) 4-(1-Methylpiperidin-4-yl)-anilin (JW Pharmlab), 0.30 ml (2.13 mMol) Triethylamin und 0.23 g (0.71 mMol) TBTU in 5.5 ml THF hergestellt.
C₂₂H₂₇Cl₂N₃O₃S x C₂HF₃O₂ (598.46)
[M+H]+ = 484/486/488
HPLC (Methode 5): Retentionszeit = 1.57 min

### Beispiel 8

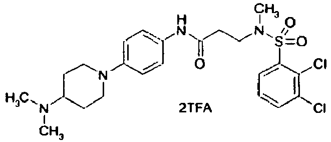

### 8a)

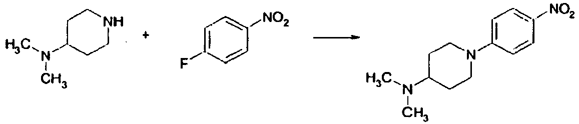

8a wird analog zu 1d aus 0.5 g (3.90 mMol) 4-Dimethylamino-piperidin (Alfa Aesar), 0.44 g (4.18 mMol) 1-Fluor-4-nitrobenzol (Aldrich) und 1.33 ml (76.59 mMol) Triethylamin in 12 ml DMF hergestellt.
C₁₃H₁₉N₃O₂ (249.31)
[M+H]⁺ = 250

### 8b)

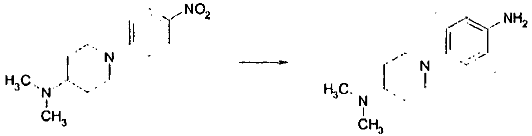

Eine Mischung aus 1.66 g (6.67 mMol) Produkt aus 8a, 0.17 g Palladium auf Kohle (5%) und 132 ml Ethanol wird im Autoklaven bei Raumtemperatur hydriert. Anschließend wird der Katalysator abgesaugt und das Filtrat im Vakuum bis zur Trockene eingeengt. C₁₃H₂₁N₃ (219.33)
[M+H]+ = 220
DC: Kieselgel, Dichlormethan / Methanol 9:1, Rf-Wert = 0.1

### 8c)

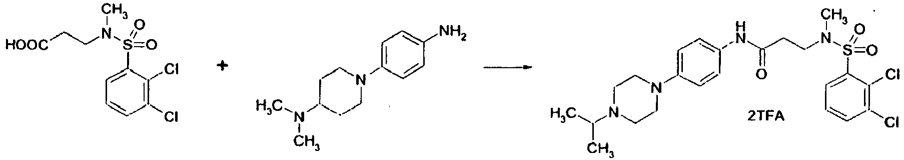

Beispiel 8 wird analog zu 1f aus 0.22 g (0.71 mMol) Produkt aus 1c, 0.16 g (0.71 mMol) Produkt aus 8b, 0.30 ml (2.13 mMol) Triethylamin und 0.23 g (0.71 mMol) TBTU in 5.5 ml THF hergestellt.
C₂₃H₃₀CL₂N₄O₃S x 2C₂HF₃O₂ (741.53)
[M+H]+ = 513/515/517
HPLC (Methode 5): Retentionszeit = 1.46 min

### Beispiel 9

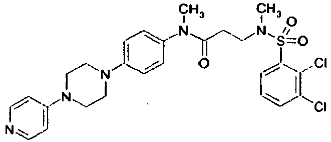

### 9a)

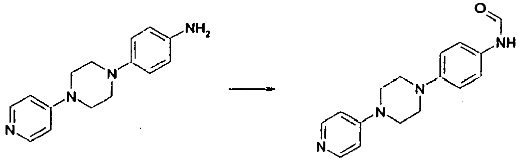

13 ml Essigsäureanhydrid werden vorgelegt und langsam mit 8 ml Ameisensäure versetzt. Die Reaktionsmischung wird 1.5 Stunden auf 50°C erhitzt und dann mit 80 ml Dichlormethan versetzt. Unter Eisbadkühlung werden anschließend 5.0 g (19.66 mMol) hinzugegeben. Es wird eine Stunde bei Raumtemperatur gerührt und danach bis zur Trockene eingeengt. Der Rückstand wird mit halbgesättigter Natriumhydrogencarbonat-Lösung versetzt und mit Dichlormethan zweimal extrahiert. Die organischen Extrakte werden mit gesättigter Natriumchlorid-Lösung gewaschen, über Natriumsulfat getrocknet und bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₆H₁₈N₄O (282.34)
[M+H]+ = 283

### 9b)

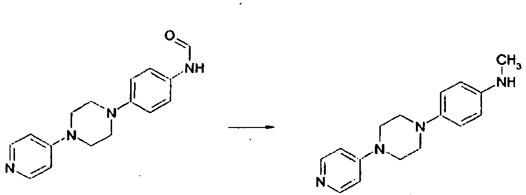

Bei 60°C wird eine Mischung aus 10.63 ml Lithiumaluminiumhydrid 2 M in THF (21.25 mMol) und 50 ml THF langsam mit 3.0 g (10.63 mMol) Produkt aus 9a versetzt. Die Reaktionsmischung wird acht Stunden bei 60°C und vier Stunden bei Raumtemperatur gerührt. Unter Eisbadkühlung werden anschließend 20 ml Wasser hinzugegeben. Es wird über Celite filtriert und mit THF und Dichlormethan nachgewaschen. Das Filtrat wird bis zur Trockene eingeengt. Der Rückstand wird mit Dichlormethan versetzt, mit Wasser und 1 M Natronlauge gewaschen, über Natriumsulfat-Lösung getrocknet und bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₆H₂₀N₄ (268.36)

### 9c)

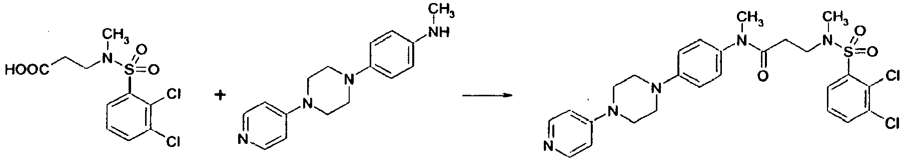

Beispiel 9 wird analog zu 1f aus 0.15 g (0.48 mMol) Produkt aus 1 c, 0.14 g (0.51 mMol) Produkt aus 9b, 0.13 ml (0.96 mMol) Triethylamin und 0.19 g (0.51 mMol) HATU in 5 ml DMF hergestellt.
C₂₆H₂₉Cl₂N₅O₃S (562.51)
[M+H]+ = 562/564/566
HPLC (Methode 2): Retentionszeit = 2.86 min

### Beispiel 10

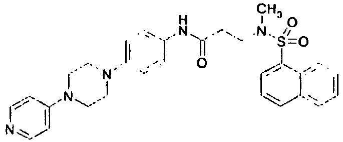

### 10a)

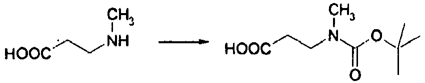

Eine Mischung aus 1.0 g (9.70 mMol) N-Methyl-β-alanin (Convertex), 24 ml Dioxan, 12 ml Wasser und 2.68 g (19.38 mMol) Kaliumcarbonat wasserfrei wird unter Eisbadkühlung mit 2.33 g (10.66 mMol) Boc-Anhydrid versetzt. Die Reaktionsmischung wird drei Tage bei Raumtemperatur gerührt. Danach wird das Dioxan im Vakuum entfernt. Der wässrige Rückstand wird mit Ethylacetat extrahiert (Ethylacetat-Phasen verwerfen), anschließend mit 1 M Salzsäure leicht sauer gestellt und dann mit Dichlormethan extrahiert. Die organischen Dichlormethan-Extrakte werden mit gesättigter Natriumchlorid-Lösung gewaschen, über Natriumsulfat getrocknet und bis zur Trockene eingeengt.
C₉H₁₇NO₄ (203.24)
[M+H]+ = 204

### 10b)

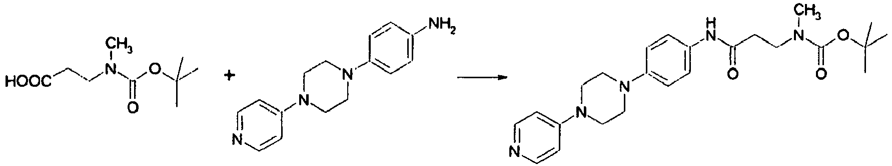

10b wird analog zu 1f aus 1.85 g (9.10 mMol) Produkt aus 10a, 2.32 g (9.10 mMol) Produkt aus 1e, 3.81 ml (27.31 mMol) Triethylamin und 2.92 g (9.10 mMol) TBTU in 80 ml DMF hergestellt.
C₂₄H₃₃N₅O₃ (439.55)
[M+H]⁺ = 440
DC: Kieselgel, Dichlormethan / Methanol 9:1, Rf-Wert = 0.49

### 10c)

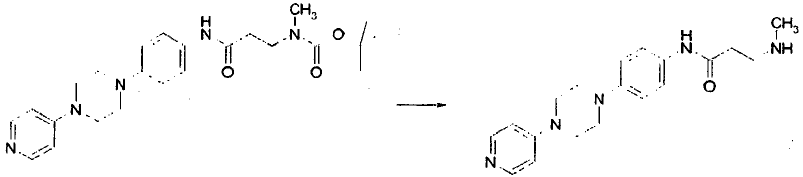

Eine Mischung aus 3.20 g (7.28 mMol) Produkt aus 10b, 20 ml TFA und 60 ml Dichlormethan wird 30 min bei Raumtemperatur gerührt. Danach wird die Reaktionsmischung im Vakuum bis zur Trockene eingeengt. Das so erhaltene Rohprodukt wird durch Säulenchromatographie über Kieselgel (Elutionsmittel: Dichlormethan / Ethanol / Ammoniak 9:1:0.1 bis 4:1:0.1) gereinigt.
C₁₉H₂₅N₅O (339.43)
[M+H]+ = 340
DC: Kieselgel, Dichlormethan / Methanol / Ammoniak 9:1:0.1, Rf-Wert = 0.25

### 10d)

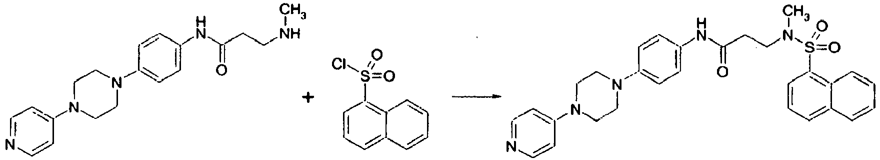

Eine Mischung aus 0.1 g (0.30 mMol) Produkt aus 10c, 0.056 g (0.25 mMol) 1-Naphthylsulfonsäurechlorid, 0.137 ml (0.98 mMol) Triethylamin und 5 ml Dichlormethan wird über Nacht bei Raumtemperatur gerührt. Danach wird die Reaktionsmischung im Vakuum bis zur Trockene eingeengt. Das so erhaltene Rohprodukt wird durch Säulenchromatographie über Kieselgel (Elutionsmittel: Dichlormethan / Ethanol / Ammoniak 12:1:0.1) gereinigt.
C₂₉H₃₁N₅O₃S (529.65)
[M+H]+ = 530
DC: Kieselgel, Dichlormethan / Methanol/Ammoniak 9:1:0.1, Rf-Wert = 0.44

### Beispiel 11

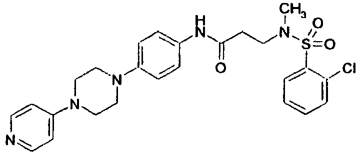

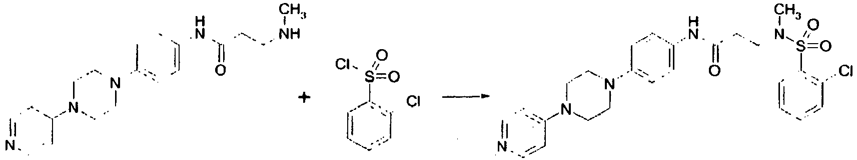

Beispiel 11 wird analog zu 10d aus 0.10 g (0.30 mMol) Produkt aus 10c, 0.052 g (0.25 mMol) 2-Chlorbenzolsulfonsäurechlorid, 0.14 ml (98 mMol) Triethylamin in 5 ml Dichlormethan hergestellt.
C₂₅H₂₈CIN₅O₃S (514.04)
[M+H]+ = 514/516
DC: Kieselgel, Dichlormethan / Methanol / Ammoniak 9:1:0.1, Rf-Wert = 0.47

### Beispiel 12

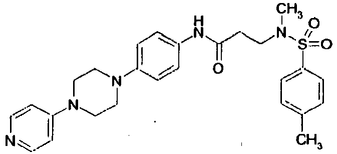

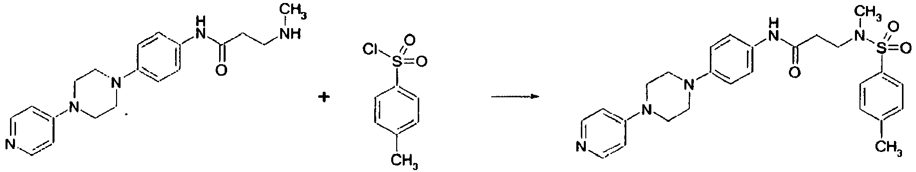

Beispiel 12 wird analog zu 10d aus 0.10 g (0.30 mMol) Produkt aus 10c, 0.047 g (0.25 mMol) p-Toluolsulfonsäurechlorid, 0.14 ml (98 mMol) Triethylamin in 5 ml Dichlormethan hergestellt.
C₂₆H₃₁N₅O₃S (493.62)
[M+H]+ = 494
DC: Kieselgel, Dichlormethan / Methanol / Ammoniak 9:1:0.1, Rf-Wert = 0.43

### Beispiel 13

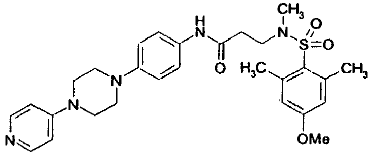

### 13a)

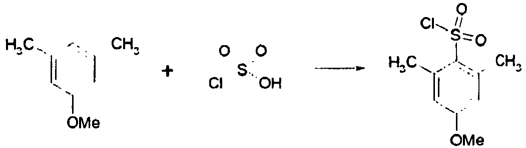

Eine Mischung aus 2.0 g (14.69 mMol) 3,5-Dimethylanisol und 20 ml Dichlormethan wird unter Eisbadkühlung mit 5.85 ml (88.0 mMol) Chlorsulfonsäure versetzt. Die Reaktionsmischung wird danach 20 min bei Raumtemperatur gerührt und anschließend auf 50 ml Eiswasser gegossen. Man extrahiert mit 100 ml Dichlormethan. Die organischen Extrakte werden mit 5%iger Natriumhydrogencarbonat-Lösung gewaschen, über Natriumsulfat getrocknet und bis zur Trockene eingeengt.
C₉H₁₁ClO₃S (234.70)
[M+H]+ = 234/236
DC: Kieselgel, Petrolether / Ethylacetat 9:1, Rf-Wert = 0.46

### 13b)

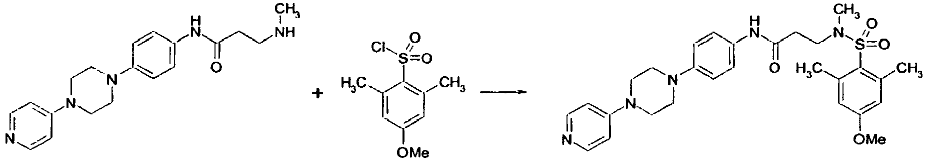

Beispiel 13 wird analog zu 10d aus 0.10 g (0.30 mMol) Produkt aus 10c, 0.058 g (0.25 mMol) Produkt aus 13a, 0.14 ml (98 mMol) Triethylamin in 5 ml Dichlormethan hergestellt.
C₂₈H₃₅N₅O₄S (537.67)
[M+H]+ = 538
DC: Kieselgel, Dichlormethan / Methanol / Amoniak 9:1:0.1, Rf-Wert = 0.62

### Beispiel 14

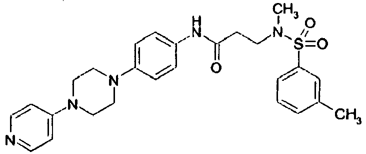

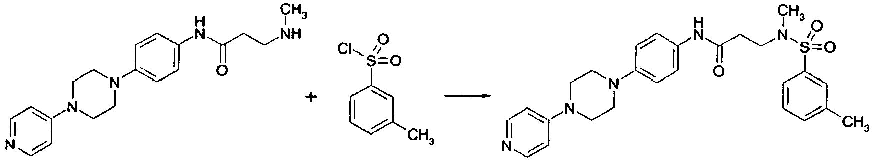

Beispiel 14 wird analog zu 10d aus 0.10 g (0.30 mMol) Produkt aus 10c, 0.047 g (0.25 mMol) m-Toluolsulfonsäurechlorid, 0.14 ml (98 mMol) Triethylamin in 5 ml Dichlormethan hergestellt.
C₂₆H₃₁N₅O₃S (493.62)
[M+H]+ = 494
DC: Kieselgel, Dichlormethan / Methanol / Ammoniak 9:1:0.1, Rf-Wert = 0.47

### Beispiel 15

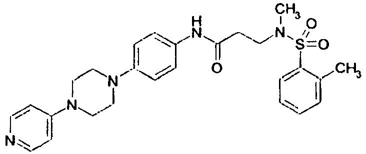

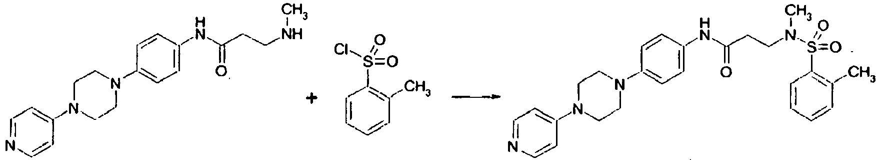

Beispiel 15 wird analog zu 10d aus 0.10 g (0.30 mMol) Produkt aus 10c, 0.047 g (0.25 mMol) o-Toluolsulfonsäurechlorid, 0.14 ml (98 mMol) Triethylamin in 5 ml Dichlormethan hergestellt.
C₂₆H₃₁N₅O₃S (493.62)
[M+H]+ = 494
HPLC (Methode 1): Retentionszeit = 2.37 min

### Beispiel 16

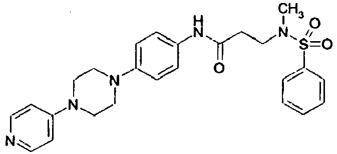

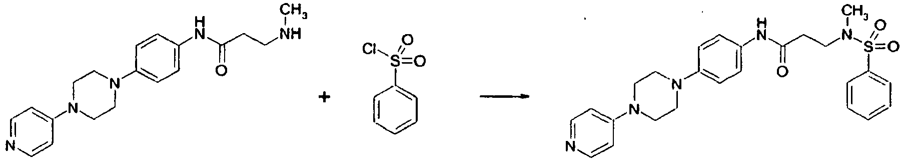

Beispiel 16 wird analog zu 10d aus 0.10 g (0.30 mMol) Produkt aus 10c, 0.043 g (0.25 mMol) Benzolsulfonsäurechlorid, 0.14 ml (98 mMol) Triethylamin in 5 ml Dichlormethan hergestellt.
C₂₅H₂₉N₅O₃S (479.60)
[M+H]+ = 480
HPLC (Methode 1): Retentionszeit = 2.40 min

### Beispiel 17

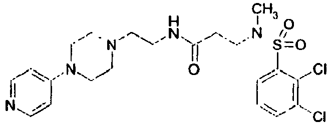

### 17a)

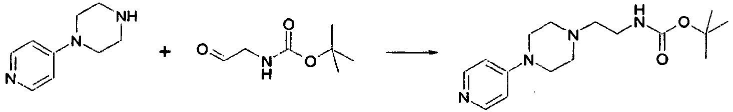

Eine Mischung aus 1.03 g (6.28 mMol) 1-(4-Pyridyl)-piperazin (Girindus) und 50 ml Dichlormethan wird mit 1.0 g (6.28 mMol) *tert*-Butyl-N-(2-oxoethyl)-carbamat (Aldrich) versetzt. Die Reaktionsmischung wird danach 30 min bei Raumtemperatur gerührt, anschließend unter Eisbadkühlung mit 2.66 g (12.56 mMol) Natrium-triacetoxyborhydrid versetzt und danach über Nacht bei Raumtemperatur gerührt. Man gibt weitere 60 ml Dichlormethan hinzu und wäscht die Reaktionsmischung mit gesättigter Natriumhydrogencarbonat-Lösung und gesättigter Natriumchlorid-Lösung. Die organische Phase wird über Natriumsulfat getrocknet und bis zur Trockene eingeengt. Das so erhaltene Rohprodukt wird durch Säulenchromatographie über Kieselgel (Elutionsmittel: Dichlormethan / Ethanol / Ammoniak 14:1:0.1 bis 10:1:0.1) gereinigt.
C₁₆H₂₆N₄O₂ (306.40)
[M+H]+ = 307

### 17b)

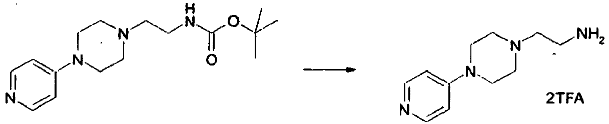

Eine Mischung aus 0.36 g (1.19 mMol) Produkt aus 17a, 1.19 ml (15.50 mMol) TFA und 2 ml Dichlormethan wird zwei Stunden bei Raumtemperatur gerührt. Danach wird die Reaktionsmischung im Vakuum bis zur Trockene eingeengt.
C₁₁H₁₈N₄ x 2C₂HF₃O₂ (434.33)
[M+H]+ = 207
HPLC (Methode 2): Retentionszeit = 0.98 min

### 17c)

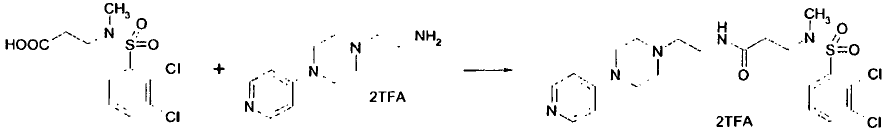

Beispiel 17 wird analog zu 1f aus 0.22 g (0.71 mMol) Produkt aus 1 c, 0.34 g (0.78 mMol) Produkt aus 17b, 0.50 ml (3.56 mMol) Triethylamin und 0.23 g (0.71 mMol) TBTU in 5.5 ml THF hergestellt.
C₂₁H₂₇Cl₂N₅O₃S x 2C₂HF₃O₂ (728.49)
[M+H]+ = 500/502/504
HPLC (Methode 2): Retentionszeit = 3.14 min

### Beispiel 18

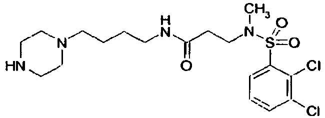

### 18a)

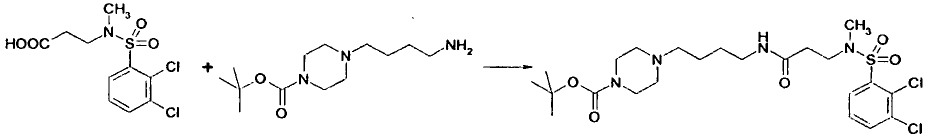

18a wird analog zu 1f aus 0.20 g (0.64 mMol) Produkt aus 1c, 0.17 g (0.64 mMol) 4-(4-Aminobutyl)-piperazin-1-carbonsäure-tert-butylester (J. Med. Chem. 47, 2004, 4300-4315), 0.27 ml (1.92 mMol) Triethylamin und 0.21 g (0.64 mMol) TBTU in 5 ml THF hergestellt.
C₂₃H₃₆CL₂N₄O₅S (551.53)
M+H]+ = 551/553/555
DC: Kieselgel, Dichlormethan / Methanol 30:1, Rf-Wert = 0.1

### 18b)

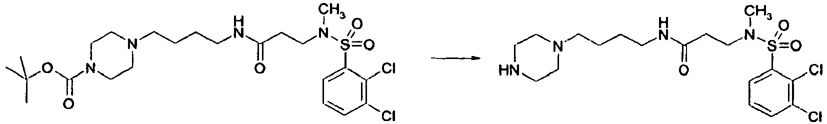

Eine Mischung aus 0.29 g (0.53 mMol) Produkt aus 18a, 0.53 ml TFA und 1 ml Dichlormethan wird zwei Stunden bei Raumtemperatur gerührt. Die Reaktionsmischung wird mit gesättigter Natriumhydrogencarbonat-Lösung gewaschen. Nach der Phasentrennung wird die wässrige Phase noch dreimal mit Dichlormethan extrahiert. Die organischen Extrakte werden über Natriumsulfat getrocknet und bis zur Trockene im Vakuum eingeengt.
C₁₈H₂₈Cl₂N₄O₃S (451.41)
[M+H]+ = 451/453/455
HPLC (Methode 2): Retentionszeit = 2.22 min

### Beispiel 19

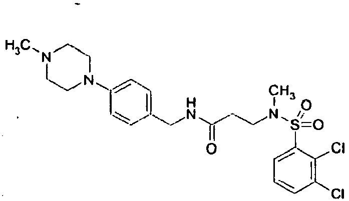

### 19a)

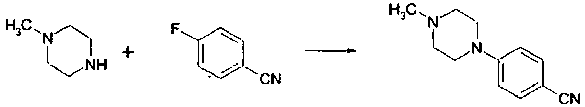

Eine Mischung aus 5.0 ml (45.13 mMol) *N*-Methylpiperazin und 0.73 g (6.00 mMol) 4-Fluorbenzonitril (Aldrich) wird 12 Stunden auf 80°C erhitzt. Danach wird bis zur Trockene eingeengt und der Rückstand mit Wasser versetzt. Es wird dreimal mit Ethylacetat extrahiert. Die organischen Extrakte werden über Natriumsulfat getrocknet und im Vakuum bis zur Trockene eingeengt.
C₁₂H₁₅N₃ (201.27)
M+H]+ = 202
DC: Kieselgel, Dichlormethan / Ethanol 95:5, Rf-Wert = 0.31

### 19b)

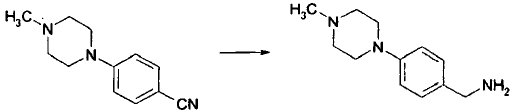

Eine Mischung aus 1.17 g (5.81 mMol) Produkt aus 19a, 0.3 g Raney-Nickel und 50 ml methanolische Ammoniaklösung wird bei 50°C im Autoklaven hydriert. Danach wird der Katalysator abfiltriert und das Filtrat im Vakuum bis zur Trockene eingeengt.
C₁₂H₁₉N₃ (205.30)
M+H]+ = 206

### 19c)

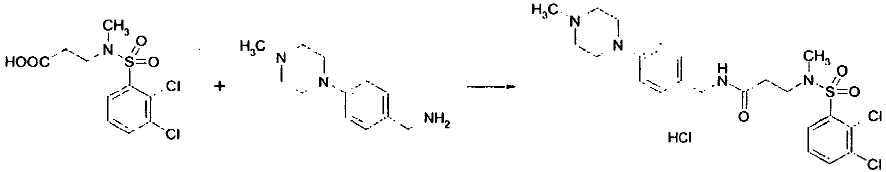

Beispiel 19 wird analog zu 1f aus 0.16 g (0.50 mMol) Produkt aus 1c, 0.10 g (0.50 mMol) Produkt aus 19b, 0.14 ml (1.00 mMol) Triethylamin und 0.16 g (0.50 mMol) TBTU in 3 ml DMF hergestellt.
C₂₂H₂₈Cl₂N₄O₃S x HCl (535.91)
[M+H]+ = 499/501/503
HPLC (Methode 3): Retentionszeit = 3.49 min

### Beispiel 20

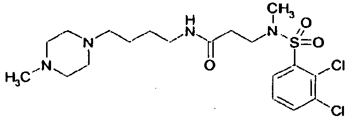

### 20a)

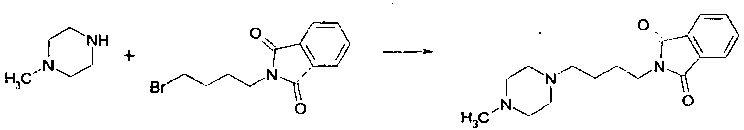

Eine Mischung aus 0.5 g (4.99 mMol) *N*-Methylpiperazin (Aldrich), 1.41 g (4.99 mMol) N-(4-Brombutyl)-phthalimid (Fluka), 0.86 ml (4.99 mMol) DIPEA und 9.3 ml Acetonitril wird 45 min in der Mikrowelle auf 120°C erhitzt. Danach wird bis zur Trockene eingeengt. Das so erhaltene Rohprodukt wird durch Säulenchromatographie über Kieselgel (Elutionsmittel: Dichlormethan / Methanol 98:2) gereinigt.
C₁₇H₂₃N₃O₂ (301.38)
M+H]+ = 302

### 20b)

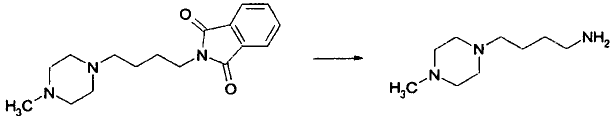

Eine Mischung aus 1.94 g (6.44 mMol) Produkt aus 20a, 1.61 g (25.75 mMol) Hydrazinhydrat Hydrat und 15 ml Ethanol absolut wird 5.5 Stunden im Autoklaven auf 120°C erhitzt. Der entstandene Niederschlag wird abfiltriert. Man engt danach das Filtrat bis zur Trockene ein. C₉H₂₁N₃ (171.28)

### 20c)

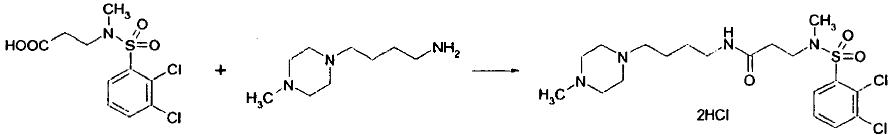

Beispiel 20 wird analog zu 1f aus 0.50 g (1.61 mMol) Produkt aus 1 c, 0.55 g (3.22 mMol) Produkt aus 20b, 0.67 ml (4.83 mMol) Triethylamin und 0.52 g (1.61 mMol) TBTU in 30 ml DMF hergestellt.
C₁₉H₃₀Cl₂N₄O₃S x 2HCl (538.36)
[M+H]+ = 465/467/469
HPLC (Methode 1): Retentionszeit = 2.15 min

### Beispiel 21

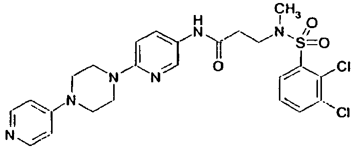

### 21a)

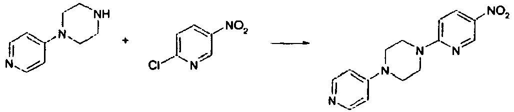

Eine Mischung aus 2.06 g (12.62 mMol) 1-Pyridin-4-yl-piperazin (Girindus), 2.00 g (12.62 mMol) 2-Chlor-5-nitropyridin (Fluka) und 50 ml Dichlormethan wird 15 min bei Raumtemperatur gerührt und anschließend mit 6.31 ml (12.62 mMol) 2 M Natronlauge versetzt. Die Reaktionsmischung wird 20 Stunden bei Raumtemperatur gerührt und danach mit 300 ml Dichlormethan und 100 ml 5%iger Natriumhydrogencarbonat-Lösung versetzt. Nach der Phasentrennung wird die organische Phase über Natriumsulfat getrocknet und im Vakuum bis zur Trockene eingeengt. Das Rohprodukt wird mit 100 ml Diethylether /Ethanol 2:1 verrührt, abfiltriert und getrocknet.
C₁₄H₁₅N₅O₂ (285.30)
[M+H]+ = 286
DC: Kieselgel, Dichlormethan / Ethanol 9:1, Rf-Wert = 0.10

### 21b)

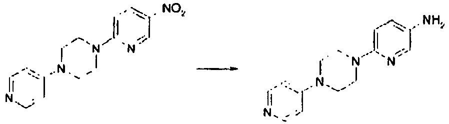

Eine Mischung aus 1.75 g (6.13 mMol) Produkt aus 21a, 0.4 g Palladium auf Kohle (10%), 100 ml Dichlormethan und 50 ml Methanol wird fünf Stunden im Autoklaven bei Raumtemperatur hydriert. Anschließend wird der Katalysator abgesaugt und das Filtrat im Vakuum bis zur Trockene eingeengt. Der Rückstand wird mit 100 ml Diethylether / Ethanol 2:1 verrührt und abgesaugt. Das so erhaltene Rohprodukt wird durch Säulenchromatographie über Kieselgel (Elutionsmittel: Dichlormethan / Methanol / Ammoniak 95:5:0.5) gereinigt.
C₁₄H₁₇N₅ (255.32)
[M+H]+ = 256
DC: Kieselgel, Dichlormethan / Methanol / Ammoniak 9:1:0.1, Rf-Wert = 0.37

### 21 c)

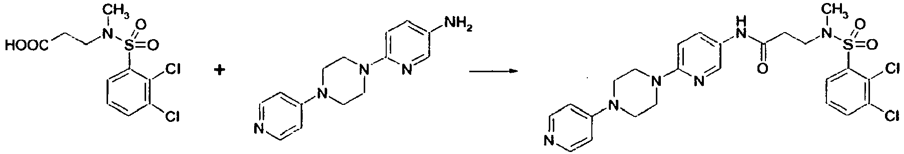

Beispiel 21 wird analog zu 1f aus 0.11 g (0.35 mMol) Produkt aus 1 c, 0.089 g (0.35 mMol) Produkt aus 21 b, 0.098 ml (0.70 mMol) Triethylamin und 0.13 g (0.42 mMol) TBTU in 15 ml THF hergestellt.
C₂₄H₂₆Cl₂N₆O₃S (549.47)
[M+H]+ = 549/551/553
HPLC (Methode 4): Retentionszeit = 2.7 min

### Beispiel 22

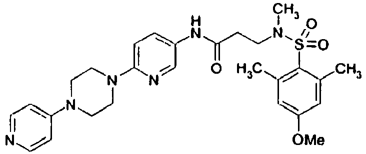

### 22a)

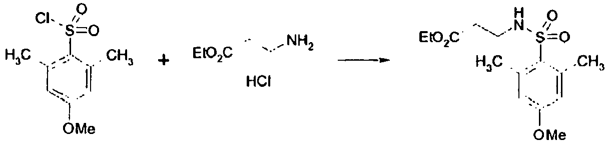

22a wird analog zu 3a aus 3.00 g (12.78 mMol) Produkt aus 13a, 2.16 g (14.06 mMol) β-Alaninethylester Hydrochlorid, 7.13 ml (51.13 mMol) Triethylamin in 70 ml Dichlormethan hergestellt.
C₁₄H₂₁NO₅S (315.39)
[M+H]+ = 316
DC: Kieselgel, Petrolether / Ethylacetat 2:1, Rf-Wert = 0.23

### 22b)

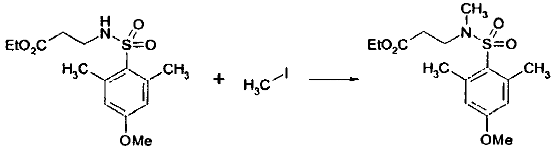

22b wird analog zu 3b aus 4.06 g (12.87 mMol) Produkt aus 22a, 2.40 ml (38.62 mMol) Methyliodid, 3.56 g (25.75 mMol) Kaliumcarbonat wasserfrei in 40 ml DMF hergestellt.
C₁₅H₂₃NO₅S (329.41)
[M+H]+ = 330
DC: Kieselgel, Petrolether / Ethylacetat 2:1, Rf-Wert = 0.36

### 22c)

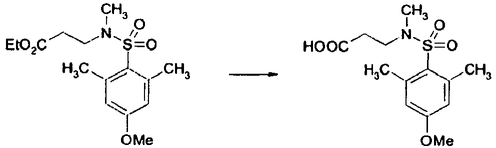

Die Säure wird analog zu 1c aus 3.83 g (11.63 mMol) Produkt aus 22b, 2.44 g (58.13 mMol) Lithiumhydroxid Monohydrat in 30 ml THF und 30 ml Wasser hergestellt.
C₁₃H₁₉NO₅S (301.36)
[M+H]+ = 302
DC: Kieselgel, Petrolether / Ethylacetat 1:1, Rf-Wert = 0.12

### 22d)

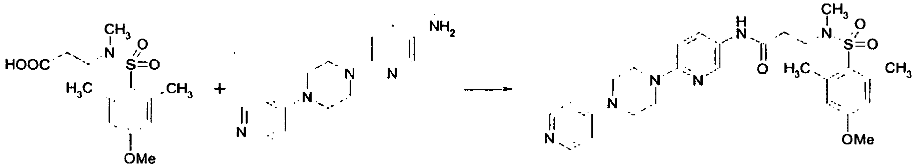

Beispiel 22 wird analog zu 1f aus 0.13 g (0.42 mMol) Produkt aus 22c, 0.089 g (0.35 mMol) Produkt aus 21 b, 0.098 ml (0.70 mMol) Triethylamin und 0.13 g (0.42 mMol) TBTU in 15 ml THF hergestellt.
C₂₇H₃₄N₆O₄S (538.66)
[M+H]+ = 539
HPLC (Methode 4): Retentionszeit = 2.6 min

### Beispiel 23

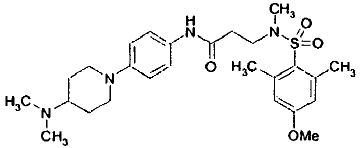

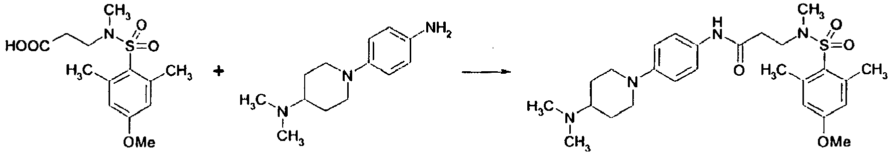

Beispiel 23 wird analog zu 1f aus 0.30 g (1.00 mMol) Produkt aus 22c, 0.22 g (1.00 mMol) Produkt aus 8b, 0.42 ml (2.99 mMol) Triethylamin und 0.32 g (1.00 mMol) TBTU in 15 ml DMF hergestellt.
C₂₆H₃₈N₄O₄S (502.67)
[M+H]+ = 503
HPLC (Methode 1): Retentionszeit = 2.47 min

### Beispiel 24

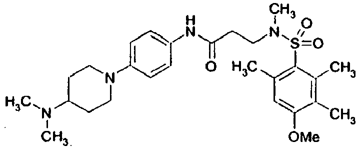

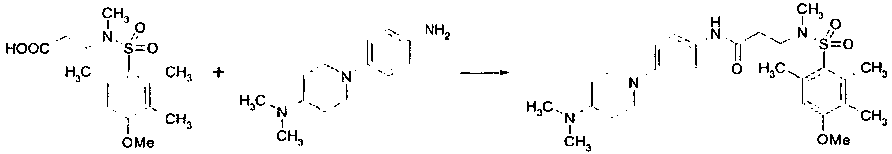

Beispiel 24 wird analog zu 1f aus 0.25 g (0.80 mMol) Produkt aus 3c, 0.18 g (0.80 mMol) Produkt aus 8b, 0.33 ml (2.39 mMol) Triethylamin und 0.26 g (0.80 mMol) TBTU in 10 ml DMF hergestellt.
C₂₇H₄₀N₄O₄S (516.70)
[M+H]+ = 517
HPLC (Methode 1): Retentionszeit = 2.50 min

### Beispiel 25

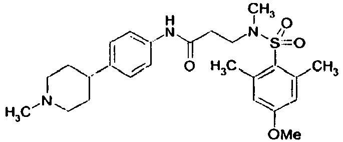

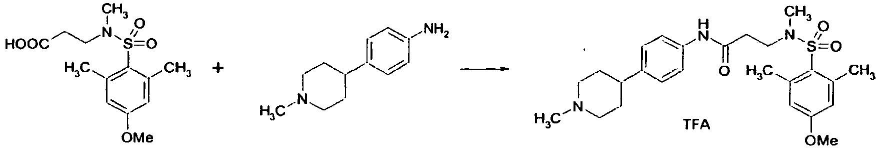

Beispiel 25 wird analog zu 1f aus 0.20 g (0.66 mMol) Produkt aus 22c, 0.14 g (0.73 mMol) 4-(1-Methylpiperidin-4-yl)-anilin (JW Pharmlab), 0.28 ml (1.99 mMol) Triethylamin und 0.21 g (0.66 mMol) TBTU in 50 ml THF hergestellt.
C₂₇H₄₀N₄O₄S x C₂HF₃O₂ (587.65)
[M+H]+ = 474
HPLC (Methode 2): Retentionszeit = 3.03 min

### Beispiel 26

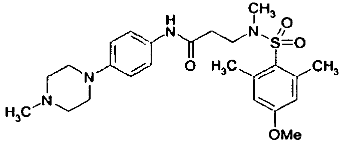

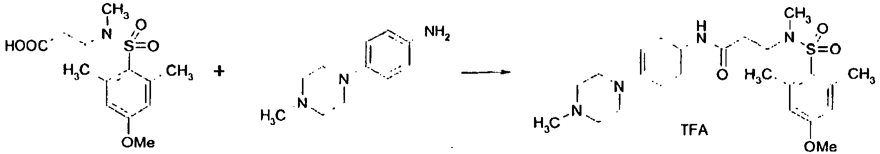

Beispiel 26 wird analog zu 1f aus 0.20 g (0.66 mMol) Produkt aus 22c, 0.14 g (0.73 mMol) 4-(4-Methylpiperazin-1-yl)-anilin (J. Med. Chem. SIR 48, 7, 2005, 2371-2387), 0.28 ml (1.99 mMol) Triethylamin und 0.21 g (0.66 mMol) TBTU in 5 ml THF hergestellt.
C₂₄H₃₄N₄O₄S x C₂HF₃O₂ (588.65)
[M+H]+ = 475
HPLC (Methode 5): Retentionszeit = 1.50 min

### Beispiel 27

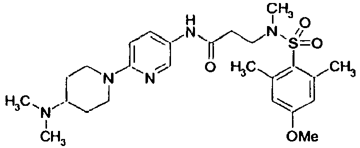

### 27a)

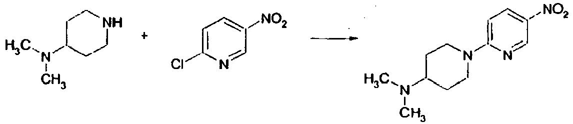

Eine Mischung aus 1.00 g (6.31 mMol) 2-Chlor-5-nitropyridin (Fluka), 1.32 ml (9.46 mMol) Triethylamin und 2 ml Methanol wird vorgelegt und langsam mit 1.13 g (8.83 mMol) 4-Dimethylamino-piperidin (Alfa Aesar) versetzt. Die Reaktionslösung wird anschließend mit halbgesättigter Natriumchlorid gequencht, der entstandene Niederschlag wird abgesaugt und getrocknet.
C₁₂H₁₈N₄O₂ (250.30)

### 27b)

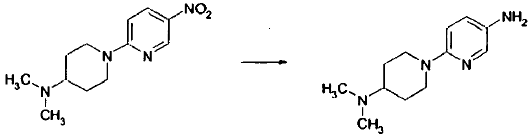

27b wird analog zu 8b aus 1.50 g (5.99 mMol) Produkt aus 27a, 0.20 g Palladium auf Kohle (10%) und 15 ml Methanol hergestellt.
C₁₂H₂₀N₄ (220.31)

### 27c)

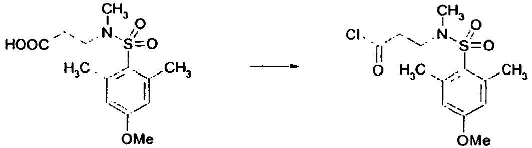

Eine Mischung aus 0.11 g (0.35 mMol) Produkt aus 22c und 2.0 ml Thionylchlorid wird zwei Stunden bei Raumtemperatur gerührt. Die Reaktionsmischung wird danach im Vakuum bis zur Trockene eingeengt.
C₁₃H₁₈ClNO₄S (319.81)

### 27d)

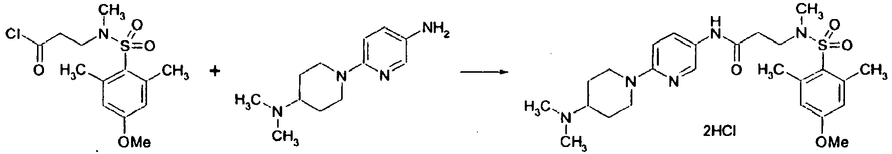

Eine Mischung aus 0.11 g (0.34 mMol) Produkt aus 27c, 0.091 g (0.41 mMol) Produkt aus 27b, 0.18 ml (1.03 mMol) DIPEA und 45 ml THF wird zwei Stunden bei Raumtemperatur gerührt. Die Reaktionsmischung wird anschließend im Vakuum bis zur Trockene eingeengt. Das Rohprodukt wird durch präparative HPLC gereinigt. Danach wird mit Hilfe von 4 M HCl in Dioxan das Hydrochlorid hergestellt.
C₂₅H₃₇N₅O₄S x 2HCl (576.58)
[M+H]+ = 504
HPLC (Methode 2): Retentionszeit = 2.73 min

### Beispiel 28

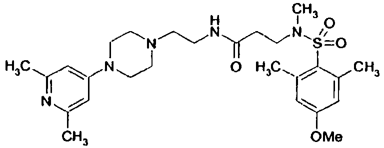

### 28a)

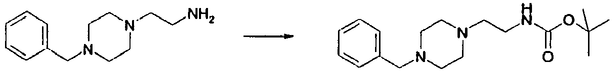

Eine Mischung aus 1.50 g (6.84 mMol) 1-(2-Aminoethyl)-4-benzylpiperazin (Maybridge), 1.64 g (7.52 mMol) Boc-Anhydrid und 30 ml Dichlormethan wird eine Stunde bei Raumtemperatur gerührt. Danach wird die Reaktionslösung mit 100 ml Dichlormethan verdünnt und mit 1 M Natronlauge und Wasser gewaschen. Die organische Phase wird über Natriumsulfat getrocknet und im Vakuum bis zur Trockene eingeengt.
C₁₈H₂₉N₃O₂ (319.44)
HPLC (Methode 4): Retentionszeit = 2.6 min

### 28b)

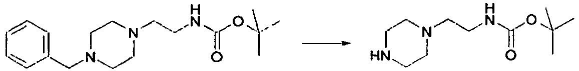

Eine Mischung aus 2.10 g (6.57 mMol) Produkt aus 28a, 0.25 g Palladium auf Kohle (10%) und 30 ml Methanol wird 15 Stunden bei Raumtemperatur im Autoklaven hydriert. Anschließend wird der Katalysator abgesaugt und das Filtrat im Vakuum bis zur Trockene eingeengt.
C₁₁H₂₃N₃O₄ (229.32)
[M+H]+ = 230
DC: Kieselgel, Dichlormethan / Ethanol 9:1, Rf-Wert = 0.08

### 28c)

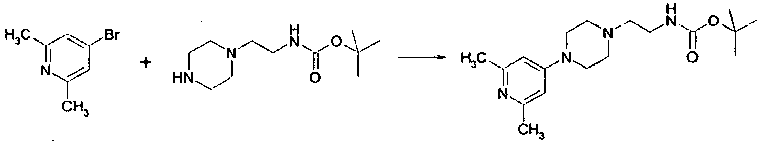

Eine Mischung aus 1.18 g (6.32 mMol) 4-Brom-2,6-dimethylpyridin (Acta Chem. Scand. Ser. B 42, 1988, 373-377), 1.45 g (6.32 mMol) Produkt aus 28b und 2.2 ml DIPEA wird 50 min auf 130°C in der Mikrowelle erhitzt. Man versetzt die Reaktionsmischung mit Ethylacetat und halbgesättigter Kaliumcarbonat-Lösung und trennt danach die Phasen. Die organische Phase wird über Natriumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 95:5:0.5) gereinigt.
C₁₈H₃₀N₄O₂S (334.46)
[M+H]+ = 335
DC: Kieselgel, Dichlormethan / Methanol / Ammoniak 9:1:0.1, Rf-Wert = 0.37

### 28d)

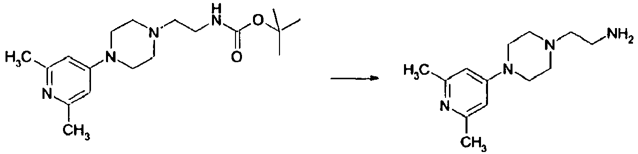

Eine Mischung aus 1.61 g (4.81 mMol) Produkt aus 28c, 3.70 ml TFA und 30 ml Dichlormethan wird sechs Stunden bei Raumtemperatur gerührt. Die Reaktionsmischung wird anschließend mit Dichlormethan verdünnt und mit 5%iger Natriumhydrogencarbonat-Lösung gewaschen. Die wässrige Phase wird noch zweimal mit Ethylacetat extrahiert. Die vereinigten organischen Phasen werden über Natriumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 90:10:1) gereinigt.
C₁₃H₂₂N₄ (234.34)
[M+H]+ = 235
DC: Kieselgel, Dichlormethan / Methanol / Ammoniak 9:1:0.1, Rf-Wert = 0.10

### 28e)

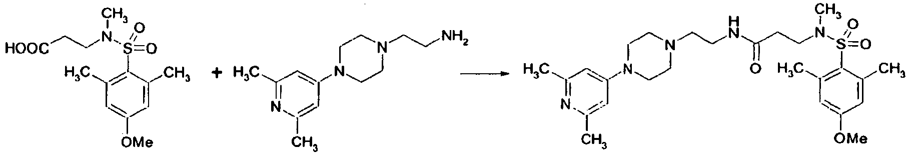

Beispiel 28 wird analog zu 1f aus 0.11 g (0.35 mMol) Produkt aus 22c, 0.082 g (0.35 mMol) Produkt aus 28d, 0.098 ml (0.70 mMol) Triethylamin und 0.13 g (0.42 mMol) TBTU in 7 ml THF und 1 ml DMF hergestellt.
C₂₆H₃₉N₅O₄S (517.69)
[M+H]+ = 518
HPLC (Methode 4): Retentionszeit = 2.4 min

### Beispiel 29

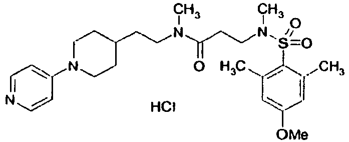

### 29a)

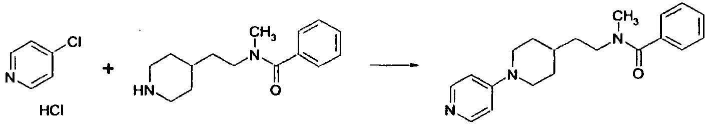

29a wird analog zu 28c aus 0.12 g (0.80 mMol) 4-Chlorpyridin Hydrochlorid (Aldrich), 0.20 g (0.80 mMol) *N*-Methyl-N-(2-piperidin-4-yl-ethyl)-benzamid (J. Med. Chem. 33, 1990, 1880-1887), 0.23 ml (1.68 mMol) Triethylamin in 5 ml Ethanol hergestellt.
C₂₀H₂₅N₃O (323.43)
[M+H]+ = 324

### 29b)

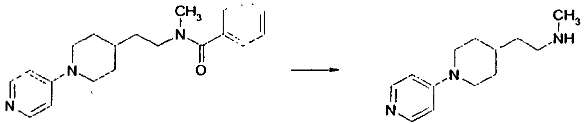

Eine Mischung aus 0.52 g (1.61 mMol) Produkt aus 29a, 10 ml 2 M Kalilauge und 10 ml Ethanol wird 30 Stunden unter Rückfluss erhitzt. Die Reaktionsmischung wird im Vakuum bis auf die Hälfte eingeengt und danach mit Dichlormethan extrahiert. Die organischen Extrakte werden über Natriumsulfat getrocknet und im Vakuum bis zur Trockene eingeengt.
C₁₃H₂₁N3 (219.33)
[M+H]+ = 220
DC: Kieselgel, Dichlormethan / Methanol / Ammoniak 8:2:0.01, Rf-Wert = 0.46

### 29c)

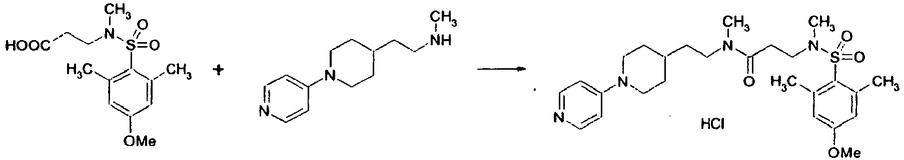

Beispiel 29 wird analog zu 1f aus 0.14 g (0.46 mMol) Produkt aus 22c, 0.10 g (0.46 mMol) Produkt aus 29b, 0.15 ml (1.09 mMol) Triethylamin und 0.16 g (0.50 mMol) TBTU in 30 ml THF und 5 ml DMF hergestellt.
C₂₆H₃₈N₄O₄S x HCl (539.13)
[M+H]+ = 503
DC: Kieselgel, Dichlormethan / Methanol / Ammoniak 8:2:0.01, Rf-Wert = 0.41

### Beispiel 30

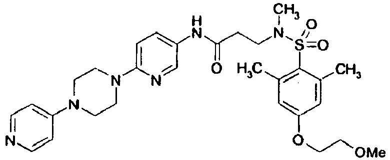

### 30a)

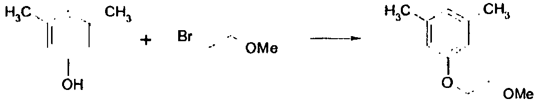

Eine Mischung aus 3.25 g (26.60 mMol) 3,5-Dimethylphenol (Aldrich), 3.20 g (28.52 mMol) Kalium-*tert*-butylat und 40 ml DMSO wird eine Stunde bei Raumtemperatur gerührt. Danach tropft man 3.80 g (27.34 mMol) Bromethylmethylether (Aldrich) hinzu und rührt weitere zwei Stunden bei Raumtemperatur. Die Reaktionsmischung wird auf Wasser gegossen und mit Diethylether extrahiert. Die organischen Extrakte werden mit Wasser und gesättigter Natriumchlorid-Lösung gewaschen, über Natriumsulfat getrocknet und im Vakuum bis zur Trockene eingeengt.
C₁₁H₁₆O₂ (180.24)
[M+H]+ = 181
DC: Kieselgel, Petrolether/Ethylacetat 9:1, Rf-Wert = 0.31

### 30b)

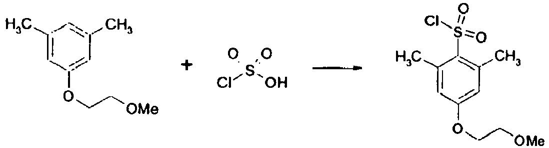

30b wird analog zu 13a aus 4.30 g (23.86 mMol) Produkt aus 30a und 5.60 g (48.06 mMol) Chlorsulfonsäure in 100 ml Dichlormethan hergestellt.
C₁₁H₁₅ClO₄S (278.75)
DC: Kieselgel, Petrolether/Ethylacetat 9:1, Rf-Wert = 0.06

### 30c)

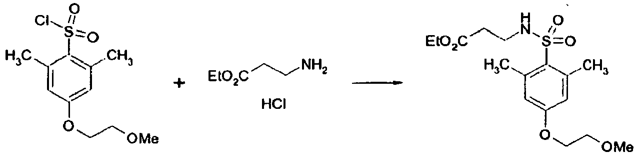

30a wird analog zu 3a aus 1.70 g (6.10 mMol) Produkt aus 30b, 1.20 g (7.81 mMol) β-Alaninethylester Hydrochlorid, 2.60 ml (18.65 mMol) Triethylamin in 30 ml Dichlormethan hergestellt.
C₁₆H₂₅NO₆S (359.44)
[M+H]+ = 360
DC: Kieselgel, Dichlormethan / Methanol 19:1, Rf-Wert = 0.51

### 30d)

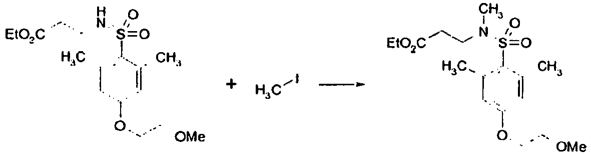

30d wird analog zu 3b aus 1.90 g (5.29 mMol) Produkt aus 30c, 1.10 g (7.75 mMol) Methyliodid, 1.50 g (10.85 mMol) Kaliumcarbonat wasserfrei in 30 ml DMF hergestellt.
C₁₇H₂₇NO₆S (373.47)
[M+H]+ = 374

### 30e)

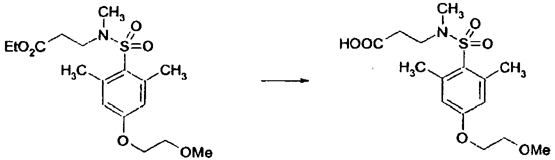

Die Säure wird analog zu 1c aus 1.70 g (4.55 mMol) Produkt aus 30d, 0.80 g (20.00 mMol) Natriumhydroxid in 30 ml Ethanol und 10 ml Wasser hergestellt.
C₁₅H₂₃NO₆S (345.41)
[M+H]+ = 346

### 30f)

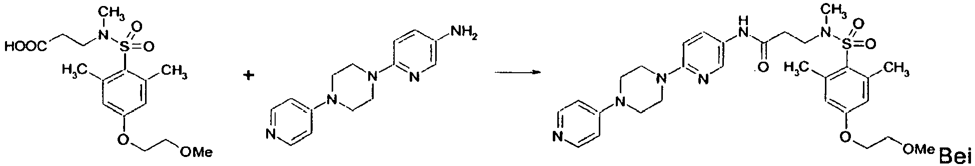

spiel 30 wird analog zu 1f aus 0.14 g (0.39 mMol) Produkt aus 30e, 0.10 g (0.39 mMol) Produkt aus 21 b, 0.10 ml (0.99 mMol) Triethylamin und 0.14 g (0.44 mMol) TBTU in 30 ml THF und 5 ml DMF hergestellt.
C₂₉H₃₈N₆O₅S (582.72)
[M+H]+ = 583
DC: Kieselgel, Dichlormethan / Methanol / Ammoniak 8:2:0.01, Rf-Wert = 0.44

### Beispiel 31

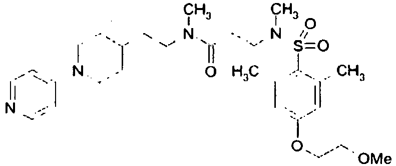

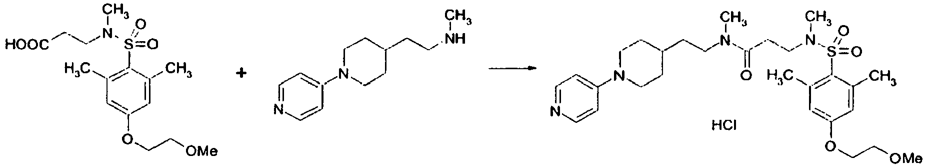

Beispiel 31 wird analog zu 1f aus 0.16 g (0.46 mMol) Produkt aus 30e, 0.10 g (0.46 mMol) Produkt aus 29b, 0.11 ml (1.09 mMol) Triethylamin und 0.16 g (0.50 mMol) TBTU in 30 ml THF und 5 ml DMF hergestellt.
C₂₈H₄₂N₄O₅S x HCl (583.18)
[M+H]+ = 547
DC: Kieselgel, Dichlormethan / Methanol / Ammoniak 8:2:0.01, Rf-Wert = 0.52

### Beispiel 32

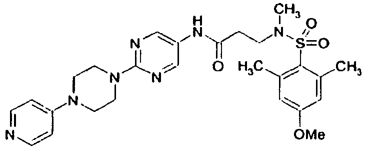

### 32a)

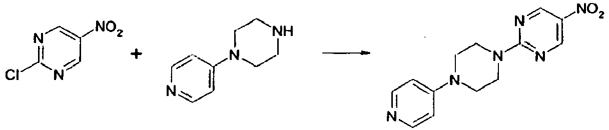

Eine Mischung aus 3.00 g (18.81 mMol) 2-Chlor-5-nitropyrimidin (Apin), 3.07 g (18.81 mMol) 1-(4-Pyridyl)-piperazin (Girindus), 9.40 ml (18.81 mMol) 2 M Natronlauge in 80 ml Dichlormethan wird 2.5 Stunden bei Raumtemperatur gerührt. Danach wird die Reaktionsmischung mit 100 ml Dichlormethan verdünnt und mit 5%iger Natriumhydrogencarbonat-Lösung gewaschen. Die organische Phase wird über Natriumsulfat getrocknet und im Vakuum bis zur Trockene eingeengt. Das Rohprodukt wird mit einer Mischung aus 50 ml Wasser und 30 ml Ethylacetat verrieben, abfiltriert und getrocknet.
C₁₃H₁₄N₆O₂ (286.29)
[M+H]+ = 287
HPLC (Methode 4): Retentionszeit = 2.6 min

### 32b)

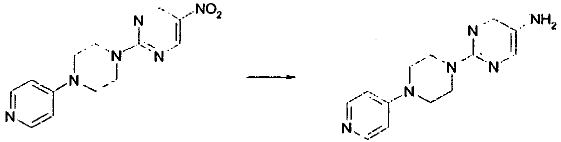

32b wird analog zu 21 b aus 1.93 g (6.74 mMol) Produkt aus 32a und 0.3 g Palladium auf Kohle (10 %) in 60 ml Dichlormethan und 30 ml Methanol hergestellt.
C₁₃H₁₆N₆ (256.31)
[M+H]+ = 257
DC: Kieselgel, Dichlormethan / Methanol 8:2, Rf-Wert = 0.11

### 32c)

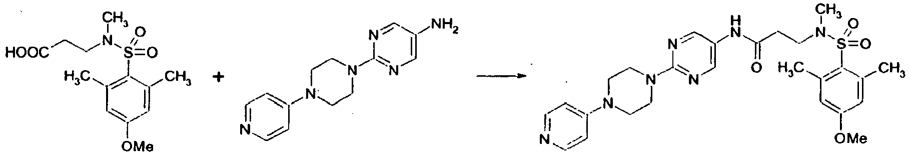

Beispiel 32 wird analog zu 1f aus 0.11 g (0.35 mMol) Produkt aus 22c, 0.090 g (0.35 mMol) Produkt aus 32b, 0.098 ml (0.70 mMol) Triethylamin und 0.13 g (0.42 mMol) TBTU in 7 ml THF und 1 ml DMF hergestellt.
C₂₆H₃₃N₇O₄S (539.65)
[M+H]+ = 540
HPLC (Methode 4): Retentionszeit = 2.9 min

### Beispiel 33

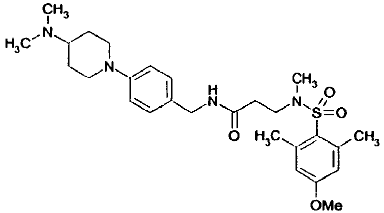

### 33a)

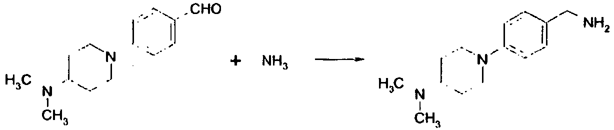

Eine Mischung aus 0.68 g (2.91 mMol) 4-(4-Dimethylamino-piperidin-1-yl)-benzaldehyd (Tetrahedron 57, 2001, 4781-4785), 15 ml 2 M Ammoniak in Ethanol und 0.10 g Raney-Nickel wird bei Raumtemperatur im Autoklaven hydriert. Danach wird der Katalysator abfiltriert und das Filtrat im Vakuum bis zur Trockene eingeengt.
C₁₄H₂₃N₃ (233.35)

### 33b)

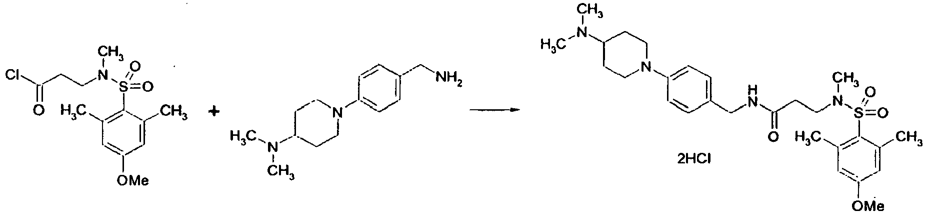

Beispiel 33 wird analog zu 27d aus 0.27 g (0.84 mMol) Produkt aus 27c, 0.63 g (2.68 mMol) Produkt aus 33a, 0.22 ml (1.26 mMol) DIPEA in 3 ml Dichlormethan hergestellt. C₂₇H₄₀N₄O₄S x 2HCl (589.62)
[M+H]+ = 517
HPLC (Methode 5): Retentionszeit = 1.46 min

### Beispiel 34

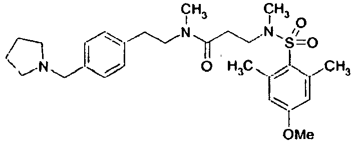

### 34a)

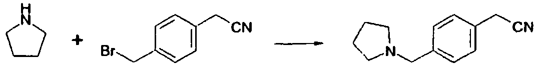

Eine Mischung aus 4.97 ml (59.50 mMol) Pyrrolidin und 100 ml Dichlormethan wird unter Eisbadkühlung langsam mit 5.00 g (23.80 mMol) (4-Brommethyl-phenyl)-acetonitril (Tetrahedron 47, 1991, 3969-3980) versetzt. Danach wird die Reaktionsmischung auf Raumtemperatur erwärmt, mit Wasser gewaschen, über Magnesiumsulfat getrocknet und im Vakuum bis zur Trockene eingeengt.
C₁₃H₁₆N₂ (200.28)
[M+H]+ = 201
DC: Kieselgel, Dichlormethan / Methanol / Ammoniak 9:1:0.1, Rf-Wert = 0.58

### 34b)

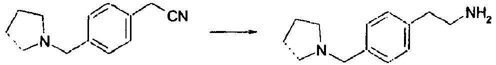

Eine Mischung aus 4.70 g (23.47 mMol) Produkt aus 34a, 0.5 g Raney-Nickel und 50 ml methanolische Ammoniak-Lösung wird im Autoklaven bei 50°C hydriert. Anschließend wird der Katalysator abfiltriert und das Filtrat im Vakuum bis zur Trockene eingeengt.
C₁₃H₂₀N₂ (204.31) [M+H]+ = 205

### 34c)

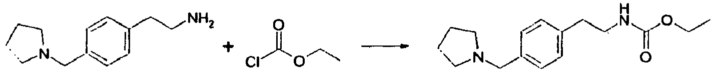

Eine Mischung aus 4.09 g (20.00 mMol) Produkt aus 34b, 5.62 ml (40.00 mMol) Triethylamin und 100 ml Dichlormethan wird unter Eisbadkühlung langsam mit 2.17 ml (22.00 mMol) Chlorameisensäureethylester (Aldrich) versetzt. Danach wird fünf Stunden bei Raumtemperatur gerührt. Die Reaktionsmischung wird anschließend mit Wasser gequencht und mit MTB-Ether extrahiert. Die organischen Extrakte werden zweimal mit Wasser gewaschen, über Magnesiumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₆H₂₄N₂O₂ (276.37)
[M+H]+ = 277

### 34d)

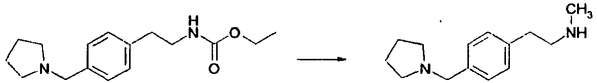

Eine Mischung aus 3.60 g (13.03 mMol) Produkt aus 34c und 25 ml THF wird langsam mit 51.05 ml (51.05 mMol) 1 M Lithiumaluminiumhydrid in THF (Aldrich) versetzt. Danach wird zwei Stunden bei Raumtemperatur und zwei Stunden bei 70°C gerührt. Die Reaktionsmischung wird anschließend mit Wasser und 15%iger Natronlauge gequencht und noch eine Stunde bei Raumtemperatur gerührt. Der entstandene Niederschlag wird abfiltriert und das Filtrat im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₄H₂₂N₂ (218.34)
[M+H]+ = 219

### 34e)

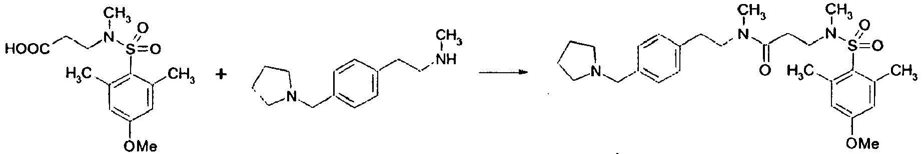

Beispiel 34 wird analog zu 1f aus 0.11 g (0.35 mMol) Produkt aus 22c, 0.076 g (0.35 mMol) Produkt aus 34d, 0.098 ml (0.70 mMol) Triethylamin und 0.13 g (0.42 mMol) TBTU in 7 ml THF und 1 ml DMF hergestellt.
C₂₇H₃₉N₃O₄S (501.68)
[M+H]+ = 502
HPLC (Methode 4): Retentionszeit = 3.1 min

### Beispiel 35

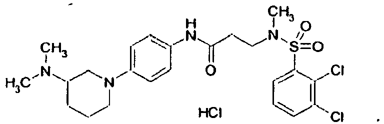

### 35a)

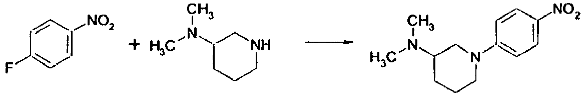

Eine Mischung aus 0.33 g (2.34 mMol) 1-Fluor-4-nitrobenzol (Aldrich), 0.30 g (2.34 mMol) 3-Dimethylamino-piperidin (Chess), 0.46 ml (3.27 mMol) Triethylamin und 4 ml DMF wird sechs Stunden bei Raumtemperatur gerührt. Die Reaktionsmischung wird danach mit Wasser gequencht und mit Dichlormethan extrahiert. Die organischen Extrakte werden mit gesättigter Natriumchlorid-Lösung gewaschen, über Natriumsulfat getrocknet und im Vakuum bis zur Trockene eingeengt.
C₁₃H₁₉N₃O₂ (249.31)
[M+H]+ = 250
DC: Kieselgel, Dichlormethan / Methanol / Ammoniak 9:1:0.1, Rf-Wert = 0.56

### 35b)

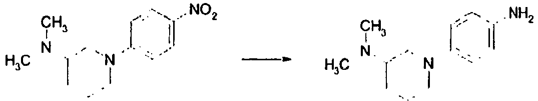

Eine Mischung aus 0.25 g (1.00 mMol) Produkt aus 35a, 30 mg Raney-Nickel und 10 ml Ethylacetat wird im Autoklaven bei Raumtemperatur hydriert. Der Katalysator wird abfiltriert und das Filtrat im Vakuum bis zur Trockene eingeengt.
C₁₃H₂₁N₃ (219.33)
DC: Kieselgel, Dichlormethan / Methanol / Ammoniak 9:1:0.1, Rf-Wert = 0.25

### 35c)

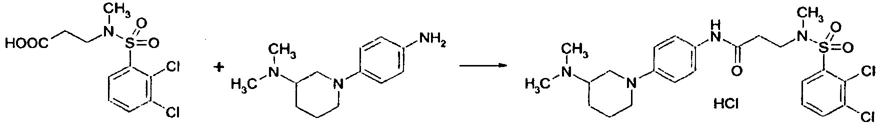

Beispiel 35 wird analog zu 1f aus 0.16 g (0.50 mMol) Produkt aus 1c, 0.11 g (0.50 mMol) Produkt aus 35b, 0.14 ml (1.00 mMol) Triethylamin und 0.16 g (0.50 mMol) TBTU in 3 ml DMF hergestellt.
C₂₃H₃₀Cl₂N₄O₃S x HCl (539.14)
[M+H]+ = 513/515/517
HPLC (Methode 1): Retentionszeit = 2.43 min

### Beispiel 36

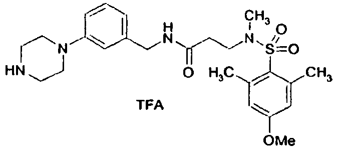

### 36a)

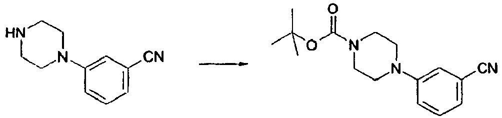

Eine Mischung aus 2.50 g (13.35 mMol) 3-Piperazin-1-yl-benzonitril (Tetrahedron 55, 1999, 13285-13300), 3.00 g (13.75 mMol) Boc-Anhydrid, 2.40 ml (13.78 mMol) DIPEA und 50 ml THF wird vier Stunden bei Raumtemperatur gerührt und danach im Vakuum bis zur Trockene eingeengt. Der Rückstand wird in Wasser aufgenommen und mit Diethylether extrahiert. Die organischen Extrakte werden über Natriumsulfat getrocknet und im Vakuum bis zur Trockene eingeengt.
C₁₆H₂₁N₃O₂ (287.36)
[M+Na]+ = 310

### 36b)

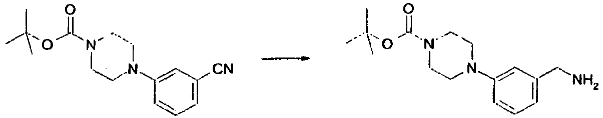

36b wird analog zu 34b aus 4.40 g (15.31 mMol) Produkt aus 36a, 0.7 g Raney-Nickel und 45 ml methanolischer Ammoniak-Lösung hergestellt.
C₁₆H₂₅N₃O₂ (291.39)
[M+H]+ = 292

### 36c)

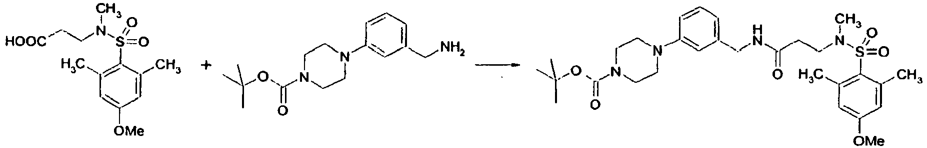

36c wird analog zu 1f aus 0.40 g (1.33 mMol) Produkt aus 22c, 0.43 g (1.46 mMol) Produkt aus 36b, 0.56 ml (3.98 mMol) Triethylamin und 0.43 g (1.33 mMol) TBTU in 10 ml THF hergestellt.
C₂₉H₄₂N₄O₆S (574.73)
M+H]+ = 575
DC: Kieselgel, Dichlormethan / Methanol 9:1, Rf-Wert = 0.53

### 36d)

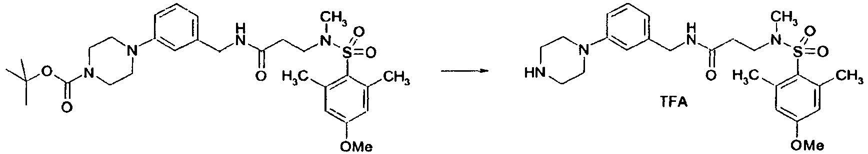

Beispiel 36 wird analog zu 18b aus 0.50 g (0.57 mMol) Produkt aus 36c, 0.44 ml TFA in 5 ml Dichlormethan hergestellt.
C₁₈H₂₈Cl₂N₄O₃S x C₂HF₃O₂ (588.64)
[M+H]+ = 475
HPLC (Methode 2): Retentionszeit = 2.95 min

### Beispiel 37

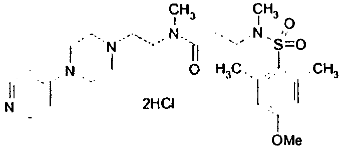

### 37a)

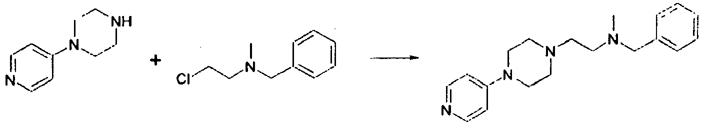

Eine Mischung aus 2.00 g (12.25 mMol) 1-(4-Pyridyl)-piperazin (Girindus), 1.65 g (14.70 mMol) Kalium-tert-butylat und 50 ml DMSO wird 30 min bei Raumtemperatur gerührt und danach mit 2.25 g (12.25 mMol) 1-Benzylmethylamino-2-chlorethan (Chem. Pharm. Bull. 45, 1997, 996-1007) versetzt. Die Reaktionsmischung wird über Nacht bei Raumtemperatur gerührt und anschließend auf Eiswasser gegossen. Man extrahiert viermal mit Dichlormethan. Die organischen Extrakte werden über Natriumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₉H₂₆N₄ (310.44)
[M+H]+ = 311
DC: Kieselgel, Dichlormethan / Methanol / Ammoniak 9:1:0.1, Rf-Wert = 0.22

### 37b)

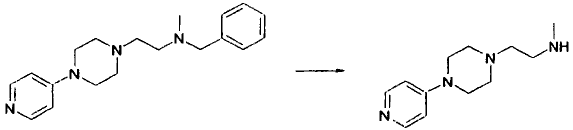

Eine Mischung aus 1.78 g (5.73 mMol) Produkt aus 37a, 0.40 g Palladiumhydroxid und 50 ml Methanol wird bei 40°C im Autoklaven hydriert. Der Katalysator wird abfiltriert und das Filtrat im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₂H₂₀N₄ (220.31)
[M+H]+ = 221
DC: Kieselgel, Dichlormethan / Methanol / Ammoniak 9:1:0.1, Rf-Wert = 0.13

### 37c)

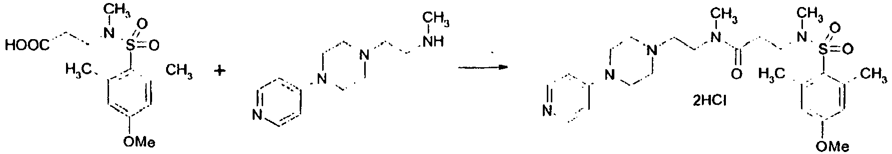

Beispiel 37 wird analog zu 1f aus 0.11 g (0.35 mMol) Produkt aus 22c, 0.072 g (0.35 mMol) Produkt aus 37b, 0.098 ml (0.70 mMol) Triethylamin und 0.13 g (0.42 mMol) TBTU in 7 ml THF hergestellt.
C₂₅H₃₇N₅O₄S x 2HCl (576.58)
[M+H]+ = 504
HPLC (Methode 4): Retentionszeit = 2.5 min

### Beispiel 38

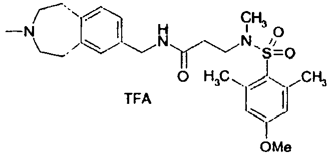

### 38a)

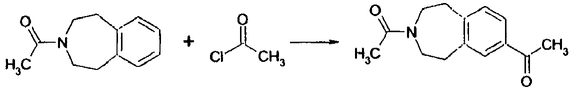

Eine Mischung aus 4.44 g (33.29 mMol) Aluminiumchlorid (Merck) und 16 ml Dichlorethan wird vorgelegt und unter Eisbadkühlung langsam mit 1.24 ml (17.44 mMol) Acetylchlorid (Aldrich) versetzt. Man lässt 30 min bei Raumtemperatur rühren. Anschließend werden 3.00 g (15.85 mMol) 1-(1,2,4,5-Tetrahydrobenzo[d]azepin-3-yl)-ethanon (J. Med.Chem. 46, 2003, 4952-4964) in 7 ml Dichlorethan langsam zur Reaktionsmischung gegeben. Nach zweistündigem Rühren bei Raumtemperatur wird die Reaktionsmischung auf eine Mischung aus 6 M HCl und Eis gegossen. Nach der Phasentrennung wird die wässrige Phase noch dreimal mit Dichlormethan extrahiert. Die vereinigten organischen Phasen werden mit Wasser gewaschen, über Natriumsulfat getrocknet und im Vakuum bis zur Trockene eingeengt. Das so erhaltene Rohprodukt wird mit Diethylether verrieben, abfiltriert und getrocknet.
C₁₄H₁₇NO₂ (231.29)
[M+H]+ = 232

### 38b)

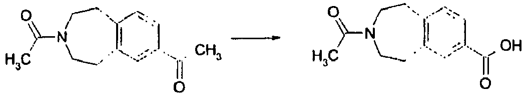

Eine Mischung aus 2.86 g (12.37 mMol) Produkt aus 38a und 79 ml 2.5 M Natronlauge wird bei Raumtemperatur langsam mit 2.48 ml (48.23 mMol) Brom versetzt. Die Reaktionsmischung wird danach eine Stunde bei Raumtemperatur gerührt. Der entstandene Niederschlag wird abfiltriert und das Filtrat mit MTB-Ether extrahiert. Die wässrige Phase wird anschließend unter Eisbadkühlung mit konzentrierter HCl und wenig Natriumdisulfit-Lösung versetzt. Der entstandene Niederschlag wird abfiltriert und im Umlufttrockenschrank bei 30°C getrocknet.
C₁₃H₁₅NO₃ (233.26)
[M+H]+ = 234

### 38c)

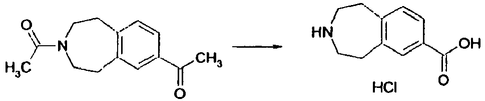

Eine Mischung aus 2.12 g (9.09 mMol) Produkt aus 38b und 20 ml 6 M HCl wird 3.5 Tage auf 100°C erhitzt. Die Reaktionsmischung wird danach mit einer Mischung aus Eis und Ethanol gekühlt. Der entstandene Niederschlag wird abfiltriert, mit wenig gekühltem Aceton und Diethylether gewaschen und im Exsikkator getrocknet.
C₁₁H₁₃NO₂ x HCl (227.69)
[M+H]+ = 192

### 38d)

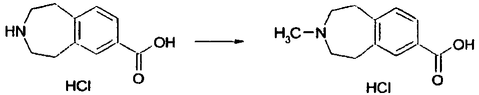

Eine Mischung aus 2.03 g (8.92 mMol) Produkt aus 38c und 3.36 ml (89.16 mMol) Ameisensäure wird erst langsam mit 0.94 ml (17.83 mMol) 50%iger Natronlauge, dann mit 2.66 ml (35.66 mMol) 37%iger Formalin-Lösung versetzt. Die Reaktionsmischung wird zwei Stunden auf 70°C erhitzt und danach im Vakuum bis zur Trockene eingeengt. Der Rückstand wird mit Wasser und konzentrierter HCl versetzt und nochmals bis zur Trockene eingeengt. Das Rohprodukt wird mit wenig eiskaltem Wasser verrieben, abfiltriert und im Umlufttrockenschrank bei 60°C getrocknet.
C₁₂H₁₅NO₂ x HCl (241.71)
[M+H]+ = 206

### 38e)

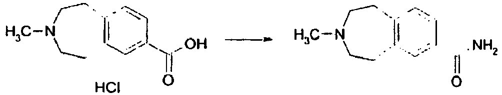

38e wird analog zu 1f aus 2.00 g (8.27 mMol) Produkt aus 38d, 18.20 ml (9.10 mMol) Ammoniak 0.5 M in Dioxan (Aldrich), 3.46 ml (24.82 mMol) Triethylamin und 3.19 g (9.93 mMol) TBTU in 30 ml THF hergestellt.
C₁₂H₁₆N₂O (204.27)
[M+H]+ = 205
HPLC (Methode 2): Retentionszeit = 1.54 min

### 38f)

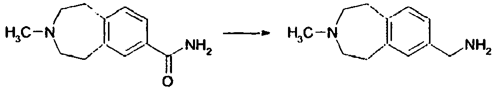

Eine Mischung aus 5.20 ml (5.20 mMol) Lithiumaluminiumhydrid 1 M in THF (Aldrich) und 18 ml THF wird auf 50°C erhitzt und langsam mit 0.84 g (2.64 mMol) Produkt aus 38e versetzt. Die Reaktionsmischung wird danach 30 min bei 50°C gerührt. Man kühlt anschließend auf -20°C ab und quencht die Reaktionsmischung erst mit einer Mischung aus Wasser und THF, dann mit 2 M Natronlauge. Es wird eine Stunde bei Raumtemperatur gerührt. Der entstandene Niederschlag wird abfiltriert, das Filtrat im Vakuum bis zur Trockene eingeengt. Der Rückstand wird in Dichlormethan aufgenommen und mit gesättigter Natriumhydrogensulfat-Lösung gewaschen. Die organische Phase wird über Natriumsulfat getrocknet und im Vakuum bis zur Trockene eingeengt.
C₁₂H₁₈N₂ (190.28)
[M+H]+ = 191
HPLC (Methode 2): Retentionszeit = 2.21 min

### 38g)

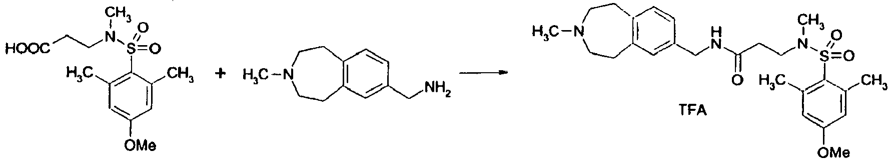

Beispiel 38 wird analog zu 1f aus 0.19 g (0.63 mMol) Produkt aus 22c, 0.12 g (0.63 mMol) Produkt aus 38f, 0.26 ml (1.89 mMol) Triethylamin und 0.20 g (0.63 mMol) TBTU in 5 ml THF hergestellt.
C₂₅H₃₅N₃O₄S x C₂HF₃O₂ (587.65)
[M+H]+ = 474
HPLC (Methode 4): Retentionszeit = 2.98 min

### Beispiel 39

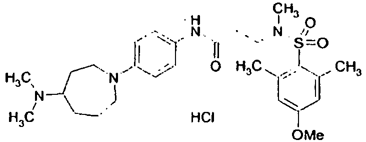

### 39a)

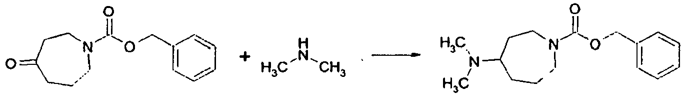

Eine Mischung aus 1.50 g (6.07 mMol) 4-Oxo-azepan-1-carbonsäurebenzylester (Tyger), 20 ml (40.00 mMol) Dimethylamin 2 M in THF (Aldrich) und 0.34 ml (6.07 mMol) Essigsäure wird 20 min bei Raumtemperatur gerührt und danach mit 3.82 g (18.00 mMol) Natriumtriacetoxyborhydrid (Aldrich) versetzt. Man lässt über Nacht bei Raumtemperatur rühren. Die Reaktionsmischung wird anschließend mit gesättigter Natriumhydrogencarbonat-Lösung versetzt und mit Ethylacetat extrahiert. Die organischen Extrakte werden mit gesättigter Natriumchlorid-Lösung gewaschen, über Natriumsulfat getrocknet und im Vakuum bis zur Trockene eingeengt. Das so erhaltene Rohprodukt wird durch Säulenchromatographie über Kieselgel (Elutionsmittel: Dichlormethan / 4-12 % (Methanol +10% Ammoniak)) gereinigt. C₁₆H₂₄N₂O₂ (276.37)
[M+H]+ = 277
HPLC (Methode 1): Retentionszeit = 2.12 min

### 39b)

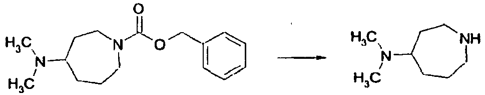

Eine Mischung aus 1.00 g (3.62 mMol) Produkt aus 39a, 0.10 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
C₈H₁₈N₂ (142.24)
[M+H]+ = 143
DC: Kieselgel, Dichlormethan / Methanol / Ammoniak 9:1:0.1, Rf-Wert = 0.12

### 39c)

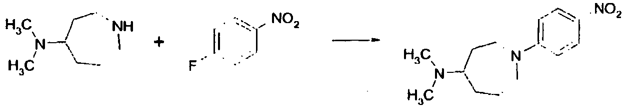

39c wird analog zu 1d aus 0.56 g (4.37 mMol) Produkt aus 39b, 0.64 g (4.50 mMol) 4-Fluor-nitrobenzol (Aldrich), 0.65 ml (4.60 mMol) Triethylamin in 5 ml DMF hergestellt.
C₁₄H₂₁N₃O₂ (263.34)
[M+H]+ = 264
DC: Kieselgel, Dichlormethan / Methanol / Ammoniak 9:1:0.1, Rf-Wert = 0.60

### 39d)

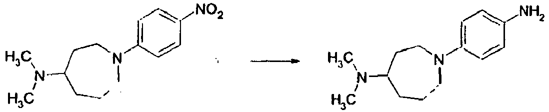

39d wird analog zu 8b aus 0.94 g (3.55 mMol) Produkt aus 39c und 0.10 g Palladium auf Kohle (10%) in 30 ml Methanol hergestellt.
C₁₄H₂₃N₃O₂ (233.35)
DC: Kieselgel, Dichlormethan / Methanol /Ammoniak 9:1:0.1, Rf-Wert = 0.15

### 39e)

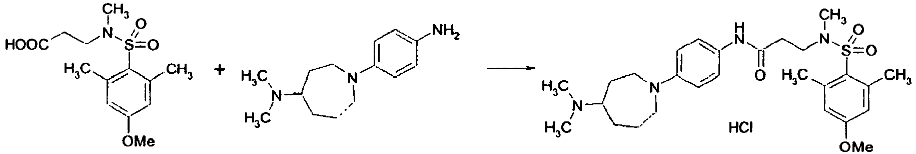

Beispiel 39 wird analog zu 1f aus 0.30 g (1.00 mMol) Produkt aus 22c, 0.23 g (1.00 mMol) Produkt aus 39d, 0.42 ml (3.00 mMol) Triethylamin und 0.32 g (1.00 mMol) TBTU in 15 ml DMF hergestellt.
C₂₇H₄₀N₄O₄S x HCl (553.16)
[M+H]+ = 517
DC: Kieselgel, Dichlormethan / Methanol / Ammoniak 9:1:0.1, Rf-Wert = 0.42
HPLC (Methode 5): Retentionszeit = 1.50 min

### Beispiel 40

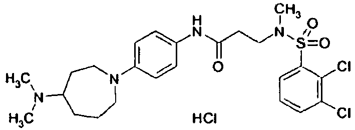

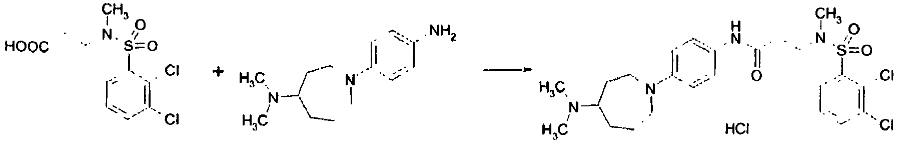

Beispiel 40 wird analog zu 1f aus 0.16 g (0.50 mMol) Produkt aus 1c, 0.12 g (0.50 mMol) Produkt aus 39d, 0.21 ml (1.50 mMol) Triethylamin und 0.16 g (0.50 mMol) TBTU in 5 ml DMF hergestellt.
C₂₄H₃₂Cl₂N₄O₃S x HCl (563,97)
[M+H]+ = 527/529/531
DC: Kieselgel, Dichlormethan / Methanol / Ammoniak 6:1:0.2, Rf-Wert = 0.48
HPLC (Methode 5): Retentionszeit = 1.53 min

### Beispiel 41

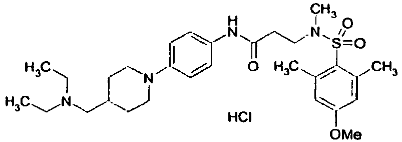

### 41a)

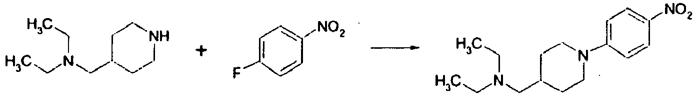

41a wird analog zu 1d aus 1.00 g (5.87 mMol) Diethyl-piperidin-4-ylmethyl-amin (Chem. Pharm. Bull. 42, 1994, 74-84), 0.83 g (5.87 mMol) 4-Fluor-nitrobenzol (Aldrich), 1.14 ml (8.20 mMol) Triethylamin in 12 ml DMF hergestellt.
C₁₆H₂₅N₃O₂ (291.39)
[M+H]+ = 292
DC: Kieselgel, Dichlormethan / Ethanol 9:1, Rf-Wert = 0.5

### 41b)

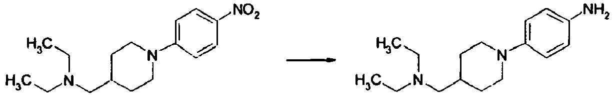

Eine Mischung aus 0.40 g (1.37 mMol) Produkt aus 41a, 0.10 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
C₁₆H₂₇N₃ (261.41)
DC: Kieselgel, Dichlormethan / Ethanol 9:1, Rf-Wert = 0.1

### 41c)

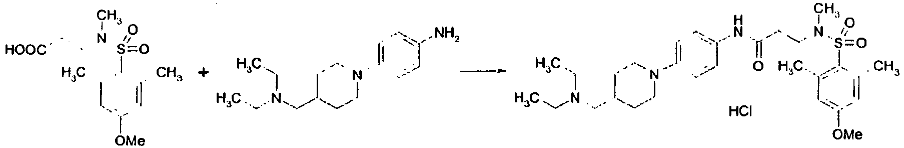

Beispiel 41 wird analog zu 1f aus 0.40 g (1.34 mMol) Produkt aus 22c, 0.35 g (1.34 mMol) Produkt aus 41 b, 0.47 ml (3.35 mMol) Triethylamin und 0.43g (1.34 mMol) TBTU in 7 ml THF und 1 ml DMF hergestellt.
C₂₉H₄₄N₄O₄S x HCl (581.21)
[M+H]+ = 545
HPLC (Methode 5): Retentionszeit = 1.42 min

### Beispiel 42

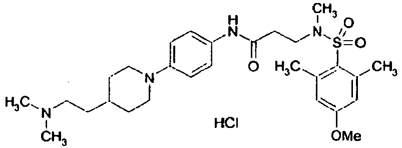

### 42a)

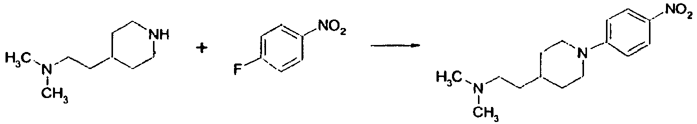

42a wird analog zu 1d aus 1.88 g (12.00 mMol) Dimethyl-(2-piperidin-4-yl-ethyl)-amin (J. Med. Chem. 36, 1993, 162-165), 1.69 g (12.00 mMol) 4-Fluor-nitrobenzol (Aldrich), 2.37 ml (17.00 mMol) Triethylamin in 15 ml DMF hergestellt.
C₁₅H₂₃N₃O₂ (277.36)
[M+H]+ = 278
DC: Kieselgel, Dichlormethan / Ethanol 9:1, Rf-Wert = 0.2

### 42b)

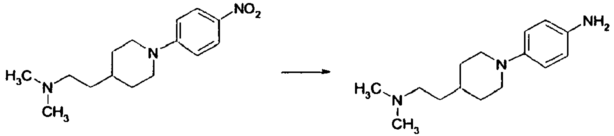

Eine Mischung aus 0.30 g (1.08 mMol) Produkt aus 42a, 0.10 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
C₁₅H₂₅N₃ (247.38)

### 42c)

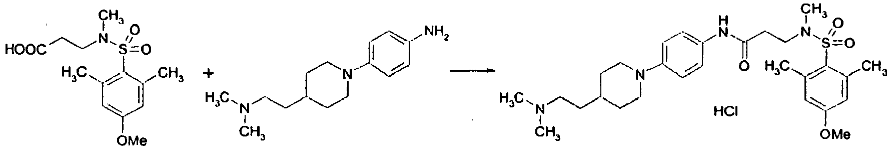

Beispiel 42 wird analog zu 1f aus 0.33 g (1.08 mMol) Produkt aus 22c, 0.27 g (1.08 mMol) Produkt aus 42b, 0.38 ml (2.70 mMol) Triethylamin und 0.35g (1.08 mMol) TBTU in 7 ml THF und 1 ml DMF hergestellt.
C₂₈H₄₂N₄O₄S (530.72)
[M+H]+ = 531
HPLC (Methode 5): Retentionszeit = 1.41 min

### Beispiel 43

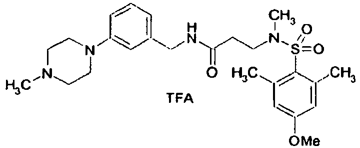

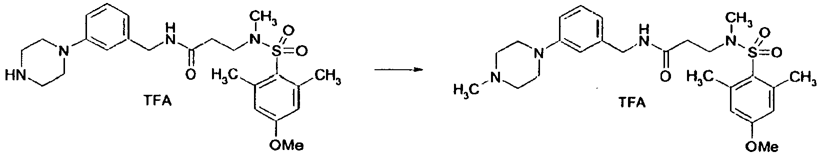

Eine Mischung aus 0.15 g (0.25 mMol) Produkt aus 36d, 0.025 ml (0.40 mMol) Methyliodid (Aldrich), 0.10 ml (0.75 mMol) Kaliumcarbonat und 5 ml Acetonitril wird über Nacht bei Raumtemperatur gerührt. Die Reaktionsmischung wird danach mit 10%iger TFA versetzt, das Produkt durch präparative HPLC abgetrennt.
C₂₅H₃₆N₄O₄S x C₂HF₃O₂ (602.67)
[M+H]+ = 489
HPLC (Methode 2): Retentionszeit = 2.99 min

### Beispiel 44

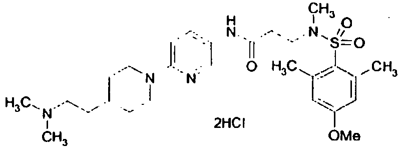

### 44a)

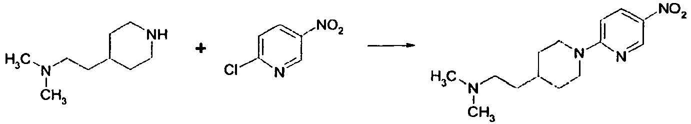

Eine Mischung aus 0.72 g (4.61 mMol) Dimethyl-(2-piperidin-4-yl-ethyl)-amin (J. Med. Chem. 36,1993,162-165),0.73 g (4.61 mMol) 2-Chlor-5-nitropyridin (Fluka), 1.30 g (9.41 mMol) Kaliumcarbonat und 100 ml THF wird über das Wochenende bei Raumtemperatur gerührt. Der Niederschlag wird abfiltriert, das Filtrat wird im Vakuum bis zur Trockene eingeengt. Das so erhaltene Rohprodukt wird durch Säulenchromatographie über Kieselgel (Elutionsmittel: Dichlormethan / Methanol 19:1 bis 4:1) gereinigt.
C₁₄H₂₂N₄O₂ (278.35)
[M+H]+ = 279

### 44b)

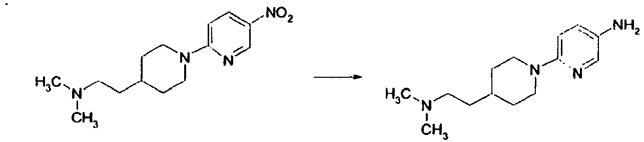

Eine Mischung aus 0.26 g (0.93 mMol) Produkt aus 44a, 0.05 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
C₁₄H₂₄N₄ (248.37)
[M+H]+ = 249

### 44c)

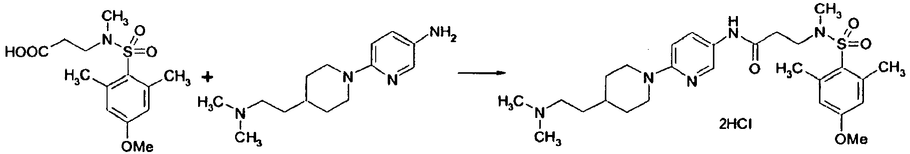

Beispiel 44 wird analog zu 1f aus 0.12 g (0.40 mMol) Produkt aus 22c, 0.10 g (0.40 mMol) Produkt aus 44b, 0.069 ml (0.50 mMol) Triethylamin und 0.14g (0.44 mMol) TBTU in 40 ml THF und 5 ml DMF hergestellt.
C₂₇H₄₁N₅O₄S x 2HCl (604.63)
[M+H]+ = 532
HPLC (Methode 5): Retentionszeit = 1.40 min

### Beispiel 45

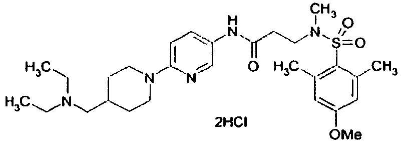

### 45a)

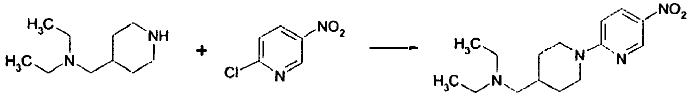

45a wird analog zu 44a aus 1.00 g (5.87 mMol) Diethyl-piperidin-4-ylmethyl-amin (Chem. Pharm. Bull. 42, 1994, 74-84), 0.93 g (5.87 mMol) 2-Chlor-5-nitropyridin (Fluka) und 1.70 g (12.30 mMol) Kaliumcarbonat in 100 ml THF hergestellt.
C₁₅H₂₄N₄O₂ (292.38)
[M+H]+ = 293

### 45b)

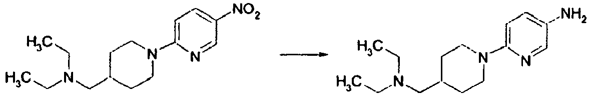

Eine Mischung aus 0.20 g (0.68 mMol) Produkt aus 45a, 0.03 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
C₁₅H₂₆N₄ (262.39)
[M+H]+ = 263

### 45c)

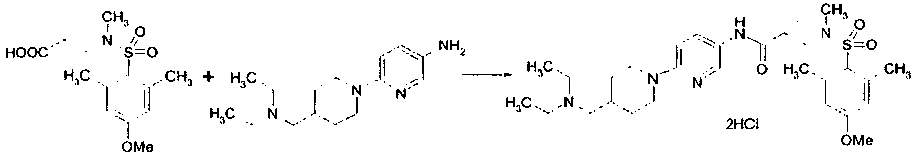

Beispiel 45 wird analog zu 1f aus 0.16 g (0.53 mMol) Produkt aus 22c, 0.14 g (0.53 mMol) Produkt aus 45b, 0.096 ml (0.69 mMol) Triethylamin und 0.19 g (0.58 mMol) TBTU in 40 ml THF und 5 ml DMF hergestellt.
C₂₈H₄₃N₅O₄S × 2HCl (618.66)
[M+H]+ = 546
HPLC (Methode 5): Retentionszeit = 1.40 min

### Beispiel 46

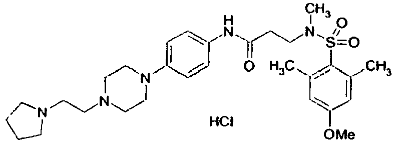

### 46a)

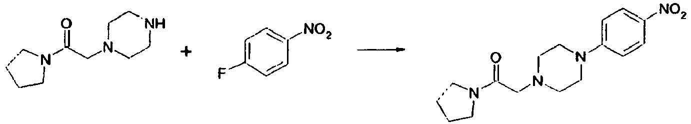

46a wird analog zu 1d aus 3.00 g (15.21 mMol) 2-Piperazin-1-yl-1-pyrrolidin-1-yl-ethanon (Chess), 2.15 g (15.21 mMol) 1-Fluor-4-nitrobenzol (Aldrich) und 3.07 ml (22.00 mMol) Triethylamin in 25 ml DMF hergestellt.
C₁₆H₂₂N₄O₃ (318.37)
[M+H]+ = 319
DC: Kieselgel, Dichlormethan / Ethanol 9:1, Rf-Wert = 0.4

### 46b)

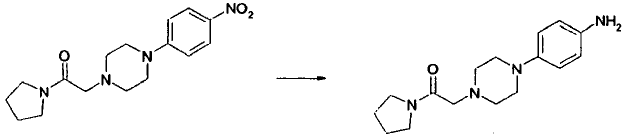

Eine Mischung aus 3.00 g (9.42 mMol) Produkt aus 46a, 0.30 g Palladium auf Kohle (10%) und 200 ml Methanol wird bei Raumtemperatur im Autoklaven hydriert. Der Katalysator wird danach abfiltriert, das Filtrat im Vakuum bis zur Trockene eingeengt.
C₁₆H₂₄N₄O (288.39)
DC: Kieselgel, Dichlormethan / Ethanol 9:1, Rf-Wert = 0.42

### 46c)

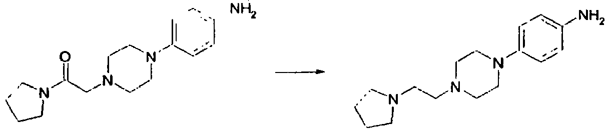

30.00 ml (30.00 mMol) Lithiumaluminiumhydrid 1 M in THF (Aldrich) wird in 50 ml THF vorgelegt und bei Raumtemperatur mit einer Mischung aus 2.70 g (9.36 mMol) Produkt aus 46b und 20 ml THF versetzt. Die Reaktionsmischung wird danach drei Stunden bei Raumtemperatur gerührt und anschließend unter Eisbadkühlung mit 20%iger Natronlauge versetzt. Der entstandene Niederschlag wird abfiltriert, das Filtrat im Vakuum bis zur Trockene eingeengt.
C₁₆H₂₆N₄ (274.40)

### 46d)

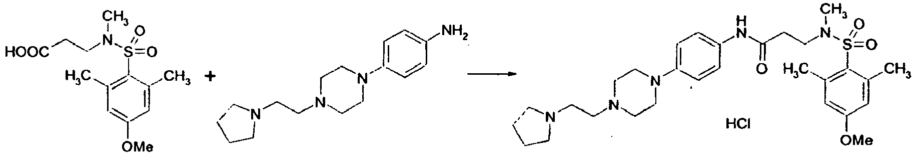

Beispiel 46 wird analog zu 1f aus 0.30 g (1.00 mMol) Produkt aus 22c, 0.27 g (1.00 mMol) Produkt aus 46c, 0.35 ml (2.50 mMol) Triethylamin und 0.32 g (1.00 mMol) TBTU in 7 ml THF und 1 ml DMF hergestellt.
C₂₉H₄₃N₅O₄S × HCl (594.21)
[M+H]+ = 558
HPLC (Methode 5): Retentionszeit = 1.43 min

### Beispiel 47

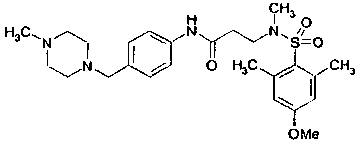

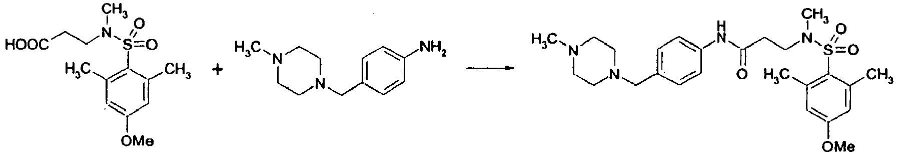

Beispiel 47 wird analog zu 1f aus 0.20 g (0.66 mMol) Produkt aus 22c, 0.14 g (0.66 mMol) 4-(4-Methyl-piperazin-1-ylmethyl)-phenylamin (Med. Chem. Res. 9, 1999, 149-161), 0.28 ml (1.99 mMol) Triethylamin und 0.21 g (0.66 mMol) TBTU in 5 ml THF hergestellt.
C₂₅H₃₆N₄O₄S (488.64)
[M+H]+ = 489
HPLC (Methode 5): Retentionszeit = 1.42 min

### Beispiel 48

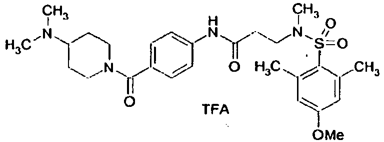

### 48a)

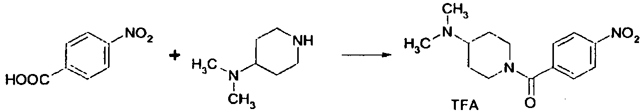

48a wird analog zu 1f aus 0.24 g (1.45 mMol) 4-Nitrobenzoesäure (Aldrich), 0.19 g (1.45 mMol) 4-Dimethylamino-piperidin (Alfa Aesar), 0.21 ml (1.52 mMol) Triethylamin und 0.49 g (1.52 mMol) TBTU in 8 ml DMF hergestellt.
C₁₄H₁₉N₃O₃ × C₂HF₃O₂ (391.34)
[M+H]+ = 278
HPLC (Methode 2): Retentionszeit = 2.29 min

### 48b)

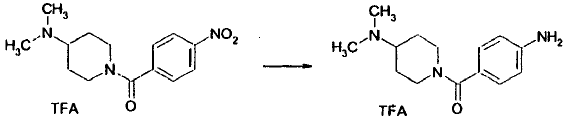

Eine Mischung aus 0.36 g (0.92 mMol) Produkt aus 48a, 0.092 g Palladium auf Kohle (10%) und 5 ml Methanol wird bei Raumtemperatur im Autoklaven hydriert. Der Katalysator wird danach abfiltriert, das Filtrat im Vakuum bis zur Trockene eingeengt.
C₁₄H₂₁N₃O x C₂HF₃O₂ (361.36)
[M+H]+ = 248
HPLC (Methode 2): Retentionszeit = 0.66 min

### 48c)

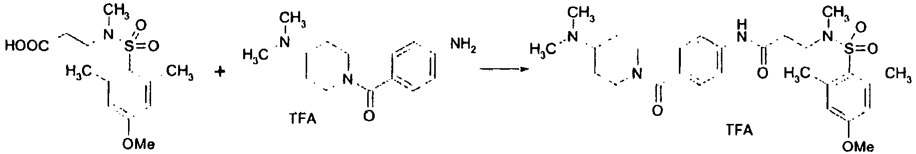

Beispiel 48 wird analog zu 1f aus 0.15 g (0.50 mMol) Produkt aus 22c, 0.18 g (0.50 mMol) Produkt aus 48b, 0.21 ml (1.49 mMol) Triethylamin und 0.16 g (0.50 mMol) TBTU in 3 ml THF hergestellt.
C₂₇H₃₈N₄O₅S x C₂HF₃O₂ (644.70)
[M+H]+ = 531
HPLC (Methode 5): Retentionszeit = 1.48 min

### Beispiel 49

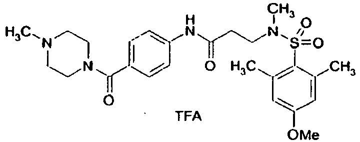

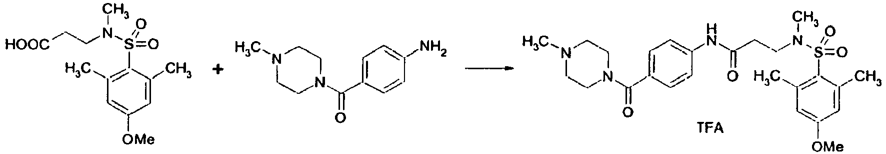

Beispiel 49 wird analog zu 1f aus 0.15 g (0.50 mMol) Produkt aus 22c, 0.11 g (0.50 mMol) (4-Aminophenyl)-(4-methylpiperazin-1-yl)-methanon (J. Org. Chem. 24, 1959, 459-463), 0.21 ml (1.49 mMol) Triethylamin und 0.16 g (0.50 mMol) TBTU in 3 ml THF hergestellt.
C₂₅H₃₄N₄O₅S x C₂HF₃O₂ (616.65)
[M+H]+ = 503
HPLC (Methode 5): Retentionszeit = 1.47 min

### Beispiel 50

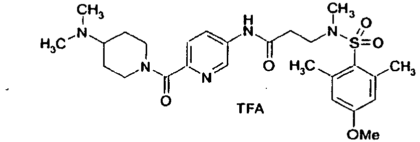

### 50a)

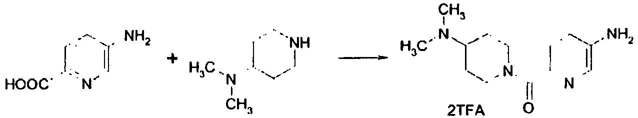

50a wird analog zu 1f aus 0.60 g (4.34 mMol) 5-Amino-pyridin-2-carbonsäure (Pharm. Acta Helv. 44, 1969, 637-643), 0.56 g (4.34 mMol) 4-Dimethylamino-piperidin (Alfa Aesar), 0.64 ml (4.56 mMol) Triethylamin und 1.46 g (4.56 mMol) TBTU in 24 ml DMF hergestellt.
C₁₃H₂₀N₄O x 2C₂HF₃O₂ (476.37)
HPLC (Methode 2): Retentionszeit = 0.65 min

### 50b)

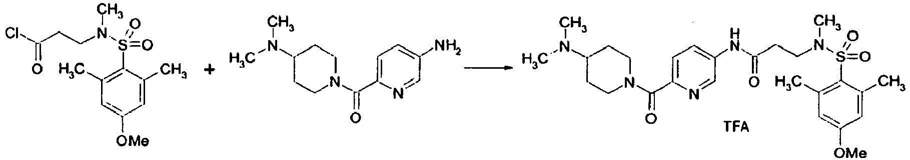

Eine Mischung aus 0.64 g (1.99 mMol) Produkt aus 27c, 1.90 g (2.39 mMol) Produkt aus 50a, 0.08 g (0.33 mMol) DMAP und 16 ml Chlorbenzol wird 39 Stunden auf 15°C erhitzt. Die Reaktionsmischung wird danach im Vakuum bis zur Trockene eingeengt. Das Produkt wird durch präparative HPLC erhalten.
C₂₆H₃₇N₅O₅S x C₂HF₃O₂ (645.69)
[M+H]+ = 532
HPLC (Methode 5): Retentionszeit = 1.44 min

### Beispiel 51

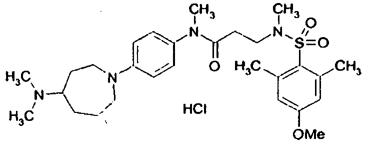

### 51a)

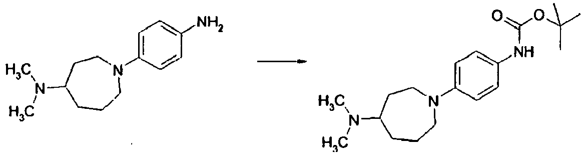

Eine Mischung aus 0.26 g (1.11 mMol) Produkt aus 39d, 0.27 g (1.22 mMol) Boc-Anhydrid, 0.17 ml (1.22 mMol) Triethylamin und 15 ml Dichlormethan wird über Nacht bei Raumtemperatur gerührt. Danach wird die Reaktionsmischung mit Dichlormethan verdünnt, mit gesättigter Natriumhydrogencarbonat-Lösung und gesättigter Natriumchlorid-Lösung gewaschen, über Natriumsulfat getrocknet und im Vakuum bis zur Trockene eingeengt.
C₁₉h₃₁N₃O₂ (333.47)
[M+H]+ = 334
HPLC (Methode 1): Retentionszeit = 2.40 min

### 51b)

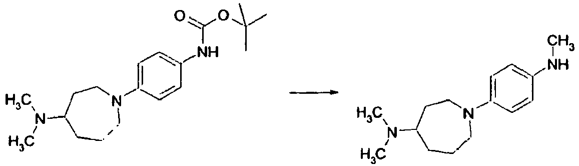

0.13 g (3.40 mMol) Lithiumaluminiumhydrid werden in 5 ml THF vorgelegt, auf 60°C erhitzt und mit 0.38 g (1.14 mMol) Produkt aus 51 a in 5 ml THF versetzt. Die Reaktionsmischung wird danach vier Stunden unter Rückfluß erhitzt und über Nacht bei Raumtemperatur gerührt. Anschließend wird die Reaktionsmischung mit Wasser und 1 M Natronlauge gequencht. Der entstandene Niederschlag wird über Celite abfiltriert, das Filtrat im Vakuum bis zur Trockene eingeengt.
C₁₅H₂₅N₃ (247.38)
DC: Kieselgel, Dichlormethan / Methanol / Ammoniak 4:1:0.2, Rf-Wert = 0.68

### 51c)

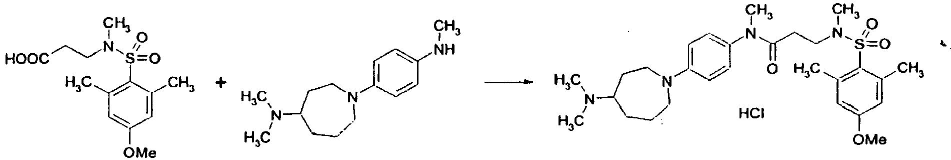

Beispiel 51 wird analog zu 1f aus 0.15 g (0.50 mMol) Produkt aus 22c, 0.12 g (0.50 mMol) Produkt aus 51 b, 0.21 ml (1.50 mMol) Triethylamin und 0.18 g (0.55 mMol) TBTU in 8 ml DMF hergestellt.
C₂₈H₄₂N₄O₄S × HCl (567.18)
[M+H]+ = 531
HPLC (Methode 1): Retentionszeit = 2.5 min

### Beispiel 52

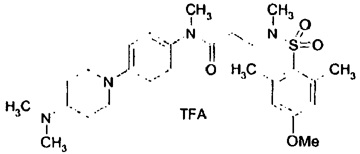

### 52a)

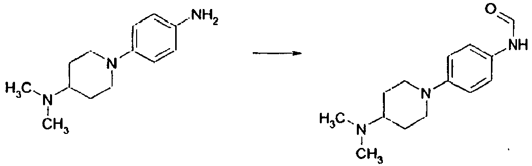

0.70 ml (7.36 mMol) Essigsäureanhydrid werden unter Stickstoff-Atmosphäre vorgelegt und unter Eisbadkühlung langsam mit 0.42 ml (9.06 mMol) Ameisensäure versetzt. Die Reaktionsmischung wird zwei Stunden auf 50-60°C erhitzt und anschließend unter Eisbadkühlung mit 0.50 g (2.28 mMol) Produkt aus 8b in 7 ml Dichlormethan versetzt. Nach 20-minütigem Rühren bei Raumtemperatur wird die Reaktionsmischung im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
C₁₄H₂₁N₃O (247.34)
[M+H]+ = 248
HPLC (Methode 5): Retentionszeit = 0.50 min

### 52b)

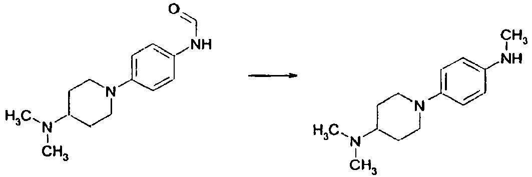

52b wird analog zu 51b aus 0.17 g (4.51mMol) Lithiumaluminiumhydrid und 0.58 g (2.34 mMol) Produkt aus 52a in 10 ml THF hergestellt.
C₁₄H₂₃N₃ (233.35)
DC: Kieselgel, Dichlormethan / Methanol / Ammoniak 9:1:0.1, Rf-Wert = 0.5

### 52c)

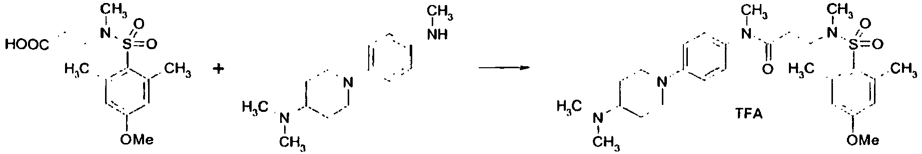

Beispiel 52 wird analog zu 1f aus 0.21 g (0.68 mMol) Produkt aus 22c, 0.24 g (0.68 mMol) Produkt aus 52b, 0.28 ml (2.04 mMol) Triethylamin und 0.22 g (0.68 mMol) TBTU in 4 ml THF hergestellt.
C₂₇H₄₀N₄O₄S × C₂HF₃O₂ (630.72)
[M+H]+=517
HPLC (Methode 5): Retentionszeit = 1.50 min

### Beispiel 83

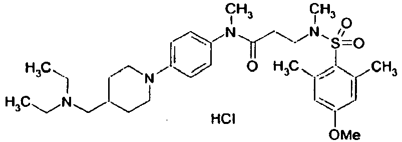

### 83a)

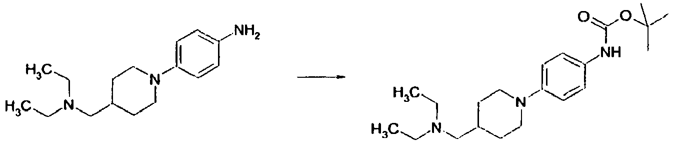

83a wird analog zu 51 a aus 1.35 g (5.16 mMol) Produkt aus 41 b, 1.24 g (5.68 mMol) Boc-Anhydrid und 0.80 ml (5.68 mMol) Triethylamin in 50 ml Dichlormethan hergestellt.
C₂₁H₃₅N₃O₂ (361.52)
[M+H]+ = 362
DC: Kieselgel, Dichlormethan / Methanol / Ammoniak 9:1:0.1, Rf-Wert = 0.42

### 83b)

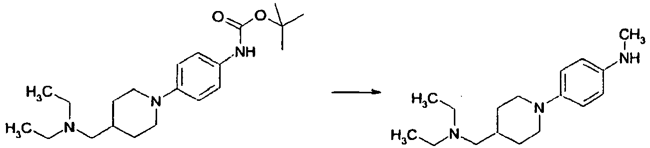

83b wird analog zu 51 b aus 1.80 g (4.98 mMol) Produkt aus 83a und 0.57 g (15.00 mMol) Lithiumaluminiumhydrid (Aldrich) in 25 ml THF hergestellt.
C₁₇H₂₉N₃ (275.43)
[M+H]+ = 276
HPLC (Methode 1): Retentionszeit = 1.77 min

### 83c)

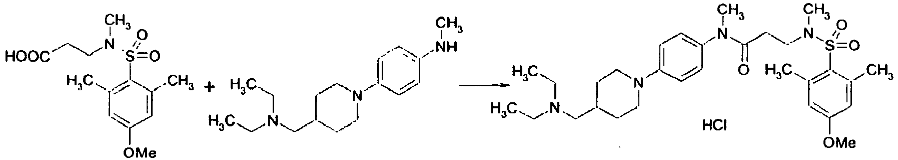

Beispiel 83 wird analog zu 1f aus 0.14 g (0.50 mMol) Produkt aus 22c, 0.15 g (0.50 mMol) Produkt aus 83b, 0.14 ml (1.00 mMol) Triethylamin und 0.18 g (0.55 mMol) TBTU in 6 ml DMF hergestellt.
C₃₀H₄₆N₄₀O₄S × HCl (595.24)
[M+H]+ = 559
HPLC (Methode 1): Retentionszeit = 2.46 min

### Beispiel 84

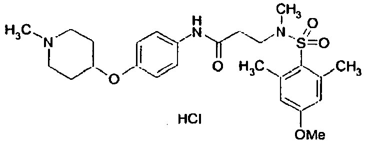

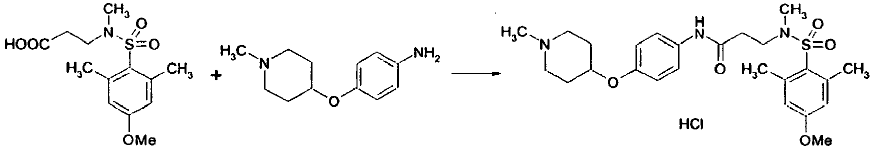

Beispiel 84 wird analog zu 1f aus 0.15 g (0.50 mMol) Produkt aus 22c, 0.10 g (0.50 mMol) 4-(1-Methylpiperidin-4-yloxy)-phenylamin (ART-CHEM), 0.14 ml (1.00 mMol) Triethylamin und 0.18 g (0.55 mMol) TBTU in 6 ml DMF hergestellt.
C₂₅H₃₅N₃O₅S × HCl (526.09)
[M+H]+ = 490
HPLC (Methode 1): Retentionszeit = 2.40 min

### Beispiel 85

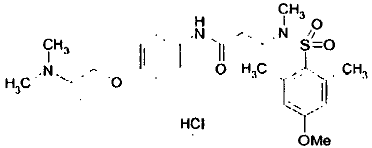

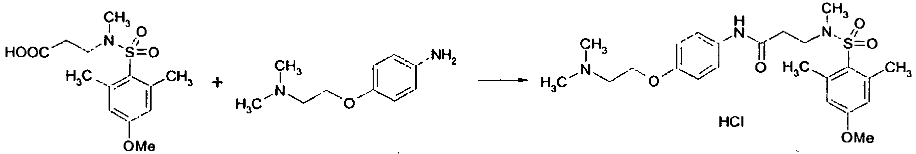

Beispiel 85 wird analog zu 1f aus 0.15 g (0.50 mMol) Produkt aus 22c, 0.09 g (0.50 mMol) 4-(2-Dimethylamino-ethoxy)-phenylamin (Collect. Czech. Chem. Commun. 55, 1990, 282-295), 0.14 ml (1.00 mMol) Triethylamin und 0.18 g (0.55 mMol) TBTU in 6 ml DMF hergestellt.
C₂₃H₃₃N₃O₅S × HCl (500.05)
[M+H]+ = 464
HPLC (Methode 1): Retentionszeit = 2.35 min

### Beispiel 86

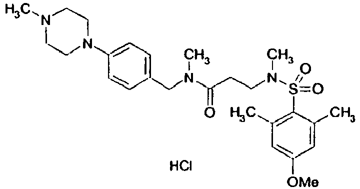

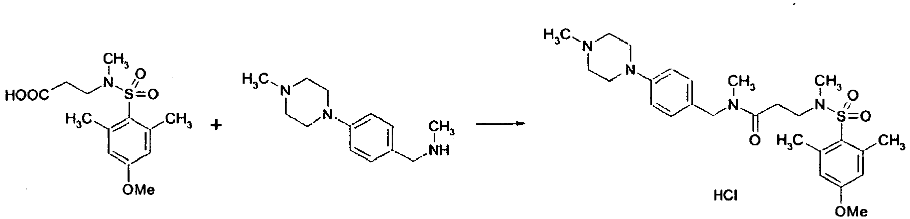

Beispiel 86 wird analog zu 1f aus 0.14 g (0.45 mMol) Produkt aus 22c, 0.099 g (0.45 mMol) Produkt aus 81a, 0.13 ml (0.90 mMol) Triethylamin und 0.17 g (0.54 mMol) TBTU in 5 ml DMF hergestellt.
C₂₆H₃₈N₄O₅S × HCl (539.13)
[M+H]+ = 503
HPLC (Methode 1): Retentionszeit = 2.43 min

### Beispiel 87

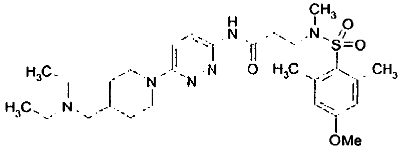

### 87a)

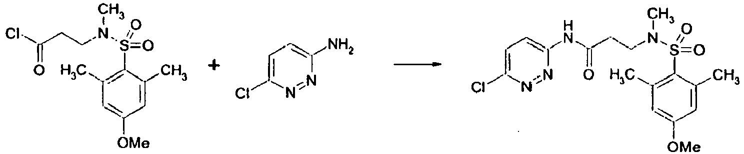

Eine Mischung aus 0.11 g (0.33 mMol) Produkt aus 27c, 0.043 g (0.33 mMol) 3-Amino-6-chlorpyridazin (Acros), 0.12 ml (0.66 mMol) DIPEA und 10 ml Dichlormethan wird drei Tage unter Rückfluss gerührt. Der Niederschlag wird danach abfiltriert. Das Filtrat wird im Vakuum bis zur Trockene eingeengt und das Rohpodukt durch präparative HPLC gereinigt.
C₁₇H₂₁CIN₄O₄S (412.89)
[M+H]+ = 413/415

### 87b)

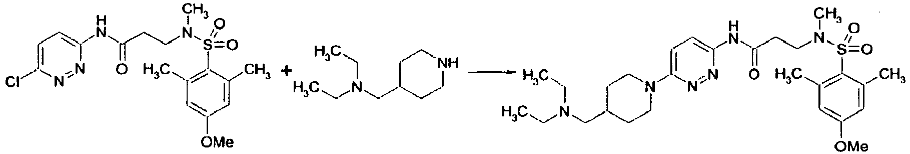

Eine Mischung aus 0.03 g (0.073 mMol) Produkt aus 87a und 0.013 g (0.073 mMol) Diethyl-piperidin-4-ylmethyl-amin (Chem. Pharm. Bull. 42, 1994, 74-84) wird bei 173°C geschmolzen. Das Produkt wird danach durch präparative HPLC aus der Reaktionsmischung gewonnen.
C₂₇H₄₂N₆O₄S (546.73)
[M+H]+ = 547
HPLC (Methode 6): Retentionszeit = 2.12 min

### Beispiel 88

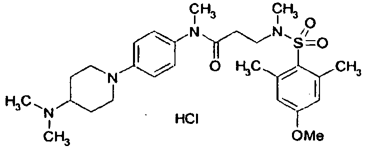

### 88a)

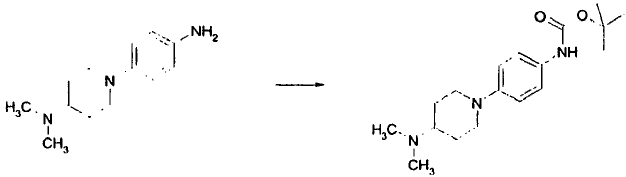

88a wird analog zu 51a aus 0.70 g (3.18 mMol) Produkt aus 27b, 0.80 g (3.65 mMol) Boc-Anhydrid und 5.50 ml (11.00 mMol) 2 M Natronlauge in 40 ml Dioxan und 20 ml Wasser hergestellt.
C₁₇H₂₈N₄O₂ (320.43)
[M+H]+ = 321
DC: Kieselgel, Dichlormethan / Methanol / Ammoniak 4:1:0.2, Rf-Wert = 0.83

### 88b)

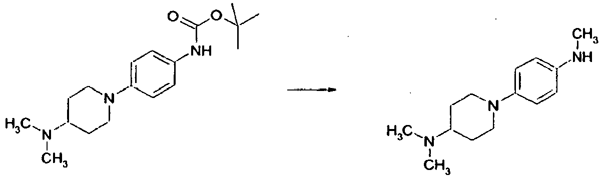

88b wird analog zu 51 b aus 0.60 g (1.87 mMol) Produkt aus 88a und 0.21 g (5.60 mMol) Lithiumaluminiumhydrid (Aldrich) in 20 ml THF hergestellt.
C₁₃H₂₂N₄ (234.34)
[M+H]+ = 235
DC: Kieselgel, Dichlormethan / Methanol / Ammoniak 4:1:0.2, Rf-Wert = 0.62

### 88c)

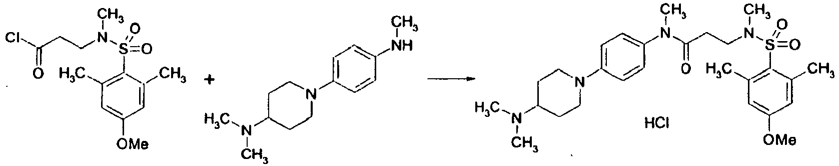

Beispiel 88 wird analog zu 27d aus 0.16 g (0.50 mMol) Produkt aus 27c, 0.12 g (0.50 mMol) Produkt aus 88b und 0.17 ml (1.00 mMol) DIPEA in 5 ml THF hergestellt.
C₂₆H₃₉N₅O₄S × HCl (554.15)
[M+H]+ = 518
DC: Kieselgel, Dichlormethan / Methanol / Ammoniak 9:1:0.1, Rf-Wert = 0.61

### Beispiel 89

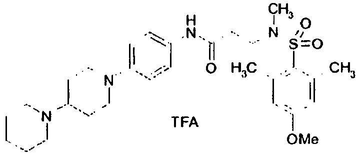

### 89a)

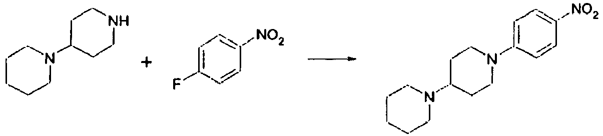

89a wird analog zu 8a aus 0.70 g (4.18 mMol) 4-Piperidinopiperidin (Aldrich), 0.44 ml (4.18 mMol) 1-Fluor-4-nitrobenzol (Acros) und 1.33 ml (9.61 mMol) Triethylamin in 12 ml DMF hergestellt.
C₁₆H₂₃N₃O₂ (289.37)
[M+H]+ = 290
DC: Kieselgel, Dichlormethan / Methanol / Ammoniak 9:1:0.1, Rf-Wert = 0.3

### 89b)

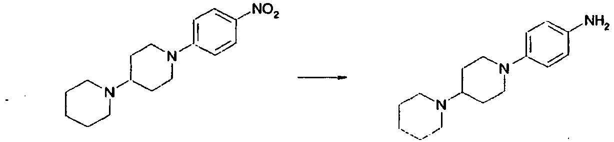

89b wird analog zu 8b aus 0.96 g (3.32 mMol) Produkt aus 89a und 0.093 g Palladium auf Kohle (5%) in 45 ml Ethanol hergestellt.
C₁₆H₂₅N₃ (259.39)
[M+H]+ = 260
DC: Kieselgel, Dichlormethan / Methanol / Ammoniak 9:1:0.1, Rf-Wert = 0.2

### 89c)

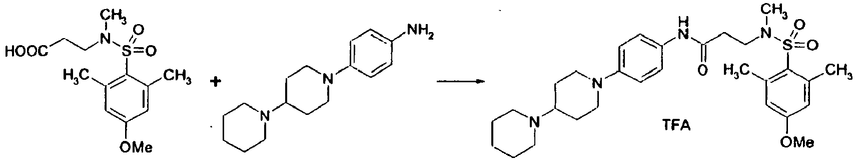

Beispiel 89 wird analog zu 1f aus 0.10 g (0.33 mMol) Produkt aus 22c, 0.086 g (0.33 mMol) Produkt aus 89b, 0.14 ml (1.00 mMol) Triethylamin und 0.11 g (0.33 mMol) TBTU in 2 ml THF hergestellt.
C₂₉H₄₂N₄O₄S x C₂HF₃O₂ (656.76)
[M+H]+ = 543
HPLC (Methode 5): Retentionszeit = 1.47 min

### Beispiel 90

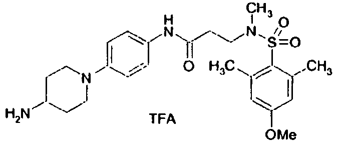

### 90a)

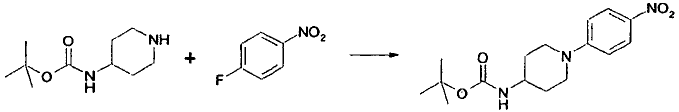

90a wird analog zu 8a aus 0.84 g (4.18 mMol) 4-N-Boc-Aminopiperidin (Acros), 0.44 ml (4.18 mMol) 1-Fluor-4-nitrobenzol (Acros) und 1.33 ml (9.61 mMol) Triethylamin in 12 ml DMF hergestellt.
C₁₆H₂₃N₃O₄ (321.37)
[M+H]+ = 322

### 90b)

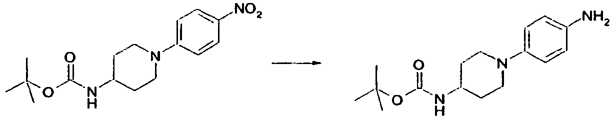

90b wird analog zu 8b aus 1.01 g (3.43 mMol) Produkt aus 90a und 0.11 g Palladium auf Kohle (5%) in 45 ml Ethanol hergestellt.
C₁₆H₂₅N₃O₂ (291.39)
[M+H]+ = 292

### 90c)

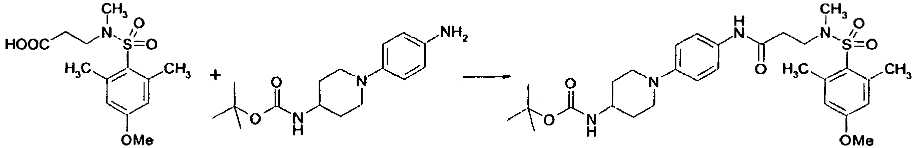

90c wird analog zu 1f aus 0.10 g (0.33 mMol) Produkt aus 22c, 0.097 g (0.33 mMol) Produkt aus 90b, 0.14ml (1.00 mMol) Triethylamin und 0.11 g (0.33 mMol) TBTU in 2 ml THF hergestellt.
C₂₉H₄₂N₄O₆S (574.73)
[M+H]+ = 575
HPLC (Methode 5): Retentionszeit = 1.62 min

### 90d)

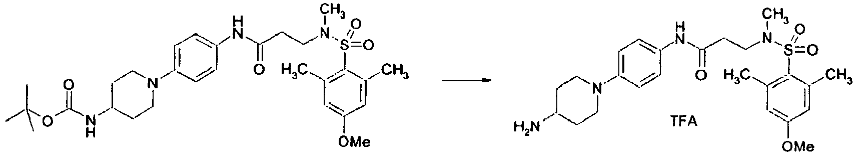

Beispiel 90 wird analog zu 18b aus 0.19 g (0.33 mMol) Produkt aus 90c und 0.33 ml TFA in 0.5 ml Dichlormethan hergestellt.
C₂₄H₃₄N₄O₄S × C₂HF₃O₂ (588.64)
[M+H]+ = 475
HPLC (Methode 5): Retentionszeit = 1.41 min

### Beispiel 91

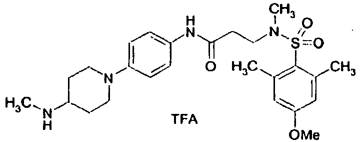

### 91a)

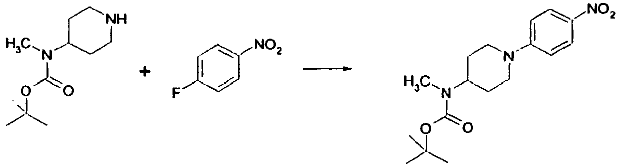

91a wird analog zu 8a aus 0.90 g (4.18 mMol) Methyl-piperidin-4-yl-carbamatsäure-tert-butylester (Fluorochem), 0.44 ml (4.18 mMol) 1-Fluor-4-nitrobenzol (Acros) und 1.33 ml (9.61 mMol) Triethylamin in 12 ml DMF hergestellt.
C₁₇H₂₅N₃O₄ (335.40)
[M+H]+ = 336
DC: Kieselgel, Dichlormethan / Methanol 30:1, Rf-Wert = 0.6

### 91b)

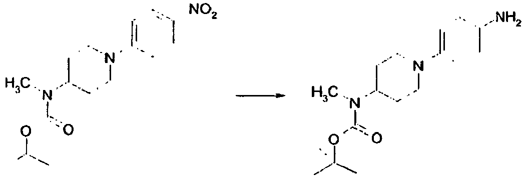

91b wird analog zu 8b aus 1.08 g (3.22 mMol) Produkt aus 91a und 0.11 g Palladium auf Kohle (5%) in 45 ml Ethanol hergestellt.
C₁₇H₂₇N₃O₂ (305.42)
[M+H]+ = 306
DC: Kieselgel, Dichlormethan / Methanol 30:1, Rf-Wert = 0.4

### 91c)

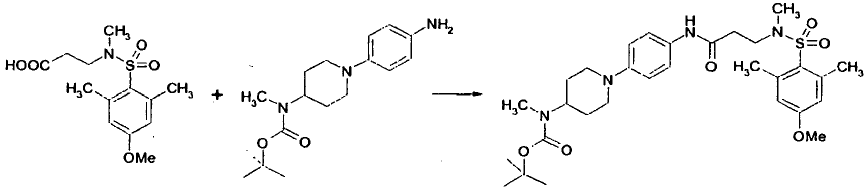

91c wird analog zu 1f aus 0.10 g (0.33 mMol) Produkt aus 22c, 0.10 g (0.33 mMol) Produkt aus 91b, 0.14 ml (1.00 mMol) Triethylamin und 0.11 g (0.33 mMol) TBTU in 2 ml THF hergestellt.
C₃₀H₄₄N₄O₆S (588.76)
[M+H]+ = 589
HPLC (Methode 5): Retentionszeit = 1.69 min

### 91d)

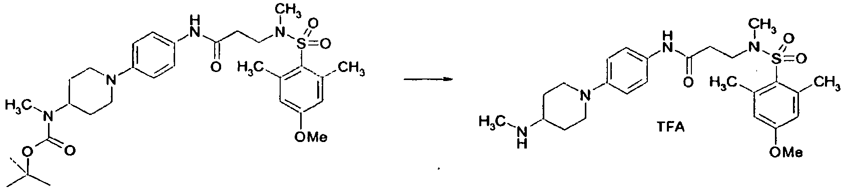

Beispiel 91 wird analog zu 18b aus 0.21 g (0.36 mMol) Produkt aus 91c und 0.36 ml TFA in 0.6 ml Dichlormethan hergestellt.
C₂₅H₃₆N₄O₄S x C₂HF₃O₂ (602.67)
[M+H]+ = 489
HPLC (Methode 5): Retentionszeit = 1.42 min

### Beispiel 92

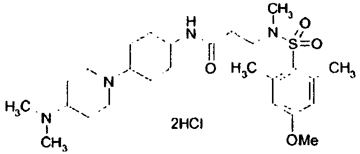

### 92a)

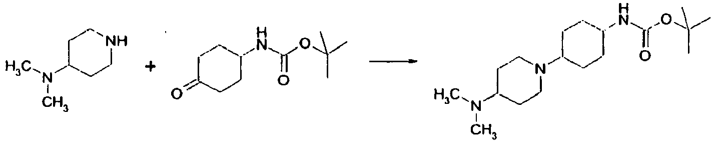

Eine Mischung aus 0.66 g (5.16 mMol) 4-Dimethylamino-piperidin (Alfa Aesar), 1.00 g (4.69 mMol) (4-Oxocyclohexyl)-carbamatsäure-tert-butylester (Fluorochem), 1.19 g (5.16 mMol) Natriumtriacetoxyborhydrid und 20 ml Dichlormethan wird unter Stickstoff vier Stunden bei Raumtemperatur gerührt. Die Reaktionsmischung wird danach mit Dichlormethan verdünnt, mit gesättigter Natriumhydrogencarbonat-Lösung gewaschen, über Natriumsulfat getrocknet und im Vakuum bis zur Trockene eingeengt.
C₁₈H₃₅N₃O₂ (325.49)

### 92b)

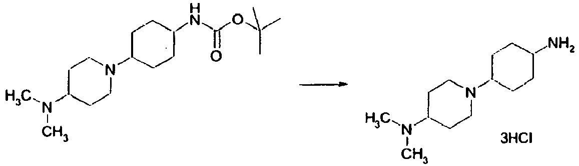

Eine Mischung aus 0.80 g (2.46 mMol) Produkt aus 92a, 4 ml 6 M HCl, 3 ml 37%ige HCl und 3 ml Methanol wird zwei Stunden bei 50°C gerührt. Die Reaktionsmischung wird danach im Vakuum bis zur Trockene eingeengt.
C₁₃H₂₇N₃ x 3HCl (334.76)

### 92c)

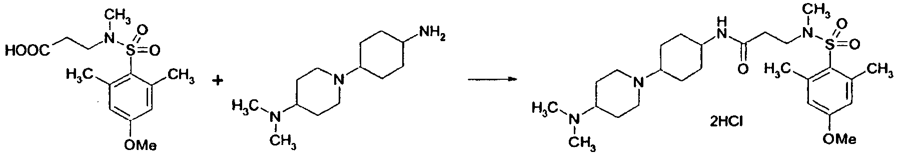

Beispiel 92 wird analog zu 1f aus 0.10 g (0.33 mMol) Produkt aus 22c, 0.13 g (0.40 mMol) Produkt aus 92b, 0.23 ml (1.66 mMol) Triethylamin und 0.13 g (0.40 mMol) TBTU in 8 ml DMF hergestellt.
C₂₆H₄₄N₄O₄S × 2HCl (581.64)
[M+H]+ = 509
HPLC (Methode 5): Retentionszeit = 1.36 min

### Beispiel 93

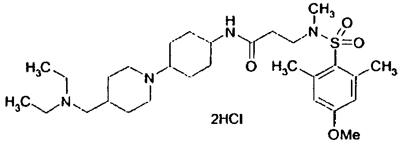

### 93a)

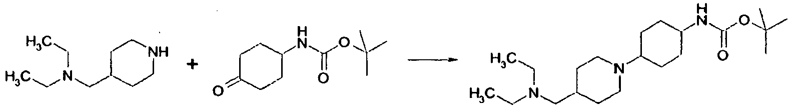

93a wird analog zu 92a aus 0.63 g (3.70 mMol) Diethyl-piperidin-4-ylmethyl-amin (Chem. Pharm. Bull. 42, 1994, 74-84), 0.72 g (3.37 mMol) (4-Oxocyclohexyl)-carbamatsäure-tert-butylester (Fluorochem) und 0.86 g (4.04 mMol) Natriumtriacetoxyborhydrid in 20 ml Dichlormethan durchgeführt.
C₂₁H₄₁N₃O₂ (367.57)

### 93b)

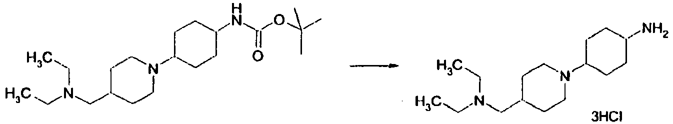

93b wird analog zu 92b aus 0.90 g (2.45 mMol) Produkt aus 93a, 4 ml 6 M HCl und 3 ml 37%ige HCl in 3 ml Methanol hergestellt.
C₁₆H₃₃N₃ × 3HCl (376.84)

### 93c)

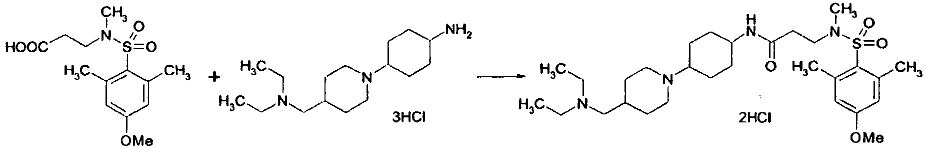

Beispiel 93 wird analog zu 1f aus 0.10 g (0.33 mMol) Produkt aus 22c, 0.14 g (0.37 mMol) Produkt aus 93b, 0.23 ml (1.66 mMol) Triethylamin und 0.13 g (0.40 mMol) TBTU in 8 ml DMF hergestellt.
C₂₉H₅₀N₄O₄S × 2HCl (623.72)
[M+H]+ = 551
HPLC (Methode 5): Retentionszeit = 1.39 min

### Beispiel 94

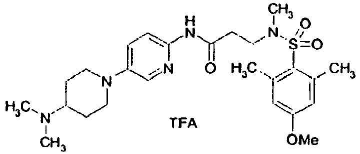

94a)

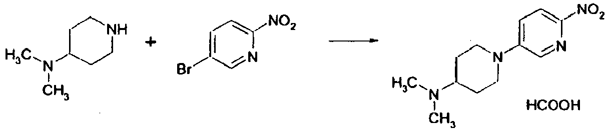

Eine Mischung aus 0.88 g (6.88 mMol) 4-Dimethylamino-piperidin (Alfa Aesar), 1.00 g (4.93 mMol) 3-Brom-6-nitropyriridin (Aldrich), 0.18 g (0.49 mMol) Tetrabutyl-ammoniumiodid, 0.74 g (5.33 mMol) Kaliumcarbonat und 5 ml DMSO wird zwei Stunden bei 80°C gerührt. Danach wird die Reaktionsmischung auf Wasser gegossen und mit Dichlormethan extrahiert. Die organischen Extrakte werden mit gesättigter Natriumhydrogencarbonat-Lösung und gesättigter Natriumchlorid-Lösung gewaschen, über Natriumsulfat getrocknet und im Vakuum bis zur Trockene eingeengt. Das so erhaltene Rohprodukt wird durch präparative HPLC gereinigt.
C₁₂H₁₈N₄O₂ x CH₂O₂ (296.32)
HPLC (Methode 1): Retentionszeit = 1.49 min

### 94b)

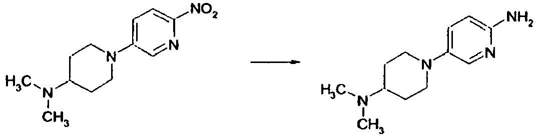

94b wird analog zu 8b aus 0.50 g (2.00 mMol) Produkt aus 94a und 0.08 g Palladium auf Kohle (10%) in 40 ml Methanol hergestellt.
C₁₂H₂₀N₄ (220.31)

### 94c)

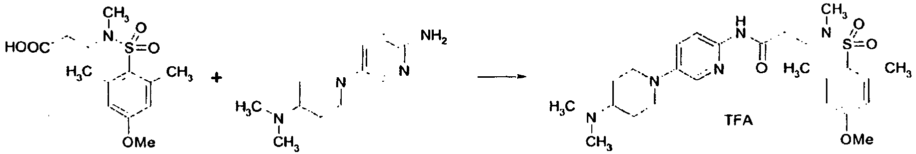

Eine Mischung aus 0.63 g (2.10 mMol) Produkt aus 22c, 0.91 g (9.00 mMol) N-Methylmorpholin, 0.45 g (2.04 mMol) Produkt aus 94b und 50 ml THF wird 10 Minuten bei Raumtemperatur gerührt und danach mit 5.22 ml (9.00 mMol) Propylphosphonsäureanhydrid 50% in Ethylacetat (Fluka) versetzt. Man lässt die Reaktionsmischung über Nacht bei Raumtemperatur rühren und engt sie anschließend im Vakuum bis zur Trockene ein. Der Rückstand wird mit 2 N Kaliumcarbonat-Lösung versetzt und mit Dichlormethan extrahiert. Die organischen Extrakte werden mit Wasser und gesättigter Natriumchlorid-Lösung gewaschen, über Natriumsulfat getrocknet und im Vakuum bis zur Trockene eingeengt. Das so erhaltene Rohprodukt wird durch präparative HPLC gereinigt.
C₂₅H₃₇N₅O₄S x C₂HF₃O₂ (617.68)
[M+H]+ = 504
HPLC (Methode 5): Retentionszeit = 1.38 min

### Beispiel 95

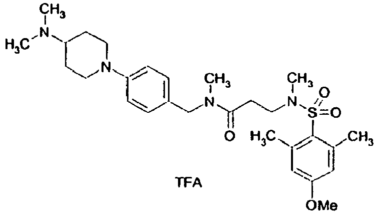

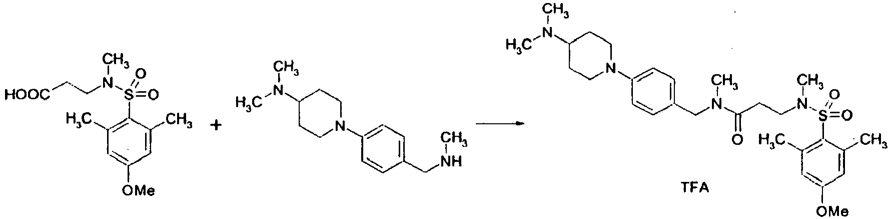

Beispiel 95 wird analog zu 1f aus 0.08 g (0.27 mMol) Produkt aus 22c, 0.066 g (0.27 mMol) Produkt aus 80a, 0.11 ml (0.80 mMol) Triethylamin und 0.085 g (0.27 mMol) TBTU in 2 ml THF hergestellt.
C₂₈H₄₂N₄O₄S × C₂HF₃O₂ (644.75)
[M+H]+ = 531
HPLC (Methode 5): Retentionszeit = 1.50 min

### Beispiel 96

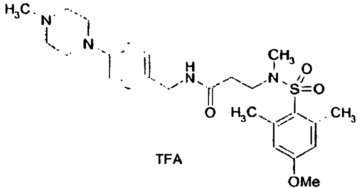

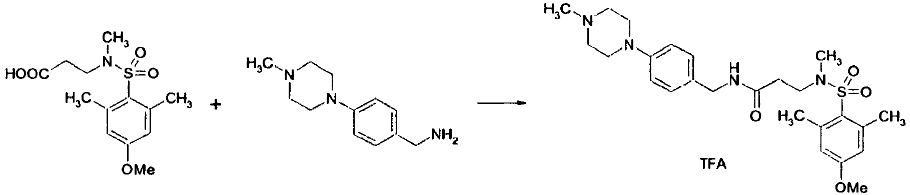

Beispiel 96 wird analog zu 1f aus 0.08 g (0.27 mMol) Produkt aus 22c, 0.054 g (0.27 mMol) Produkt aus 19b, 0.11 ml (0.80 mMol) Triethylamin und 0.085 g (0.27 mMol) TBTU in 2 ml THF hergestellt.
C₂₅H₃₆N₄O₄S x C₂HF₃O₂ (602.67)
[M+H]+ = 489
HPLC (Methode 5): Retentionszeit = 1.49 min

### Beispiel 97

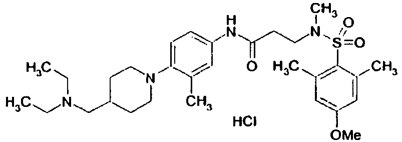

### 97a)

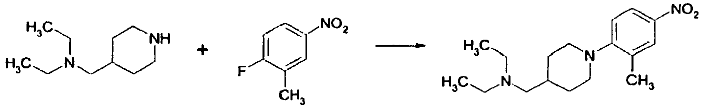

97a wird analog zu 8a aus 1.00 g (5.87 mMol) Diethyl-piperidin-4-ylmethyl-amin (Chem. Pharm. Bull. 42, 1994, 74-84), 0.91 g (5.87 mMol) 1-Fluor-2-methyl-4-nitrobenzol (ABCR) und 1.14 ml (8.20 mMol) Triethylamin in 12 ml DMF hergestellt.
C₁₇H₂₇N₃O₂ (305.42)
[M+H]+ = 306

### 97b)

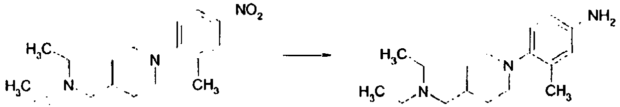

97b wird analog zu 8b aus 0.91 g (2.98 mMol) Produkt aus 97a und 0.20 g Palladium auf Kohle (10%) in 50 ml Methanol hergestellt.
C₁₇H₂₉N₃ (275.43)

### 97c)

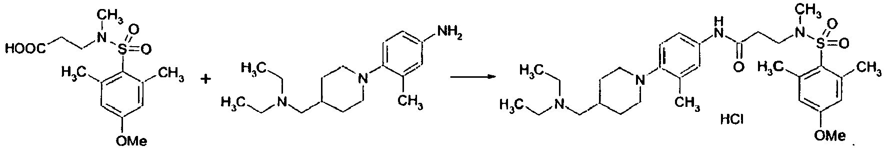

Beispiel 97 wird analog zu 1f aus 0.15 g (0.50 mMol) Produkt aus 22c, 0.14 g (0.50 mMol) Produkt aus 97b, 0.21 ml (1.50 mMol) Triethylamin und 0.16 g (0.50 mMol) TBTU in 7 ml THF und 1 ml DMF hergestellt.
C₃₀H₄₆N₄O₄S × HCl (595.24)
[M+H]+ = 559
HPLC (Methode 5): Retentionszeit = 1.44 min

### Beispiel 98

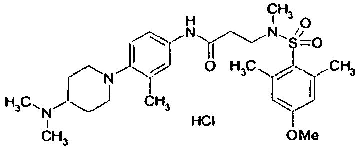

### 98a)

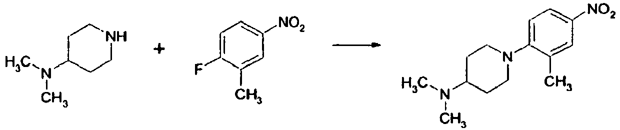

98a wird analog zu 8a aus 0.75 g (5.87 mMol) 4-Dimethylamino-piperidin (Alfar Aesar), 0.91 g (5.87 mMol) 1-Fluor-2-methyl-4-nitrobenzol (ABCR) und 1.14 ml (8.20 mMol) Triethylamin in 12 ml DMF hergestellt.
C₁₄H₂₁N₃O₂ (263.34)

### 98b)

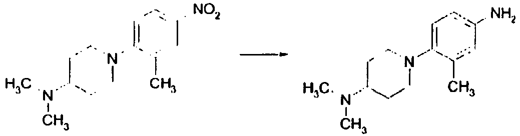

98b wird analog zu 8b aus 0.30 g (1.14 mMol) Produkt aus 98a und 0.10 g Palladium auf Kohle (10%) in 25 ml Methanol hergestellt.
C₁₄H₂₃N₃ (233.35)

### 98c)

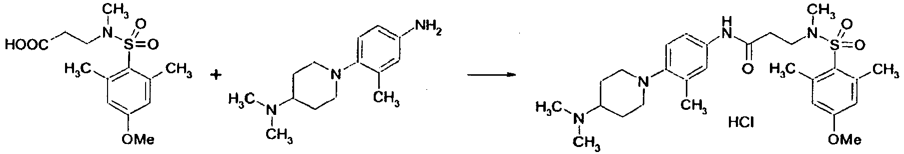

Beispiel 98 wird analog zu 1f aus 0.32 g (1.07 mMol) Produkt aus 22c, 0.25 g (1.07 mMol) Produkt aus 98b, 0.42 ml (3.00 mMol) Triethylamin und 0.34 g (1.07 mMol) TBTU in 7 ml THF und 1 ml DMF hergestellt.
C₂₇H₄₀N₄O₄S × HCl (553.16)
[M+H]+ = 517
HPLC (Methode 5): Retentionszeit = 1.52 min

### Beispiel 99

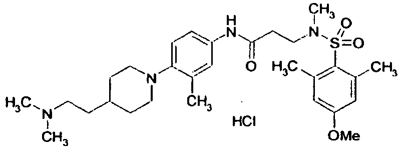

### 99a)

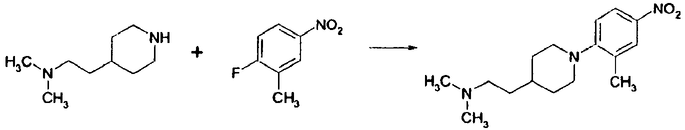

99a wird analog zu 8a aus 0.92 g (5.87 mMol) Dimethyl-(2-piperidin-4-yl-ethyl)-amin (J. Med. Chem. 36, 1993, 162-165), 0.91 g (5.87 mMol) 1-Fluor-2-methyl-4-nitrobenzol (ABCR) und 2.49 g (18.00 mMol) Kaliumcarbonat in 12 ml DMF hergestellt.
C₁₆H₂₅N₃O₂ (291.39)
[M+H]+ = 292

### 99b)

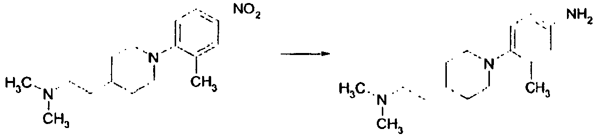

99b wird analog zu 8b aus 0.60 g (1.14 mMol) Produkt aus 99a und 0.20 g Palladium auf Kohle (10%) in 50 ml Methanol hergestellt.
C₁₆H₂₇N₃ (261.41)

### 99c)

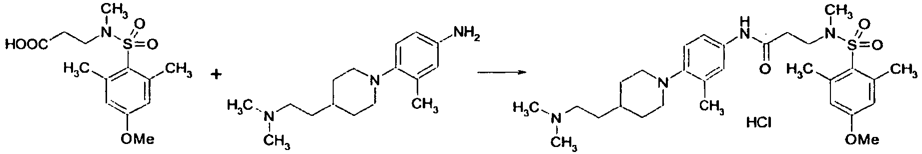

Beispiel 99 wird analog zu 1f aus 0.15 g (0.50 mMol) Produkt aus 22c, 0.13 g (0.50 mMol) Produkt aus 99b, 0.21 ml (1.50 mMol) Triethylamin und 0.16 g (0.50 mMol) TBTU in 7 ml THF und 1 ml DMF hergestellt.
C₂₉H₄₄N₄O₄S × HCl (581.21)
[M+H]+ = 545
HPLC (Methode 5): Retentionszeit = 1.42 min

### Beispiel 100

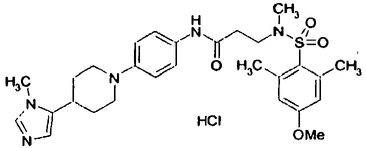

### 100a)

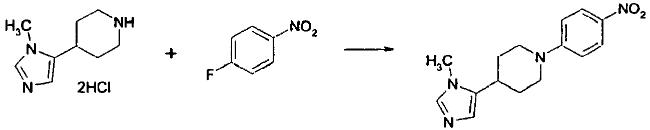

100a wird analog zu 8a aus 1.00 g (4.47 mMol) 4-(3-Methyl-3H-imidazol-4-yl)-piperidin (J. Med. Chem. 46, 2003, 5445-5457), 0.63 g (4.47 mMol) 4-Fluor-nitrobenzol (ABCR) und 2.10 g (15.20 mMol) Kaliumcarbonat in 50 ml DMF hergestellt.
C₁₄H₁₆N₄O₂ (272.30)
[M+H]+ = 273

100b)

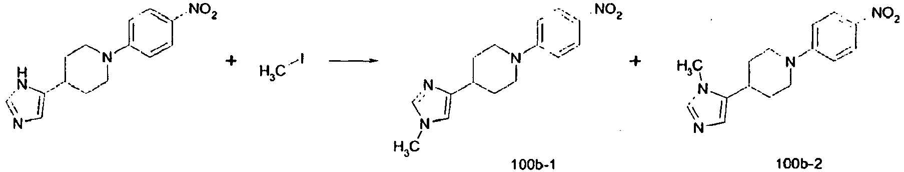

100b-1 und 100b-2 werden analog zu 62a aus 1.10 g (4.04 mMol) Produkt aus 100a, 0.60 g (4.23 mMol) Methyliodid und 0.46 g (4.10 mMol) Kalium-tert-butylat in 50 ml DMSO hergestellt. Das so entstandene Isomerengemisch wird durch Säulen-chromatographie über Kieselgel (Elutionsmittel: Dichlormethan / Methanol 100:1 1 bis 30: 1) aufgetrennt.
1 00b-1: C₁₅H₁₈N₄O₂ (286.33)
   [M+H]+ = 287
   DC: Kieselgel, Dichlormethan / Methanol 9:1, Rf-Wert = 0.50
100b-2: C₁₅H₁₈N₄O₂ (286.33)
   [M+H]+ = 287
   DC: Kieselgel, Dichlormethan / Methanol 9:1, Rf-Wert = 0.38

### 100c)

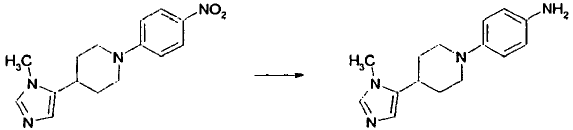

100c wird analog zu 8b aus 0.10 g (0.35 mMol) 100b-2 und 0.20 g Palladium auf Kohle (10%) in 30 ml Methanol hergestellt.
C₁₅H₂₀N₄ (256.35)
DC: Kieselgel, Dichlormethan / Methanol 9:1, Rf-Wert = 0.10

### 100d)

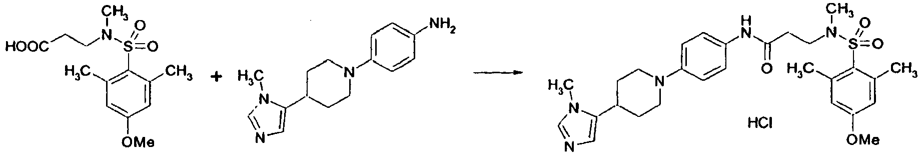

Beispiel 100 wird analog zu 1f aus 0.085 g (0.28 mMol) Produkt aus 22c, 0.070 g (0.27 mMol) Produkt aus 100c, 0.048 ml (0.35 mMol) Triethylamin und 0.095 g (0.30 mMol) TBTU in 20 ml THF und 3 ml DMF hergestellt.
C₂₈H₃₇N₅O₄S × HCl (576.15)
[M+H]+ = 540
HPLC (Methode 5): Retentionszeit = 1.41 min

### Beispiel 101

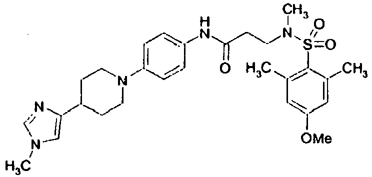

### 101a)

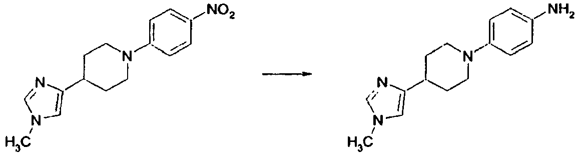

101a wird analog zu 8b aus 0.35 g (1.22 mMol) 100b-1 und 0.50 g Palladium auf Kohle (10%) in 50 ml Methanol hergestellt.
C₁₅H₂₀N₄ (256.35)
DC: Kieselgel, Dichlormethan / Methanol 9:1, Rf-Wert = 0.14

### 101b)

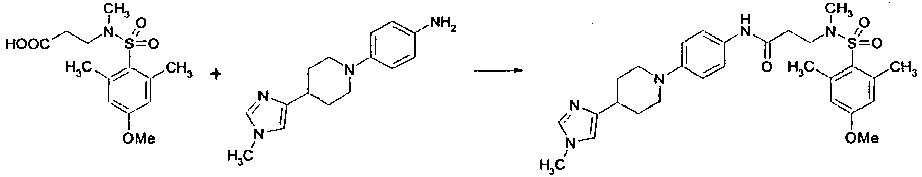

Beispiel 101 wird analog zu 1f aus 0.12 g (0.40 mMol) Produkt aus 22c, 0.10 g (0.39 mMol) Produkt aus 101a, 0.05 ml (0.50 mMol) Triethylamin und 0.14 g (0.42 mMol) TBTU in 30 ml THF und 5 ml DMF hergestellt.
C₂₈H₃₇N₅O₄S (539.69)
[M+H]+ = 540
DC: Kieselgel, Dichlormethan / Methanol 9:1, Rf-Wert = 0.47

Die folgenden Verbindungen wurden analog zu Beispiel 22 hergestellt:

### Beispiel 140

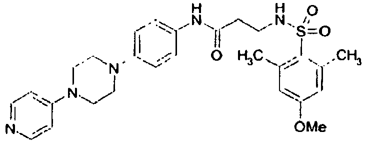

C₂₇H₃₃N₅O₄S x C₂HF₃O₂ (637.67)
[M+H]+ = 524
HPLC (Methode 6): Retentionszeit = 2.43 min

### Beispiel 141

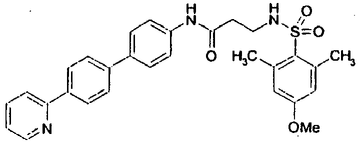

C₂₉H₂₉N₃O₄S x C₂HF₃O₂ (629.65)
[M+H]+ = 516
HPLC (Methode 6): Retentionszeit = 3.39 min

### Beispiel 142

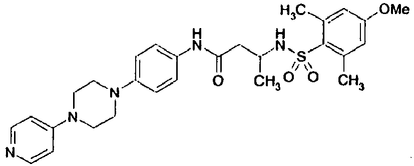

C₂₈H₃₅N₅O₄S (537.67)
[M+H]+ = 538
HPLC (Methode 6): Retentionszeit = 2.45 min

### Beispiel 143

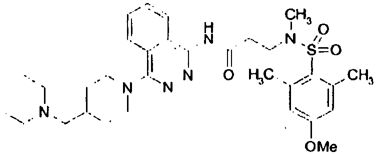

C₃₁H₄₄N₆O₄S x C₂HF₃O₂ (710.81)
[M+H]+ = 597
HPLC (Methode 6): Retentionszeit = 2.30 min

### Beispiel 144

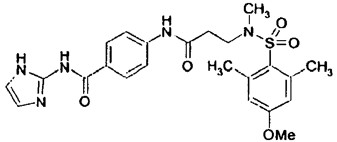

C₂₃H₂₇N₅O₅S x C₂HF₃O₂ (599.58)
[M+H]+ = 486
HPLC (Methode 6): Retentionszeit = 2.46 min

### Beispiel 145

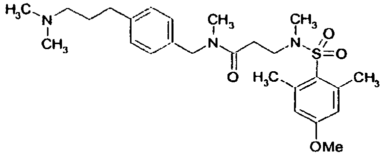

C₂₆H₃₉N₃O₄S (489.67)
[M+H]+ = 490
HPLC (Methode 6): Retentionszeit = 2.66 min

### Beispiel 146

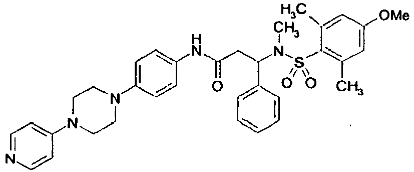

C₃₄H₃₉N₅O₄S (613.77)
[M+H]+ = 614
HPLC (Methode 6): Retentionszeit = 3.07 min

### Beispiel 147

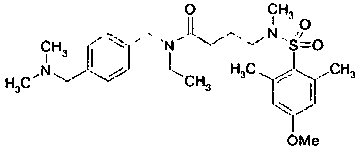

C₂₆H₃₉N₃O₄S x C₂HF₃O₂ (603.70)
[M+H]+ = 490
HPLC (Methode 6): Retentionszeit = 2.60 min

### Beispiel 148

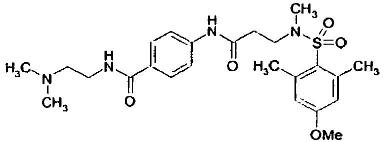

C₂₄H₃₄N₄O₅S x CH₂O₂ (536.64)
[M+H]+ = 491
HPLC (Methode 6): Retentionszeit = 2.29 min

### Beispiel 149

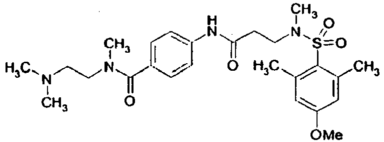

C₂₆H₃₆N₄O₅S × CH₂O₂ (550.67)
[M+H]+ = 505
HPLC (Methode 6): Retentionszeit = 2.32 min

### Beispiel 150

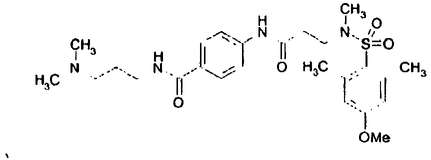

C₂₅H₃₆N₄O₅S (504.64)
[M+H]+ = 505
HPLC (Methode 6): Retentionszeit = 2.31 min

### Beispiel 151

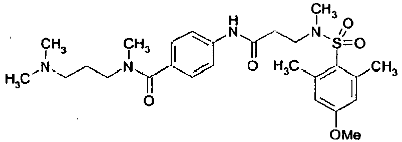

C₂₆H₃₈N₄O₅S x CH₂O₂ (564.70)
[M+H]+ = 519
HPLC (Methode 6): Retentionszeit = 2.34 min

### Beispiel 152

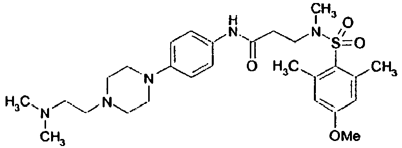

C₂₇H₄₁N₅O₄S x 2C₂HF₃O₂ (759.76)
[M+H]+ = 532
HPLC (Methode 5): Retentionszeit = 1.40 min

### Beispiel 153

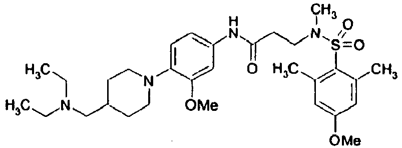

C₃₀H₄₆N₄O₅S × HCl (611.24)
[M+H]+ = 575
HPLC (Methode 1): Retentionszeit = 2.12 min

### Beispiel 154

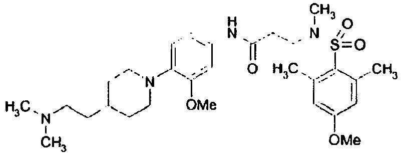

C₂₉H₄₄N₄O₅S x HCl (597.21)
[M+H]+ = 561
HPLC (Methode 8): Retentionszeit = 3.12 min

### Beispiel 155

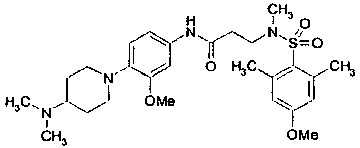

C₂₇H₄₀N₄O₅S x HCl (569.16)
[M+H]+ = 533
HPLC (Methode 11): Retentionszeit = 1.67 min

### Beispiel 156

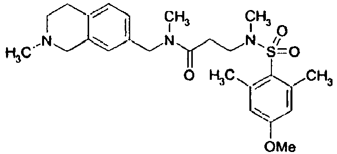

C₂₅H₃₅N₃O₄S x HCl (510.09)
[M+H]+ = 474
HPLC (Methode 7): Retentionszeit = 1.90 min

### Beispiel 157

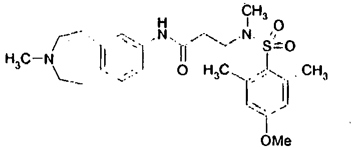

C₂₄H₃₃N₃O₄S x C₂HF₃O₂ (573.63)
[M+H]+ = 460
HPLC (Methode 5): Retentionszeit = 1.52 min

### Beispiel 158

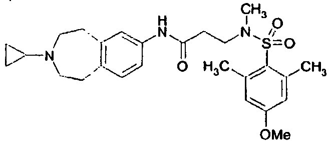

C₂₆H₃₅N₃O₄S x C₂HF₃O₂ (599.66)
[M+H]+ = 486
HPLC (Methode 5): Retentionszeit = 1.55 min

### Beispiel 159

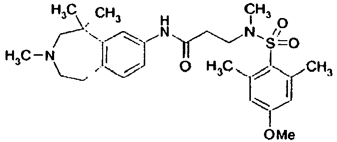

C₂₆H₃₇N₃O₄S x C₂HF₃O₂ (601.68)
[M+H]+ = 488
HPLC (Methode 5): Retentionszeit = 1.54 min

### Beispiel 160

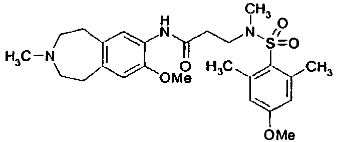

C₂₅H₃₅N₃O₅S x C₂HF₃O₂ (603.65)
[M+H]+ = 490
HPLC (Methode 5): Retentionszeit = 1.54 min

### Beispiel 161

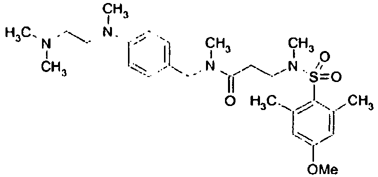

C₂₆H₄₀N₄O₄S × HCl (541.15)
[M+H]+ = 505
HPLC (Methode 5): Retentionszeit = 1.59 min

### Beispiel 162

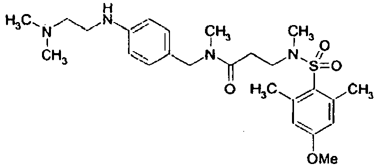

C₂₅H₃₈N₄O₄S × HCl (527.12)
[M+H]+ = 491
HPLC (Methode 5): Retentionszeit = 1.55 min

### Beispiel 163

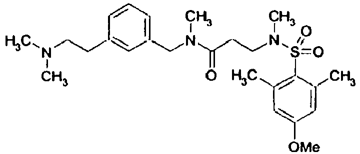

C₂₅H₃₇N₃O₄S × HCl (512.11)
[M+H]+ = 476
HPLC (Methode 5): Retentionszeit = 1.56 min

### Beispiel 164

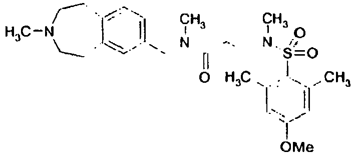

C₂₆H₃₇N₃O₄S × HCl (524.12)
[M+H]+=488
HPLC (Methode 5): Retentionszeit = 1.54 min

### Beispiel 165

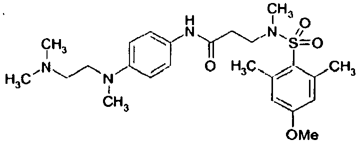

C₂₄H₃₆N₄O₄S × HCl (513.09)
[M+H]+ = 477
HPLC (Methode 7): Retentionszeit = 1.88 min

### Beispiel 166

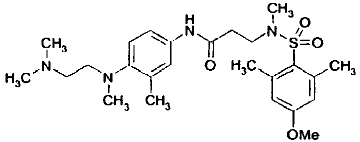

C₂₅H₃₈N₄O₄S × HCl (527.12)
[M+H]+ = 491
HPLC (Methode 7): Retentionszeit = 1.92 min

### Beispiel 167

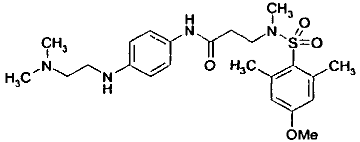

C₂₃H₃₄N₄O₄S × HCl (499.07)
[M+H]+ = 463
HPLC (Methode 7): Retentionszeit = 1.79 min

### Beispiel 168

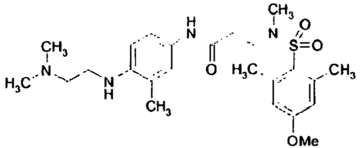

C₂₄H₃₆N₄O₄S × HCl (513.09)
[M+H]+ = 477
HPLC (Methode 7): Retentionszeit = 1.86 min

### Beispiel 169

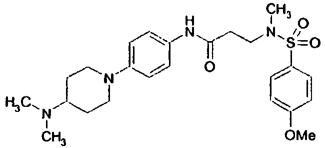

C₂₄H₃₄N₄O₄S x C₂HF₃O₂ (588.64)
[M+H]+ = 475
HPLC (Methode 5): Retentionszeit = 1.39 min

### Beispiel 170

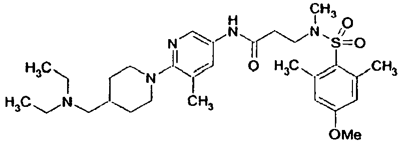

C₂₉H₄₅N₅O₄S × 2HCl (632.69)
[M+H]+ = 560
DC: Kieselgel, Dichlormethan / Ethanol 9:1, Rf-Wert = 0.22

### Beispiel 171

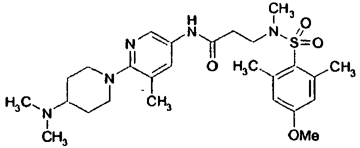

C₂₆H₃₉N₅O₄S × 2HCl (590.61)
[M+H]+ = 518
DC: Kieselgel, Dichlormethan / Ethanol 4:1, Rf-Wert = 0.68

### Bespiel 172

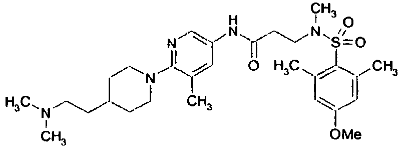

C₂₈H₄₃N₅O₄S × 2HCl (618.66)
[M+H]+ = 546
HPLC (Methode 5): Retentionszeit = 1.26 min

### Beispiel 173

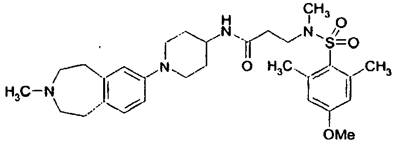

C₂₉H₄₂N₄O₄S (542.73)
[M+H]+ = 543
HPLC (Methode 4): Retentionszeit = 2.8 min

### Beispiel 174

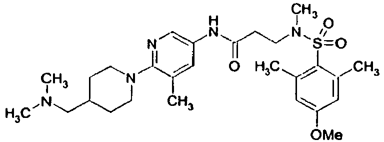

C₂₇H₄₁N₅O₄S × 2HCl (604.63)
[M+H]+ = 532
HPLC (Methode 5): Retentionszeit = 1.39 min

### Beispiel 175

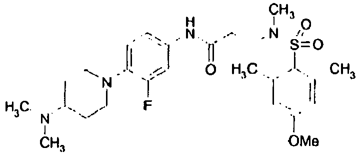

C₂₆H₃₇FN₄O₄S × HCl (557.12)
[M+H]+ = 521
DC: Kieselgel, Dichlormethan / Ethanol 9:1, Rf-Wert = 0.25

### Beispiel 176

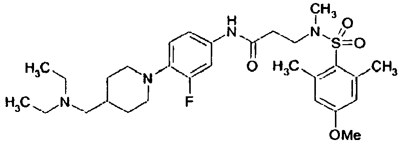

C₂₉H₄₃FN₄O₄S x HCl (599.20)
[M+H]+=563
DC: Kieselgel, Dichlormethan / Ethanol 9:1, Rf-Wert = 0.40

### Beispiel 177

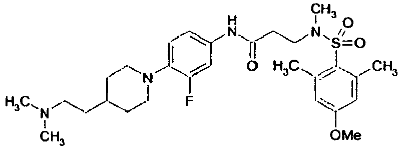

C₂₈H₄₁FN₄O₄S × HCl (585.17)
[M+H]+ = 549
DC: Kieselgel, Dichlormethan / Ethanol 9:1, Rf-Wert = 0.35

### Beispiel 178

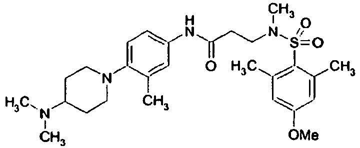

C₂₇H₄₀N₄O₄S (516.70)
[M+H]+ = 517
DC: Kieselgel, Dichlormethan / Ethanol / Ammoniak 8:2:0.01, Rf-Wert = 0.41

### Beispiel 179

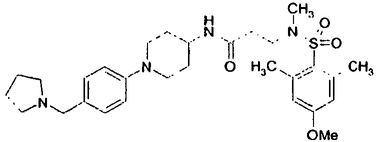

C₂₉H₄₂N₄O₄S × HCl (579.19)
[M+H]+ = 543
DC: Kieselgel, Dichlormethan / Ethanol / Ammoniak 8:2:0.01, Rf-Wert = 0.47

### Beispiel 180

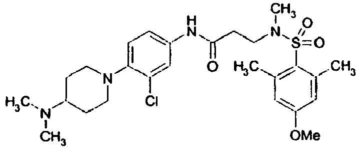

C₂₆H₃₇ClN₄O₄S x HCl (573.58)
[M+H]+ = 537/539
DC: Kieselgel, Dichlormethan / Ethanol 9:1, Rf-Wert = 0.26

### Beispiel 181

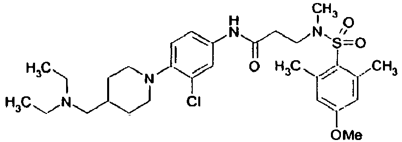

C₂₉H₄₃ClN₄O₄S x HCl (615.66)
[M+H]+ = 579/581
DC: Kieselgel, Dichlormethan / Ethanol 9:1, Rf-Wert = 0.45

### Beispiel 182

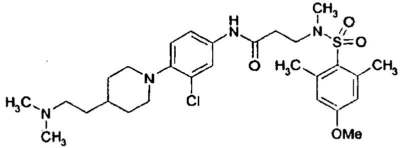

C₂₈H₄₁ClN₄O₄S × HCl (601.63)
[M+H]+ = 565/567
DC: Kieselgel, Dichlormethan / Ethanol 9:1, Rf-Wert = 0.30

### Beispiel 183

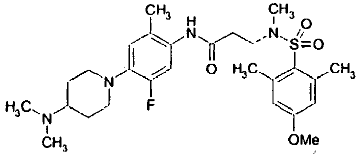

C₂₇H₃₉FN₄O₄S × HCl (571.15)
[M+H]+ = 535
DC: Kieselgel, Dichlormethan / Ethanol / Ammoniak 8:2:0.01, Rf-Wert = 0.26

### Beispiel 184

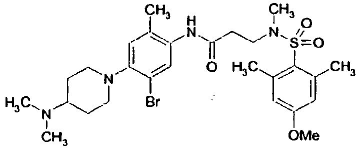

C₂₇H₃₉BrN₄O₄S × HCl (632.05)
[M+H]+ = 595/597
DC: Kieselgel, Dichlormethan / Ethanol / Ammoniak 8:2:0.01, Rf-Wert = 0.51

### Beispiel 185

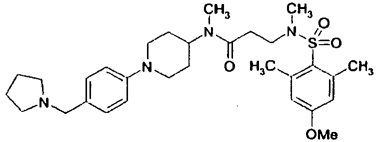

C₃₀H₄₄N₄O₄S × HCl (593.22)
[M+H]+ = 557
DC: Kieselgel, Dichlormethan / Ethanol / Ammoniak 8:2:0.01, Rf-Wert = 0.63

### Beispiel 186

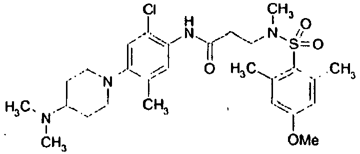

C₂₇H₃₉ClN₄O₄S × HCl (587.60)
[M+H]+ = 551
DC: Kieselgel, Dichlormethan / Ethanol / Ammoniak 8:2:0.01, Rf-Wert = 0.58

### Beispiel 187

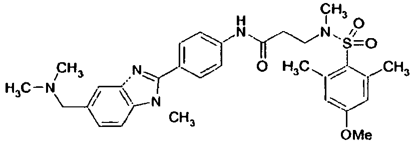

C₃₀H₃₇N₅O₄S x HCl (600.17)
[M+H]+ = 564
HPLC (Methode 4): Retentionszeit = 3.0 min

### Beispiel 188

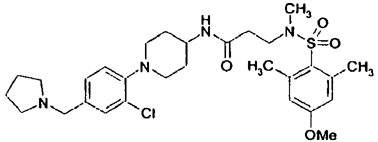

C₂₉H₄₁ClN₄O₄S × HCl (613.64)
[M+H]+ = 577/579
DC: Kieselgel, Dichlormethan / Methanol 9:1, Rf-Wert = 0.24

### Beispiel 189

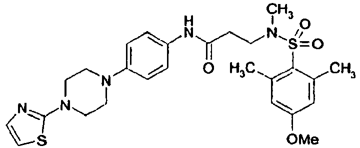

C₂₆H₃₃N₅O₄S₂ (543.70)
[M+H]+ = 544
HPLC (Methode 6): Retentionszeit = 3.11 min

### Beispiel 190

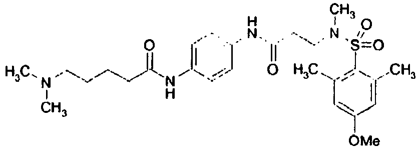

C₂₆H₃₈N₄O₅S (518.67)
[M+H]+ = 519
HPLC (Methode 6): Retentionszeit = 2.44 min

### Beispiel 191

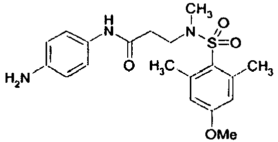

C₁₉H₂₅N₃O₄S x C₂HF₃O₂ (505.51)
[M+H]+ = 392
HPLC (Methode 6): Retentionszeit = 2.04 min

### Beispiel 192

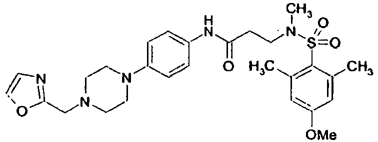

C₂₇H₃₅N₅O₅S (541.66)
[M+H]+ = 542
HPLC (Methode 6): Retentionszeit = 2.51 min

### Beispiel 193

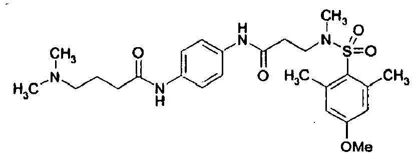

C₂₅H₃₆N₄O₅S (504.64)
[M+H]+ = 505
HPLC (Methode 6): Retentionszeit = 2.43 min

### Beispiel 194

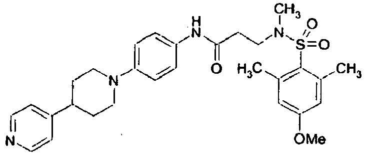

C₂₉H₃₆N₄O₄S (536.69)
[M+H]+ = 537
HPLC (Methode 6): Retentionszeit = 2.19 min

### Beispiel 195

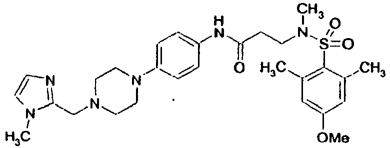

C₂₈H₃₈N₆O₄S (554.71)
[M+H]+ = 555
HPLC (Methode 6): Retentionszeit = 2.37 min

### Beispiel 196

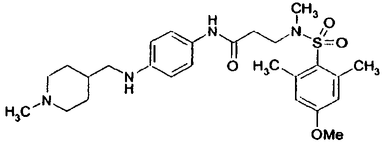

C₂₆H₃₈N₄O₄S (502.67)
[M+H]+ = 503
HPLC (Methode 6): Retentionszeit = 2.35 min

### Beispiel 197

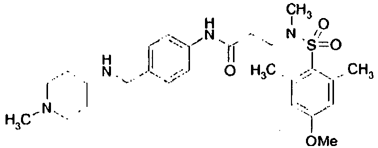

C₂₆H₃₈N₄O₄S (502.67)
[M+H]+ = 503
HPLC (Methode 6): Retentionszeit = 2.00 min

### Beispiel 198

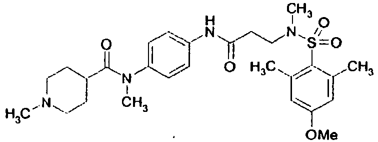

C₂₇H₃₈N₄O₅S x C₂HF₃O₂ (644.70)
[M+H]+ = 531
HPLC (Methode 6): Retentionszeit = 2.45 min

### Beispiel 199

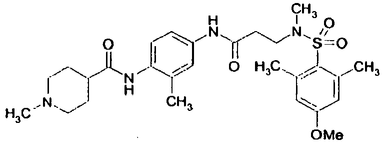

C₂₇H₃₈N₄O₅S (530.68)
[M+H]+ = 531
HPLC (Methode 6): Retentionszeit = 2.53 min

### Beispiel 200

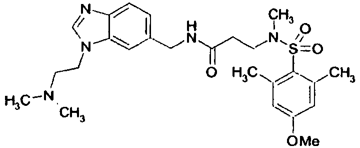

C₂₅H₃₅N₅O₄S (501.64)
[M+H]+ = 502
HPLC (Methode 6): Retentionszeit = 2.05 min

### Beispiel 201

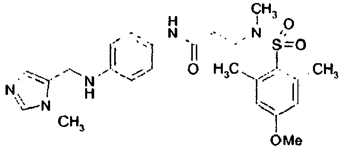

C₂₄H₃₁N₅O₄S (485.60)
[M+H]+ = 486
HPLC (Methode 6): Retentionszeit = 2.41 min

### Beispiel 202

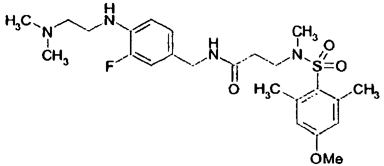

C₂₄H₃₅FN₄O₄S x CH₂O₂ (540.65)
[M+H]+ = 495
HPLC (Methode 6): Retentionszeit = 2.50 min

### Beispiel 203

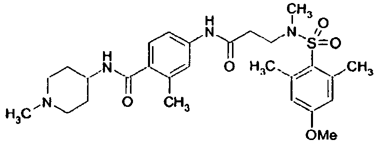

C₂₇H₃₈N₄O₅S (530.68)
[M+H]+ = 531
HPLC (Methode 6): Retentionszeit = 2.36 min

### Beispiel 204

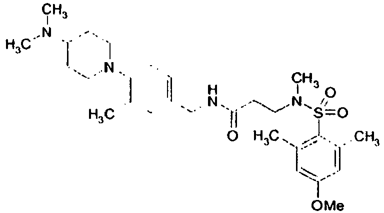

C₂₈H₄₂N₄O₄S (530.72)
[M+H]+ = 531
HPLC (Methode 6): Retentionszeit = 2.60 min

### Beispiel 205

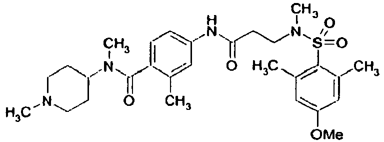

C₂₈H₄₀N₄O₅S (544.71)
[M+H]+ = 545
HPLC (Methode 6): Retentionszeit = 2.21 min

### Beispiel 206

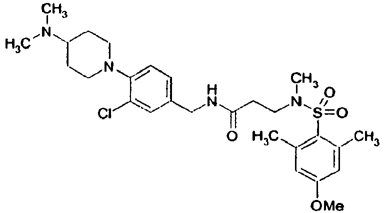

C₂₇H₃₉ClN₄O₄S (551.14)
[M+H]+ = 551/553
HPLC (Methode 6): Retentionszeit = 2.65 min

### Beispiel 207

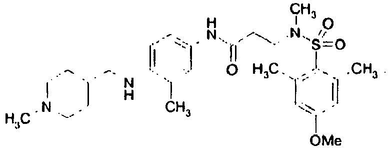

C₂₇H₄₀N₄O₄S x C₂HF₃O₂ (630.72)
[M+H]+ = 517
HPLC (Methode 6): Retentionszeit = 2.51 min

### Beispiel 208

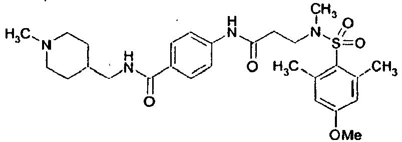

C₂₇H₃₈N₄O₅S x CH₂O₂ (576.71)
[M+H]+=531
HPLC (Methode 6): Retentionszeit = 2.33 min

### Beispiel 209

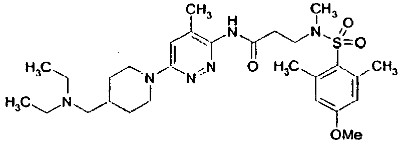

C₂₈H₄₄N₆O₄S x C₂HF₃O₂ (674.78)
[M+H]+ = 561
HPLC (Methode 6): Retentionszeit = 2.14 min

### Beispiel 210

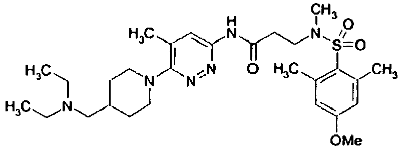

C₂₈H₄₄N₆O₄S x C₂HF₃O₂ (674.78)
[M+H]+ = 561
HPLC (Methode 6): Retentionszeit = 2.43 min

### Beispiel 211

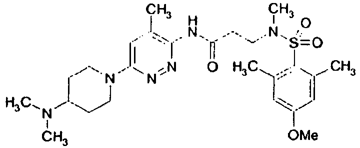

C₂₅H₃₈N₆O₄S x C₂HF₃O₂ (632.70)
[M+H]+ = 519
HPLC (Methode 6): Retentionszeit = 2.11 min

### Beispiel 212

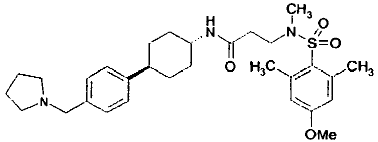

C₃₀H₄₃N₃O₄S x C₂HF₃O₂ (655.77)
[M+H]+ = 542
HPLC (Methode 6): Retentionszeit = 2.75 min

### Beispiel 213

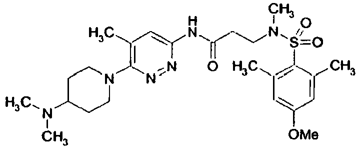

C₂₅H₃₈N₆O₄S x C₂HF₃O₂ (632.70)
[M+H]+=519
HPLC (Methode 6): Retentionszeit = 2.30 min

### Beispiel 214

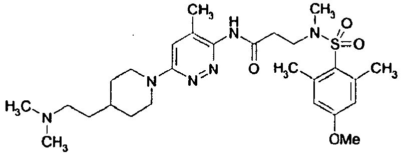

C₂₇H₄₂N₆O₄S x C₂HF₃O₂ (660.75)
[M+H]+ = 547
HPLC (Methode 6): Retentionszeit = 2.08 min

### Beispiel 215

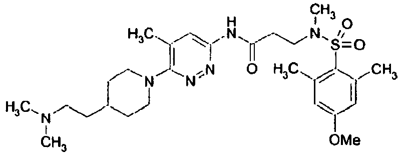

C₂₇H₄₂N₆O₄S x C₂HF₃O₂ (660.75)
(M+H]+ = 547
HPLC (Methode 6): Retentionszeit = 2.34 min

### Beispiel 216

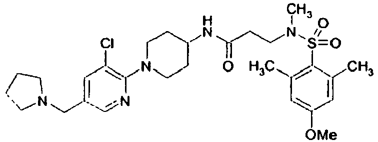

C₂₈H₄₀ClN₅O₄S x CH₂O₂ (624.19)
[M+H]+ = 578/580
HPLC (Methode 6): Retentionszeit = 2.64 min

### Beispiel 217

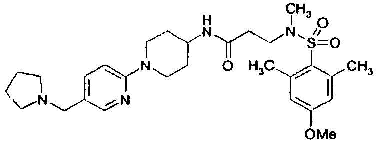

C₂₈H₄₁N₅O₄S x CH₂O₂ (589.75)
[M+H]+ = 544
HPLC (Methode 6): Retentionszeit = 2.09 min

### Beispiel 218

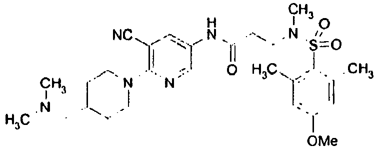

C₂₇H₃₈N₆O₄S x CH₂O₂ (588.72)
[M+H]+ = 543
HPLC (Methode 9): Retentionszeit = 1.67 min

### Beispiel 219

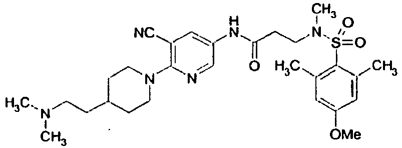

C₂₈H₄₀N₆O₄S (556.72)
[M+H]+ = 557
HPLC (Methode 9): Retentionszeit = 1.71 min

### Beispiel 220

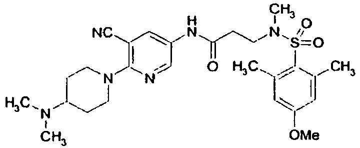

C₂₆H₃₆N₆O₄S x CH₂O₂ (574.69)
[M+H]+ = 529
HPLC (Methode 9): Retentionszeit = 1.61 min

### Beispiel 221

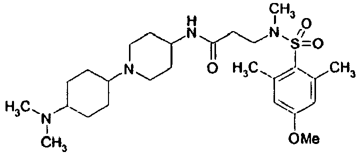

C₂₆H₄₄N₄O₄S (508.72)
[M+H]+ = 509
HPLC (Methode 9): Retentionszeit = 1.23 min

### Beispiel 222

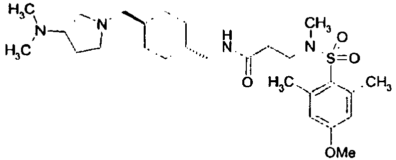

C₂₇H₄₆N₄O₄S x 2C₂HF₃O₂ (750.79)
[M+H]+ = 523
HPLC (Methode 9): Retentionszeit = 1.30 min

### Beispiel 223

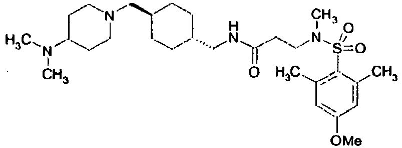

C₂₈H₄₈N₄O₄S x 2C₂HF₃O₂ (764.82)
[M+H]+ = 537
HPLC (Methode 9): Retentionszeit = 1.31 min

### Beispiel 224

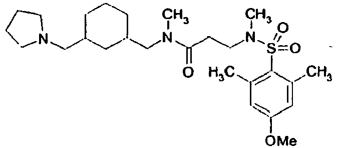

C₂₆H₄₃N₃O₄S (493.70)
[M+H]+ = 494
HPLC (Methode 9): Retentionszeit = 1.72 min

### Beispiel 225

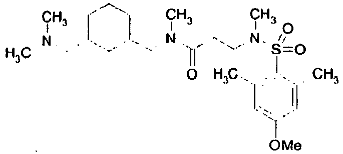

C₂₄H₄N₃O₄S x C₂HF₃O₂ (581.69)
[M+H]+ = 468
HPLC (Methode 9): Retentionszeit = 1.69 min

### Beispiel 226

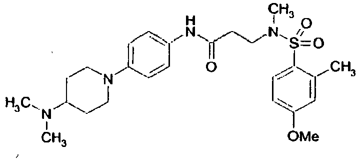

C₂₅H₃₆N₄O₄S x C₂HF₃O₂ (602.67)
[M+H]+ = 489
HPLC (Methode 5): Retentionszeit = 1.39 min

### Beispiel 227

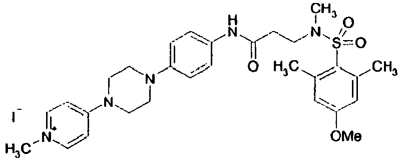

C₂₉H₃₈N₅O₄S x I (679.61)
[M+H]+ = 552
HPLC (Methode 5): Retentionszeit = 1.55 min

### Beispiel 228

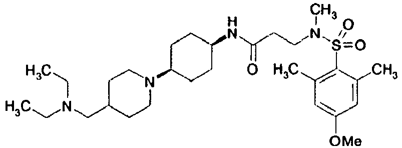

C₂₉H₅₀N₄O₄S (550.80)
[M+H]+ = 551
HPLC (Methode 5): Retentionszeit = 1.38 min

### Beispiel 229

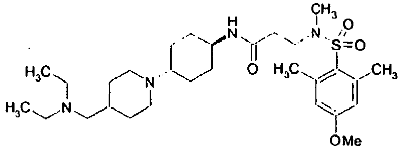

C₂₉H₅₀N₄O₄S (550.80)
[M+H]+ = 551
HPLC (Methode 5): Retentionszeit = 1.40 min

### Beispiel 230

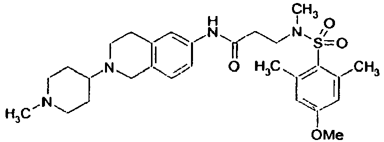

C₂₈H₄₀N₄O₄S x 2HCl (601.63)
[M+H]+ = 529
HPLC (Methode 5): Retentionszeit = 1.41 min

### Beispiel 231

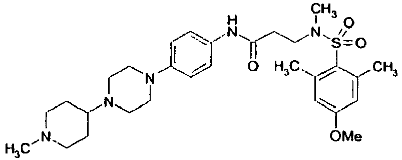

C₂₉H₄₃N₅O₄S (557.75)
[M+H]+ = 558
HPLC (Methode 1): Retentionszeit = 1.90 min

### Beispiel 232

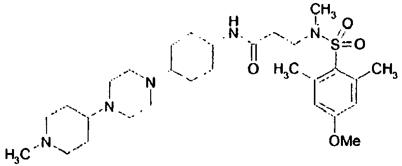

C₂₉H₄₉N₅O₄S (563.80)
[M+H]+ = 564
HPLC (Methode 5): Retentionszeit = 1.33 min

### Beispiel 233

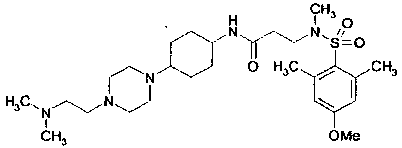

C₂₇H₄₇N₅O₄S x 2HCl (610.68)
[M+H]+ = 538
HPLC (Methode 7): Retentionszeit = 1.74 min

### Beispiel 234

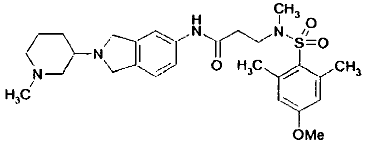

C₂₇H₃₈N₄O₄S (610.68)
[M+H]+ = 515 HPLC (Methode 5): Retentionszeit = 1.41 min

### Beispiel 235

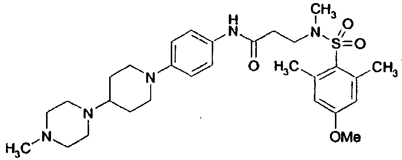

C₂₉H₄₃N₅O₄S (557.75)
[M+H]+ = 558
HPLC (Methode 5): Retentionszeit = 1.43 min

### Beispiel 236

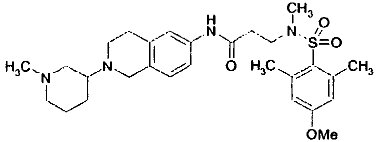

C₂₈H₄₀N₄O₄S x 2C₂HF₃O₂ (756.75)
[M+H]+ = 529
HPLC (Methode 5): Retentionszeit = 1.42 min

### Beispiel 237

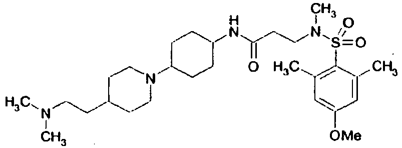

C₂₈H₄₈N₄O₄S x 2HCl (609.69)
[M+H]+ = 537
HPLC (Methode 7): Retentionszeit = 1.70 min

### Beispiel 238

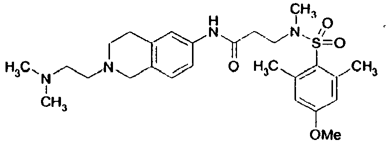

C₂₆H₃₈N₄O₄S x 2C₂HF₃O₂ (730.72)
[M+H]+ = 503
HPLC (Methode 5): Retentionszeit = 1.40 min

### Beispiel 239

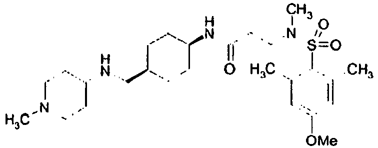

C₂₆H₄₄N₄O₄S x 2HCl (581.64)
[M+H]+ = 509
HPLC (Methode 5): Retentionszeit = 1.38 min

### Beispiel 240

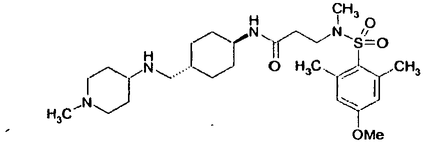

C₂₆H₄₄N₄O₄S x 2HCl (581.64)
[M+H]+ = 509
HPLC (Methode 5): Retentionszeit = 1.40 min

### Beispiel 241

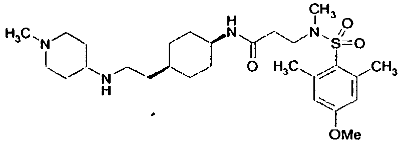

C₂₇H₄₆N₄O₄S x 2HCl (595.67)
[M+H]+ = 523
DC: Kieselgel, Dichlormethan / Methanol / Ammoniak 9:1:0.1, Rf-Wert = 0.13

### Beispiel 242

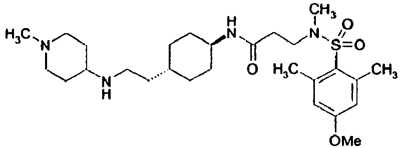

C₂₇H₄₆N₄O₄S x 2HCl (595.67)
[M+H]+ = 523
DC: Kieselgel, Dichlormethan / Methanol / Ammoniak 9:1:0.1, Rf-Wert = 0.13

### Beispiel 243

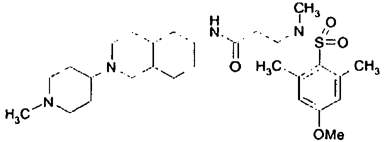

C₂₈H₄₆N₄O₄S x 2HCl (607.68)
[M+H]+ = 535
HPLC (Methode 5): Retentionszeit = 1.10 min

### Beispiel 244

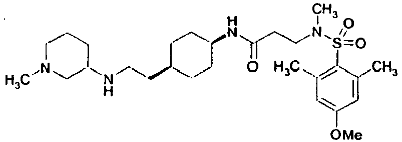

C₂₇H₄₆N₄O₄S x 2HCl (595.67)
[M+H]+ = 523
DC: Kieselgel, Dichlormethan / Methanol / Ammoniak 9:1:0.1, Rf-Wert = 0.25

### Beispiel 245

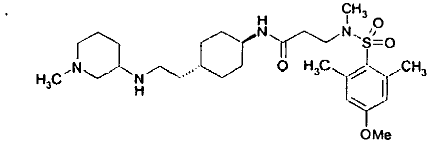

C₂₇H₄₆N₄O₄S x 2HCl (595.67)
[M+H]+ = 523
DC: Kieselgel, Dichlormethan / Methanol / Ammoniak 9:1:0.1, Rf-Wert = 0.25

### Beispiel 246

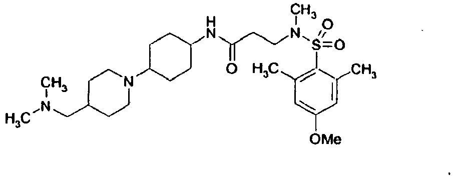

C₂₇H₄₆N₄O₄S x 2C₂HF₃O₂ (750.79)
[M+H]+ = 523
HPLC (Methode 5): Retentionszeit = 1.38 min

### Beispiel 247

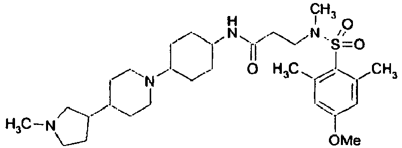

C₂₉H₄₈N₄O₄S x 2C₂HF₃O₂ (776.83)
[M+H]+ = 549
HPLC (Methode 5): Retentionszeit = 1.39 min

### Beispiel 248

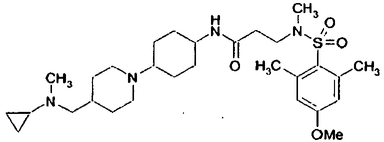

C₂₉H₄₈N₄O₄S x 2C₂HF₃O₂ (776.83)
[M+H]+ = 549
HPLC (Methode 5): Retentionszeit = 1.39 min

### Beispiel 249

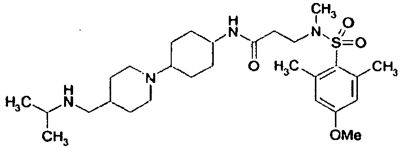

C₂₈H₄₈N₄O₄S x 2HCl (609.69)
[M+H]+ = 537
HPLC (Methode 5): Retentionszeit = 1.38 min

### Beispiel 250

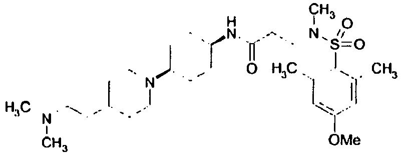

C₂₈H₄₈N₄O₄S x 2HCl (609.69)
[M+H]+ = 537
HPLC (Methode 11): Retentionszeit = 1.60 min

### Beispiel 251

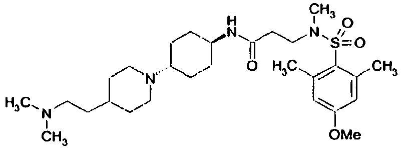

C₂₈H₄₈N₄O₄S x 2HCl (609.69)
[M+H]+ = 537
HPLC (Methode 7): Retentionszeit = 1.71 min

### Beispiel 252

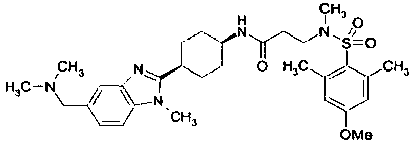

C₃₀H₄₃N₅O₄S x HCl (606.22)
[M+H]+ = 570
DC: Kieselgel, Dichlormethan / Methanol / Ammoniak 9:1:0.1, Rf-Wert = 0.29

### Beispiel 253

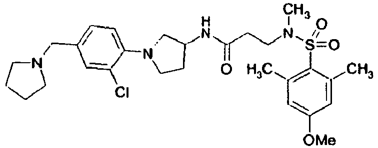

C₂₈H₃₉ClN₄O₄S x HCl (599.61)
[M+H]+ = 563/565
HPLC (Methode 5): Retentionszeit = 1.59 min

### Beispiel 254

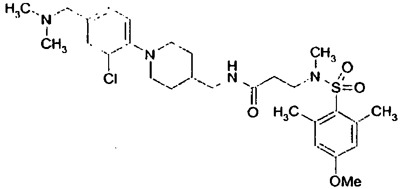

C₂₈H₄₁ClN₄O₄S x HCl (601.63)
[M+H]+ = 565/567
DC: Kieselgel, Dichlormethan / Methanol / Ammoniak 9:1:0.1, Rf-Wert = 0.46

### Beispiel 255

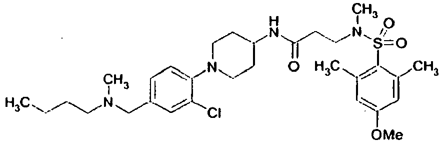

C₃₀H₄₅ClN₄O₄S x HCl (629.68)
[M+H]+ = 593/595
DC: Kieselgel, Dichlormethan / Methanol 9:1, Rf-Wert = 0.42

### Beispiel 256

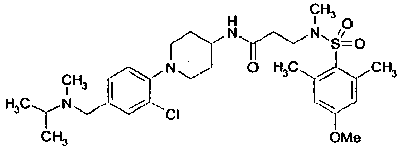

C₂gH₄₃ClN₄O₄S x HCl (615.66)
[M+H]+ = 579/581
DC: Kieselgel, Dichlormethan / Methanol 9:1, Rf-Wert = 0.23

### Beispiel 257

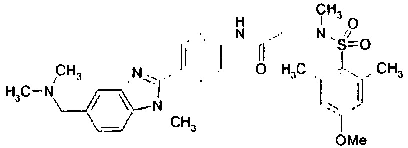

C₃₀H₃₇N₅O₄S x HCl (600.17)
[M+H]+ = 564
DC: Kieselgel, Dichlormethan / Methanol / Ammoniak 8:2:0.1, Rf-Wert = 0.67

### Beispiel 258

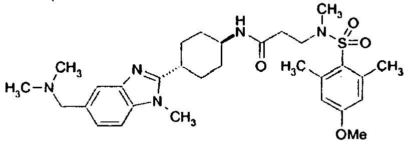

C₃₀H₄₃N₅O₄S (569.76)
[M+H]+ = 570 DC: Kieselgel, Dichlormethan / Methanol / Ammoniak 8:2:0.2, Rf-Wert = 0.65

### Beispiel 259

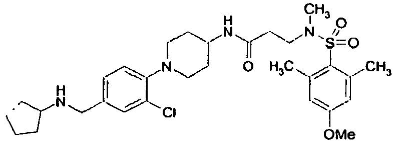

C₃₀H₄₃ClN₄O₄S x HCl (627.67)
[M+H]+ = 591/593
DC: Kieselgel, Dichlormethan / Methanol 9:1, Rf-Wert = 0.20

### Beispiel 260

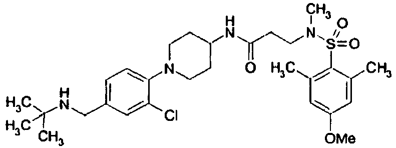

C₂₉H₄₃ClN₄O₄S x HCl (615.66)
[M+H]+ = 579/581
DC: Kieselgel, Dichlormethan / Methanol 9:1, Rf-Wert = 0.17

### Beispiel 261

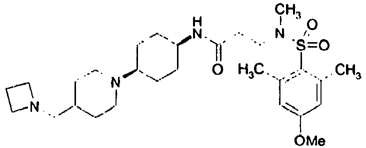

C₂₈H₄₆N₄O₄S x 2HCl (607.68)
[M+H]+ = 535
DC: Kieselgel, Dichlormethan / Methanol / Ammoniak 8:2:0.2, Rf-Wert = 0.23

### Beispiel 262

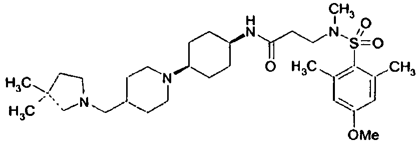

C₃₁H₅₂N₄O₄S x 2HCl (649.76)
[M+H]+ = 577
DC: Kieselgel, Dichlormethan / Methanol / Ammoniak 8:2:0.2, Rf-Wert = 0.69

### Beispiel 263

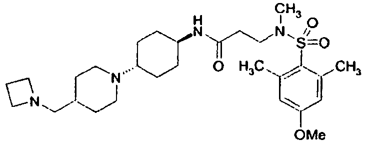

C₂₈H₄₆N₄O₄S (534.76)
[M+H]+ = 535
DC: Kieselgel, Dichlormethan / Methanol / Ammoniak 8:2:0.2, Rf-Wert = 0.58

### Beispiel 264

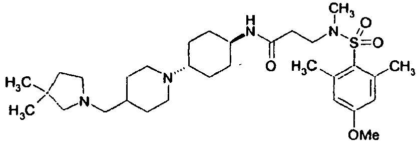

C₃₁H₅₂N₄O₄S (576.84)
[M+H]+ = 577
DC: Kieselgel, Dichlormethan / Methanol / Ammoniak 8:2:0.2, Rf-Wert = 0.56

### Beispiel 265

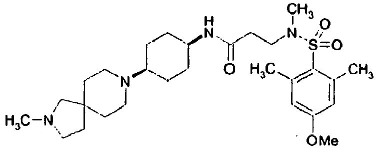

C₂₈H₄₆N₄O₄S x 2HCl (607.68)
[M+H]+ = 535
DC: Kieselgel, Dichlormethan / Methanol / Ammoniak 8:2:0.2, Rf-Wert = 0.58

### Beispiel 266

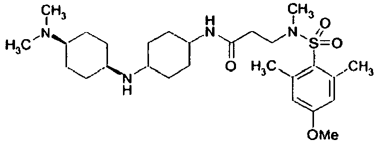

C₂₇H₄₆N₄O₄S (522.74)
[M+H]+ = 523
HPLC (Methode 9): Retentionszeit = 1.30 min

### Beispiel 267

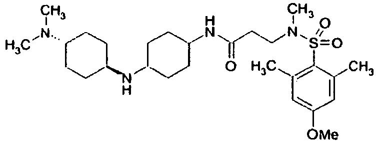

C₂₇H₄₆N₄O₄S (522.74)
[M+H]+ = 523
HPLC (Methode 9): Retentionszeit = 1.28 min

### Beispiel 268

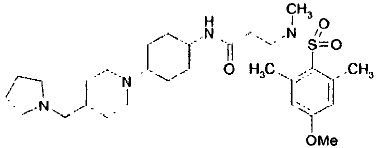

C₂₉H₄₈N₄O₄S x CH₂O₂ (594.81)
[M+H]+=549
HPLC (Methode 6): Retentionszeit = 1.96 min

### Beispiel 269

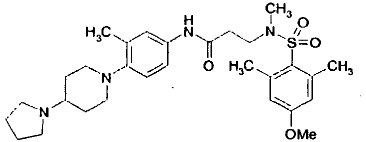

C₃₀₉H₄₄N₄O₄S (556.76)
[M+H]+ = 557
HPLC (Methode 9): Retentionszeit = 1.71 min

### Beispiel 270

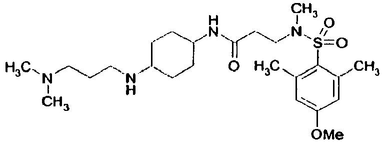

C₂₄H₄₂N₄O₄S x C₂HF₃O₂ (596.70)
[M+H]+ = 483
HPLC (Methode 9): Retentionszeit = 1.23 min

### Beispiel 271

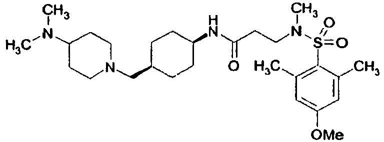

C₂₇H₄₆N₄O₄S (522.74)
[M+H]+ = 523
HPLC (Methode 9): Retentionszeit = 1.23 min

### Beispiel 272

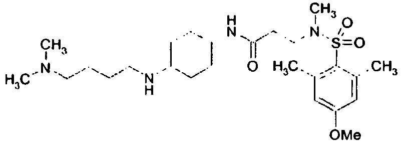

C₂₅H₄₄N₄O₄S (496.71)
[M+H]+ = 497
HPLC (Methode 9): Retentionszeit = 1.25 min

### Beispiel 273

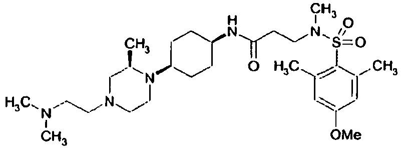

C₂₈H₄₉N₅O₄S x 2HCl (624.71)
[M+H]+ = 552
HPLC (Methode 10): Retentionszeit = 1.06 min

### Beispiel 274

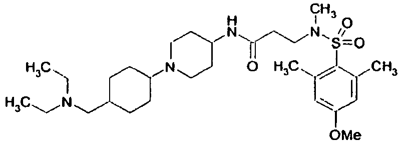

C₂₉H₅₀N₄O₄S x CH₂O₂ (596.82)
[M+H]+ = 551
HPLC (Methode 9): Retentionszeit = 1.31 min

### Beispiel 275

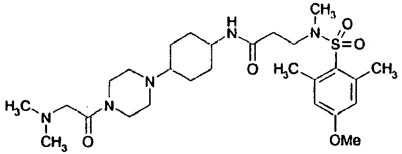

C₂₇H₄₅N₅O₅S x CH₂O₂ (597.77)
[M+H]+ = 552
HPLC (Methode 9): Retentionszeit = 1.20 min

### Beispiel 276

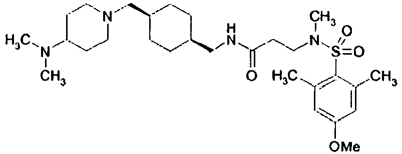

C₂₈H₄₈N₄O₄S (536.77)
[M+H]+ = 537
HPLC (Methode 6): Retentionszeit = 1.32 min

### Beispiel 277

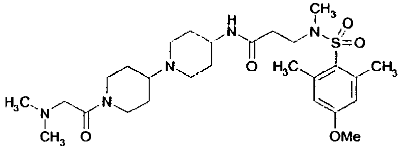

C₂₇H₄₅N₅O₅S x C₂HF₃O₂ (665.77)
[M+H]+ = 552
HPLC (Methode 9): Retentionszeit = 1.18 min

### Beispiel 278

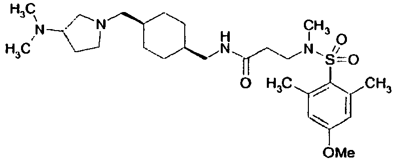

C₂₇H₄₆N₄O₄S x 2 C₂HF₃O₂ (750.79)
[M+H]+ = 523
HPLC (Methode 9): Retentionszeit = 1.29 min

### Beispiel 279

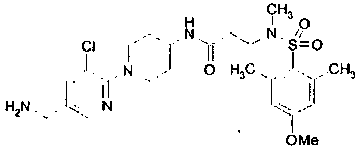

C₂₄H₃₄ClN₅O₄S (524.08)
[M+H]+ = 524/526
HPLC (Methode 9): Retentionszeit = 1.60 min

### Beispiel 280

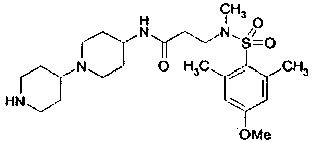

C₂₃H₃₈N₄O₄S x 2C₂HF₃O₂ (694.69)
[M+H]+ = 467
HPLC (Methode 9): Retentionszeit = 1.16 min

### Beispiel 281

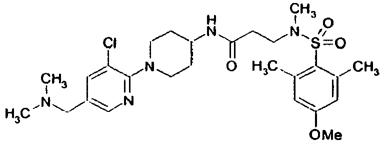

C₂₆H₃₈ClN₅O₄S x C₂HF₃O₂ (666.15)
[M+H]+ = 552/554
HPLC (Methode 9): Retentionszeit = 1.68 min

### Beispiel 282

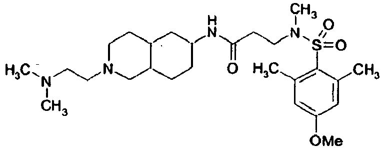

C₂₆H₄₄N₄O₄S x 2HCl (581.64)
[M+H]+ = 509
HPLC (Methode 5): Retentionszeit = 1.36 min

### Beispiel 283

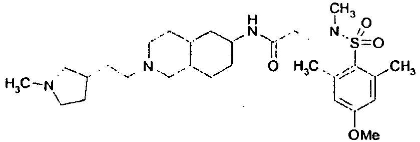

C₂₉H₅₀N₄O₄S x 2HCl (621.70)
[M+H]+ = 549
HPLC (Methode 5): Retentionszeit = 1.38 min

### Beispiel 284

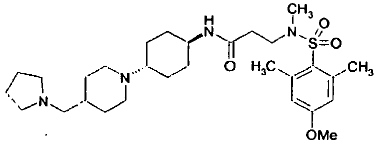

C₂₉H₄₈N₄O₄S (548.78)
[M+H]+ = 549
HPLC (Methode 5): Retentionszeit = 1.38 min

### Beispiel 285

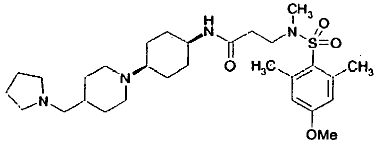

C₂₉H₄₈N₄O₄S (548.78)
[M+H]+=549
HPLC (Methode 5): Retentionszeit = 1.36 min

### Beispiel 286

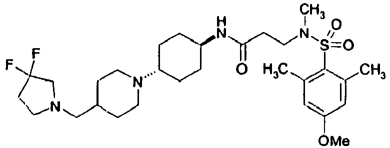

C₂₉H₄₆F₂N₄O₄S (584.76)
[M+H]+ = 585
HPLC (Methode 5): Retentionszeit = 1.38 min

### Beispiel 287

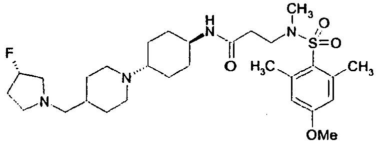

C₂₉H₄₇FN₄O₄S (566.77)
[M+H]+ = 567
HPLC (Methode 5): Retentionszeit = 1.38 min

### Beispiel 288

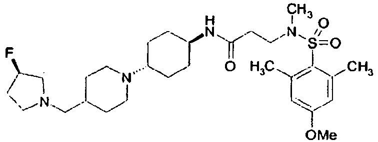

C₂₉H₄₇FN₄O₄S (566.77)
[M+H]+ = 567
HPLC (Methode 5): Retentionszeit = 1.36 min

### Beispiel 289

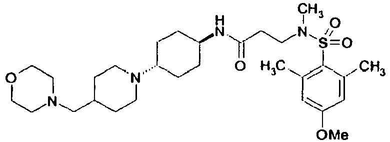

C₂₉H₄₈N₄O₅S (564.78)
[M+H]+ = 565
HPLC (Methode 5): Retentionszeit = 1.37 min

### Beispiel 290

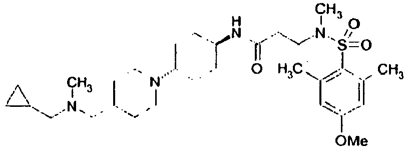

C₂₉H₅₀N₄O₄S (562.81)
[M+H]+ = 563
HPLC (Methode 5): Retentionszeit = 1.39 min

### Beispiel 291

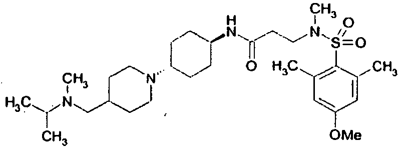

C₂₉H₅₀N₄O₄S (550.80)
[M+H]+ = 551
HPLC (Methode 5): Retentionszeit = 1.36 min

### Beispiel 292

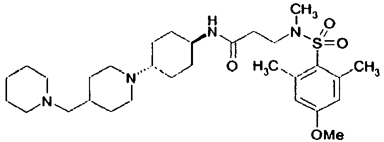

C₃₀H₅₀N₄O₄S (562.81)
[M+H]+=563
HPLC (Methode 5): Retentionszeit = 1.28 min

### Beispiel 293

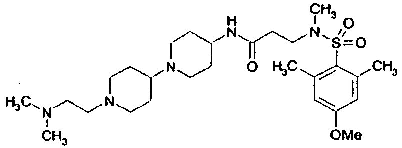

C₂₇H₄₇N₅O₄S x 3C₂HF₃O₂ (879.83)
[M+H]+ = 538
HPLC (Methode 5): Retentionszeit = 1.33 min

### Beispiel 294

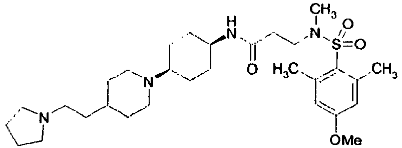

C₃₀H₅₀N₄O₄S x 2HCl (635.73)
[M+H]+ = 563
HPLC (Methode 5): Retentionszeit = 1.37 min

### Beispiel 295

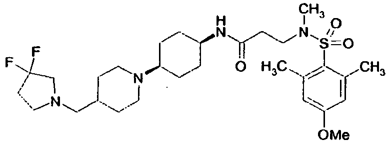

C₂₉H₄₆F₂N₄O₄S x 2C₂HF₃O₂ (584.76)
[M+H]+ = 585
HPLC (Methode 5): Retentionszeit = 1.38 min

### Beispiel 296

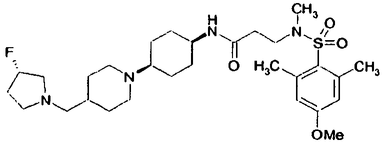

C₂₉H₄₇FN₄O₄S x 2C₂HF₃O₂ (794.82)
[M+H]+ = 567
HPLC (Methode 5): Retentionszeit = 1.35 min

### Beispiel 297

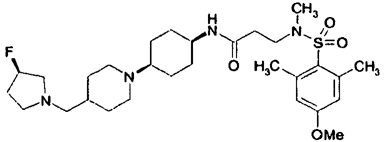

C₂₉H₄₇FN₄O₄S x 2C₂HF₃O₂ (794.82)
[M+H]+ = 567
HPLC (Methode 5): Retentionszeit = 1.36 min

### Beispiel 298

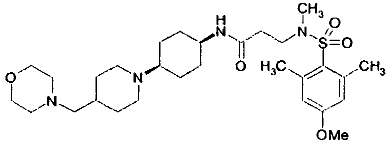

C₂₉H₄₈N₄O₅S (564.78)
[M+H]+ = 565
HPLC (Methode 5): Retentionszeit = 1.36 min

### Beispiel 299

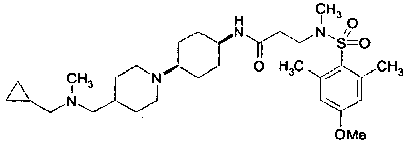

C₃₀H₅₀N₄O₄S (562.81)
[M+H]+ = 563
HPLC (Methode 5): Retentionszeit = 1.37 min

### Beispiel 300

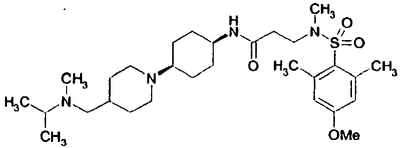

C₂₉H₅₀N₄O₄S (550.80)
[M+H]+ = 551
HPLC (Methode 5): Retentionszeit = 1.37 min

### Beispiel 301

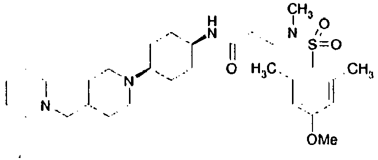

C₃₀H₅₀N₄O₄S (562.81)
[M+H]+ = 563
HPLC (Methode 5): Retentionszeit = 1.37 min

### Beispiel 302

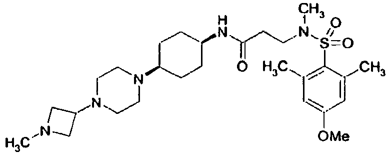

C₃₀H₅₀N₄O₄S x 3C₂HF₃O₂ (877.81)
[M+H]+ = 536
HPLC (Methode 5): Retentionszeit = 1.34 min

### Beispiel 303

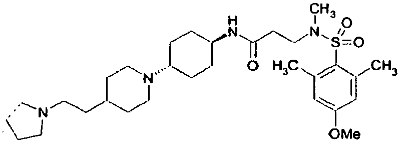

C₃₀H₅₀N₄O₄S (562.81)
[M+H]+ = 563
HPLC (Methode 5): Retentionszeit = 1.37 min

### Beispiel 304

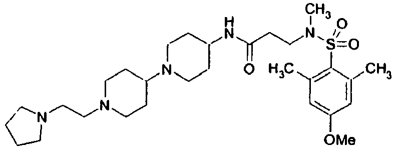

C₂₉H₄₉N₅O₄S x 3C₂HF₃O₂ (905.87)
[M+H]+ = 564
HPLC (Methode 5): Retentionszeit = 1.08 min

### Beispiel 305

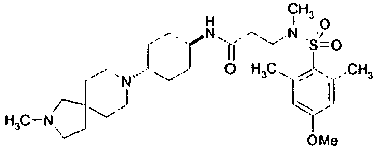

C₂₈H₄₆N₄O₄S x 2HCl (607.68)
[M+H]+ = 535
DC: Kieselgel, Dichlormethan / Methanol / Ammoniak 8:2:0.2, Rf-Wert = 0.19

### Beispiel 306

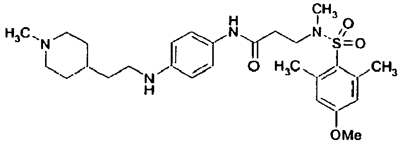

C₂₇H₄₀N₄O₄S x C₂HF₃O₄ (630.72)
[M+H]+ = 517
HPLC (Methode 9): Retentionszeit = 1.40 min

### Beispiel 307

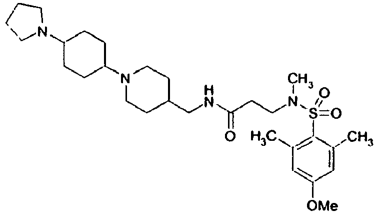

C₂₉H₄₈N₄O₄S (548.78)
[M+H]+ = 549
HPLC (Methode 6): Retentionszeit = 1.24 min

### Bespiel 308

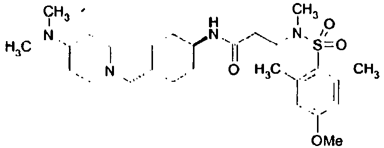

C₂₇H₄₆N₄O₄S (522.74)
[M+H]+ = 523
HPLC (Methode 9): Retentionszeit = 1.29 min

### Beispiel 309

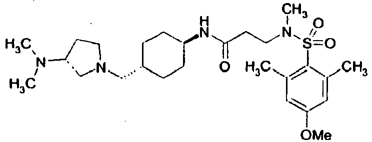

C₂₆H₄₄N₄O₄S (508.72)
[M+H]+ = 509
HPLC (Methode 9): Retentionszeit = 1.30 min

### Beispiel 310

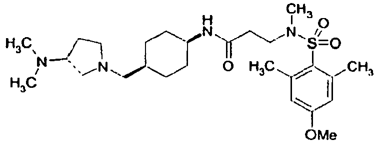

C₂₆H₄₄N₄O₄S (508.72)
[M+H]+ = 509
HPLC (Methode 9): Retentionszeit = 1.23 min

### Beispiel 311

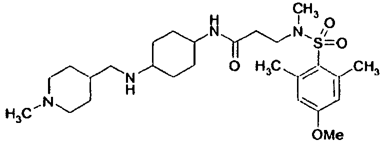

C₂₆H₄₄N₄O₄S (508.72)
[M+H]+ = 509
HPLC (Methode 6): Retentionszeit = 1.20 min

### Beispiel 312

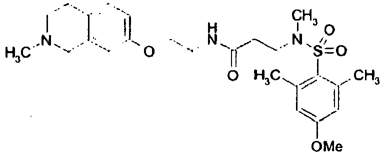

C₂₅H₃₅N₃O₅S x HCl (526.09)
[M+H]+ = 490
HPLC (Methode 10): Retentionszeit = 1.16 min

### Beispiel 313

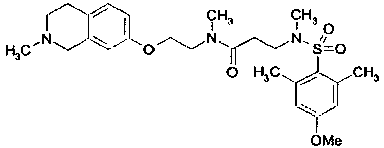

C₂₆H₃₇N₃O₅S x HCl (540.12)
[M+H]+ = 504
HPLC (Methode 10): Retentionszeit = 1.22 min

### Beispiel 314

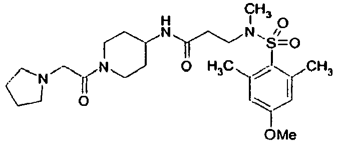

C₂₄H₃₈N₄O₅S x C₂HF₃O₂ (608.67)
[M+H]+=495
HPLC (Methode 9): Retentionszeit = 1.47 min

### Beispiel 315

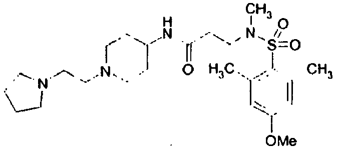

C₂₄H₄₀N₄O₄S (480.66)
[M+H]+ = 481
HPLC (Methode 9): Retentionszeit = 1.21 min

### Beispiel 316

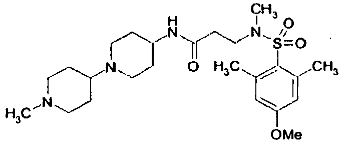

C₂₄H₄₀N₄O₄S x C₂HF₃O₂ (594.69)
[M+H]+ = 481
HPLC (Methode 9): Retentionszeit = 1.19 min

### Beispiel 317

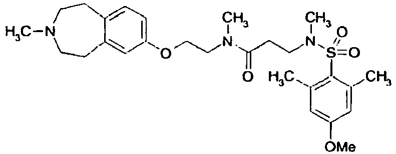

C₂₇H₃₉N₃O₅S x HCl (554.14)
[M+H]+ = 518
HPLC (Methode 5): Retentionszeit = 1.36 min

### Beispiel 593

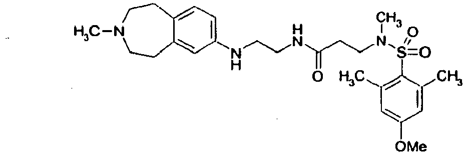

C₂₆H₃₈N₄O₄S x HCl (539.13)
[M+H]+ = 503
HPLC (Methode 5): Retentionszeit = 1.29 min

### Beispiel 594

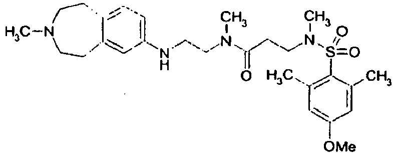

C₂₇H₄₀N₄O₄S x HCl (553.16)
[M+H]+ = 517
HPLC (Methode 5): Retentionszeit = 1.35 min

### Beispiel 595

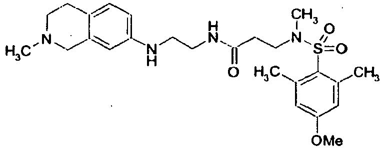

C₂₅H₃₆N₄O₄S x HCl (525.10)
[M+H]+ = 489
HPLC (Methode 5): Retentionszeit = 1.31 min

### Beispiel 596

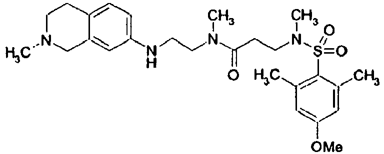

C₂₆H₃₈N₄O₄S x HCl (539.13)
[M+H]+ = 503
HPLC (Methode 5): Retentionszeit = 1.35 min

### Beispiel 597

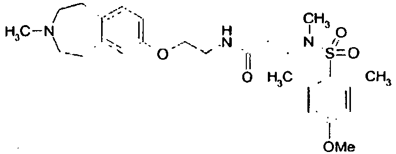

C₂₆H₃₇N₃O₅S x HCl (540.12)
[M+H]+ = 504
HPLC (Methode 10): Retentionszeit = 1.18 min

### Beispiel 598

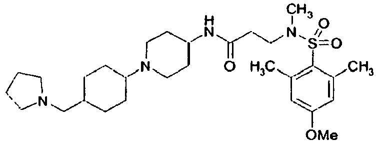

C₂₉H₄₈N₄O₄S x C₂HF₃O₂ (662.81)
[M+H]+ = 549
HPLC (Methode 9): Retentionszeit = 1.27 min

### Beispiel 599

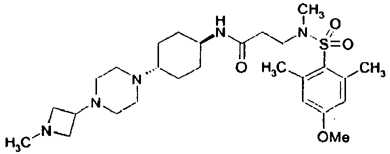

C₂₇H₄₅N₅O₄S x 3HCl (645.13)
[M+H]+ = 536
HPLC (Methode 5): Retentionszeit = 1.14 min

### Beispiel 600

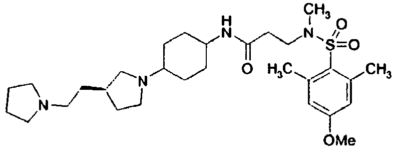

C₂₉H₅₀N₄O₄S x 2HCl (621.70)
[M+H]+ = 549
HPLC (Methode 5): Retentionszeit = 1.16 min

### Beispiel 601

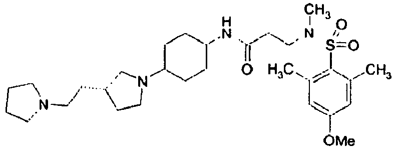

C₂₉H₄₈N₄O₄S (548.78)
[M+H]+ = 549
HPLC (Methode 5): Retentionszeit = 1.16 min

### Beispiel 602

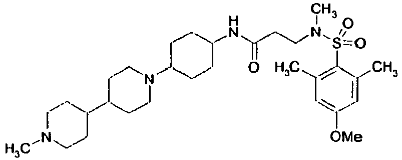

C₃₀H₅₀N₄O₄S x C₂HF₃O₂ (676.83)
[M+H]+ = 563
HPLC (Methode 9): Retentionszeit = 1.14 min

### Beispiel 608

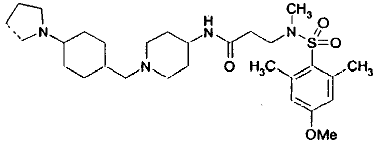

C₂₉H₄₈N₄O₄S x C₂HF₃O₂ (662.81)
[M+H]+ = 549
HPLC (Methode 9): Retentionszeit = 1.30 min

### Beispiel 609

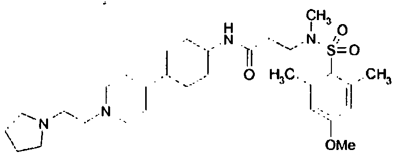

C₃₀H₅₀N₄O₄S x 2HCl (635.73)
[M+H]+ = 563
HPLC (Methode 11): Retentionszeit = 1.70 min

### Beispiel 610

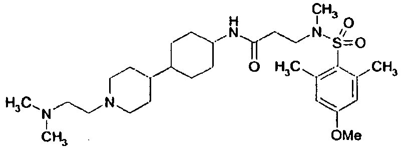

C₂₈H₄₈N₄O₄S x 2HCl (609.69)
[M+H]+ = 537
HPLC (Methode 11): Retentionszeit = 1.67 min

### Beispiel 611

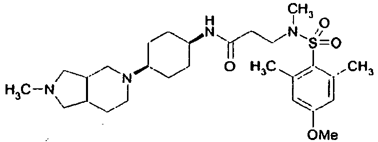

C₂₇H₄₄N₄O₄S x 2HCl (593.65)
[M+H]+ = 521
HPLC (Methode 11): Retentionszeit = 1.61 min

### Beispiel 636

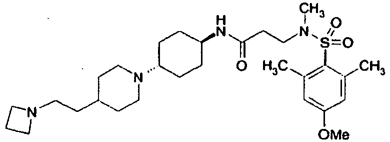

C₂₉H₄₈N₄O₄S x 2HCl (621.70)
[M+H]+=549
HPLC (Methode 4): Retentionszeit = 2.39 min

### Beispiel 637

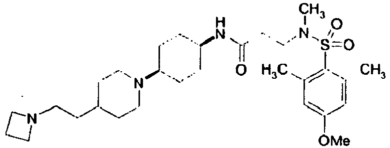

C₂₉H₄₈N₄O₄S x 2HCl (621.70)
[M+H]+ = 549
HPLC (Methode 4): Retentionszeit = 2.34 min

### Beispiel 549

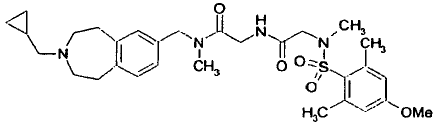

C₃₁H₄₄N₄O₅S x HCl (621.23)
[M+H]+ = 585
HPLC (Methode 12): Retentionszeit = 3.0 min

### Beispiel 551

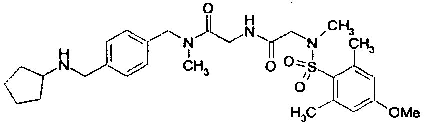

C₂₉H₄₂N₄O₅S x HCl (595.19)
[M+H]+ = 559
DC: Kieselgel, Dichlormethan / Methanol / Ammoniak 9:1:0.1, Rf-Wert = 0.32

### Beispiel 563

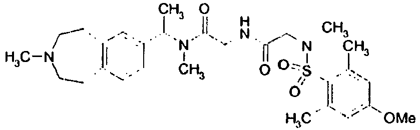

C₂₉H₄₂N₄O₅S x HCl (595.19)
[M+H]+ = 559
HPLC (Methode 12): Retentionszeit = 2.8 min

### Beispiel 606

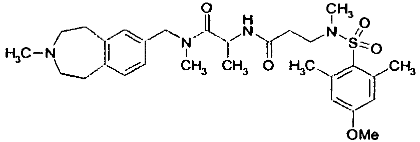

C₂₉H₄₂N₄O₅S x HCl (595.19)
[M+H]+ = 559
DC: Kieselgel, Dichlormethan / Methanol / Ammoniak 8:2:0.01, Rf-Wert = 0.68

Die folgende Verbindung wurde analog zu Beispiel 138 hergestellt:

### Beispiel 573

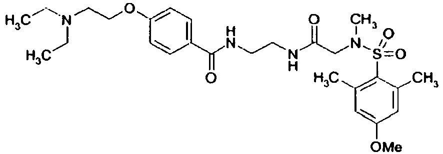

### 573a)

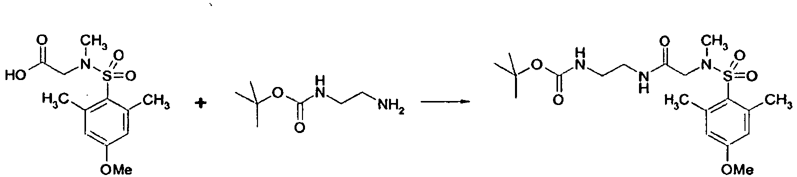

573a wird analog zu 1f aus 2.16 g (7.50 mMol) Produkt aus 121b, 1.20 g (7.50 mMol) N-Bocethylendiamin (Fluka), 3.14 ml (22.50 mMol) Triethylamin und 2.41 g (7.50 mMol) TBTU in 28 ml THF und 4 ml DMF hergestellt.
C₁₉H₃₁N₃O₆S (429.53)
DC: Kieselgel, Dichlormethan / Ethanol 19:1, Rf-Wert = 0.35

### 573b)

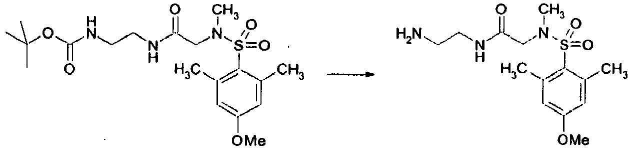

573b wird analog zu 28d aus 2.70 g (6.29 mMol) Produkt aus 573a und 7 ml TFA in 50 ml Dichlormethan hergestellt.
C₁₄H₂₃N₃O₄S (329.42)
DC: Kieselgel, Dichlormethan / Ethanol 9:1, Rf-Wert = 0.15

### 573c)

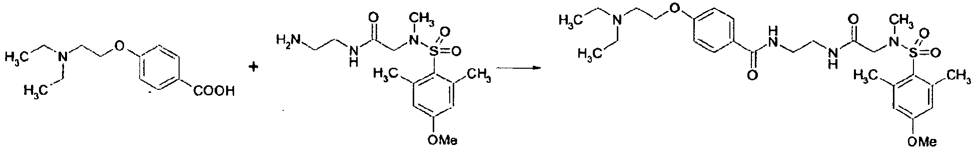

Beispiel 573 wird analog zu 1f aus 0.119 g (0.50 mMol) 4-(2-Diethylaminoethoxy)-benzoesäure (J. Med. Chem. 14, 1971, 836-842), 0.165 g (0.50 mMol) Produkt aus 573b, 0.21 ml (1.50 mMol) Triethylamin und 0.16 g (0.50 mMol) TBTU in 7 ml THF und 1 ml DMF hergestellt.
C₂₇H₄₀N₄O₆S x HCl (585.16)
HPLC (Methode 5): Retentionszeit = 1.44 min

Die folgenden Verbindungen wurde analog zu Beispiel 573 hergestellt:

### Beispiel 607

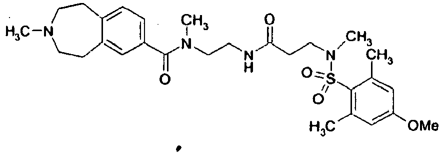

C₂₈H₄N₄O₅S x HCl (581.17)
[M+H]+ = 545
HPLC (Methode 12): Retentionszeit = 3.51 min

Die nachfolgenden Beispiele beschreiben pharmazeutischer Darreichungsformen, die als Wirkstoff eine beliebige Verbindung der allgemeinen Formel I enthalten:

### Beispiel I

Trockenampulle mit 75 mg Wirkstoff pro 10 ml

### Zusammensetzung:

| | |
|---|---|
| Wirkstoff | 75.0 mg |
| Mannitol | 500 mg |
| Wasser für Injektionszwecke ad | 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel II

Tablette mit 50 mg Wirkstoff

### Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215,0 mg |

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 9 mm.

### Beispiel III

Tablette mit 350 mg Wirkstoff

### Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 12 mm.

### Beispiel IV

Kapseln mit 50 mg Wirkstoff

### Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

### Beispiel V

Kapseln mit 350 mg Wirkstoff

### Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Gr6Be 0 abgefüllt.

### Beispiel VI

Suppositorien mit 100 mg Wirkstoff

### 1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

## Patentansprüche

1. Verbindungen, nämlich 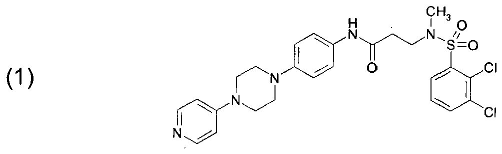 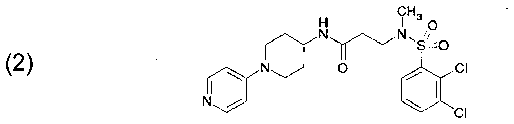 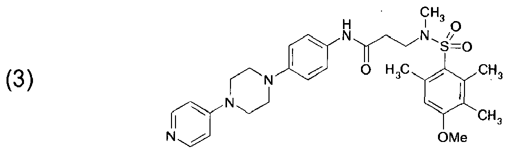 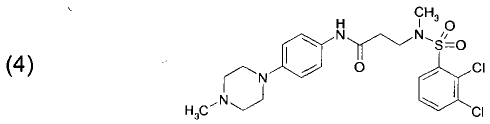 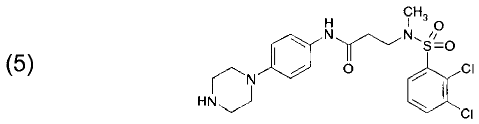 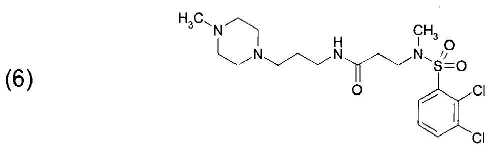 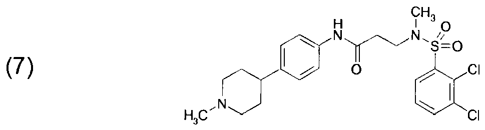 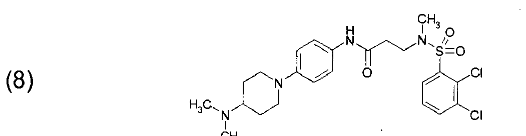 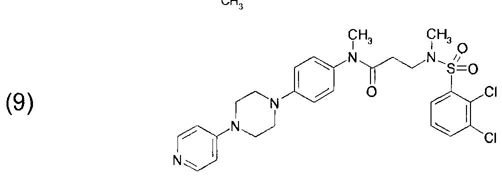 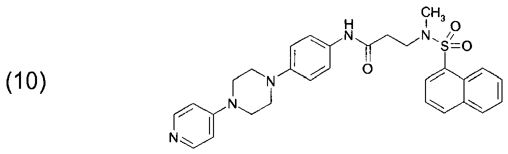 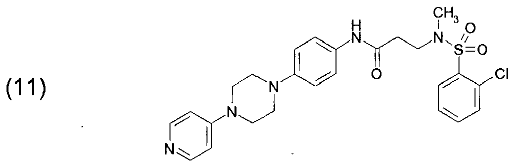 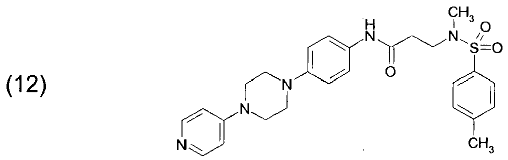 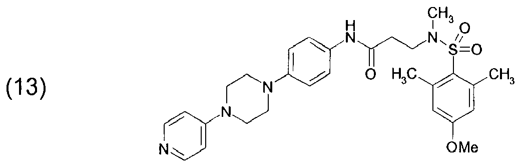 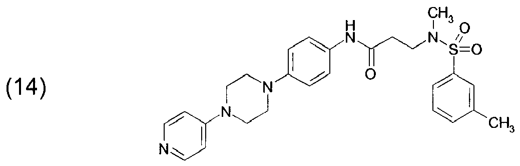 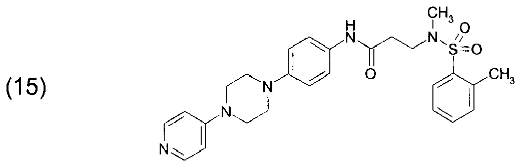 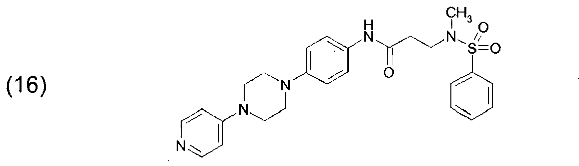 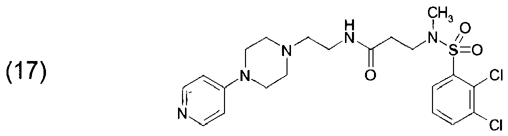 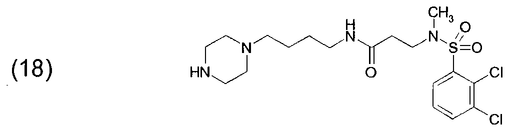 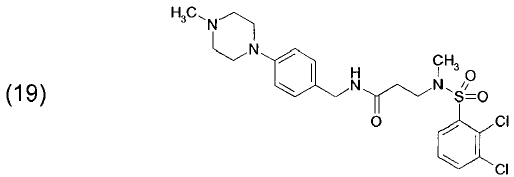 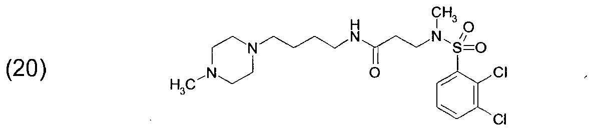 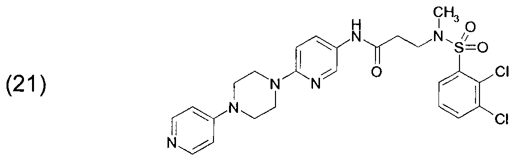 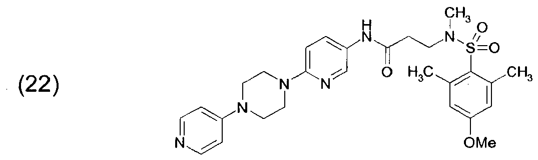 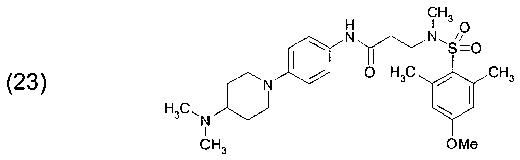 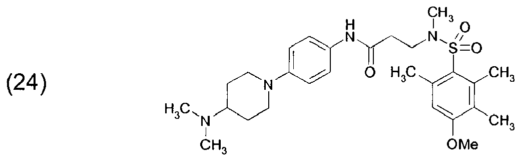 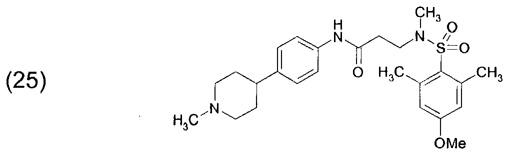 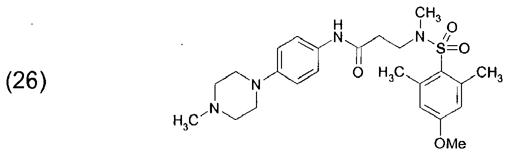 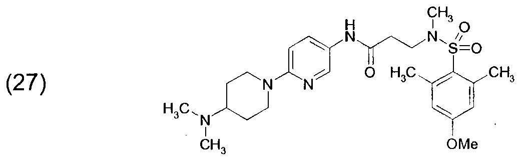 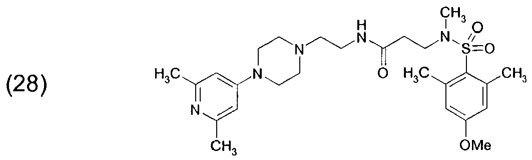 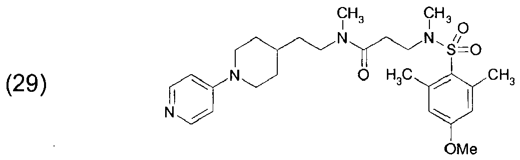 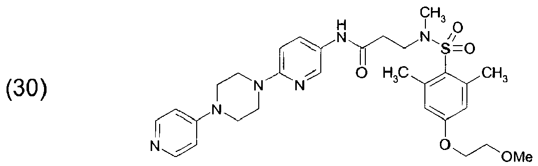 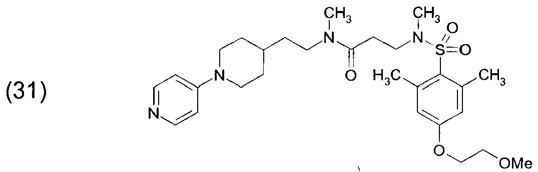 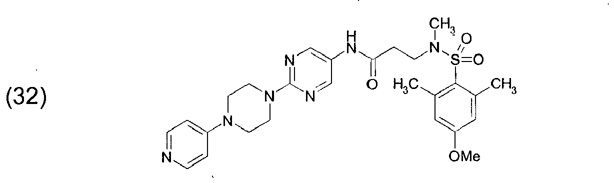 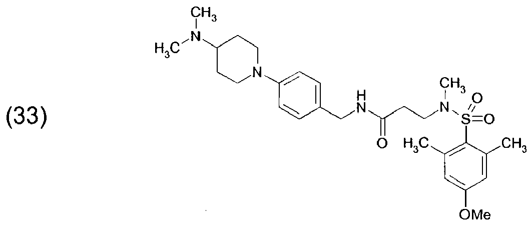 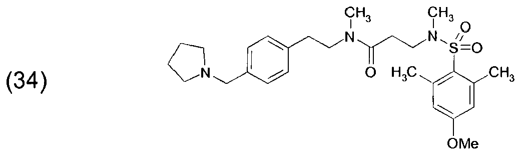 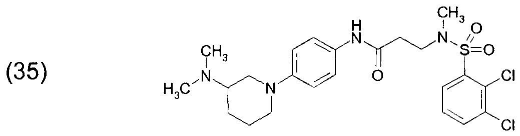 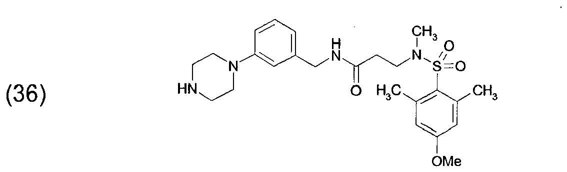 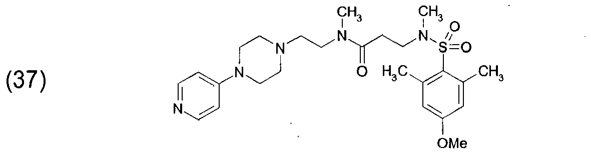 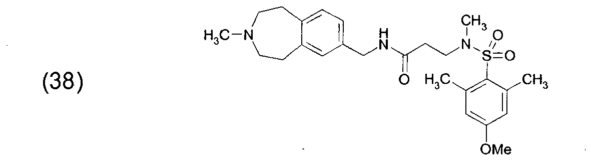 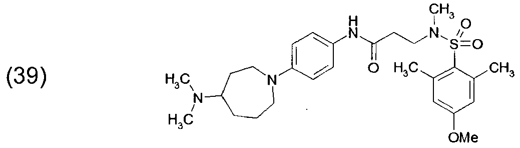 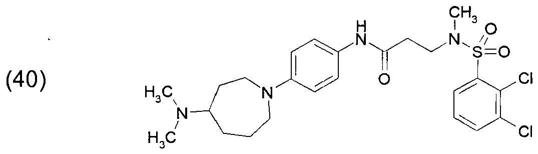 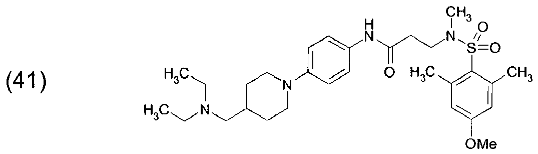 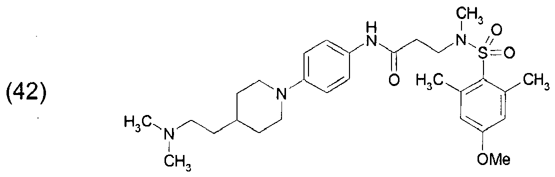 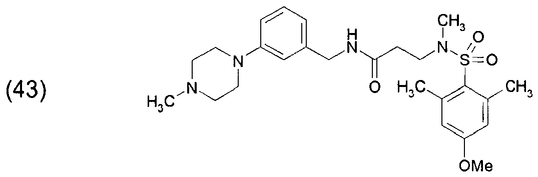 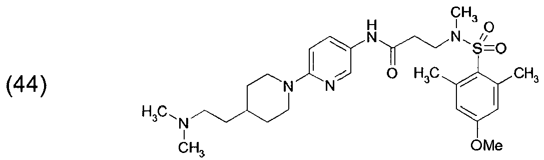 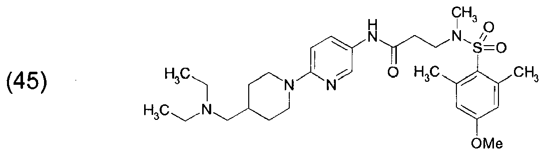 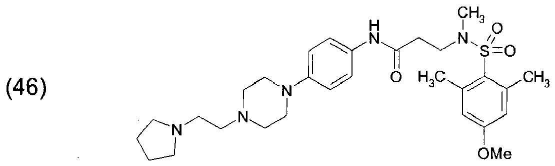 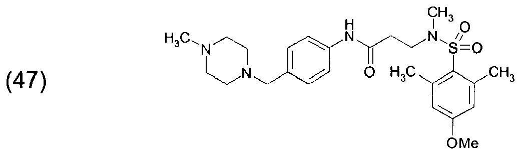 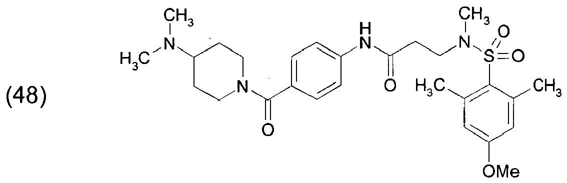 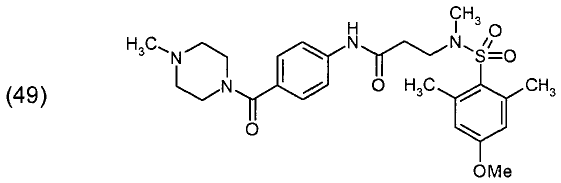 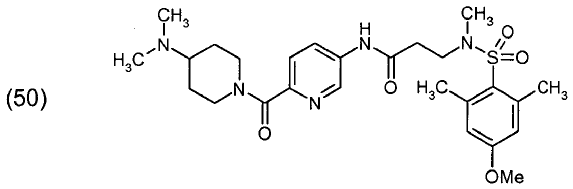 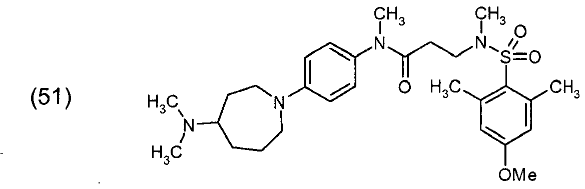 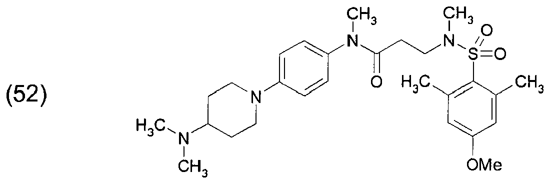 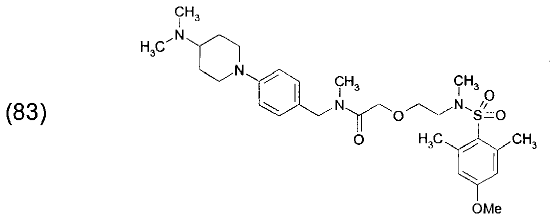 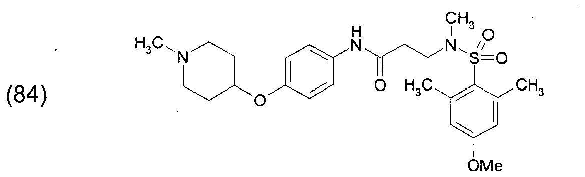 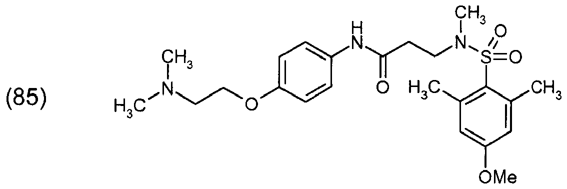 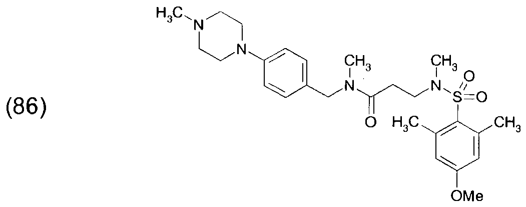 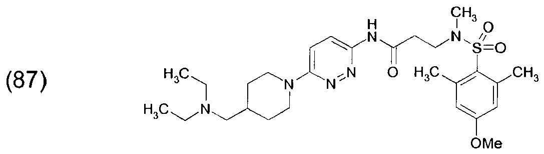 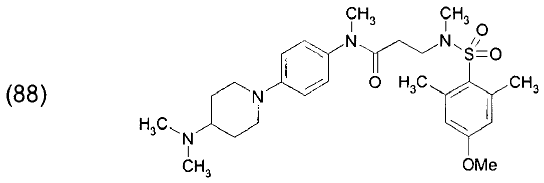 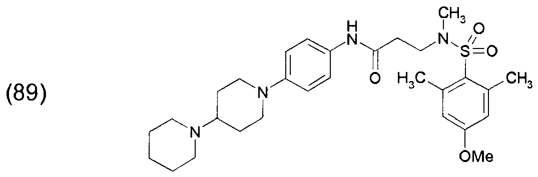 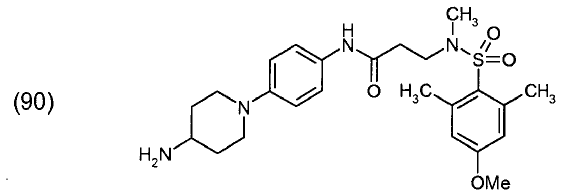 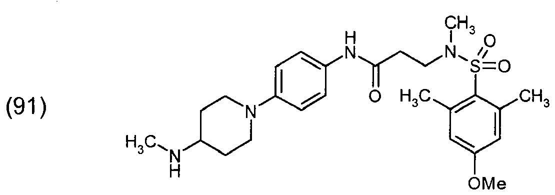 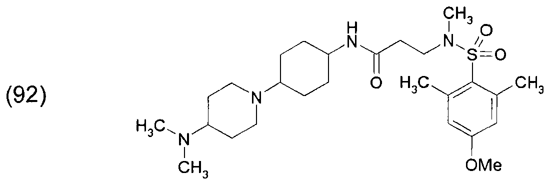 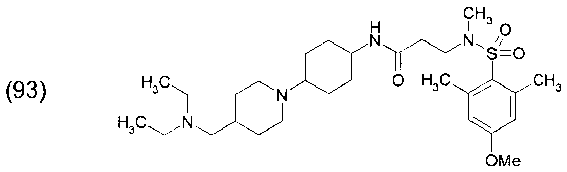 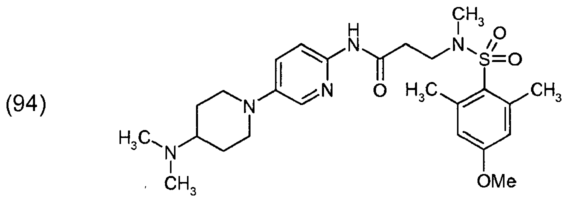 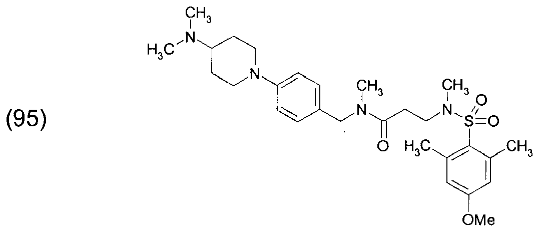 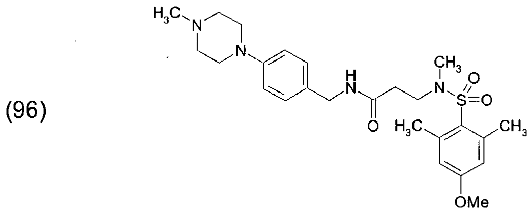 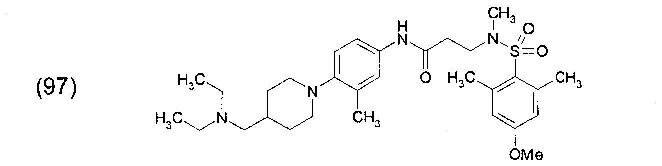 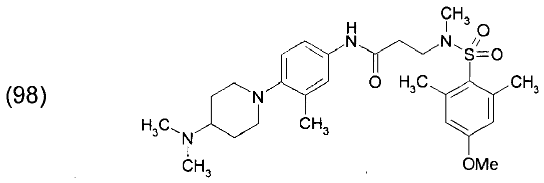 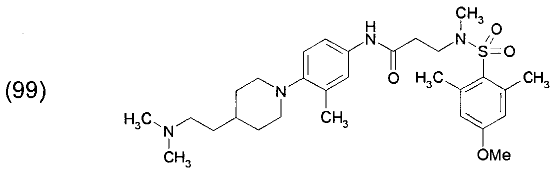 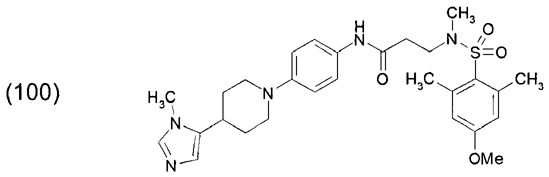 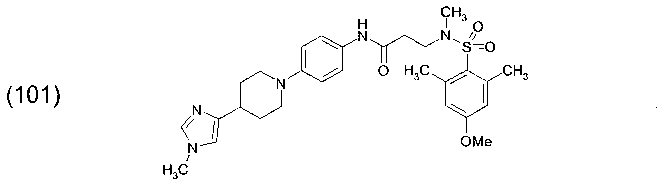 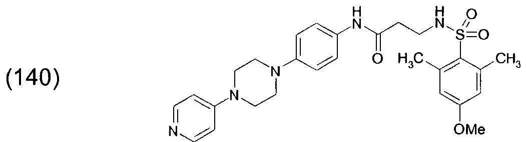 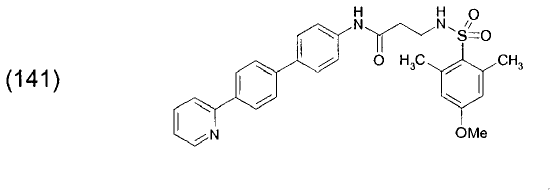 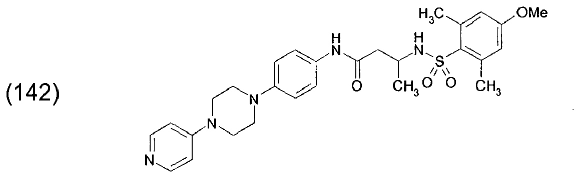 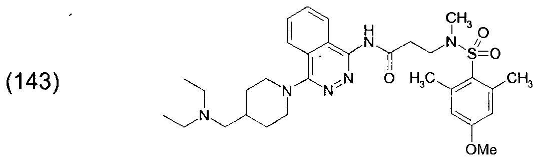 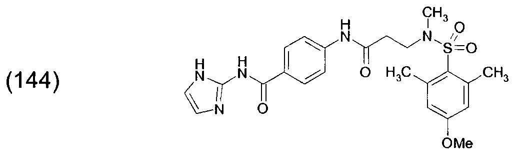 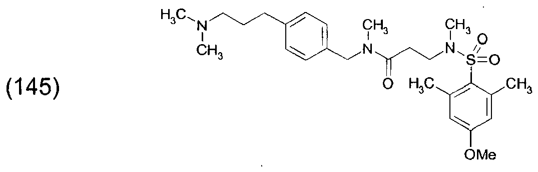 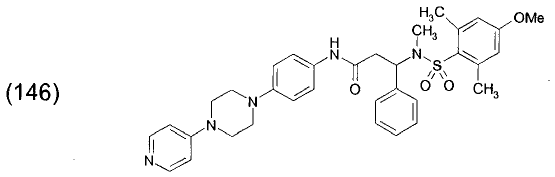 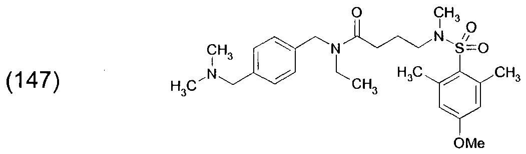 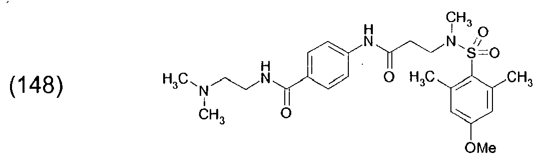 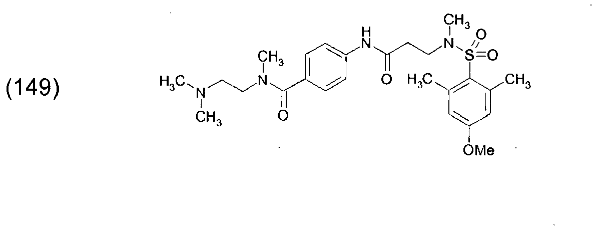 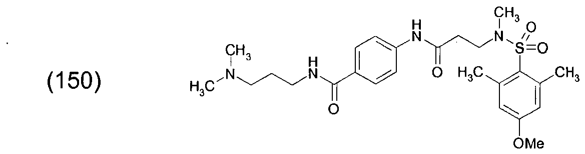 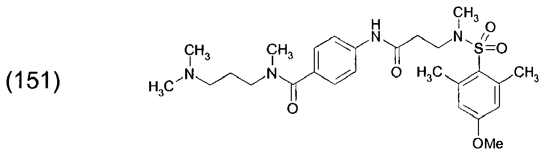 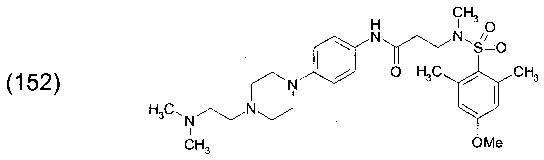 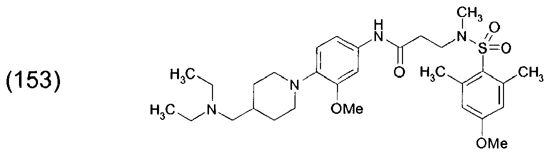 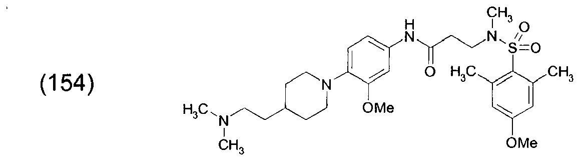 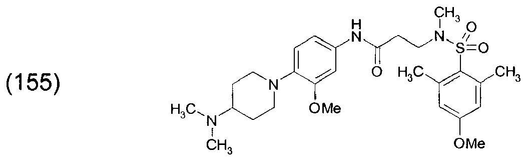 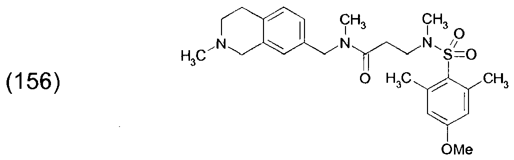 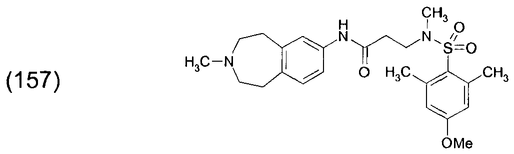 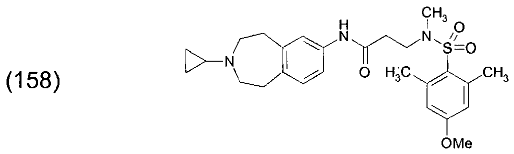 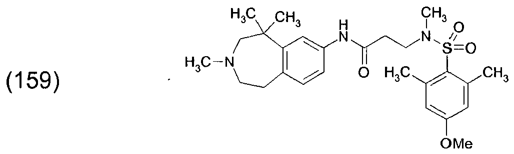 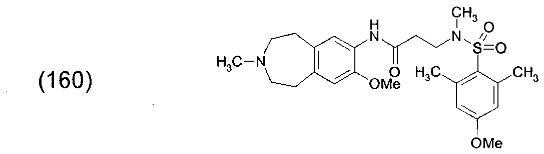 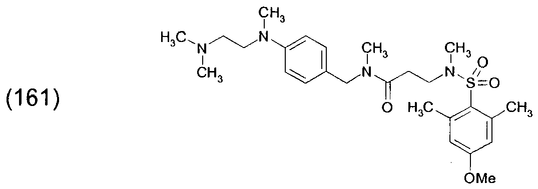 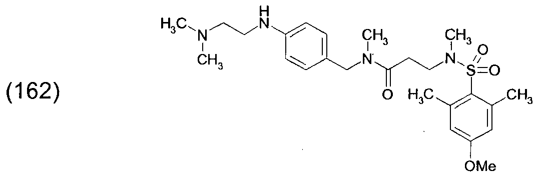 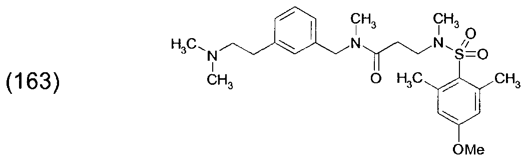 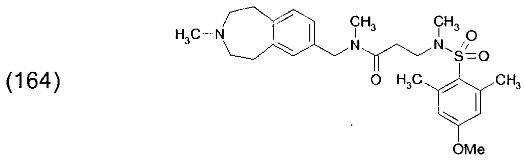 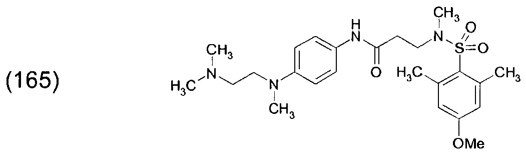 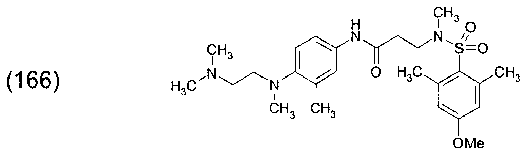 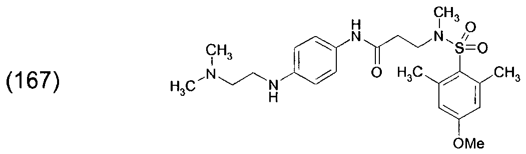 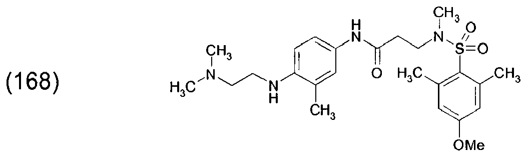 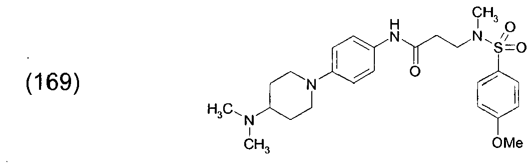 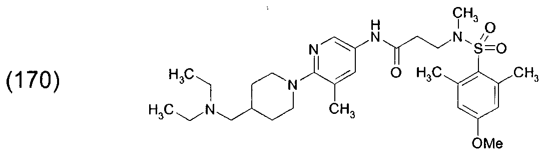 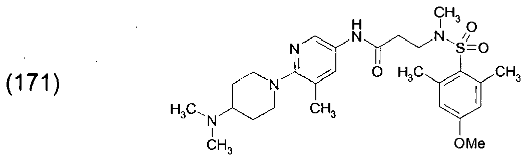 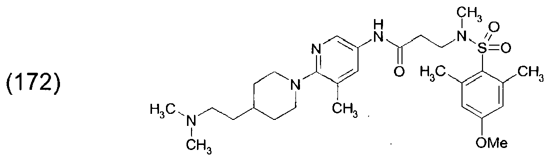 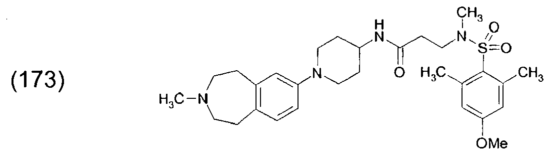 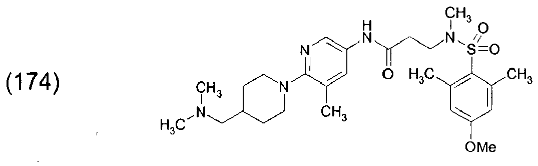 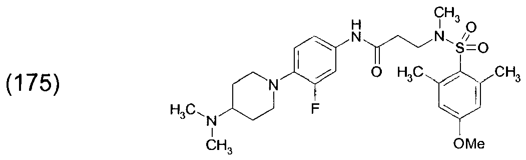 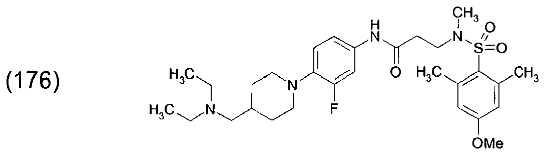 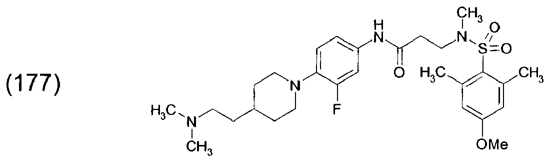 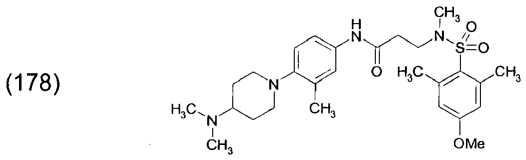 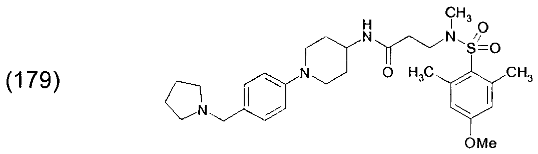 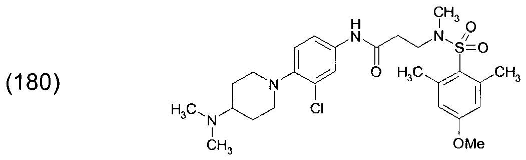 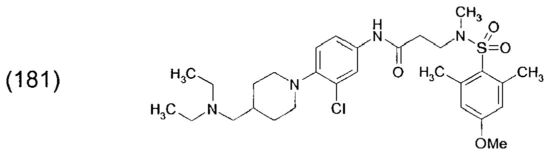 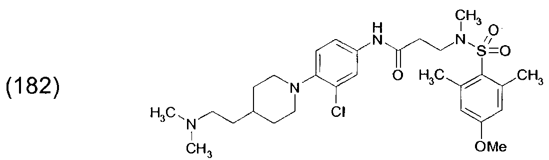 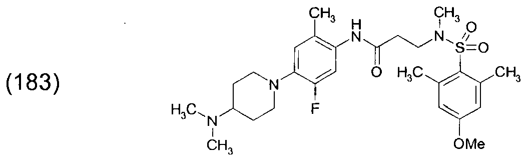 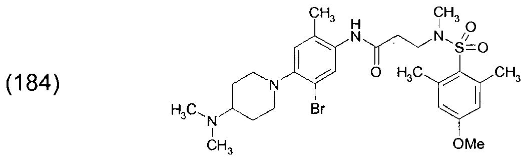 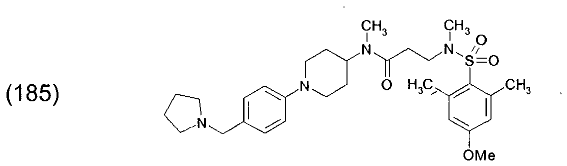 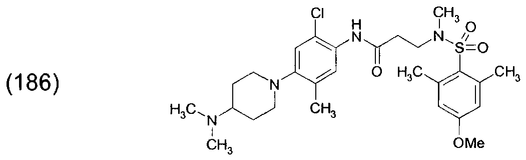 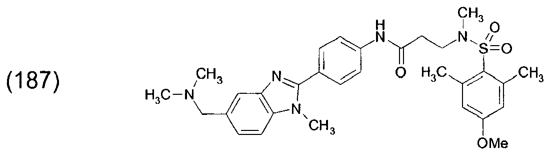 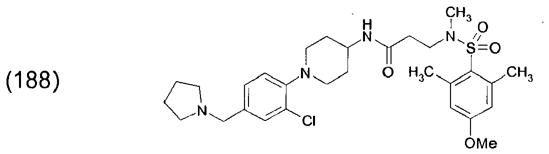 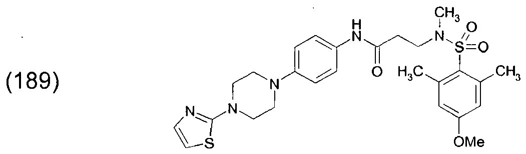 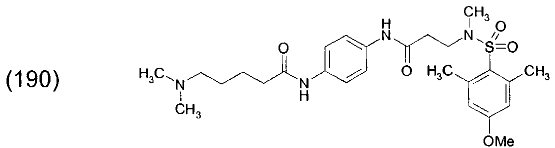 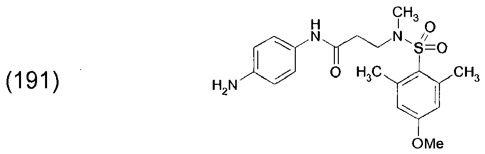 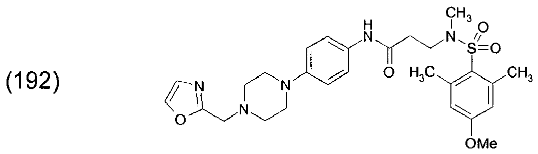 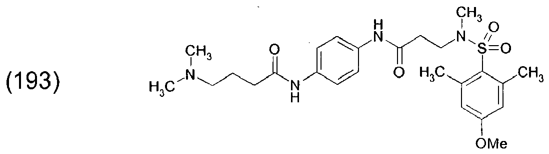 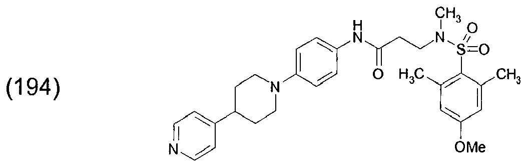 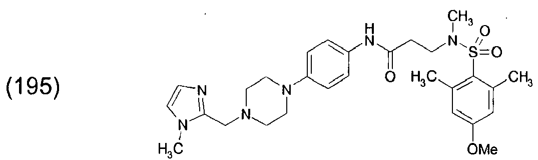 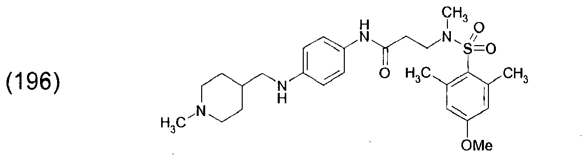 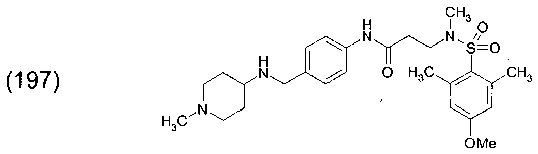 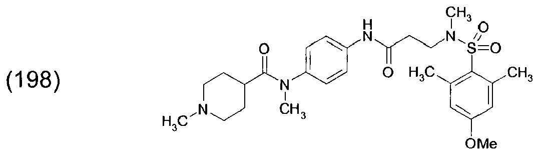 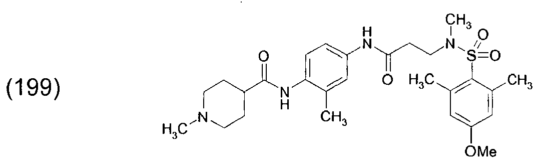 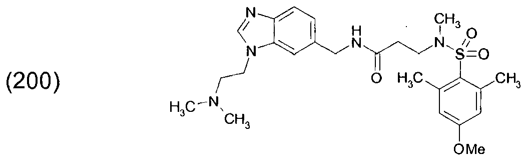 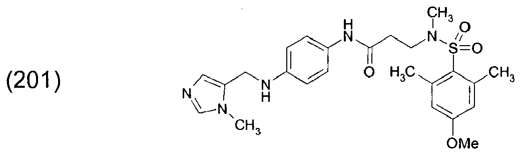 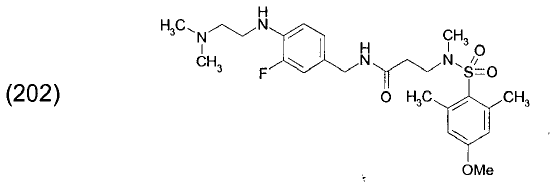 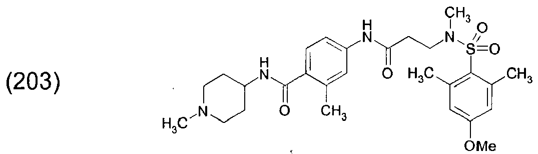 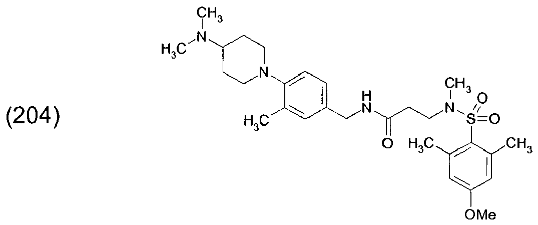 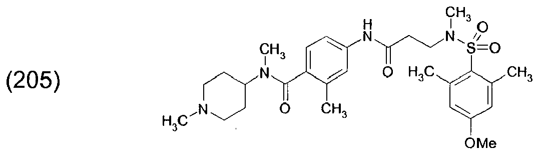 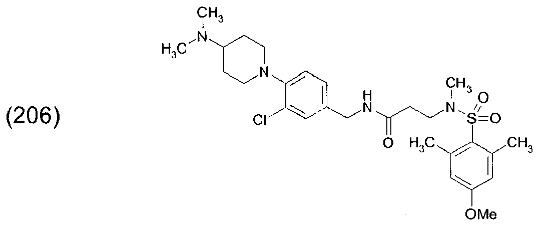 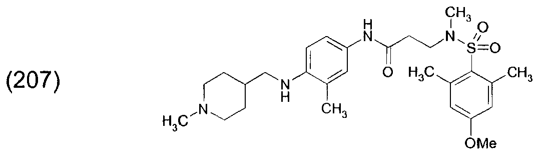 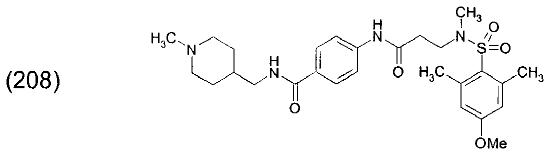 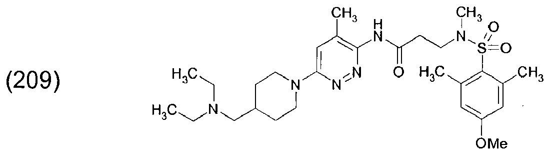 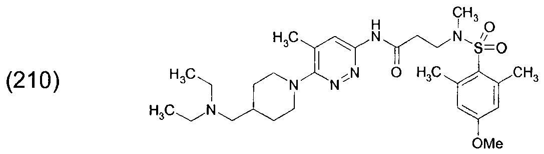 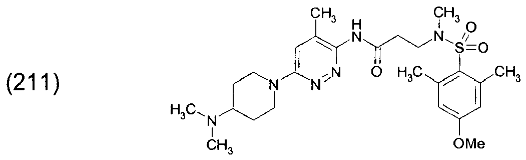 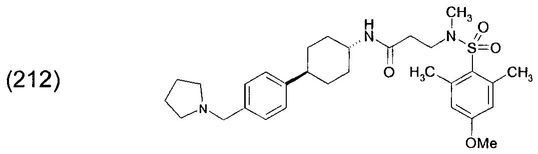 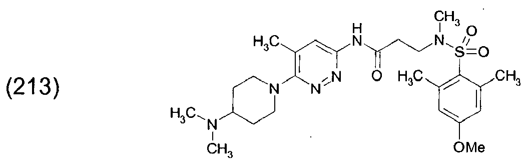 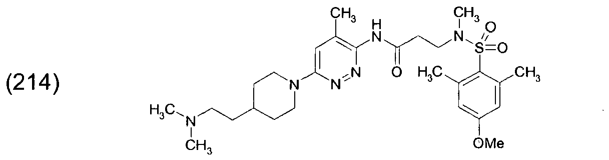 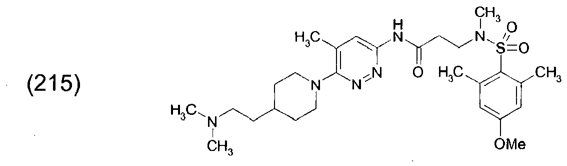 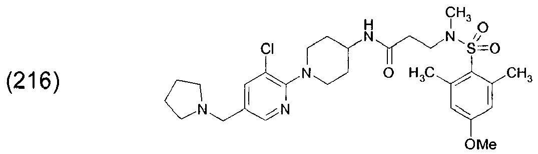 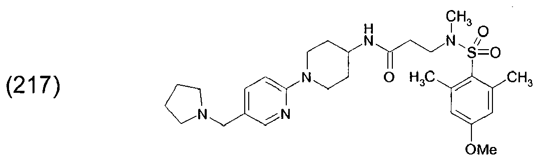 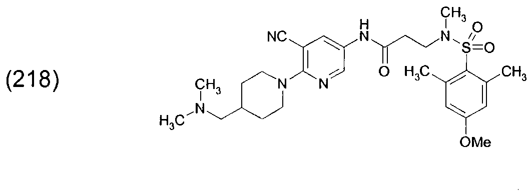 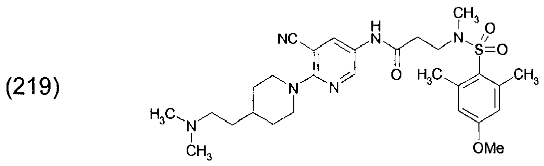 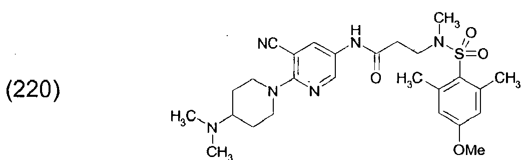 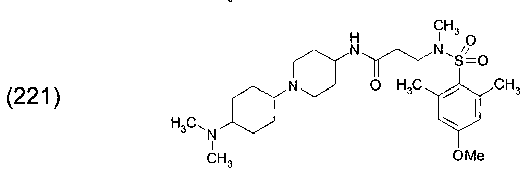 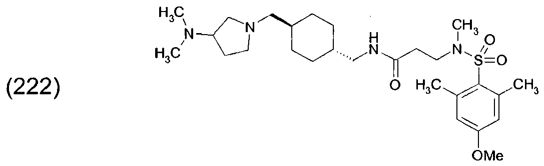 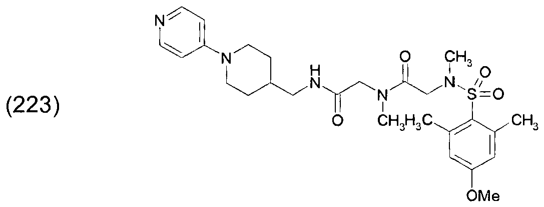 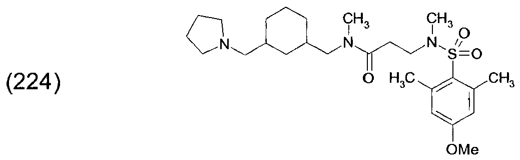 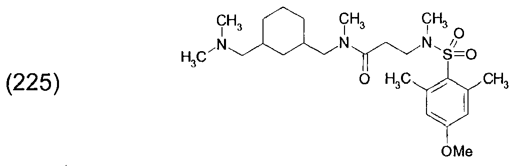 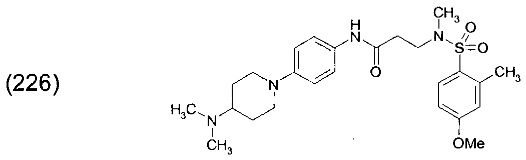 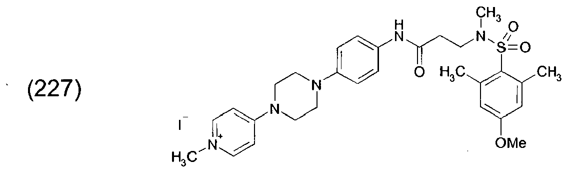 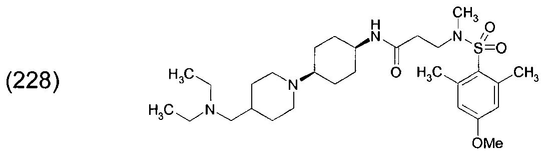 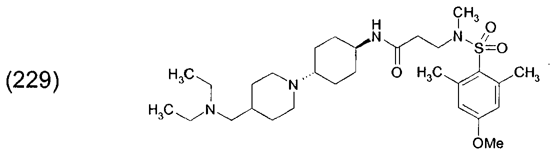 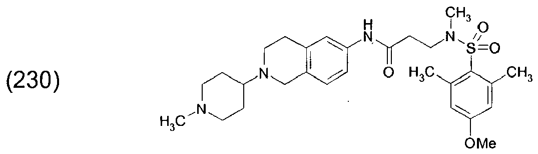 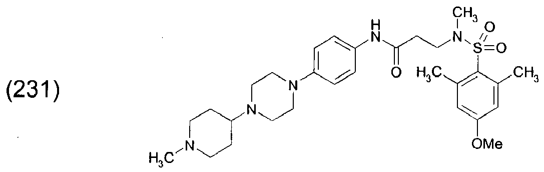 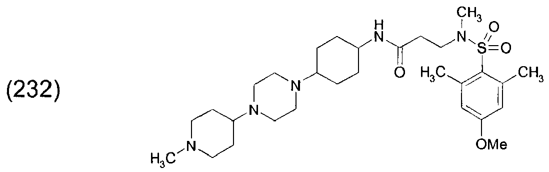 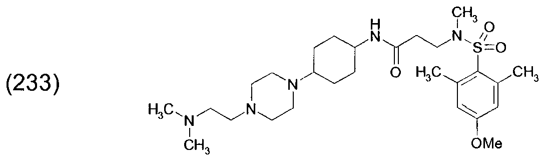 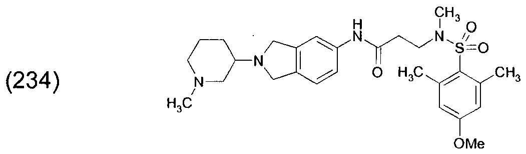 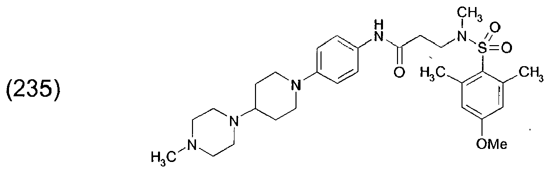 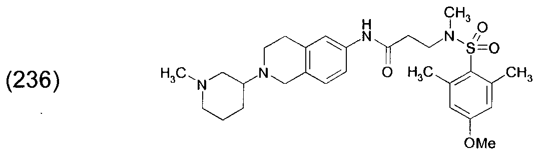 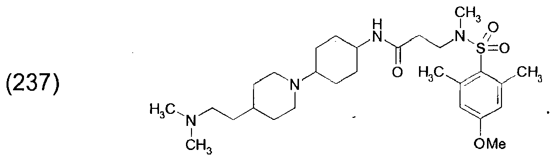 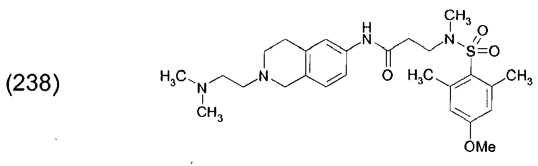 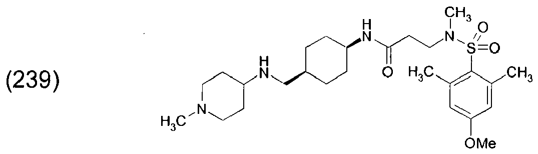 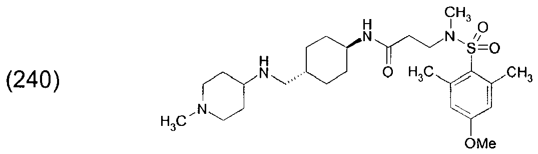 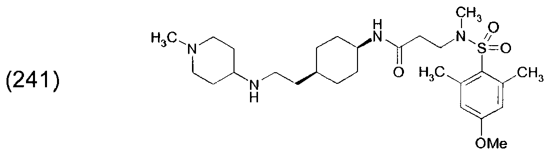 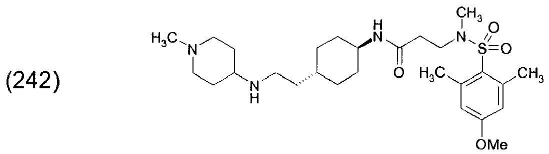 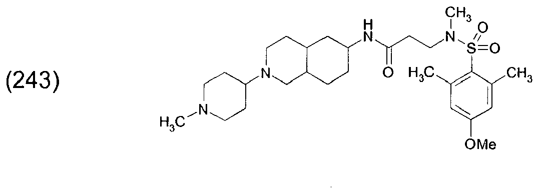 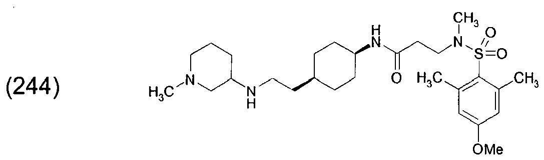 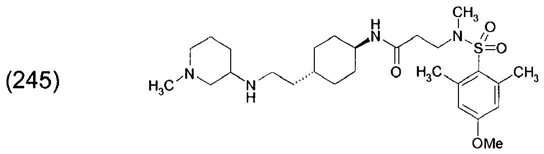 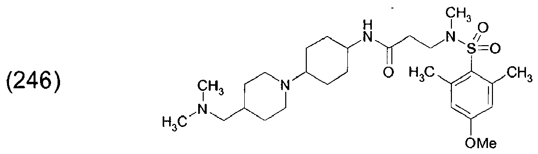 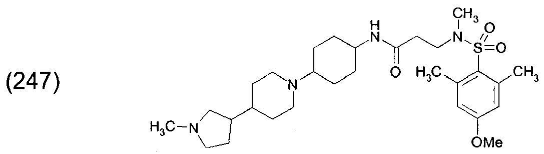 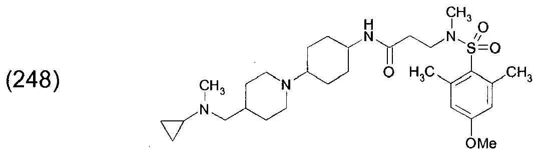 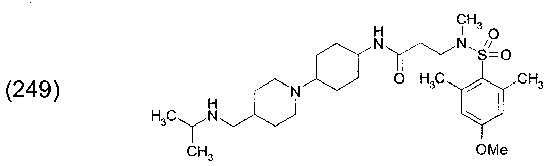 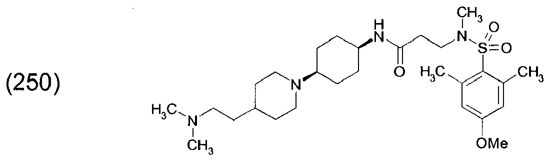 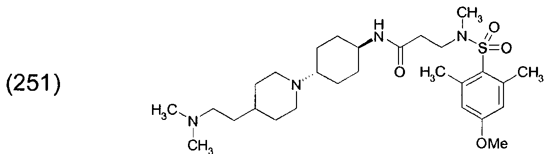 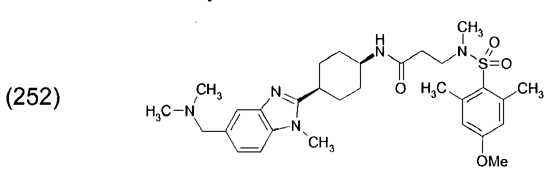 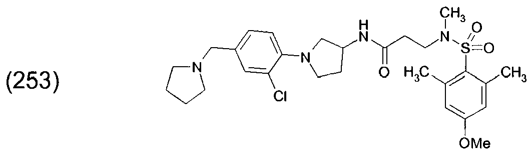 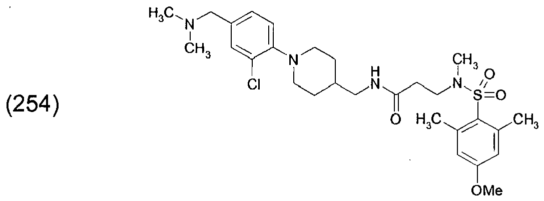 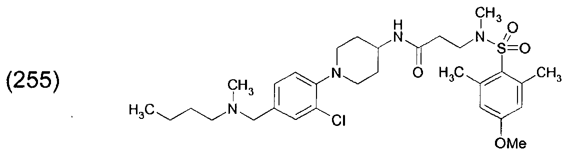 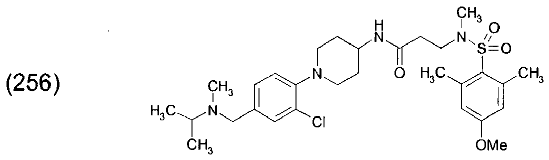 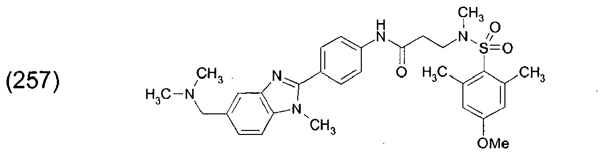 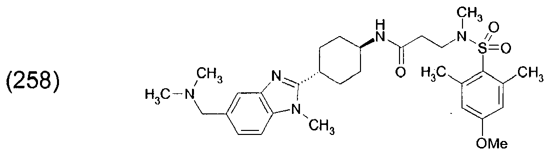 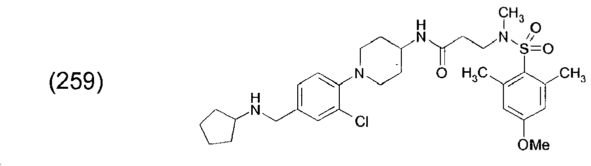 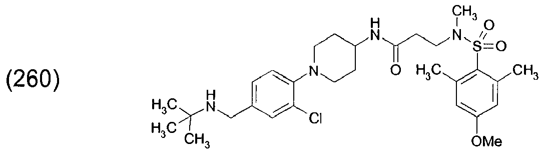 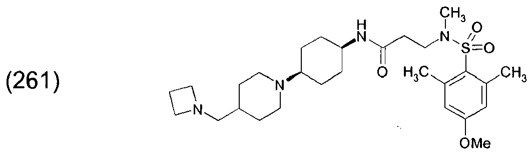 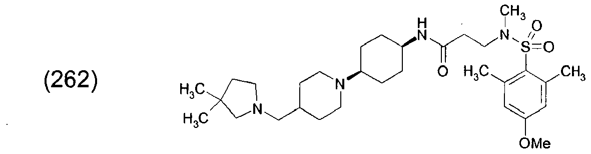 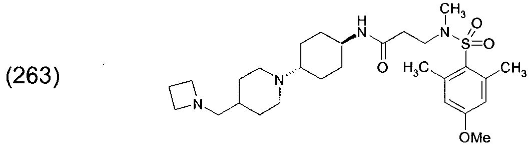 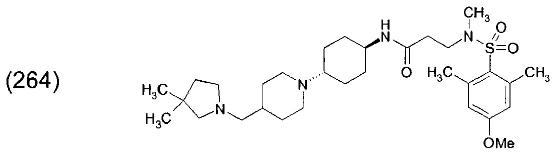 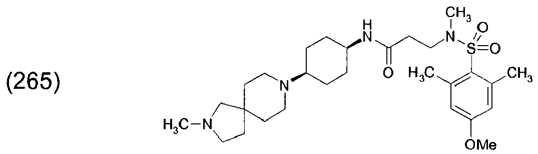 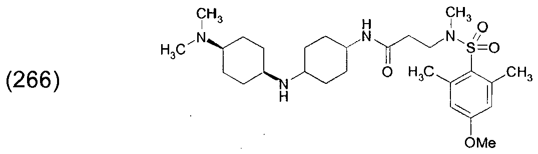 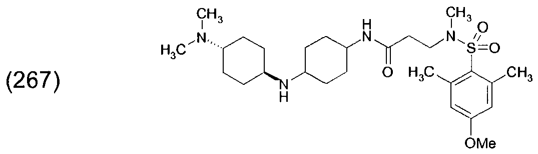 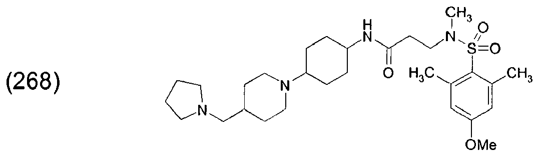 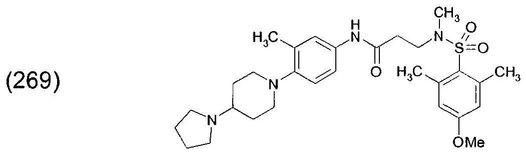 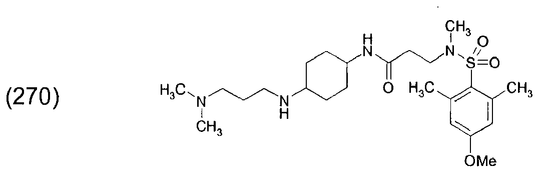 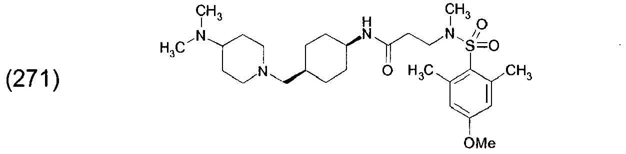 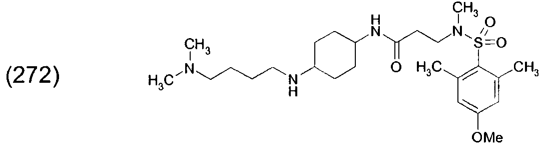 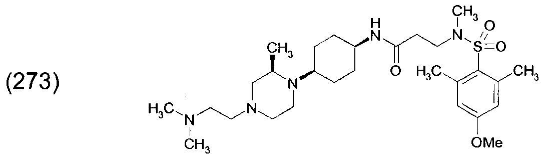 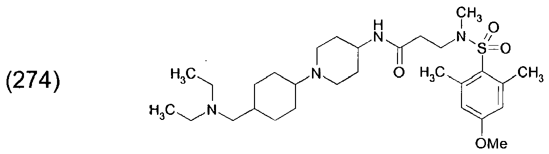 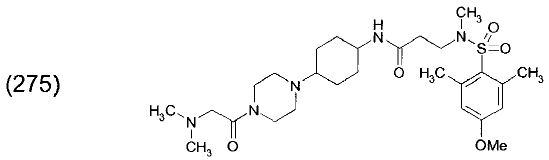 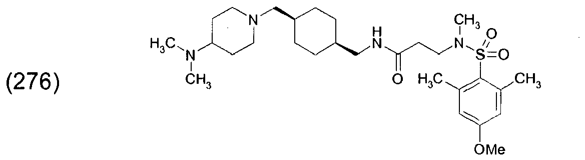 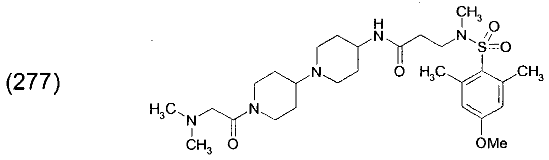 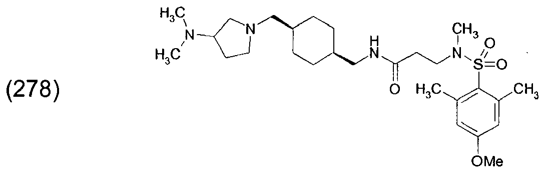 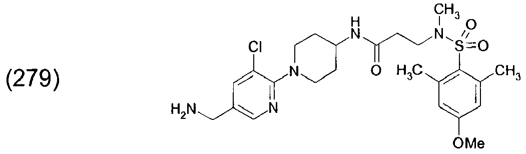 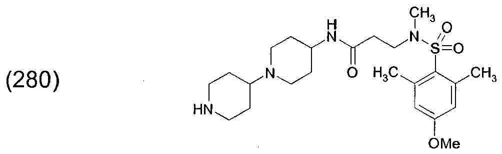 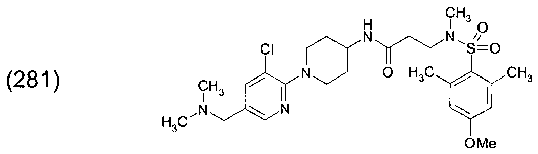 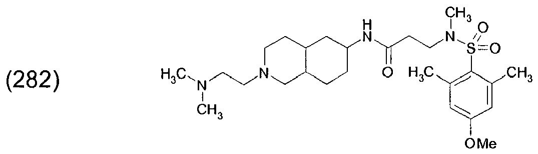 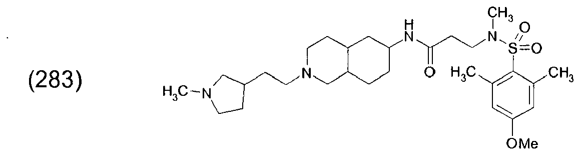 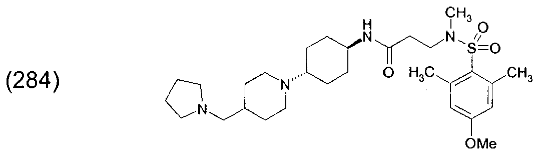 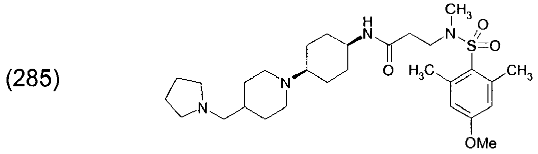 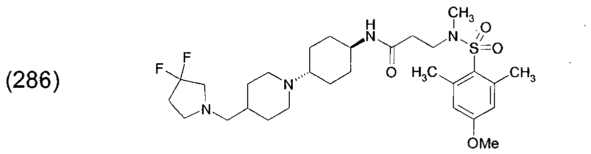 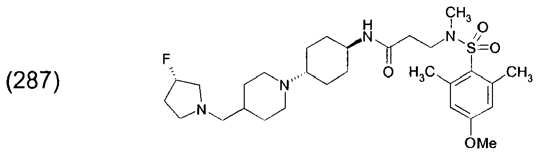 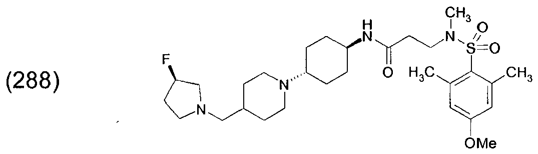 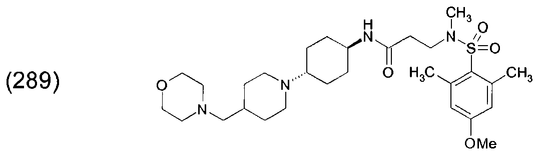 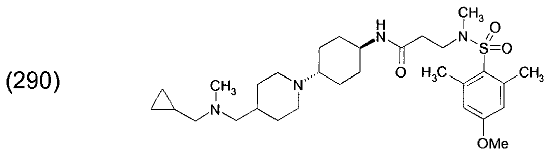 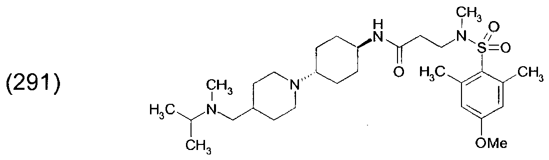 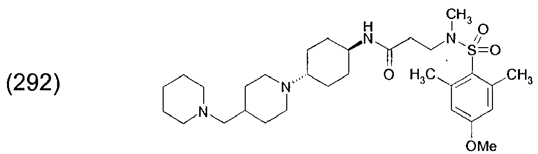 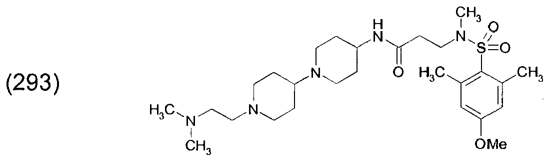 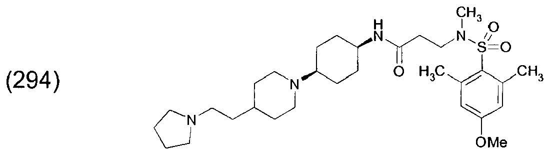 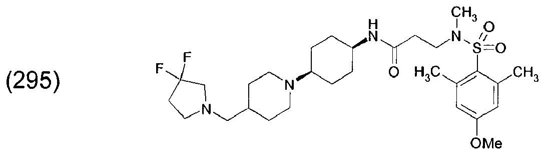 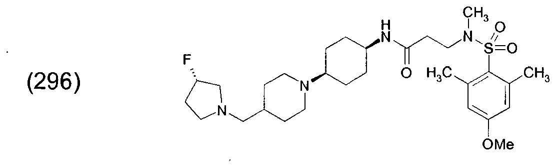 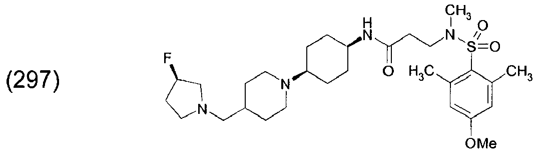 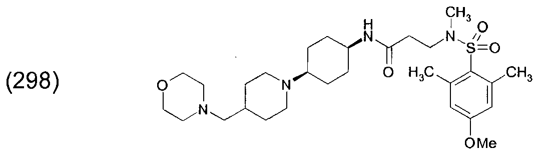 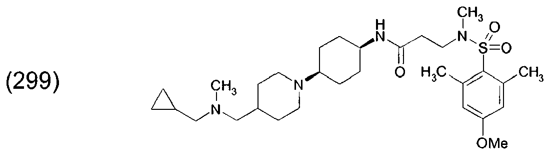 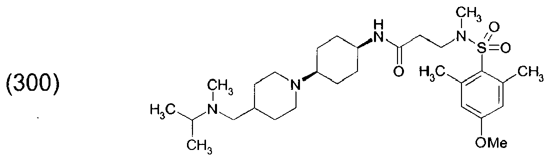 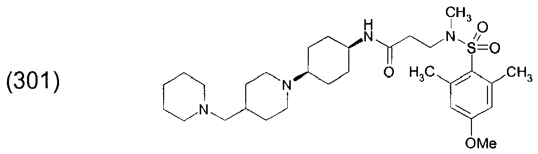 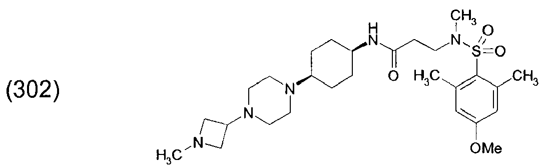 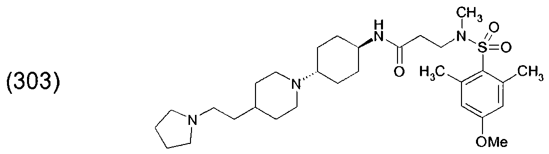 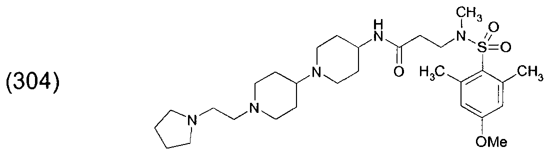 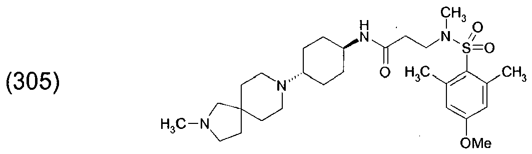 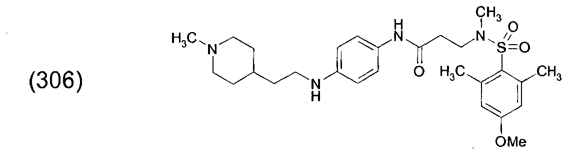 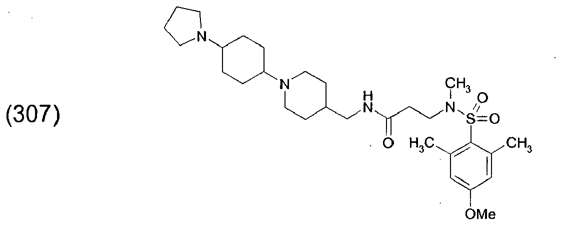 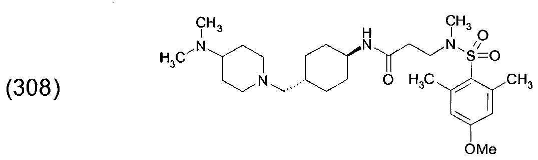 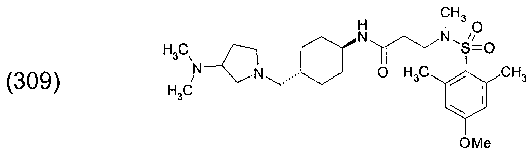 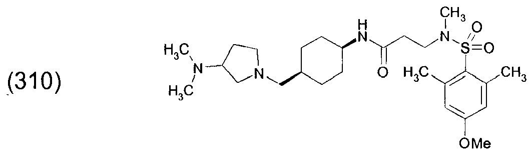 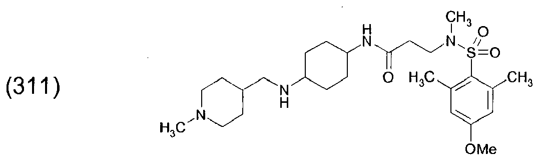 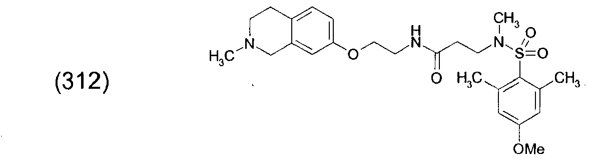 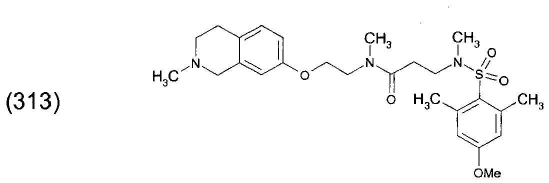 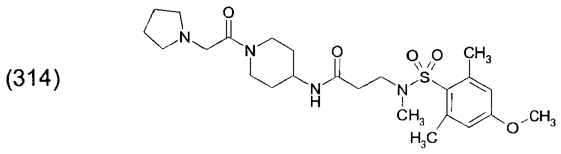 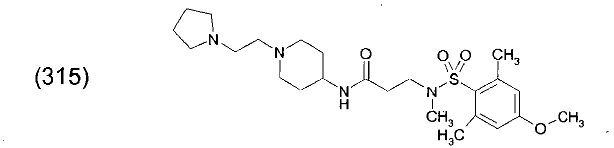 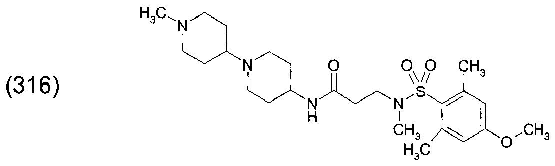 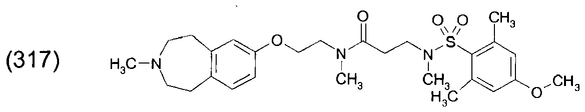 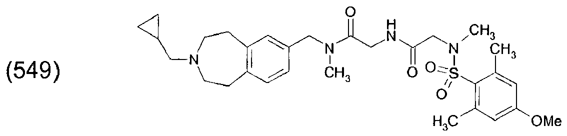 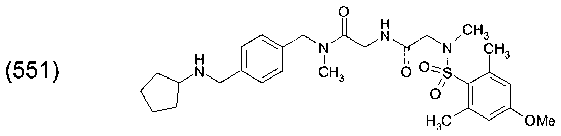 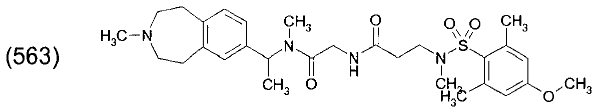 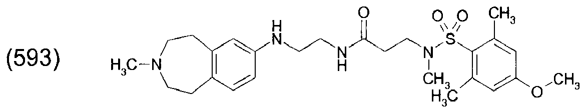 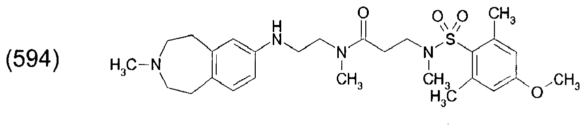 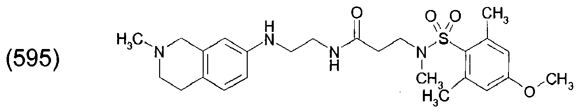 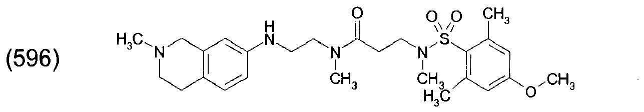 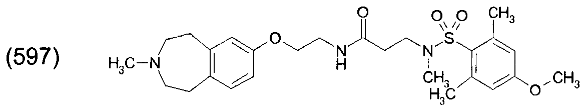 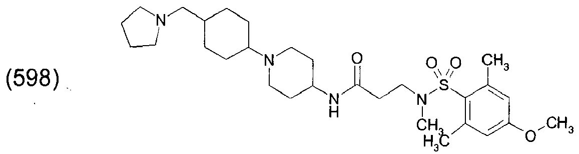 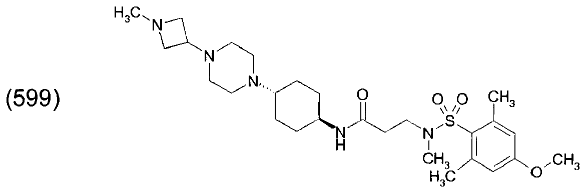 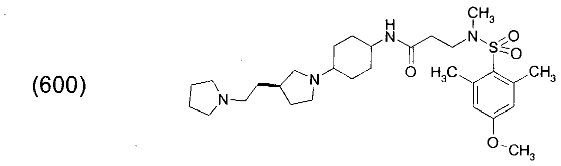 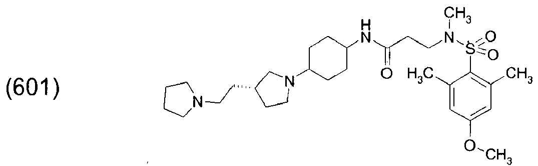 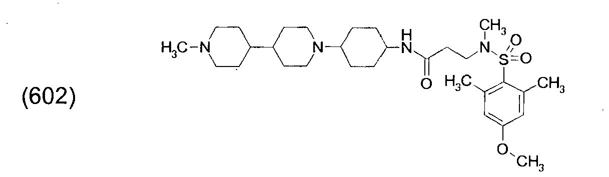 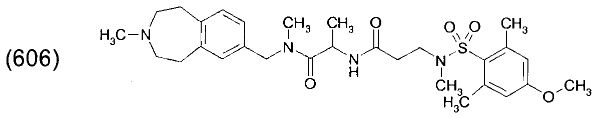 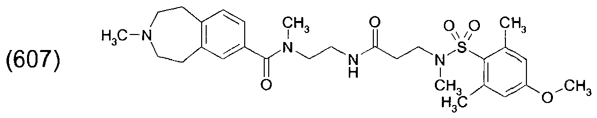 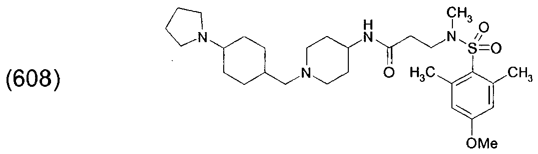 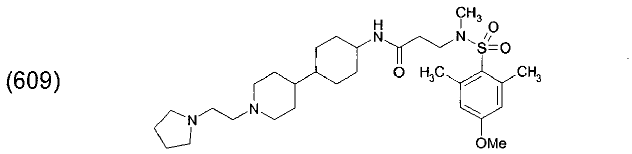 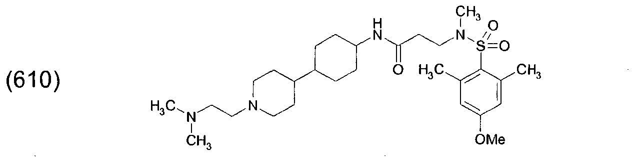 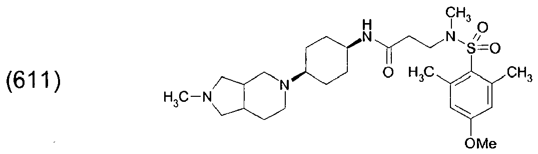 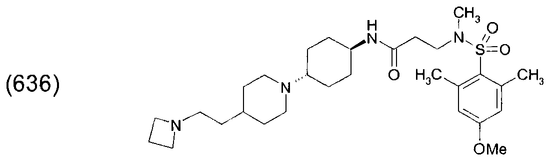 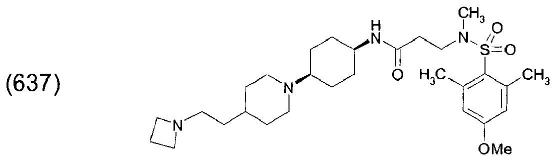 deren Enantiomere, deren Diastereomere, deren Gemische und deren Salze, insbesondere deren physiologisch verträgliche Salze mit organischen oder anorganischen Säuren oder Basen.

2. Physiologisch verträgliche Salze der Verbindungen nach Anspruch 1 mit anorganischen oder organischen Säuren oder Basen.

3. Arzneimittel, enthaltend eine Verbindung nach Anspruch 1 oder ein physiologisch verträgliches Salz gemäß Anspruch 2 neben gegebenenfalls einem oder mehreren inerten Trägerstoffen und/oder Verdünnungsmitteln.

4. Verbindungen nach Anspruch 1 oder 2 zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen und Kopfschmerz-Erkrankungen.

5. Verfahren zur Herstellung eines Arzneimittels gemäß Anspruch 3, **dadurch gekennzeichnet, dass** auf nichtchemischem Weg eine Verbindung nach mindestens einem der Ansprüche 1 bis 2 in einen oder mehrere inerte Trägerstoffe und/oder Verdünnungsmittel eingearbeitet wird.

## Claims

1. Compounds, namely 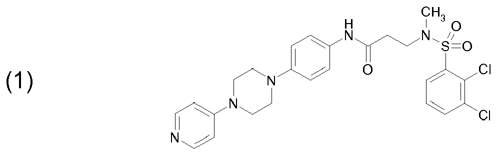 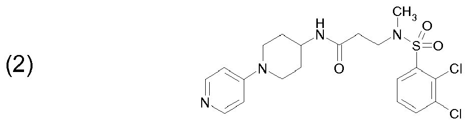 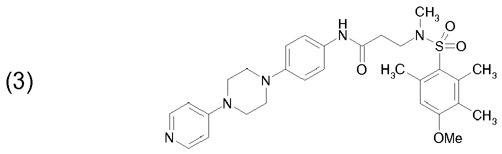 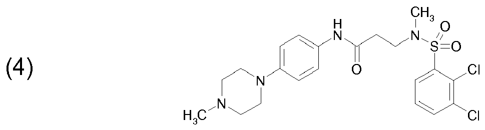 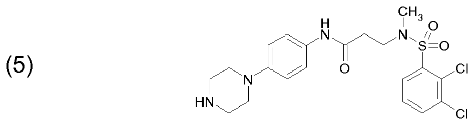 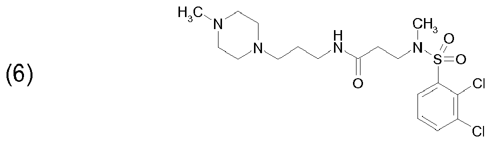 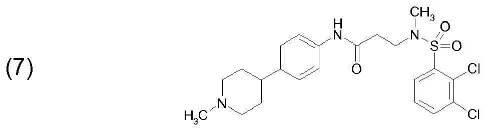 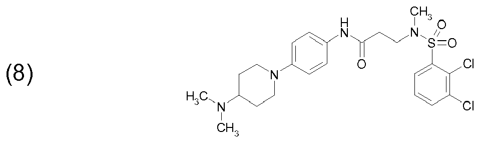 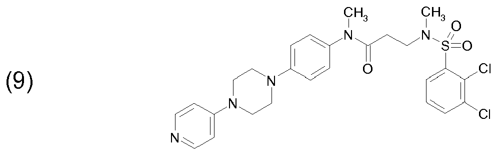 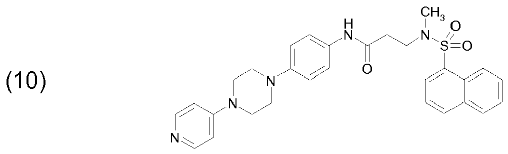 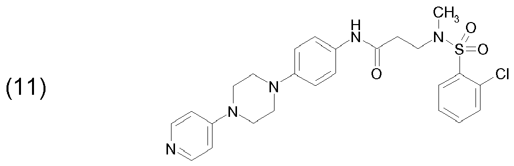 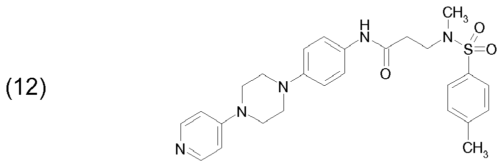 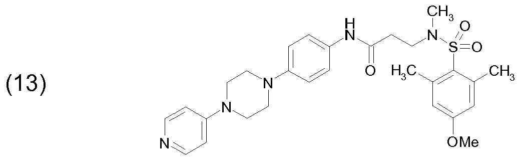 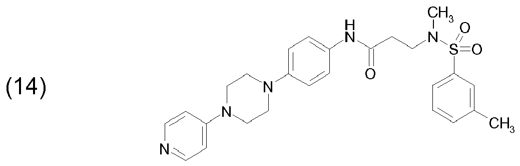 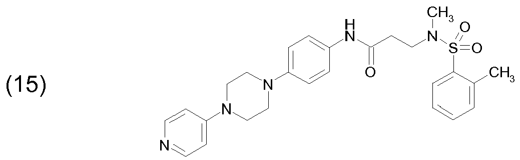 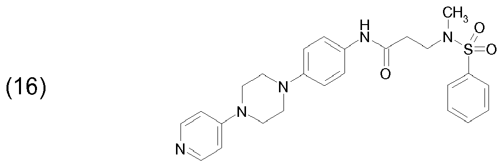 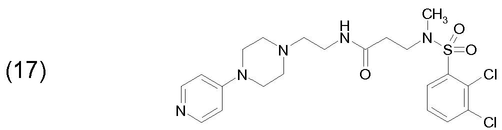 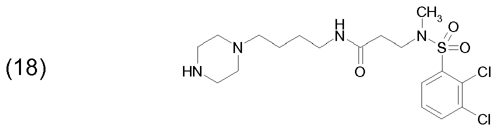 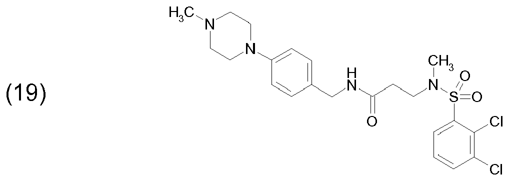 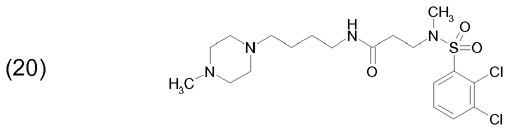 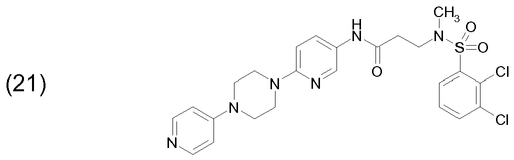 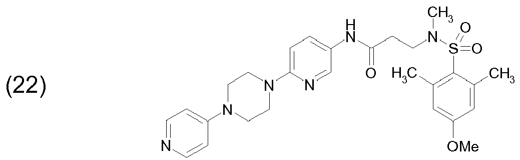 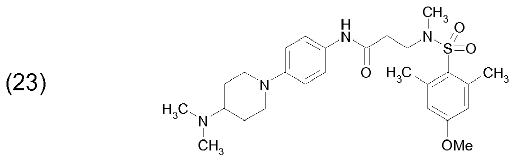 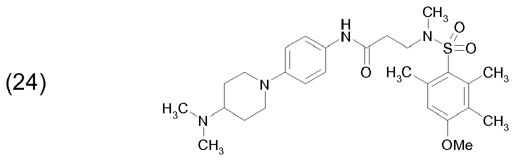 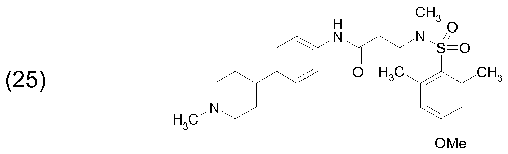 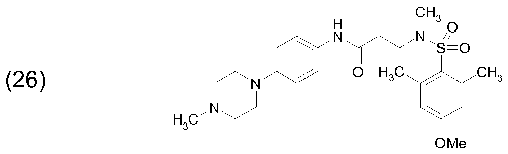 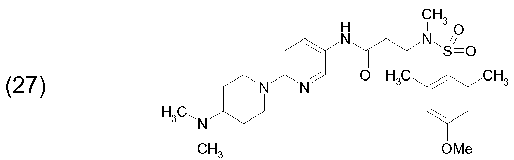 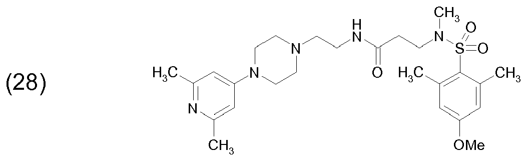 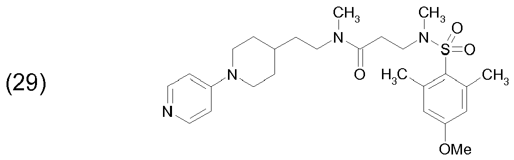 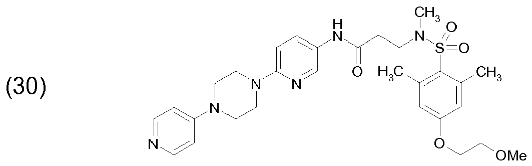 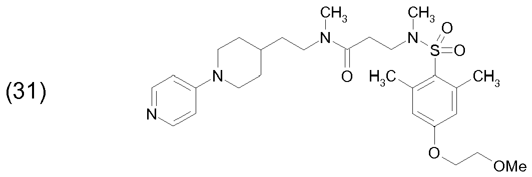 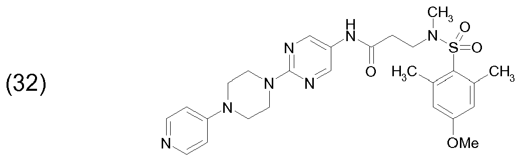 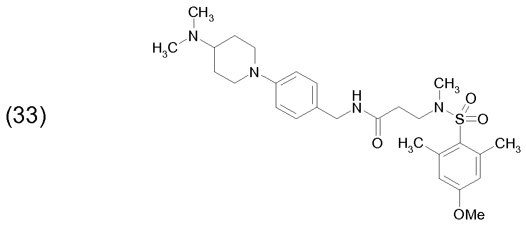 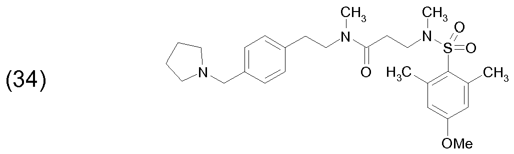 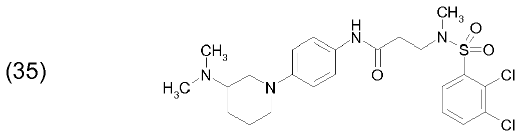 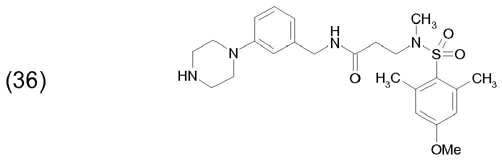 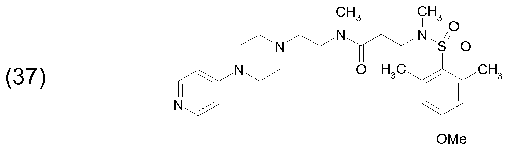 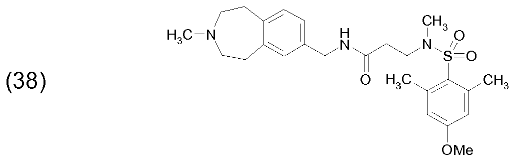 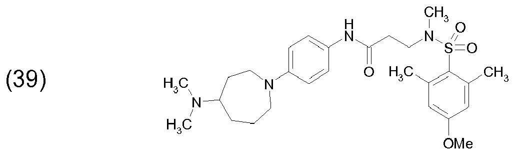 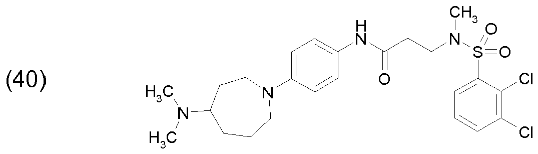 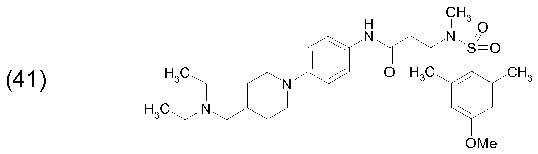 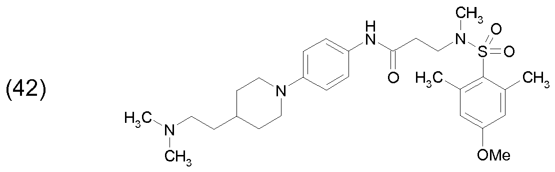 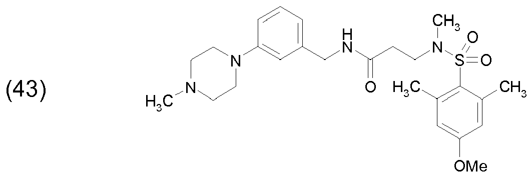 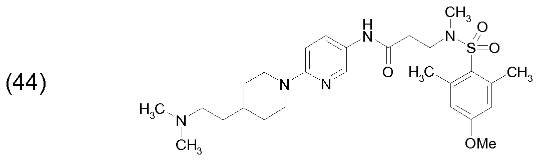 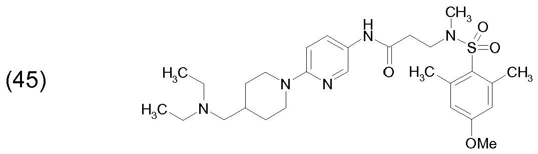 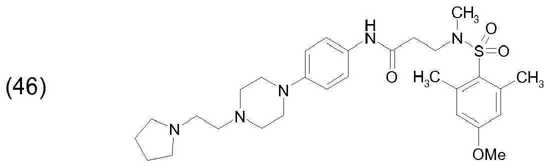 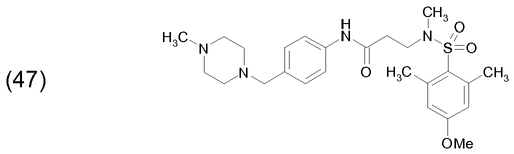 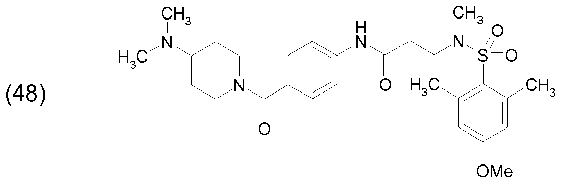 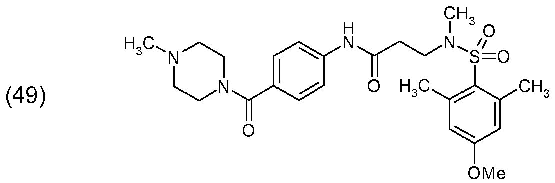 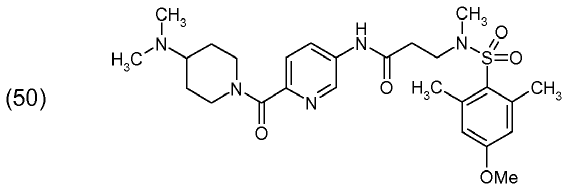 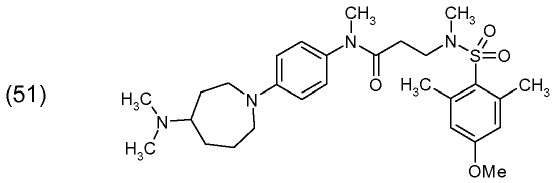 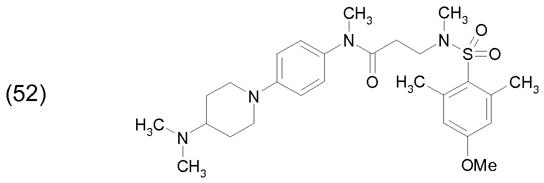 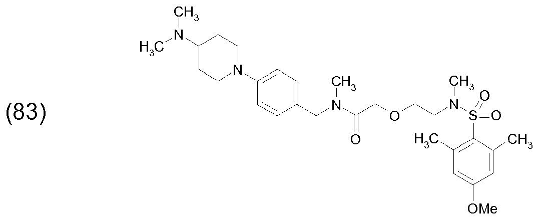 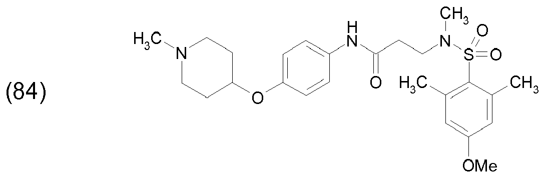 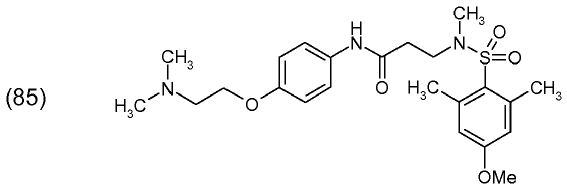 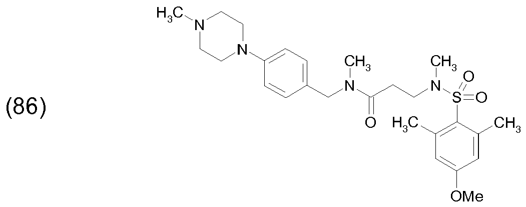 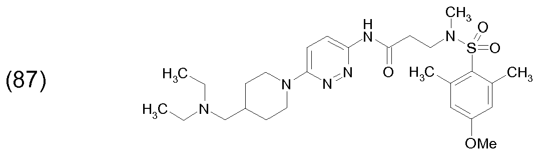 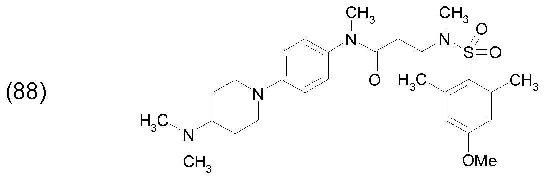 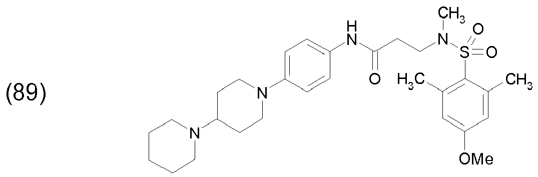 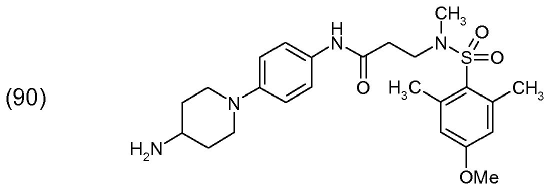 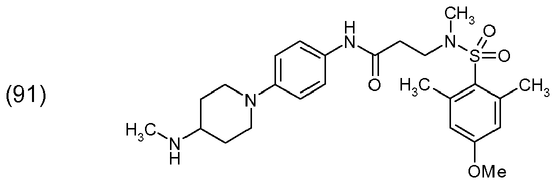 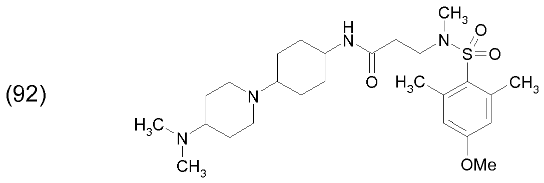 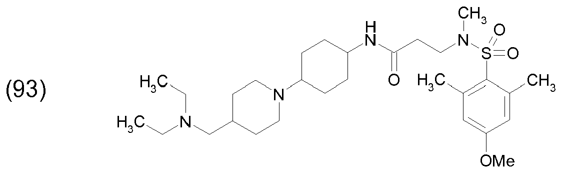 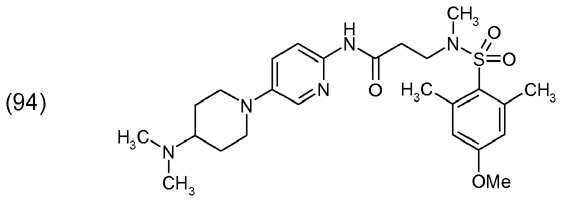 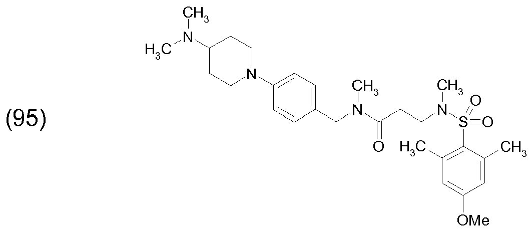 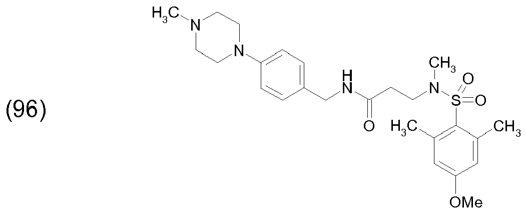 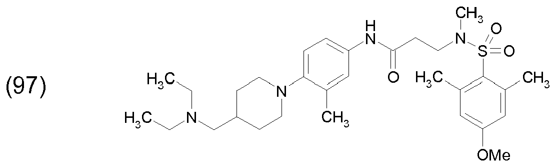 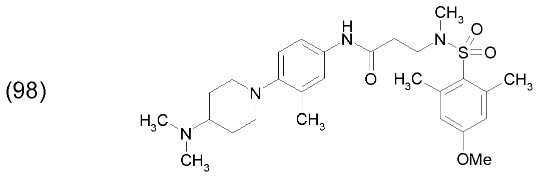 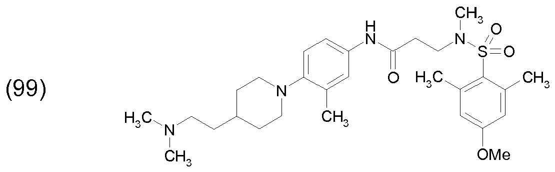 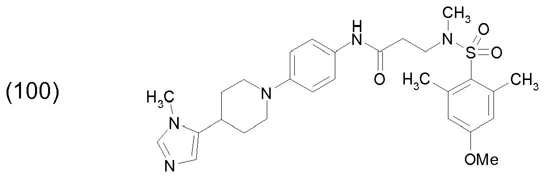 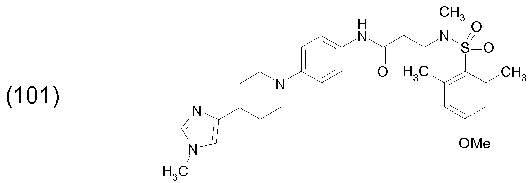 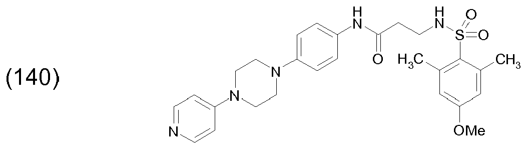 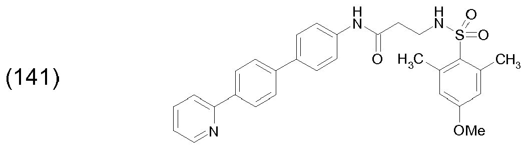 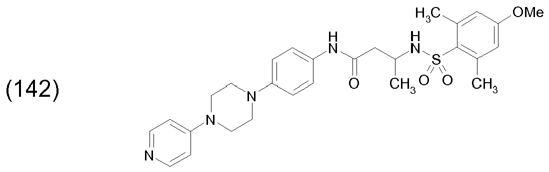 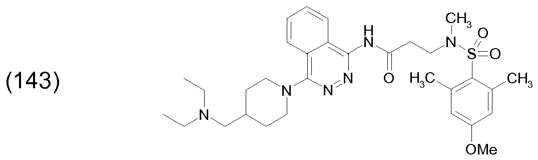 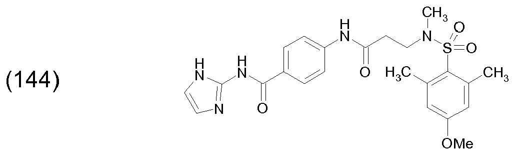 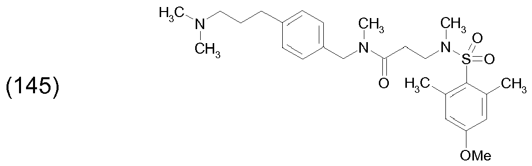 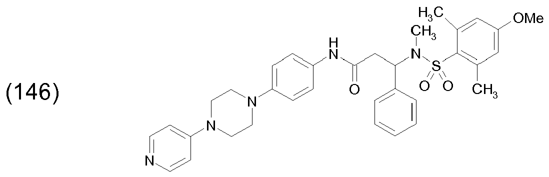 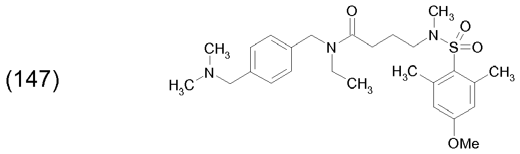 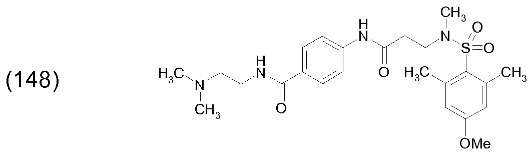 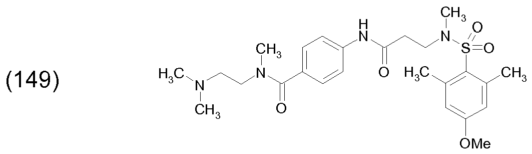 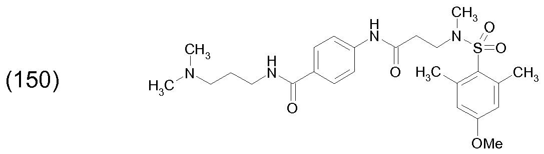 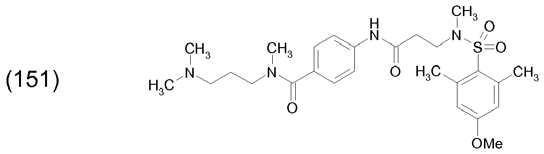 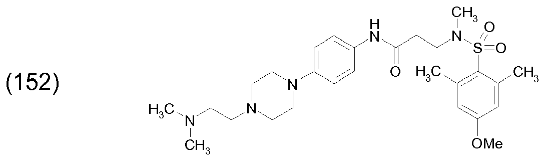 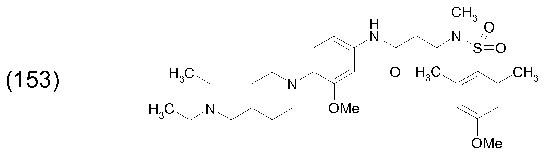 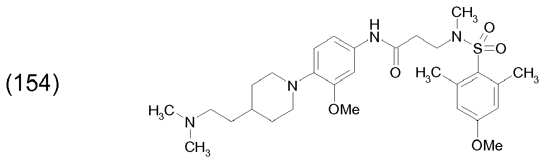 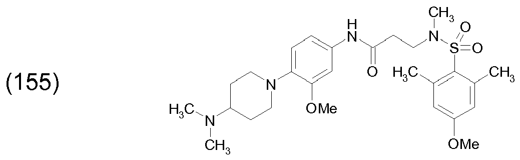 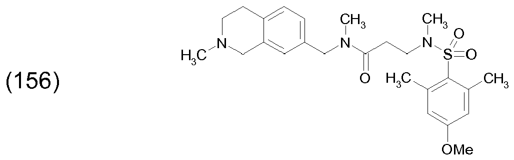 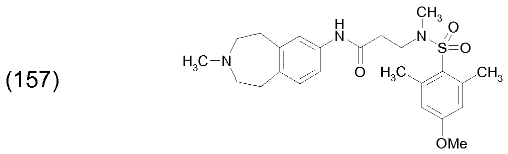 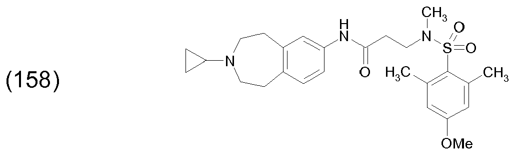 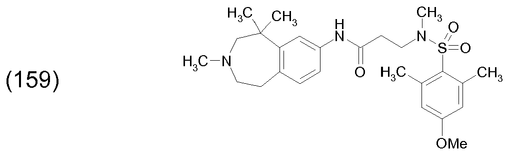 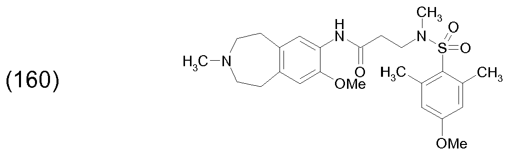 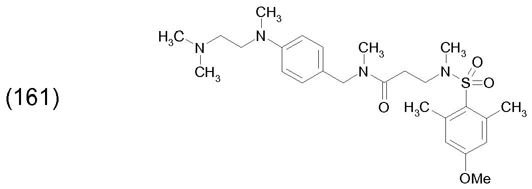 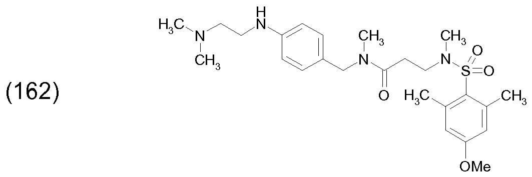 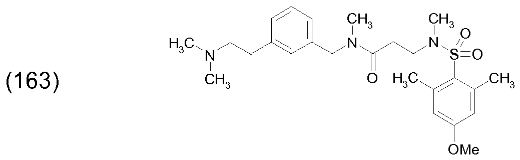 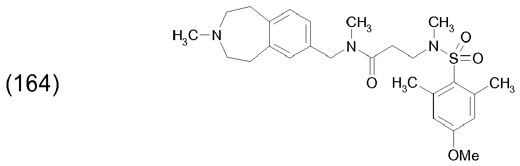 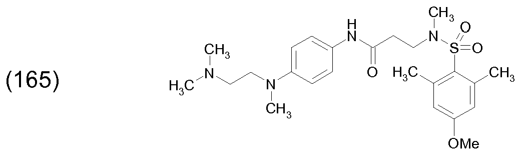 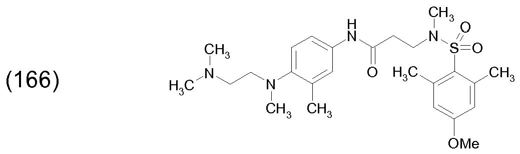 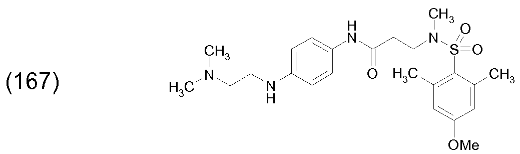 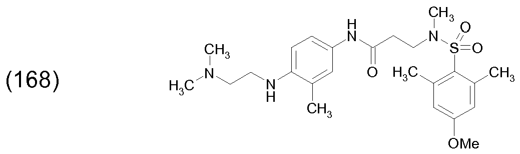 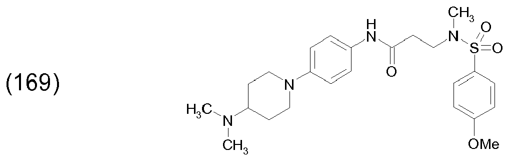 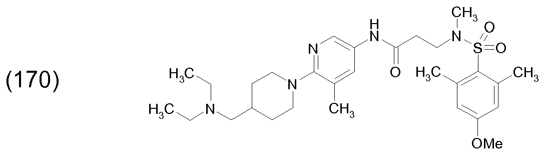 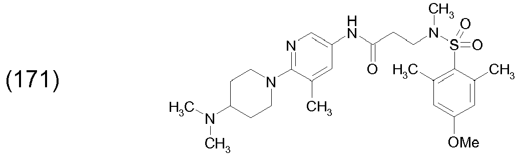 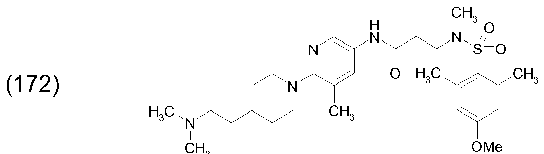 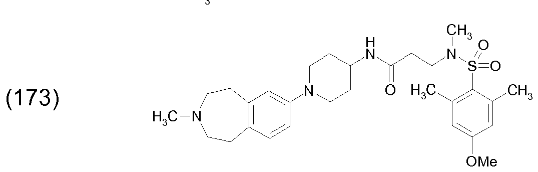 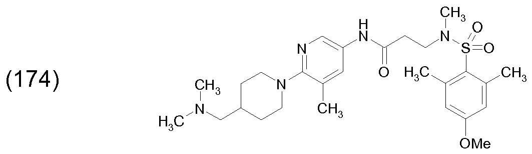 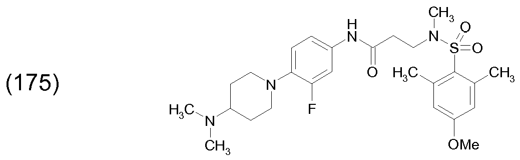 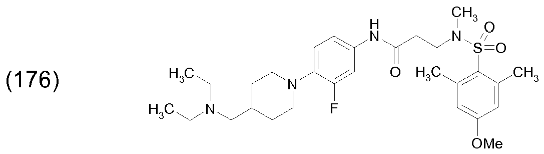 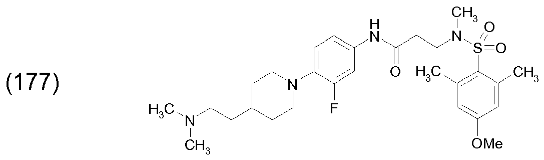 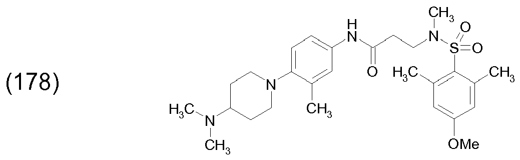 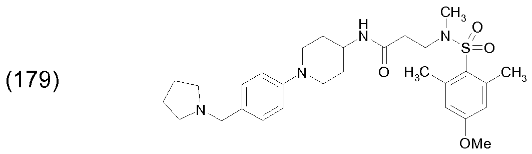 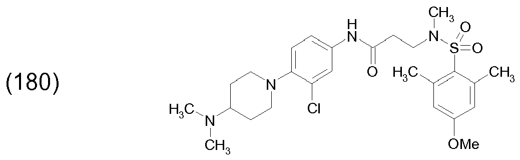 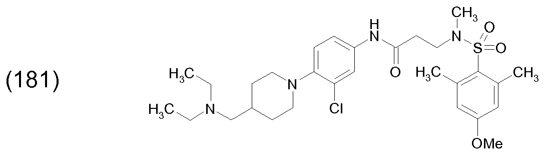 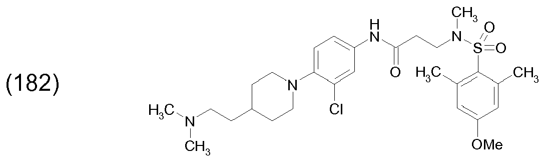 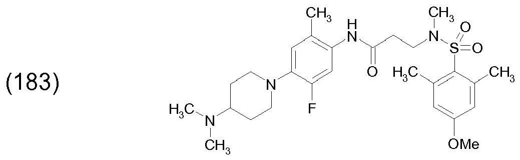 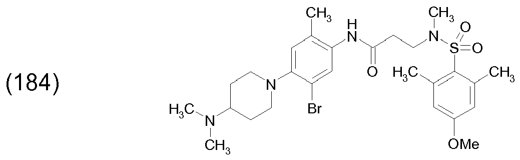 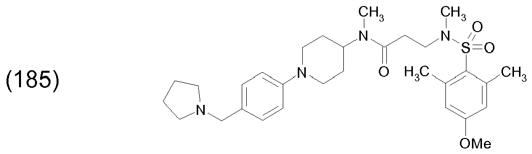 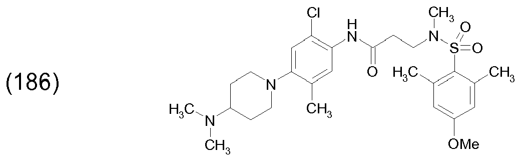 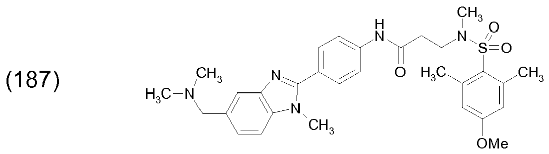 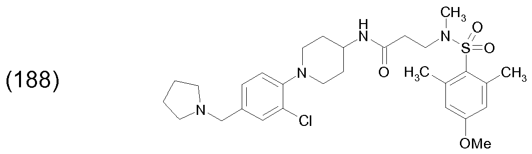 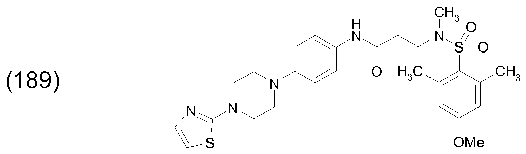 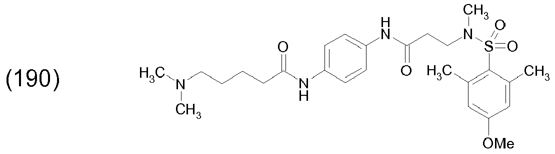 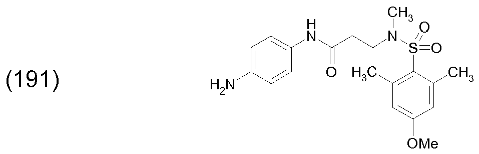 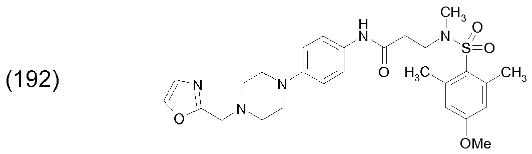 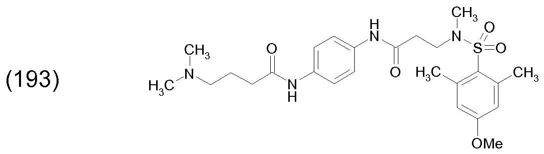 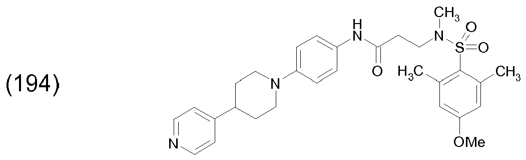 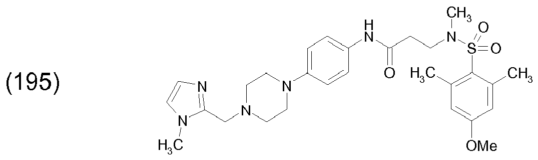 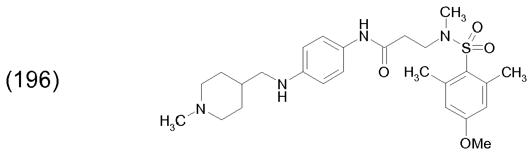 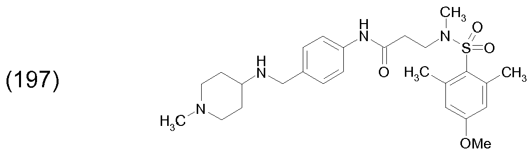 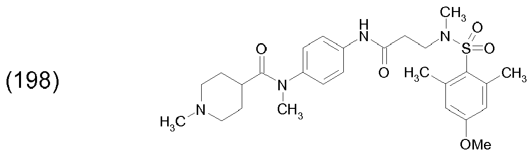 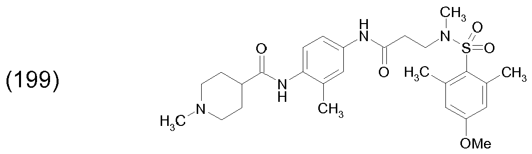 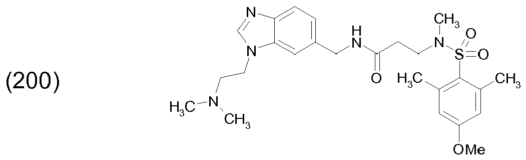 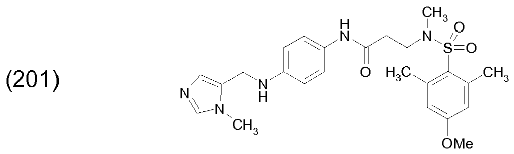 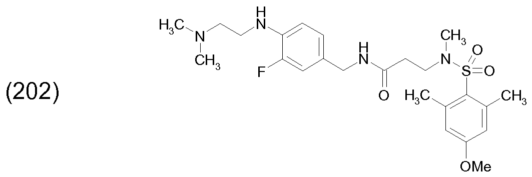 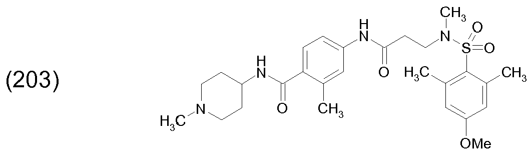 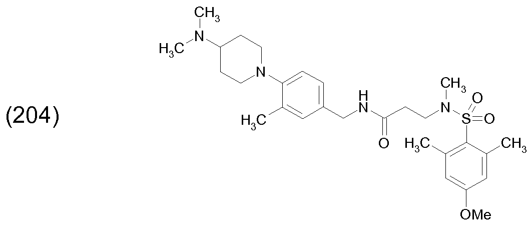 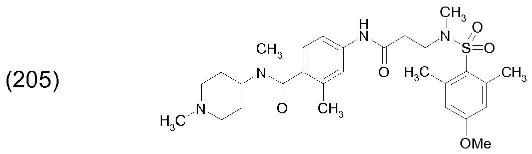 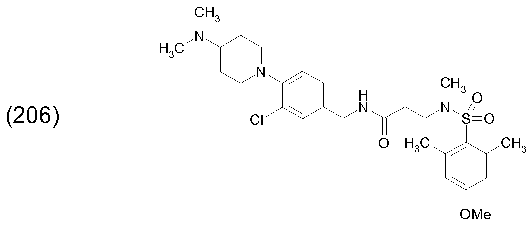 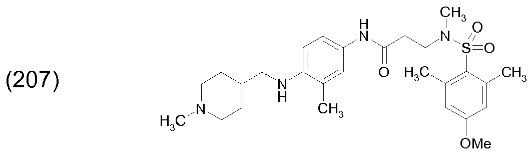 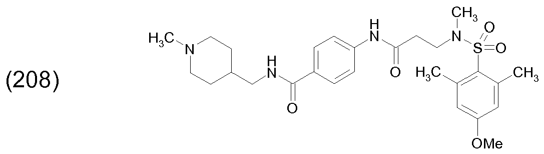 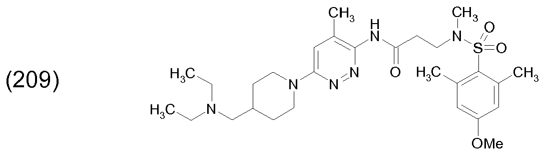 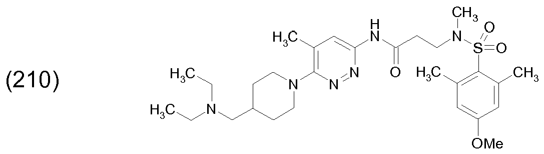 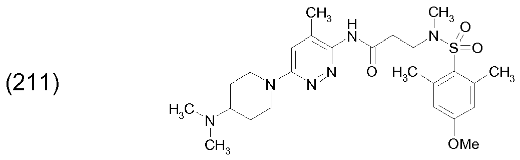 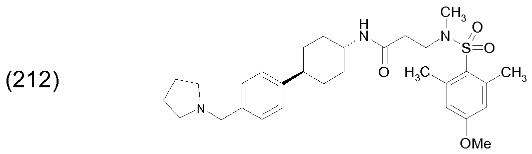 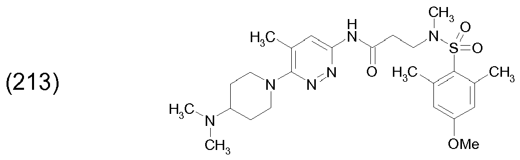 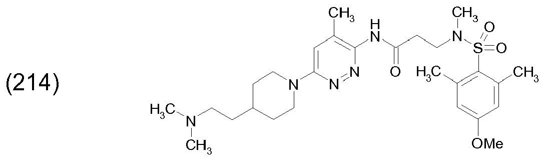 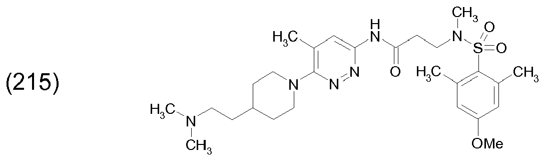 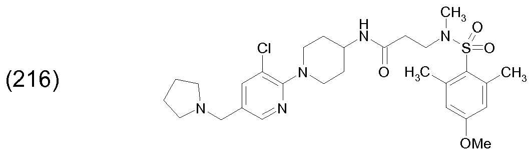 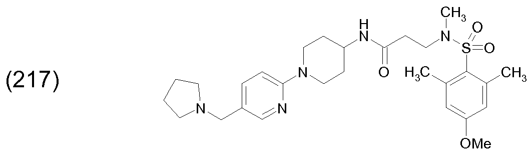 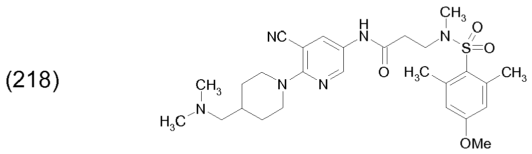 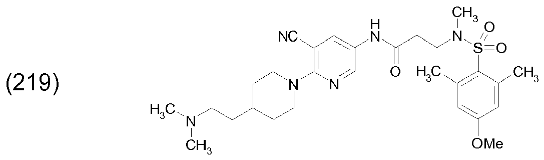 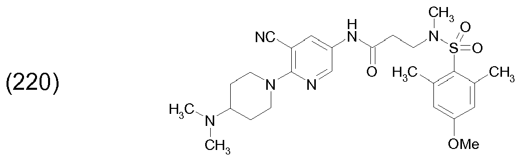 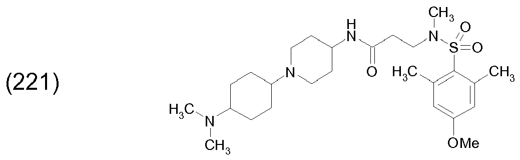 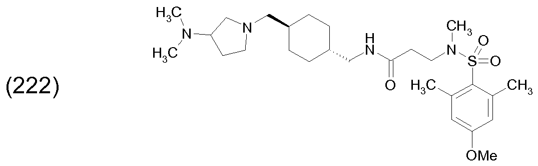 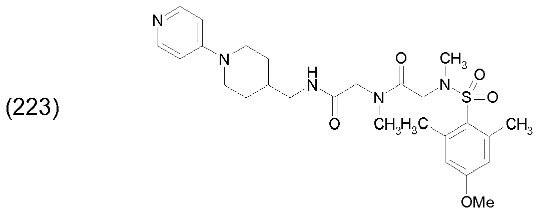 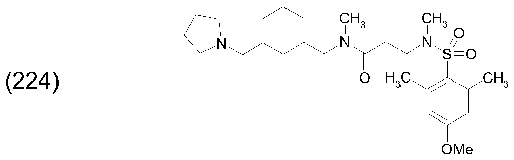 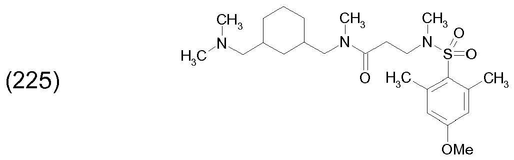 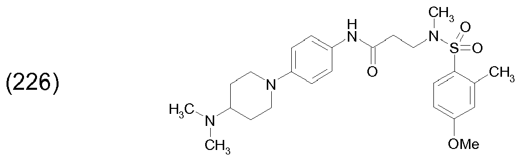 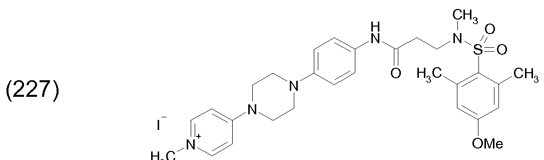 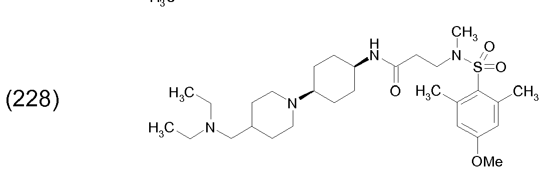 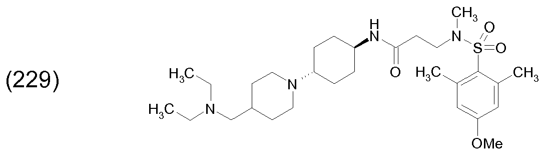 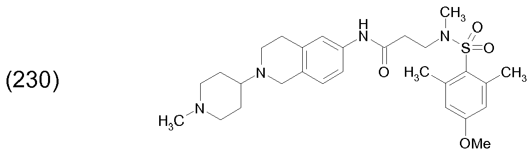 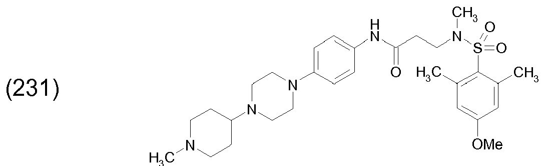 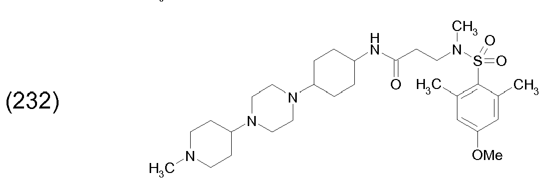 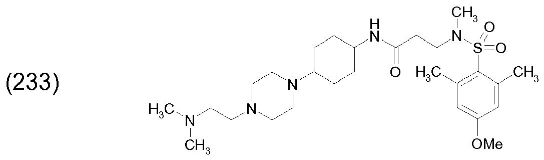 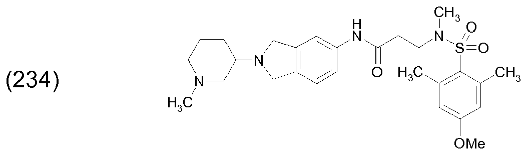 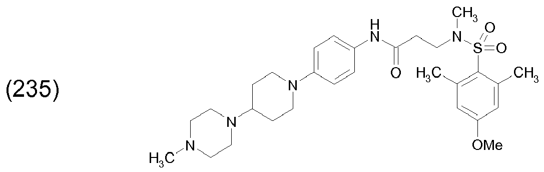 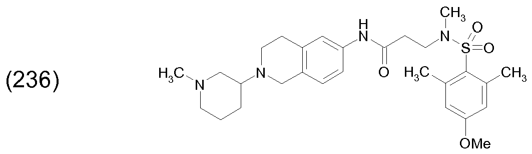 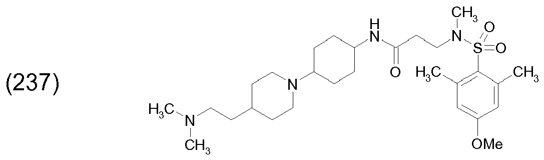 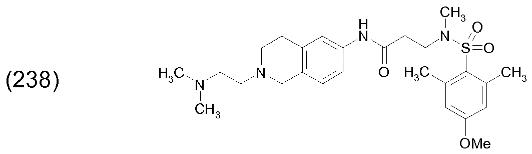 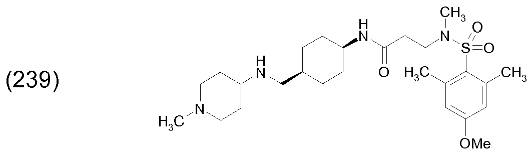 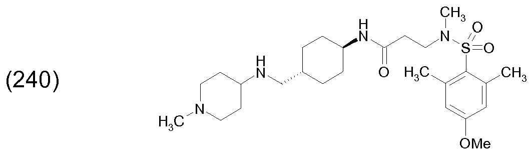 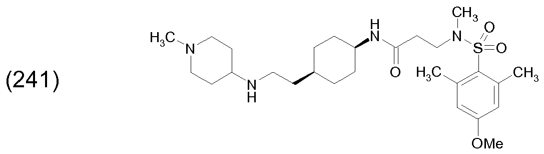 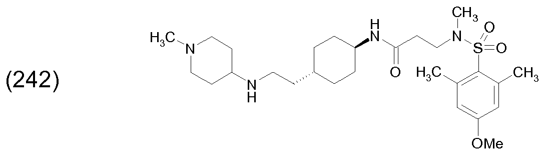 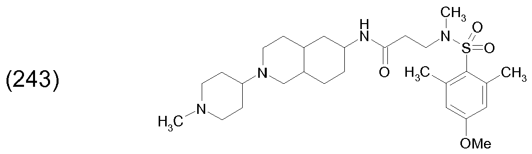 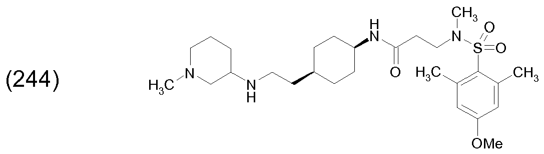 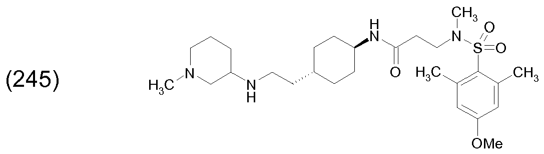 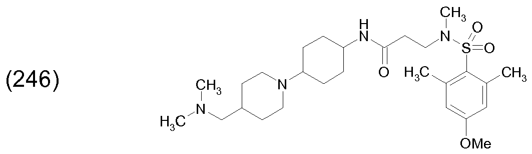 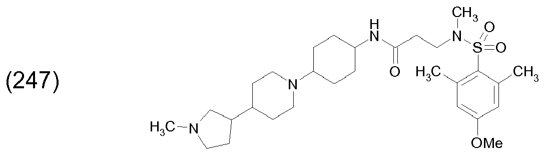 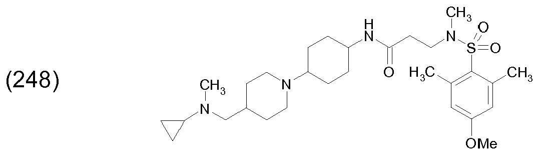 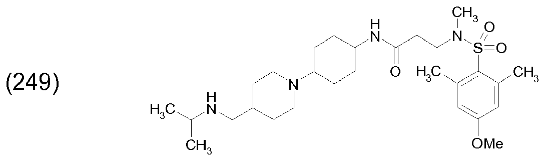 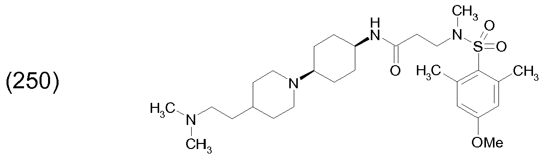 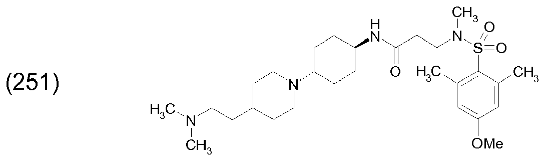 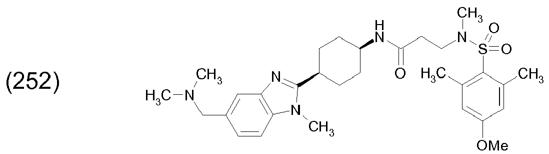 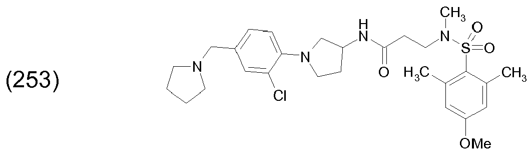 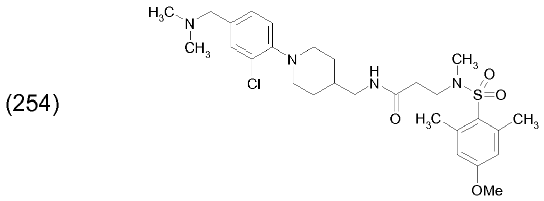 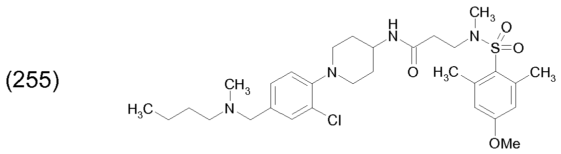 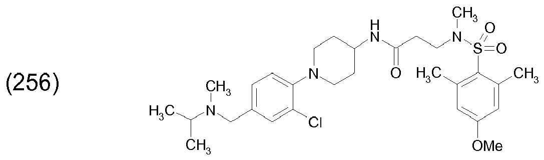 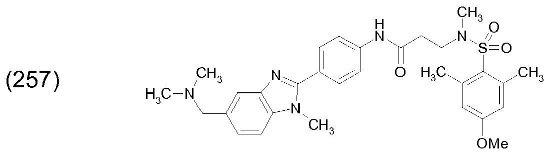 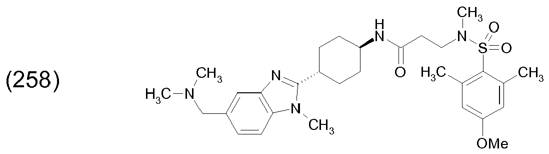 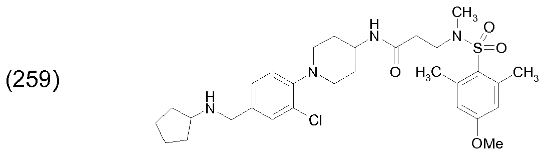 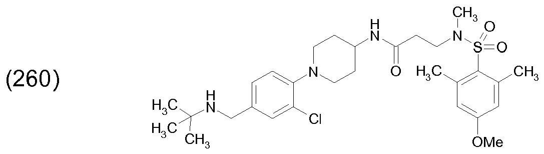 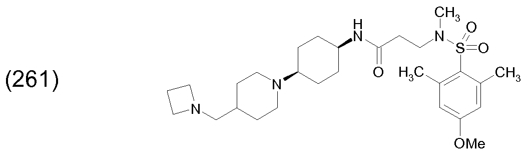 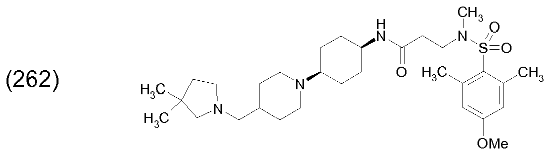 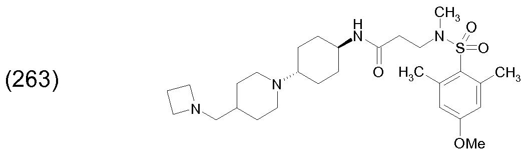 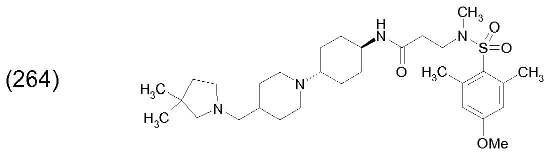 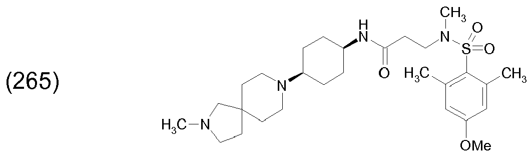 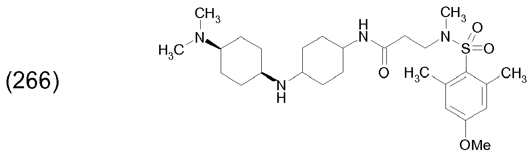 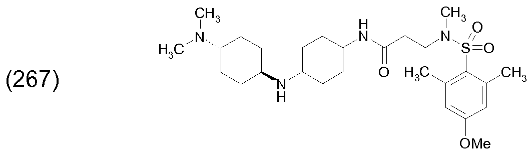 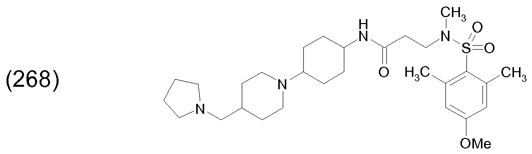 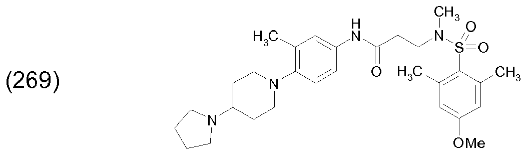 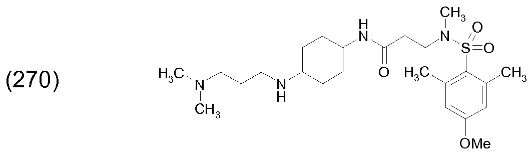 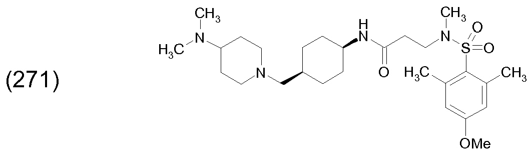 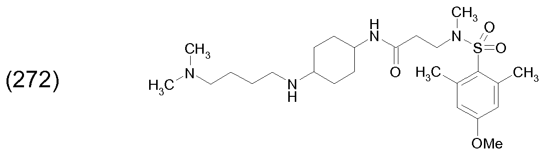 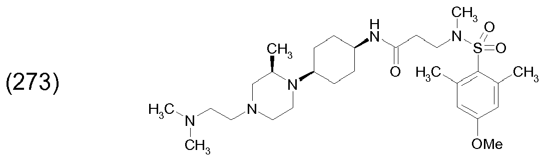 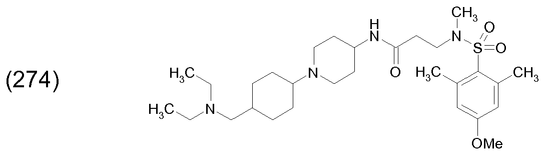 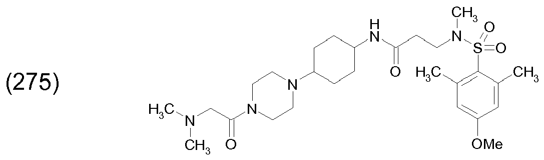 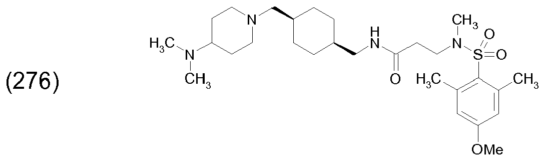 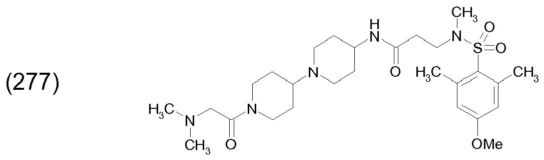 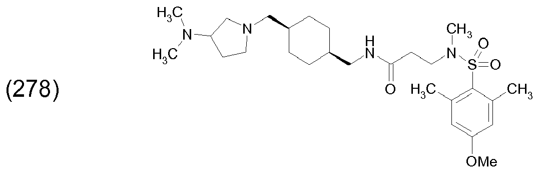 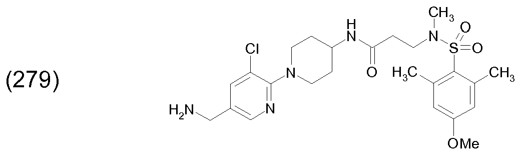 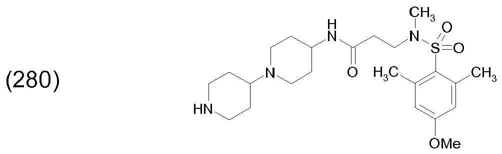 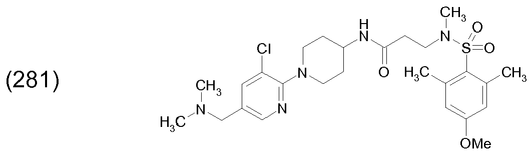 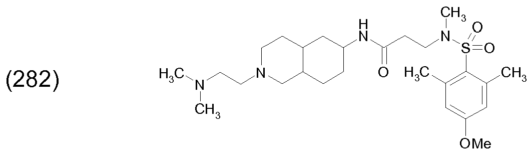 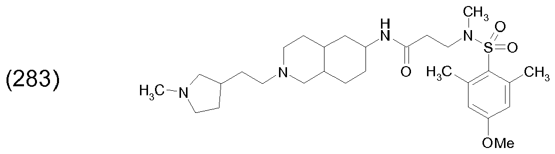 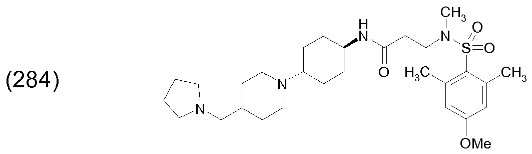 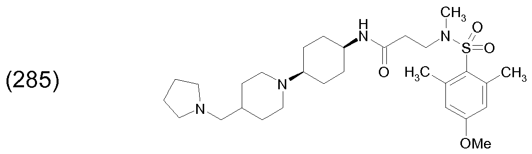 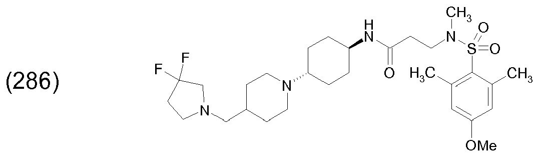 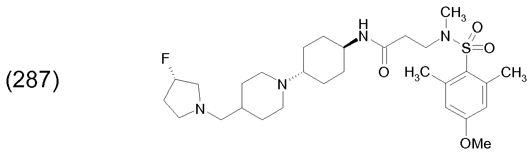 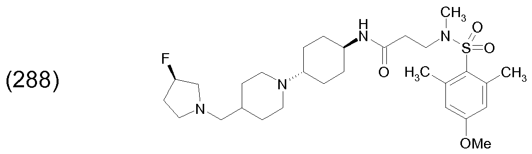 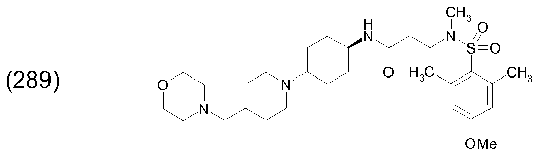 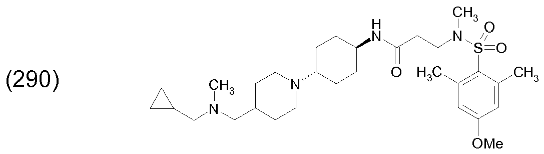 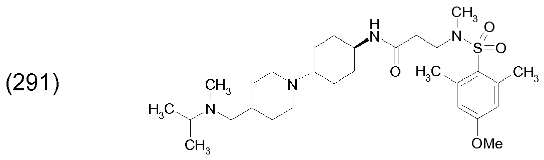 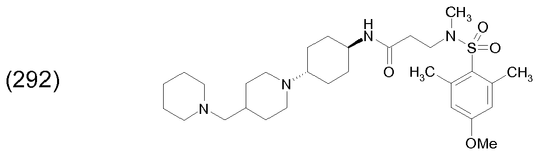 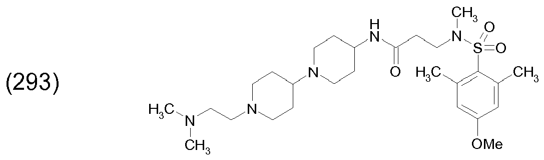 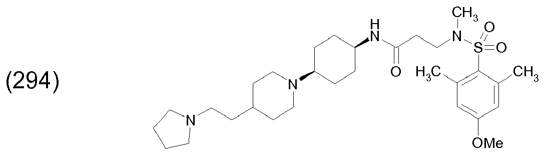 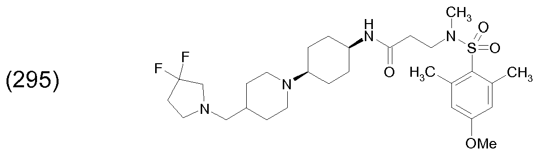 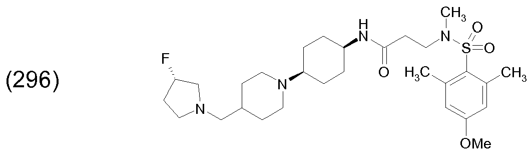 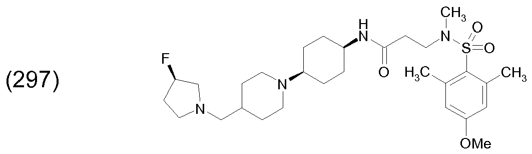 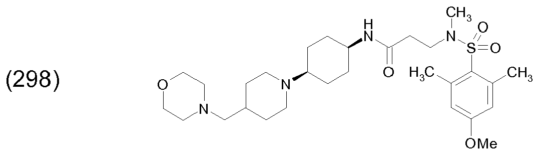 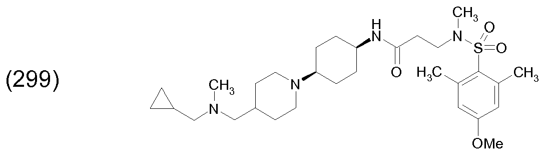 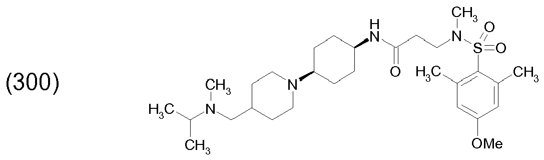 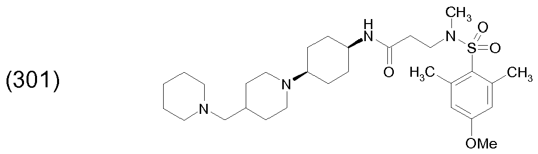 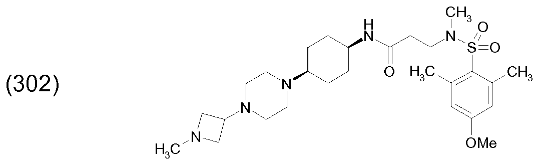 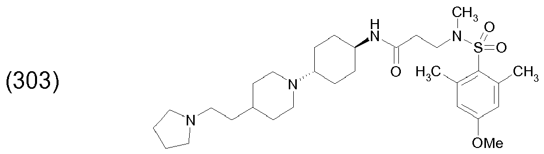 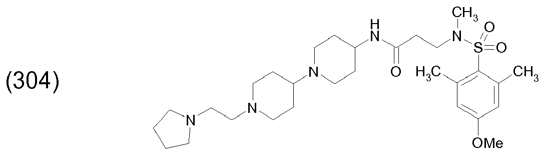 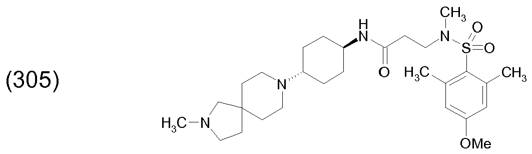 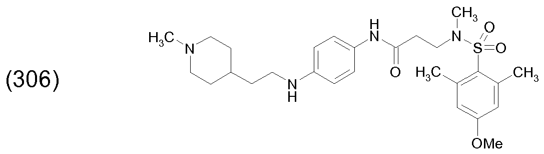 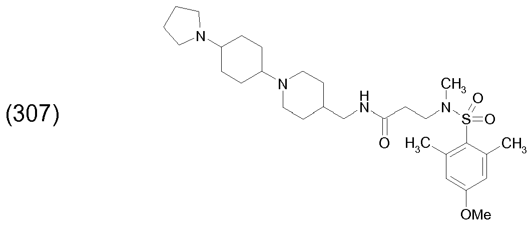 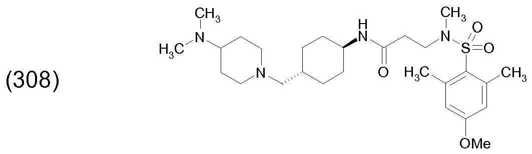 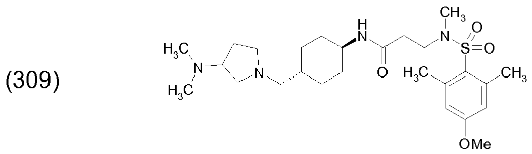 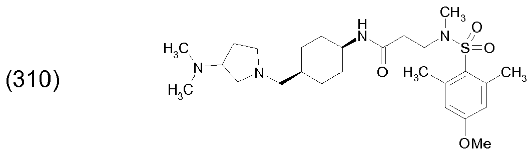 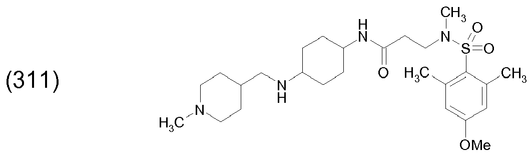 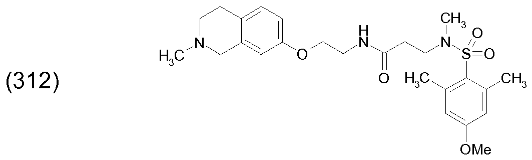 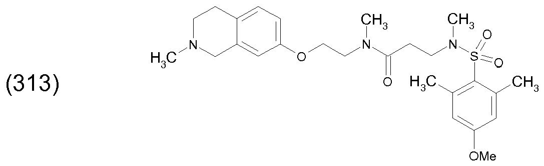 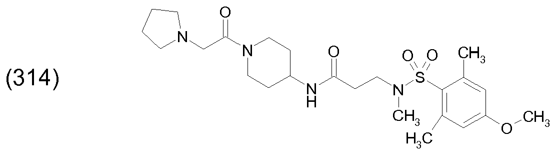 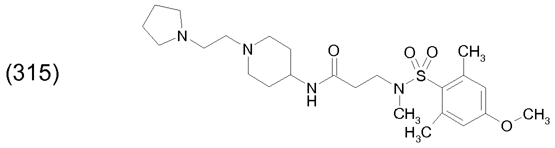 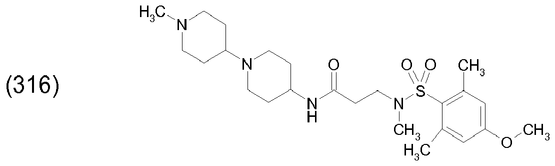 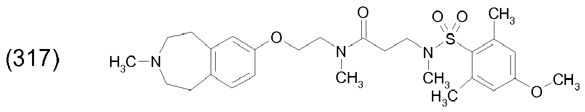 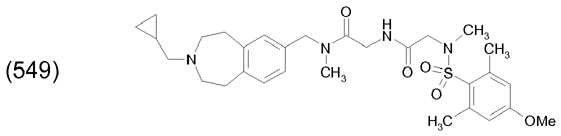 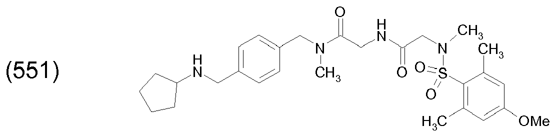 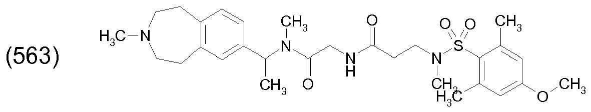 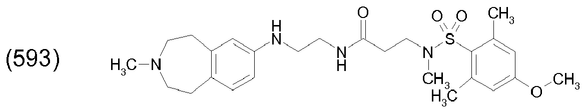 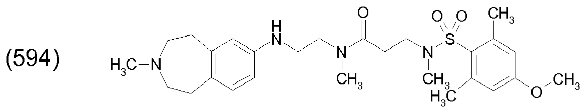 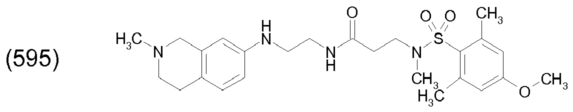 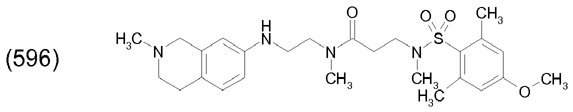 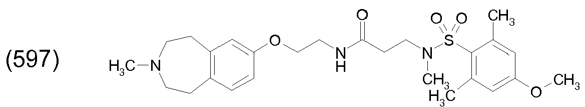 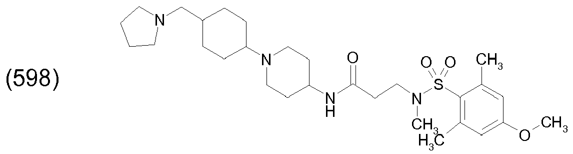 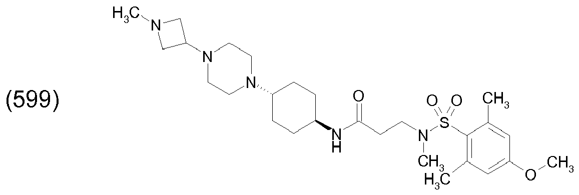 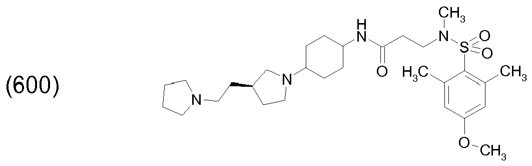 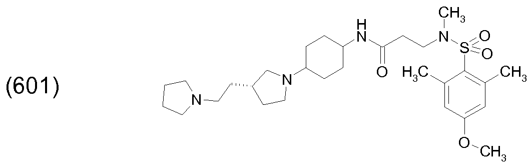 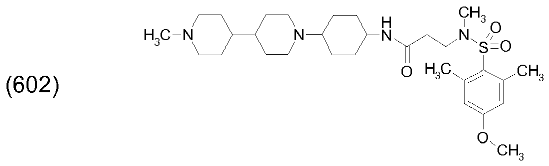 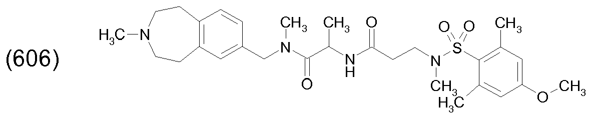 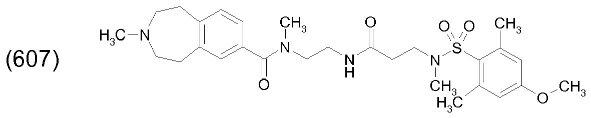 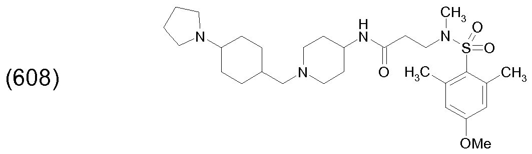 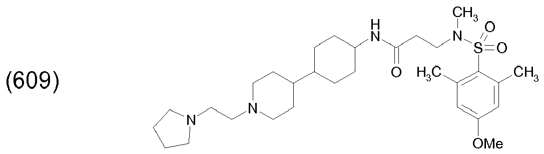 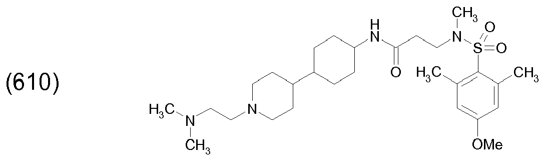 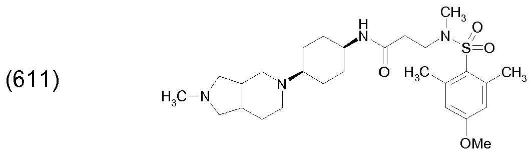 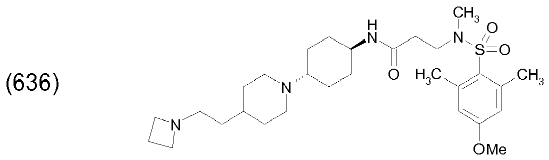 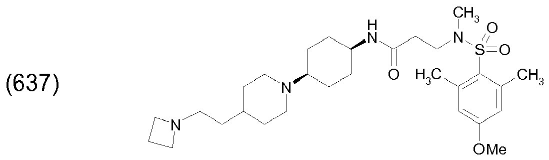 the enantiomers, the diastereomers, the mixtures and the salts thereof, particularly the physiologically acceptable salts thereof with organic or inorganic acids or bases.

2. Physiologically acceptable salts of the compounds according to claim 1 with inorganic or organic acids or bases.

3. Medicament, containing a compound according to claim 1 or a physiologically acceptable salt according to claim 8 optionally together with one or more inert carriers and/or diluents.

4. Compounds according to claim 1 or 2 for the acute and prophylactic treatment of acute pain, visceral pain, neuropathic pain, inflammatory/pain receptor-mediated pain, tumour pain and headache diseases.

5. Process for preparing a medicament according to claim 3, **characterised in that** a compound according to at least one of claims 1 to 2 is incorporated in one or more inert carriers and/or diluents by a non-chemical method.

## Revendications

1. Composés, à savoir : 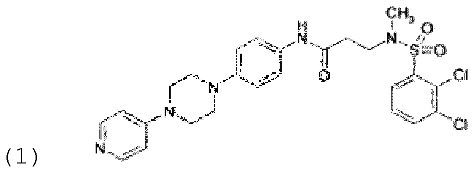 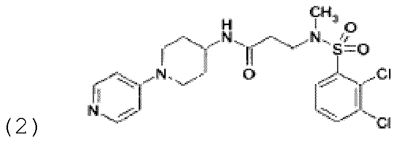 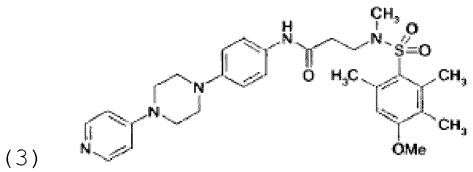 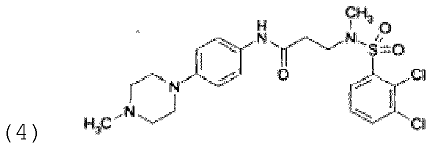 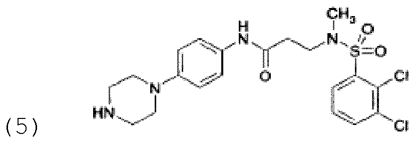 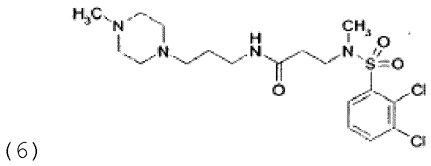 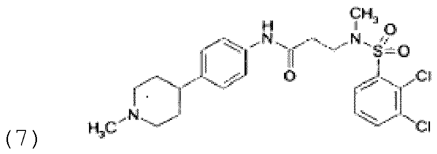 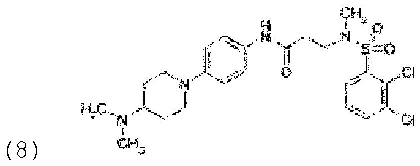 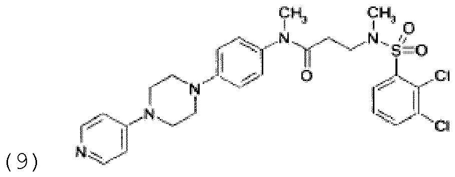 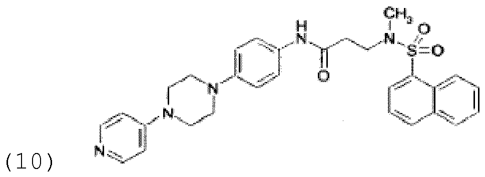 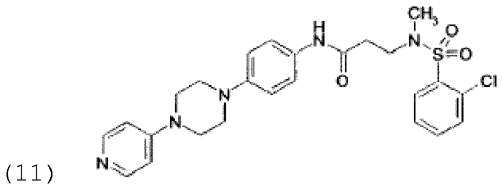 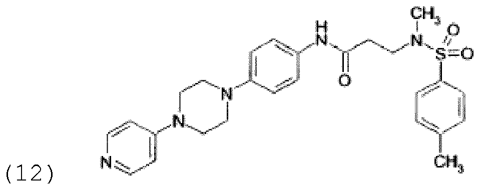 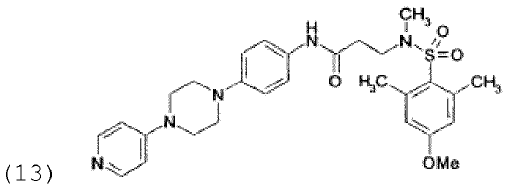 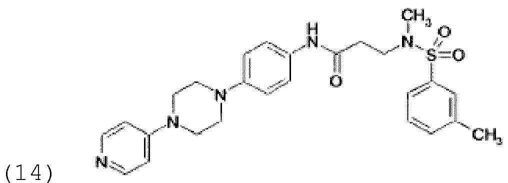 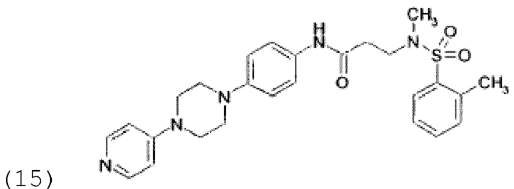 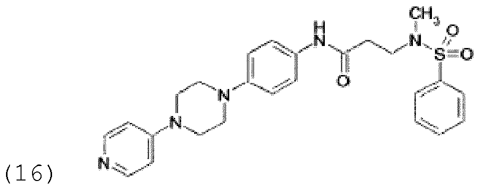 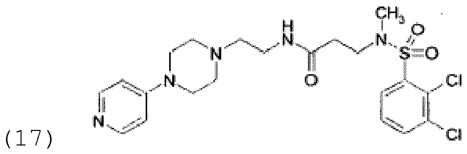 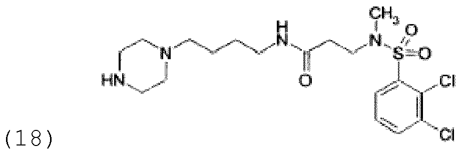 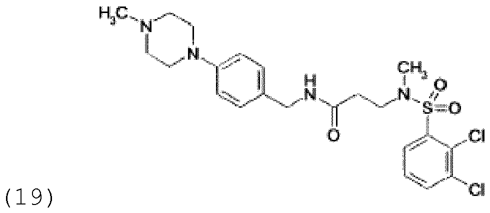 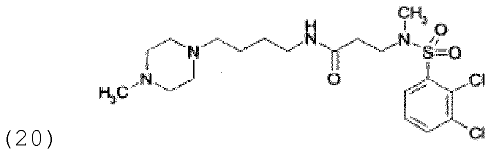 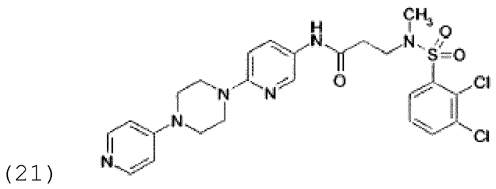 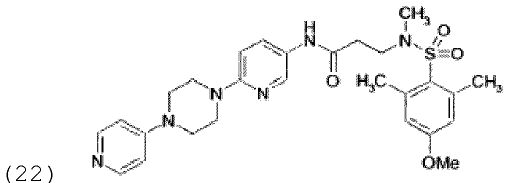 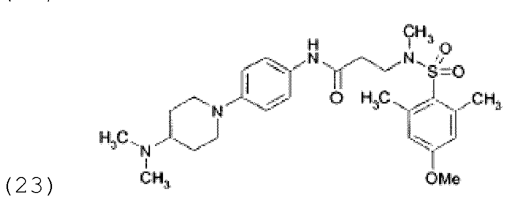 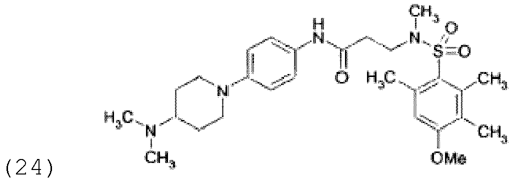 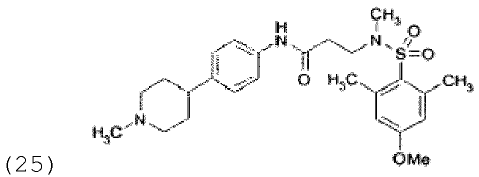 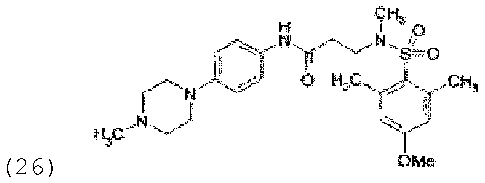 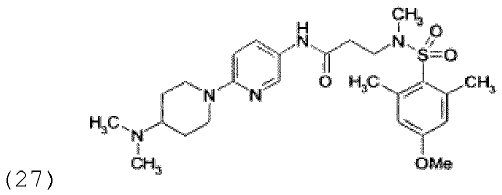 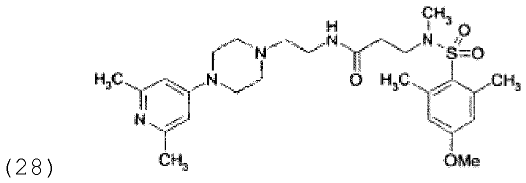 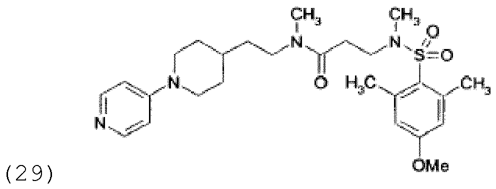 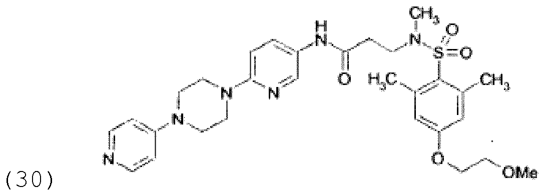 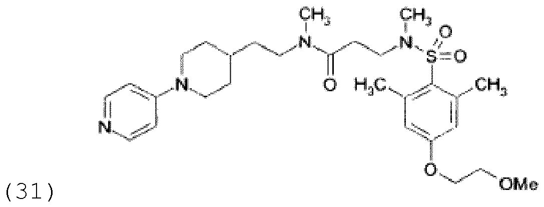 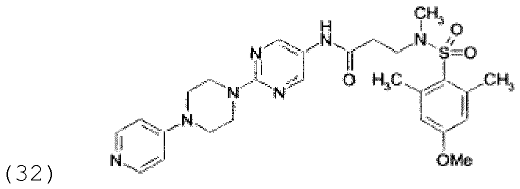 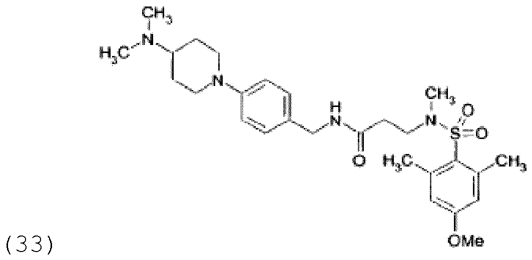 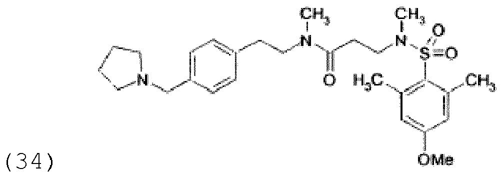 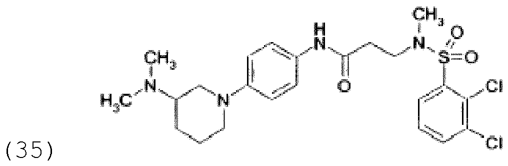 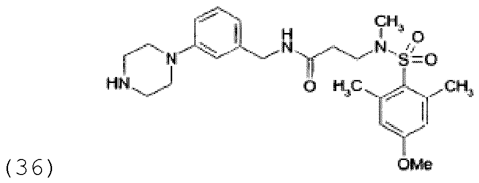 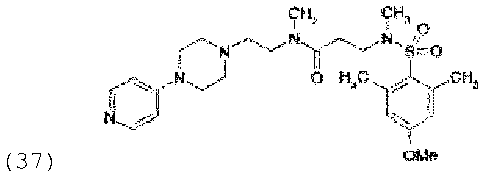 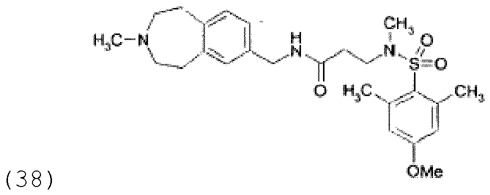 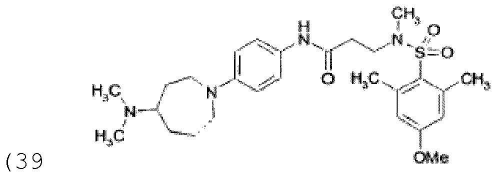 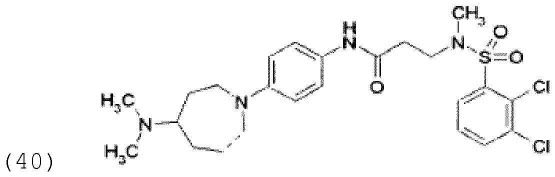 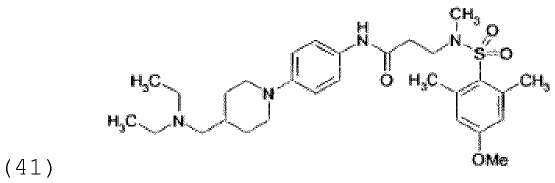 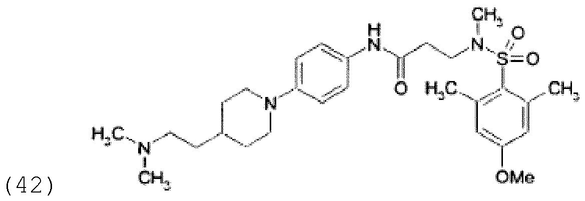 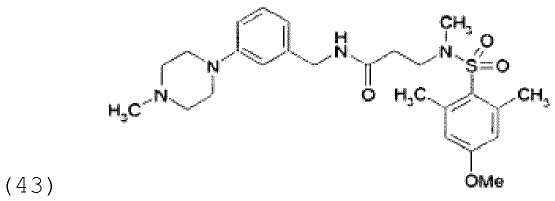 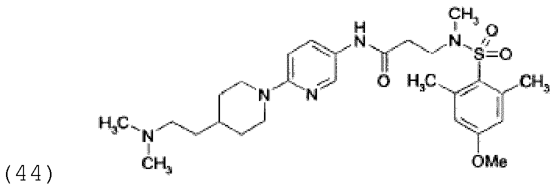 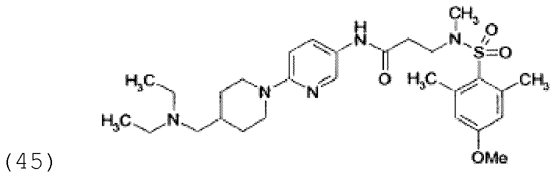 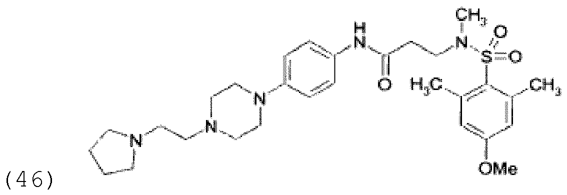 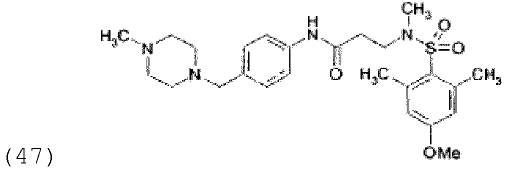 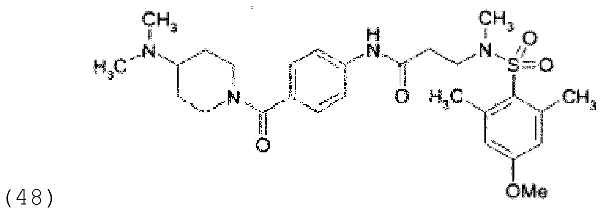 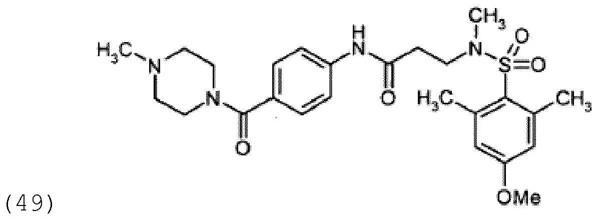 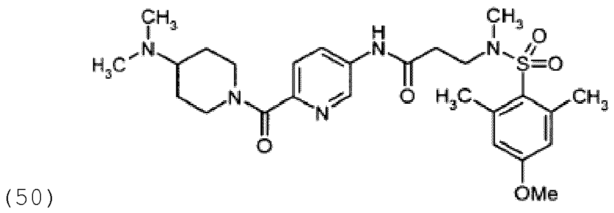 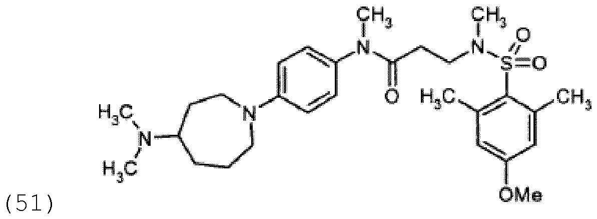 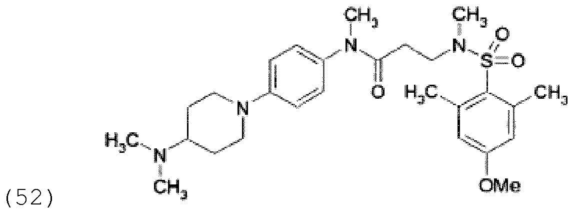 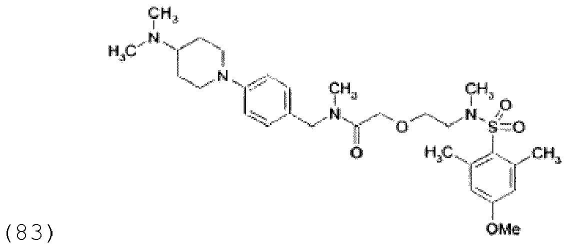 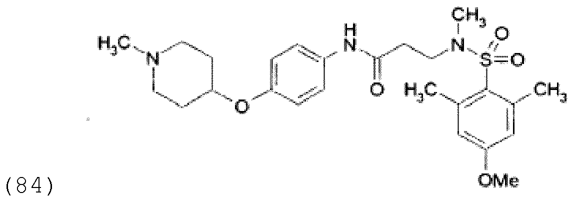 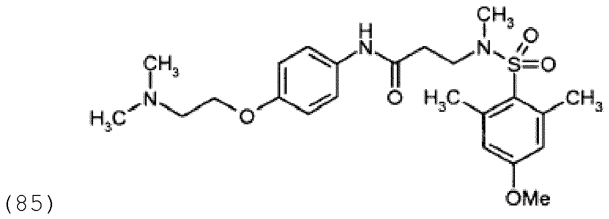 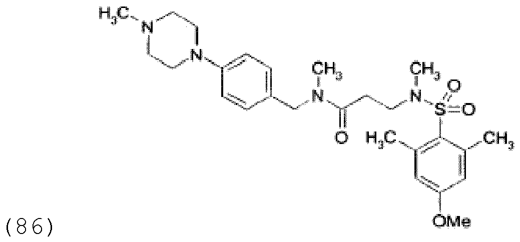 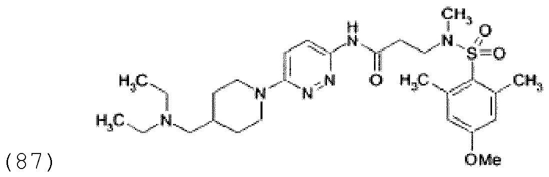 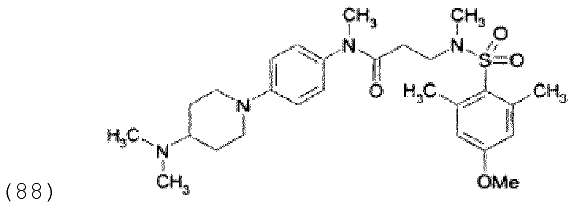 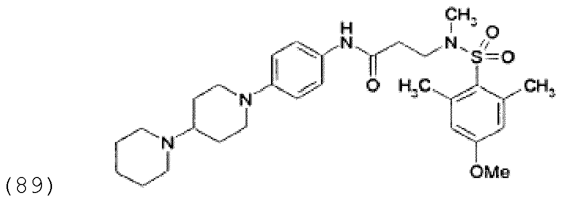 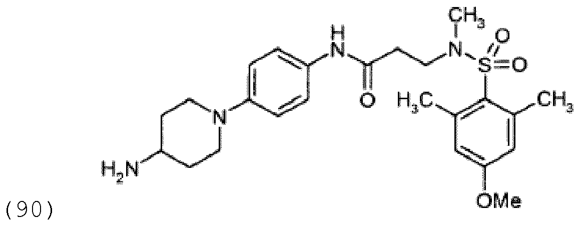 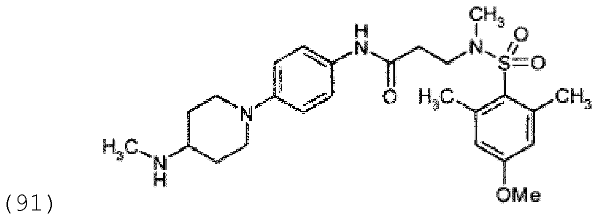 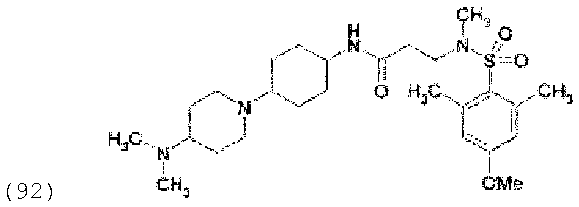 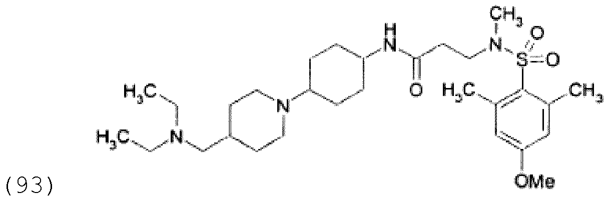 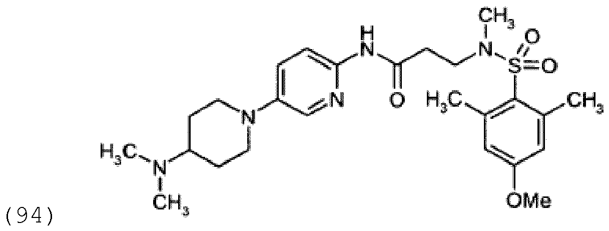 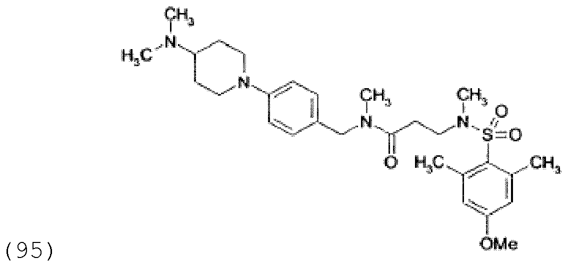 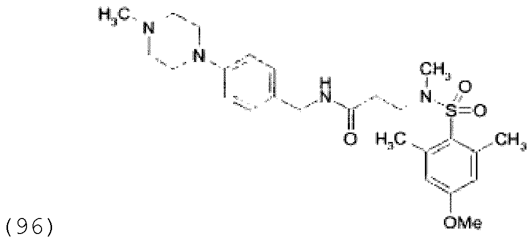 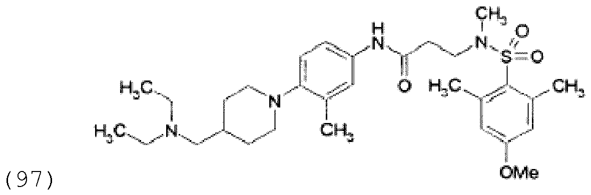 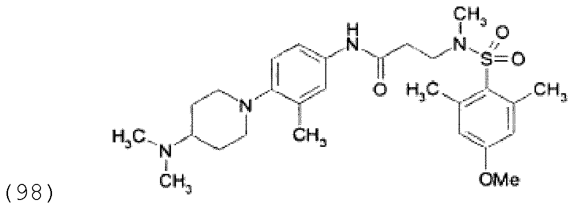 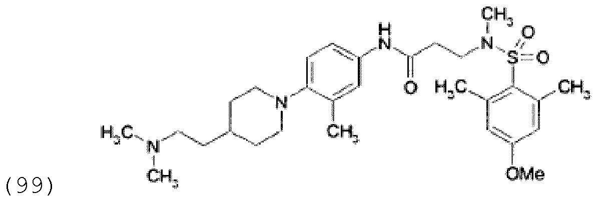 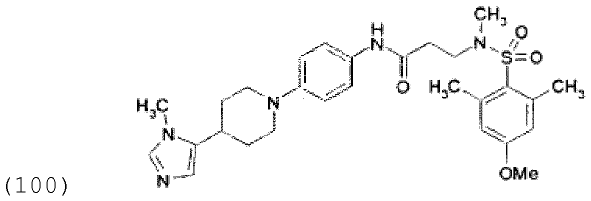 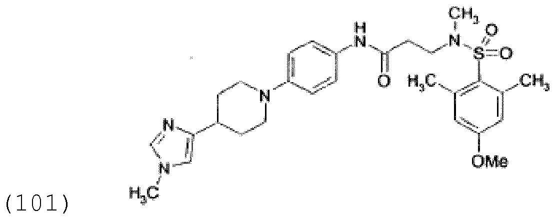 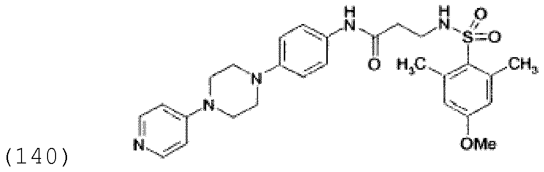 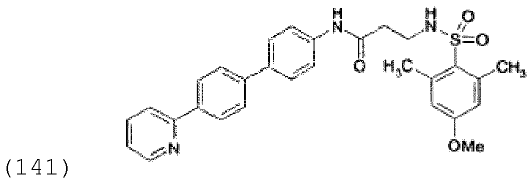 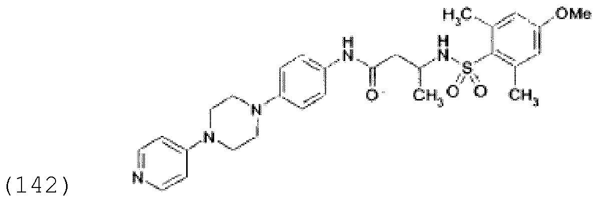 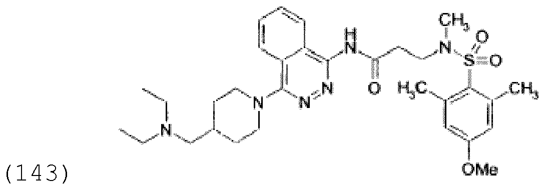 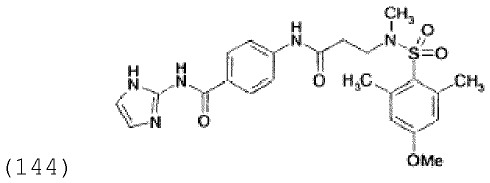 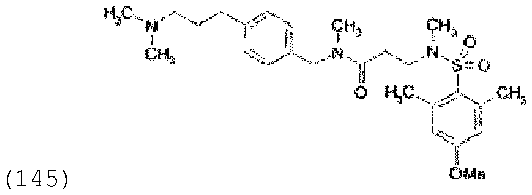 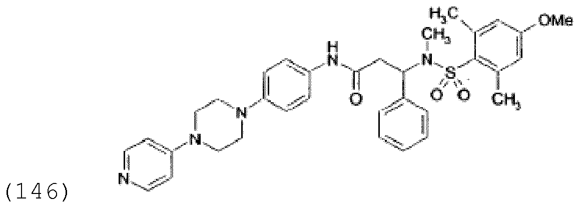 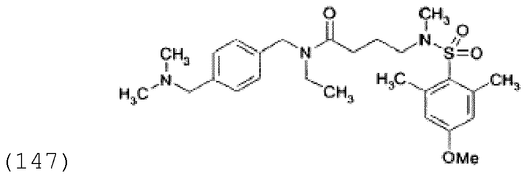 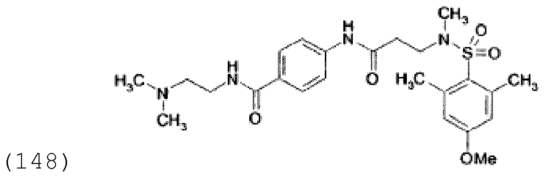 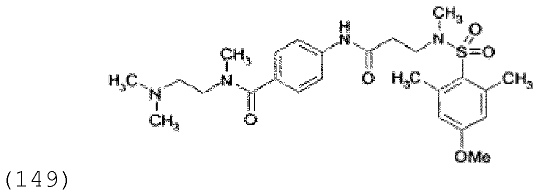 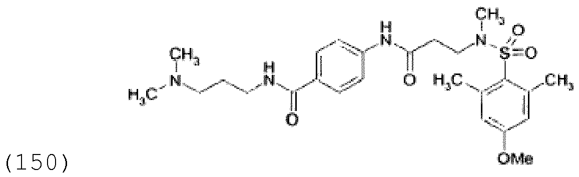 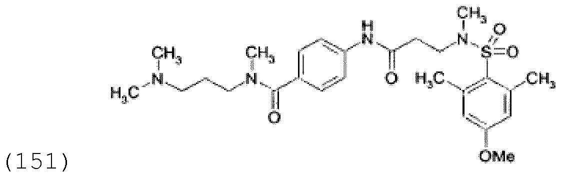 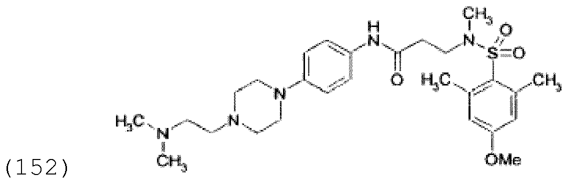 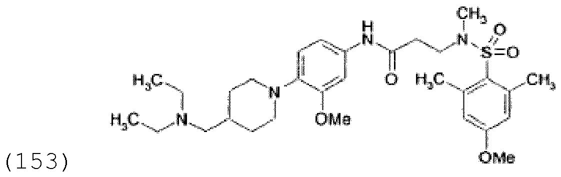 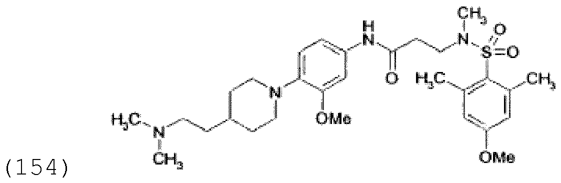 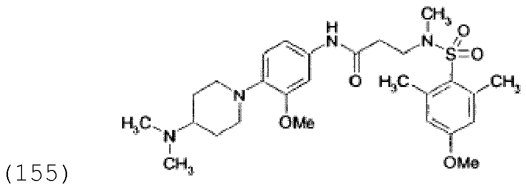 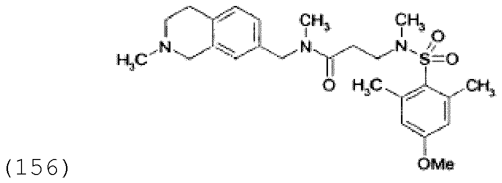 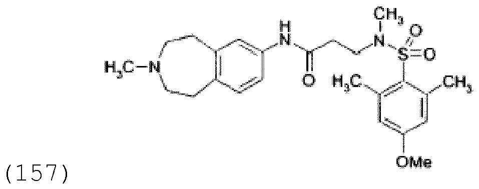 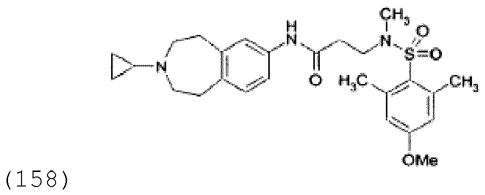 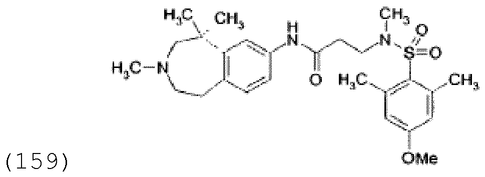 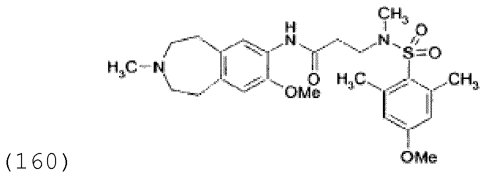 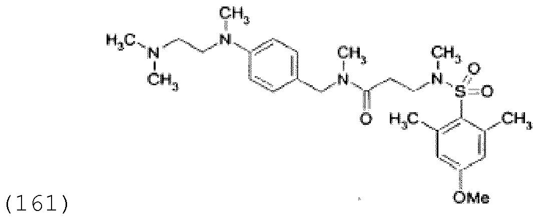 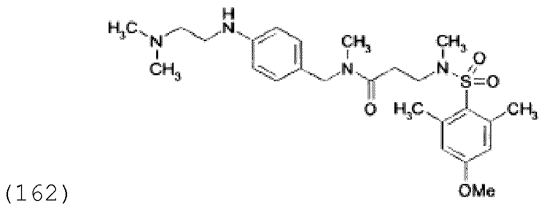 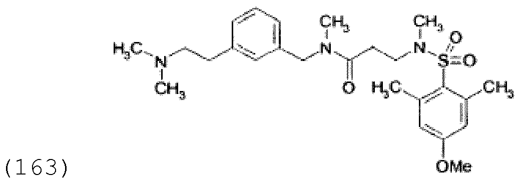 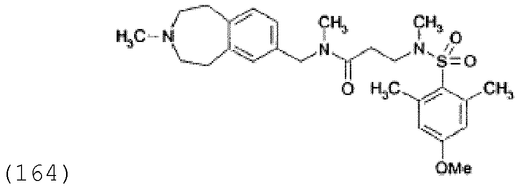 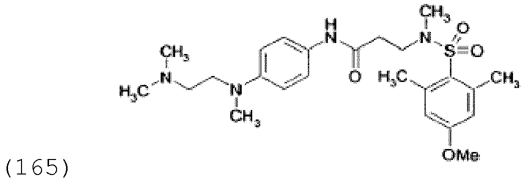 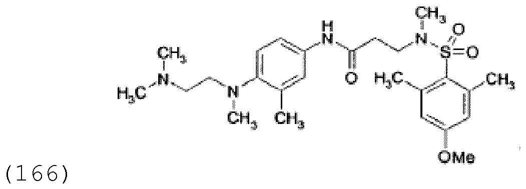 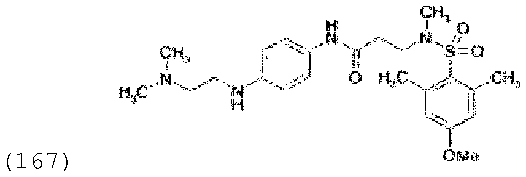 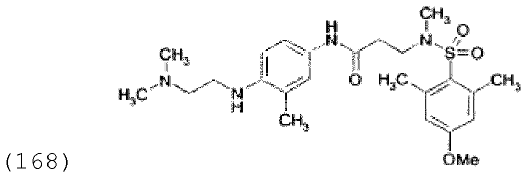 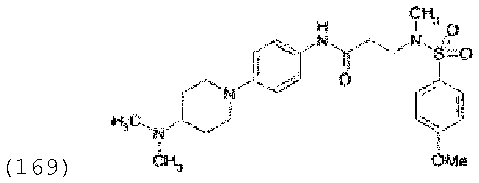 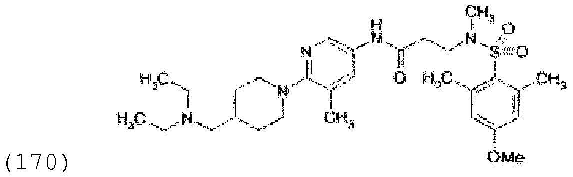 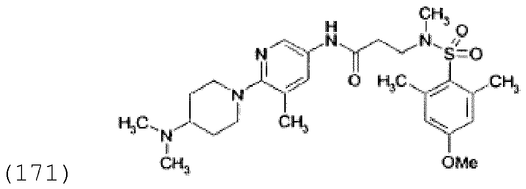 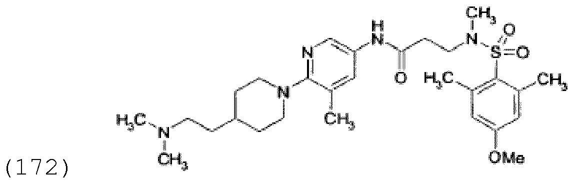 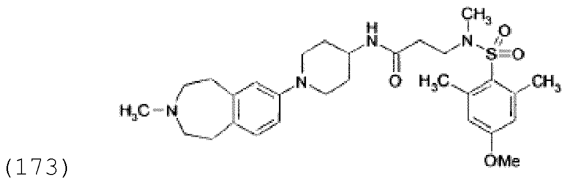 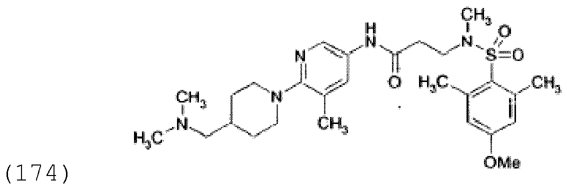 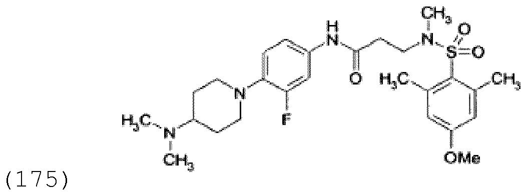 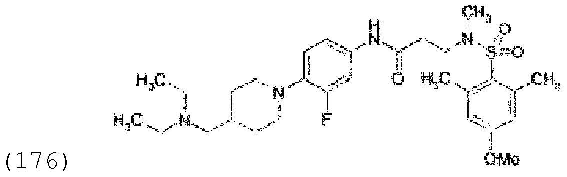 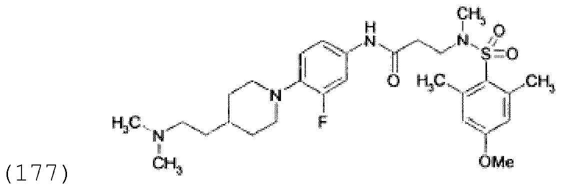 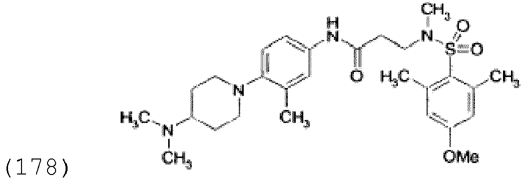 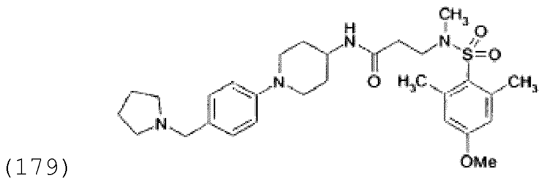 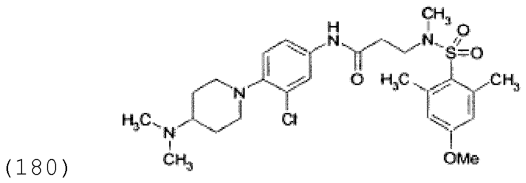 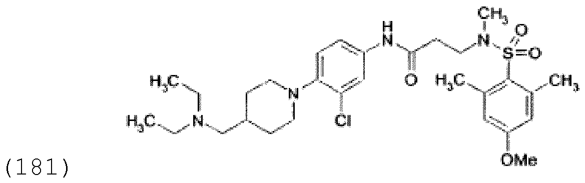 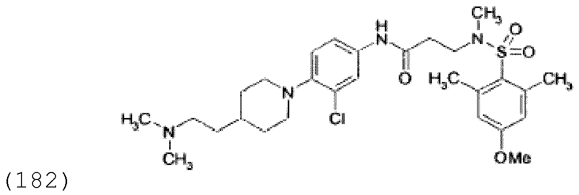 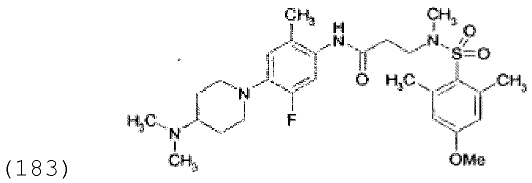 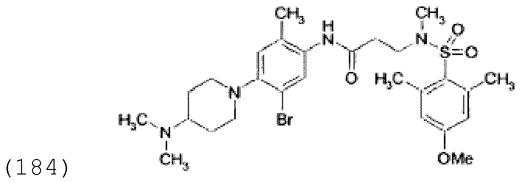 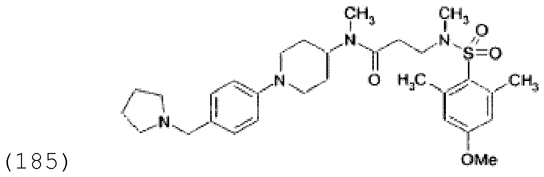 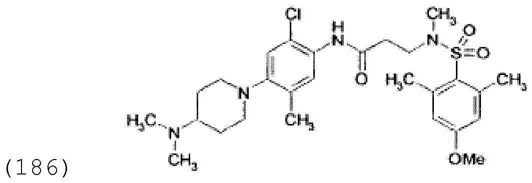 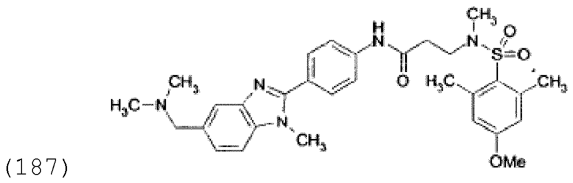 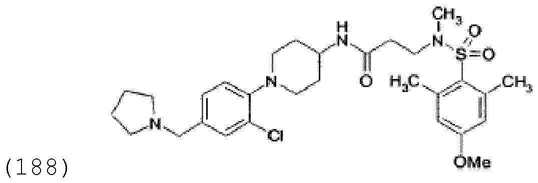 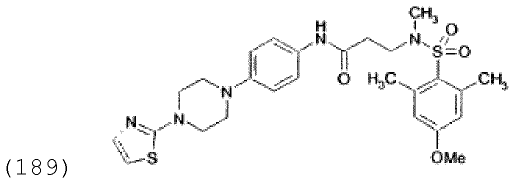 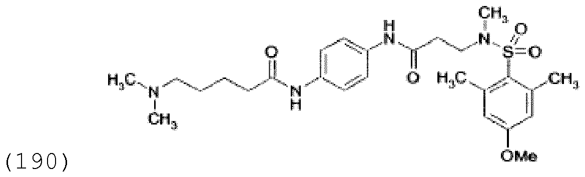 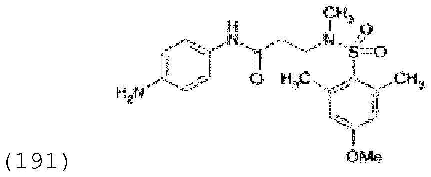 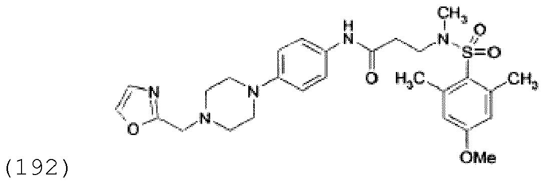 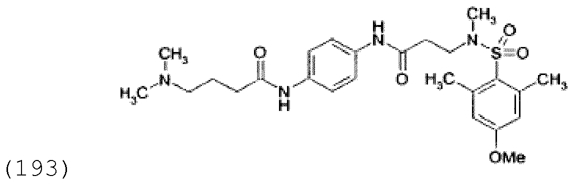 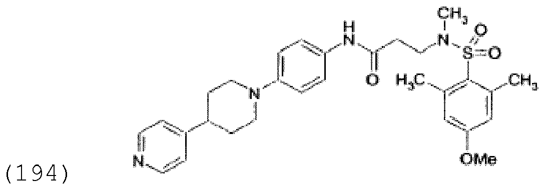 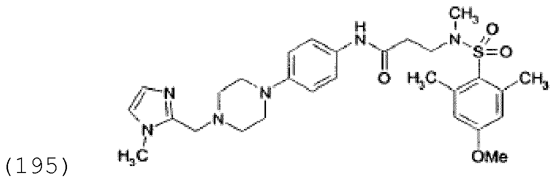 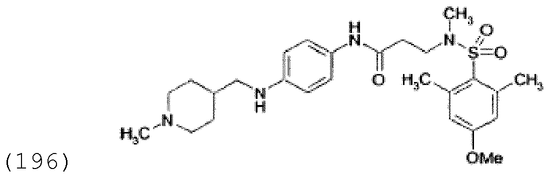 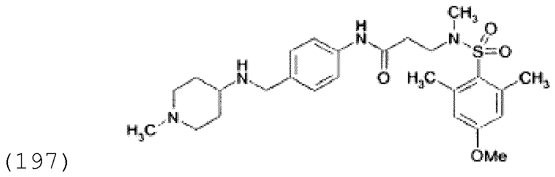 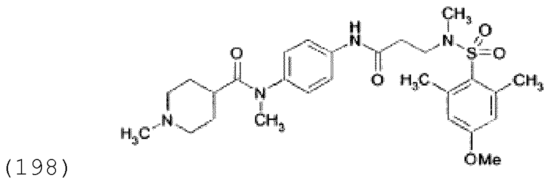 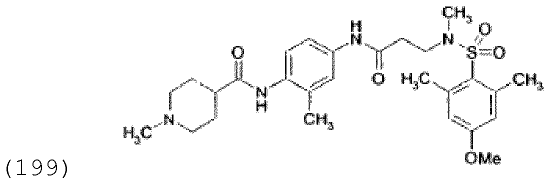 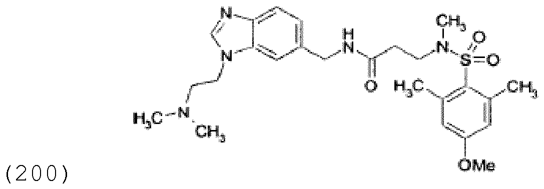 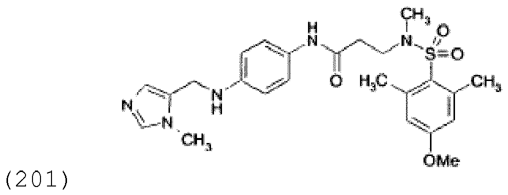 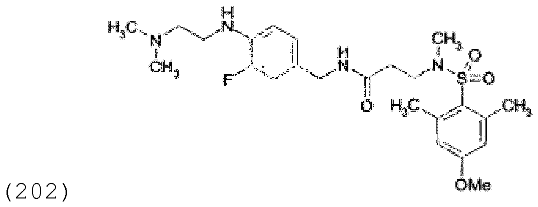 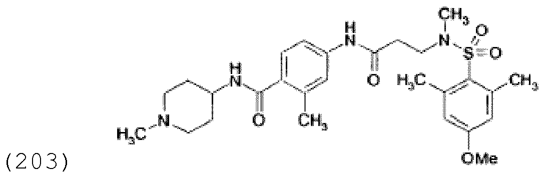 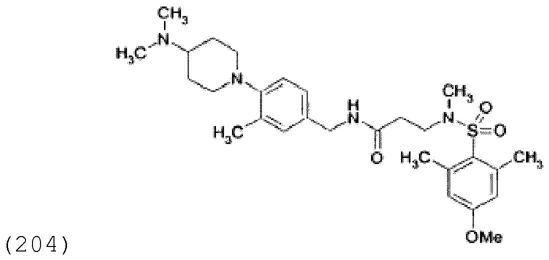 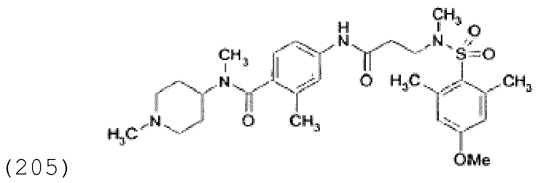 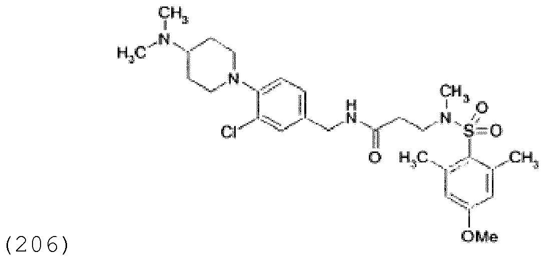 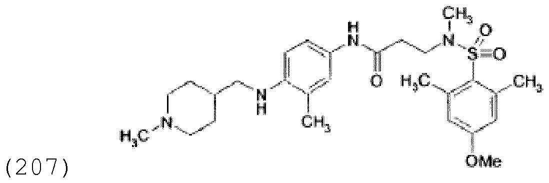 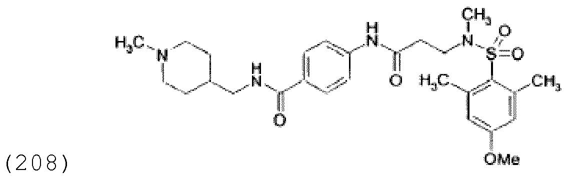 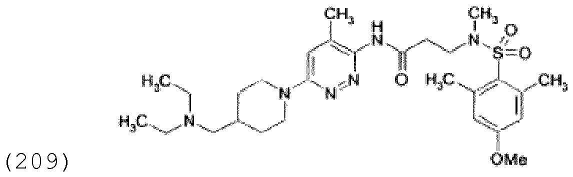 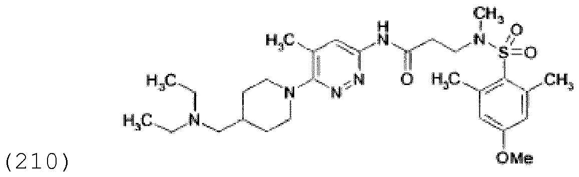 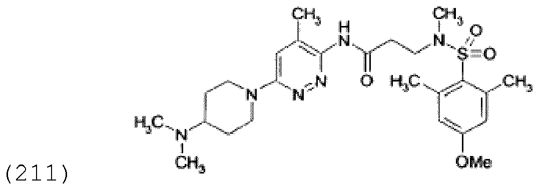 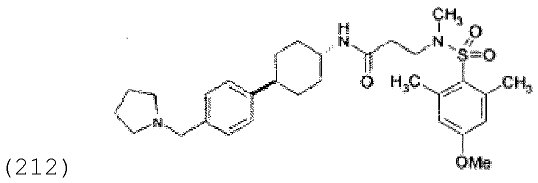 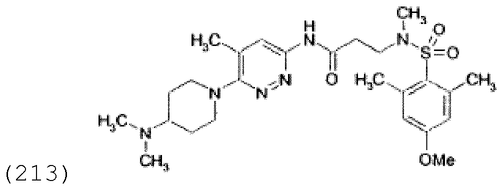 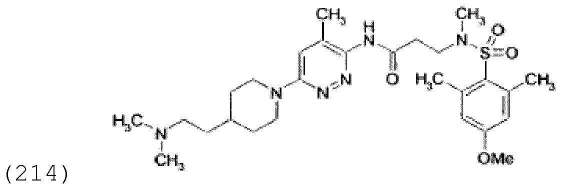 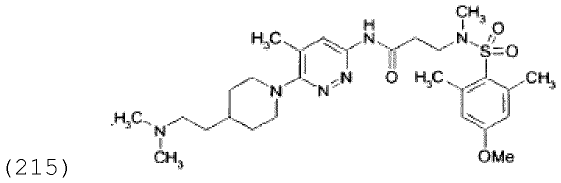 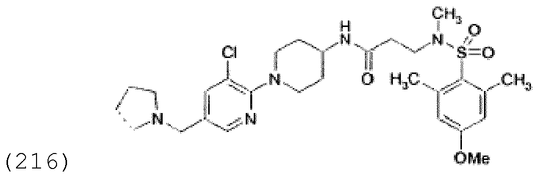 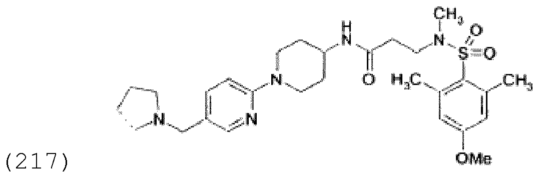 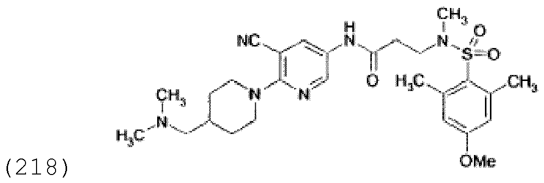 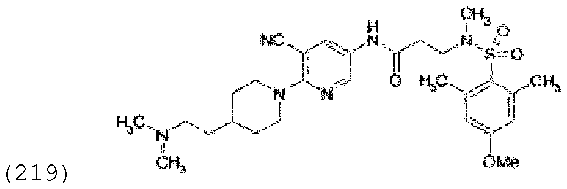 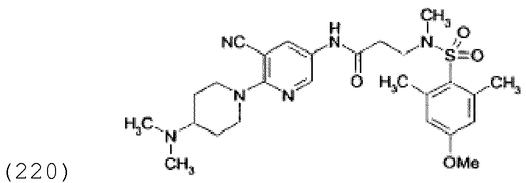 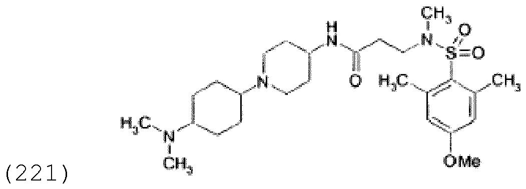 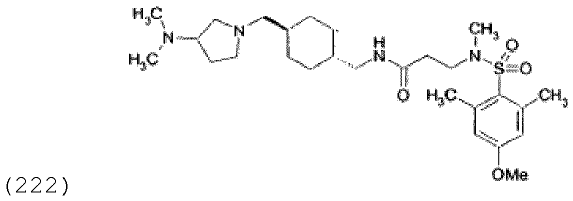 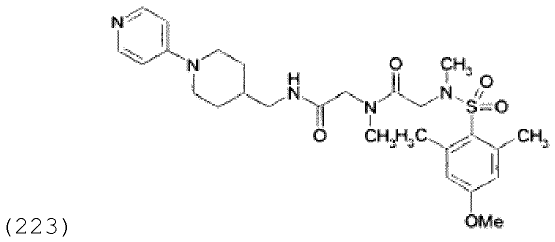 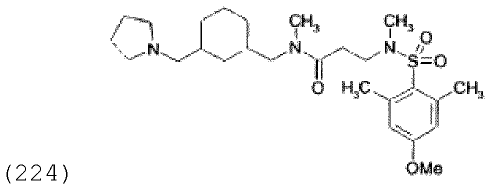 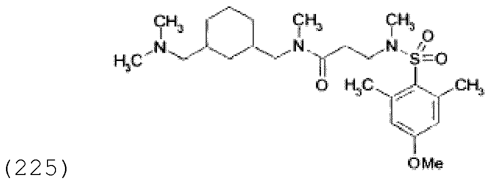 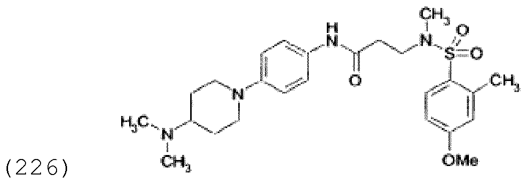 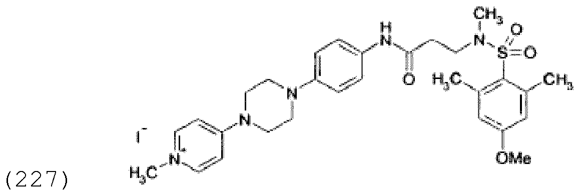 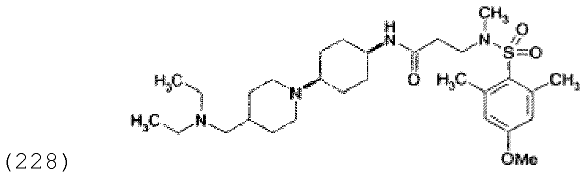 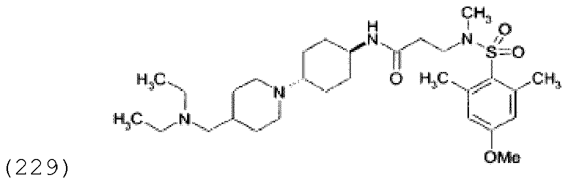 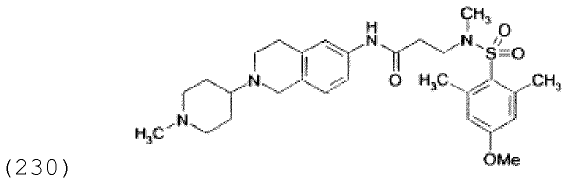 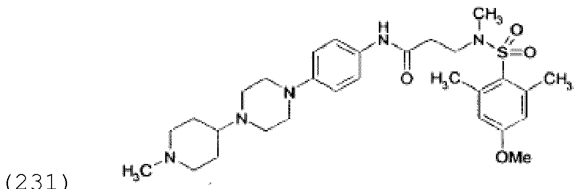 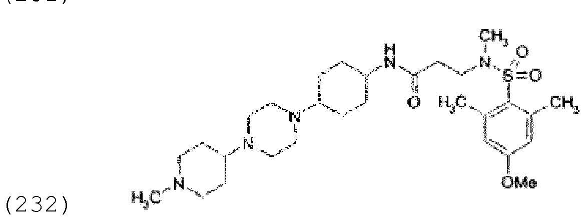 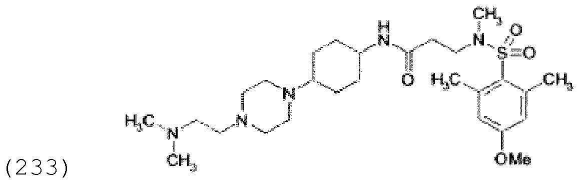 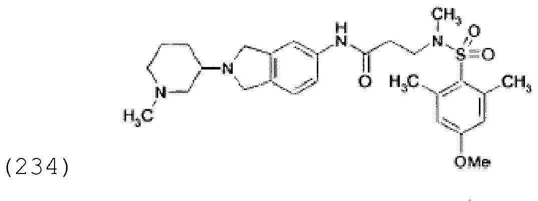 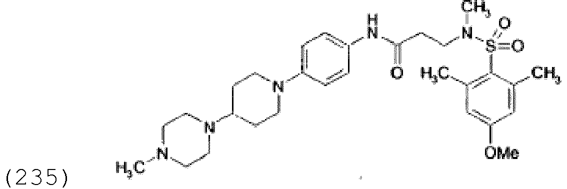 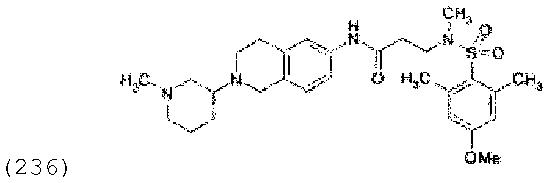 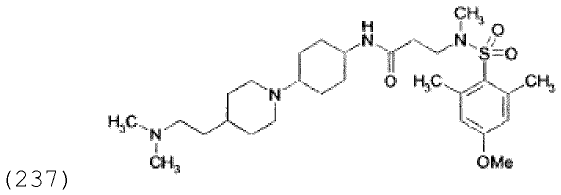 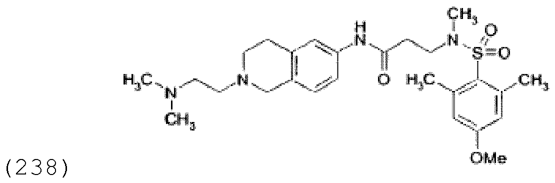 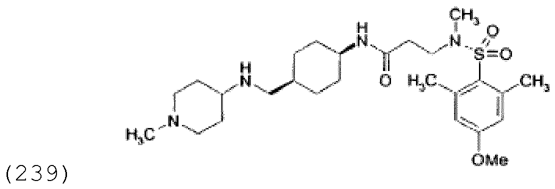 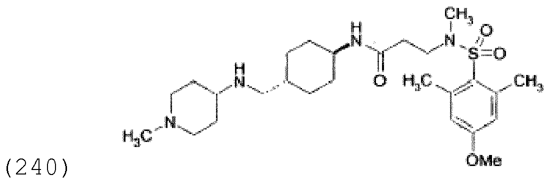 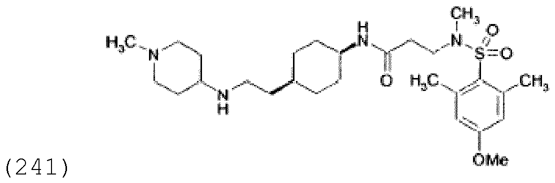 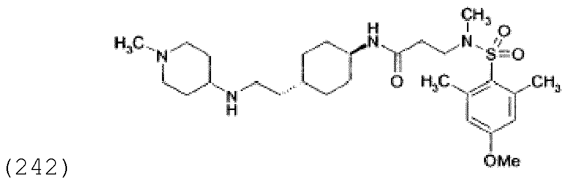 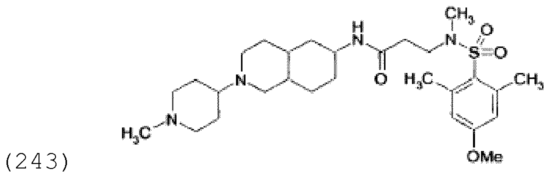 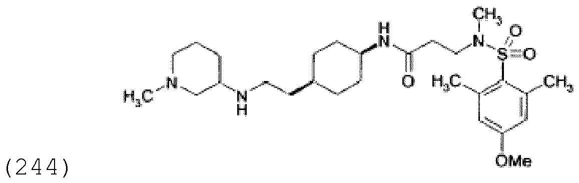 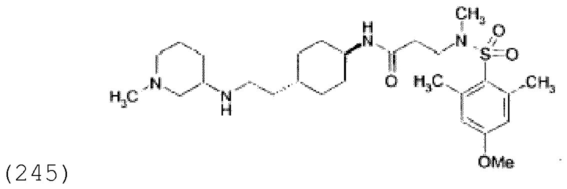 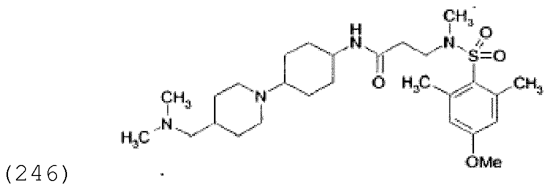 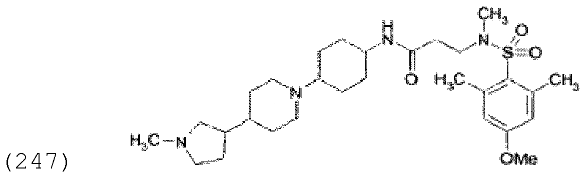 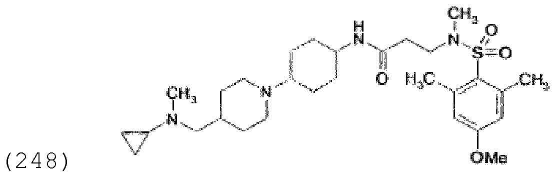 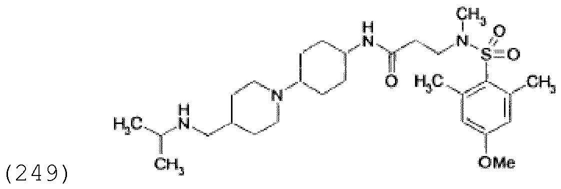 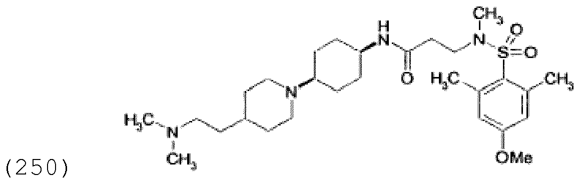 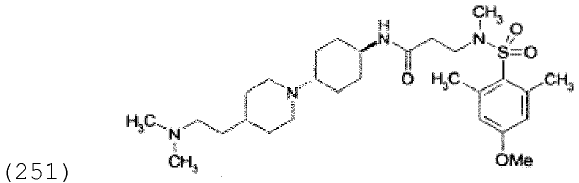 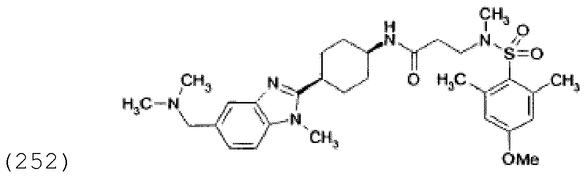 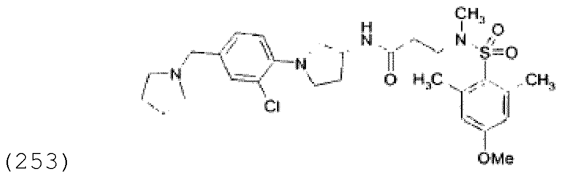 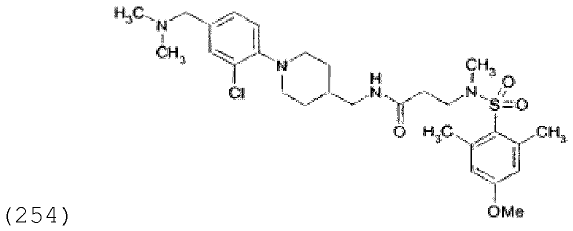 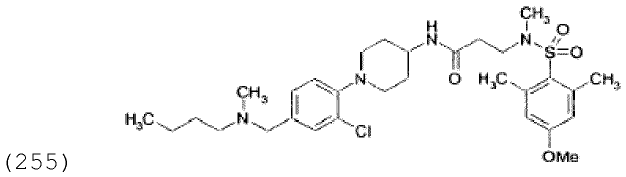 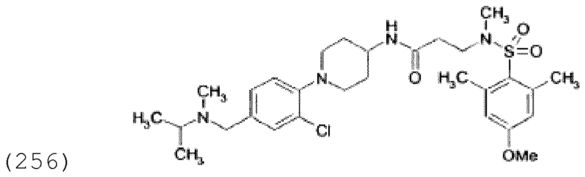 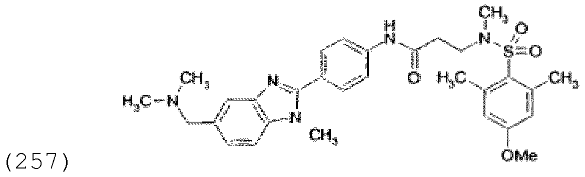 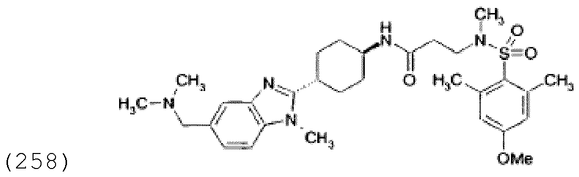 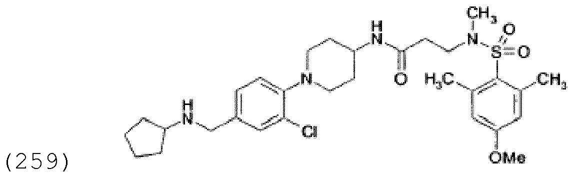 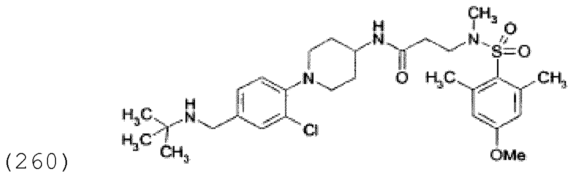 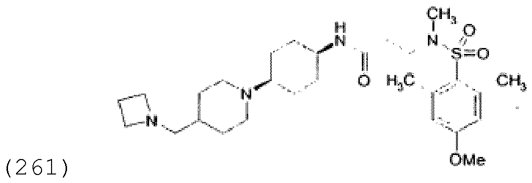 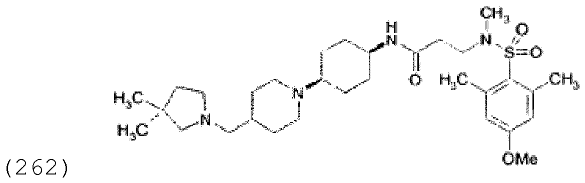 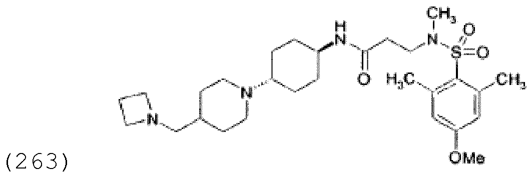 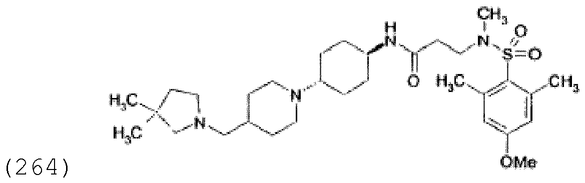 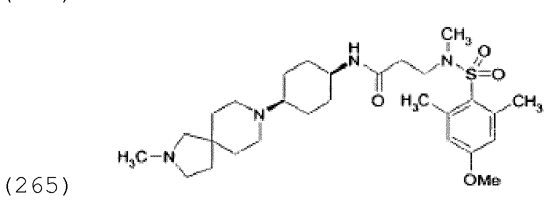 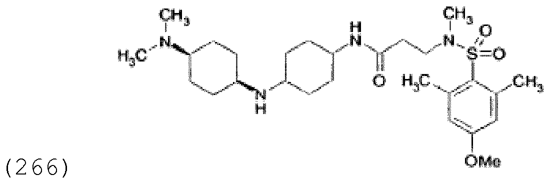 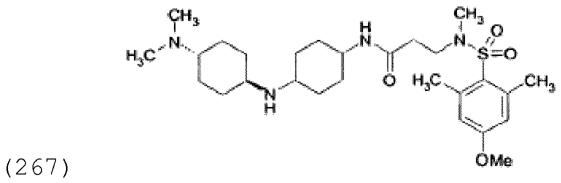 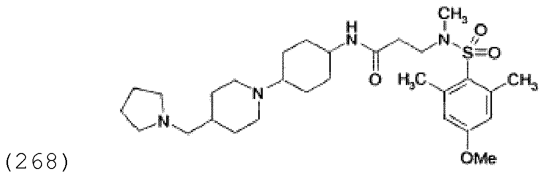 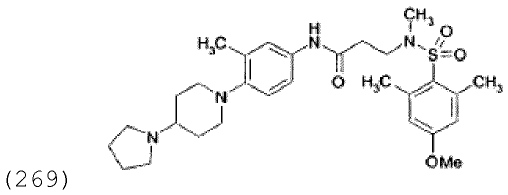 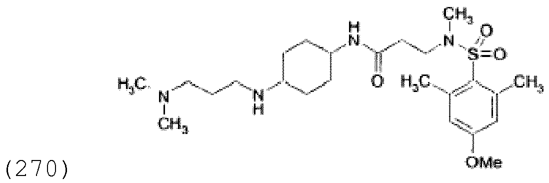 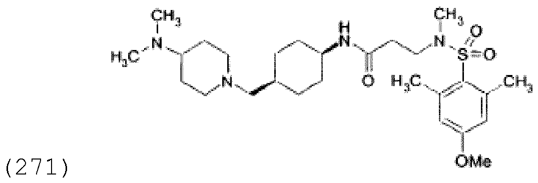 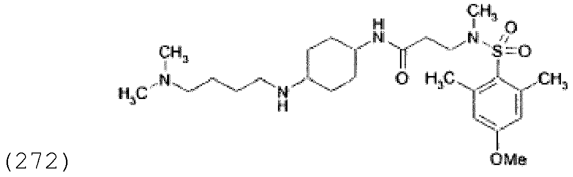 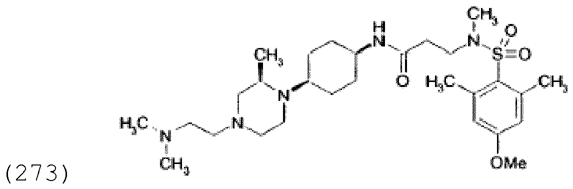 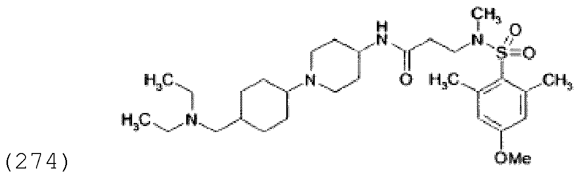 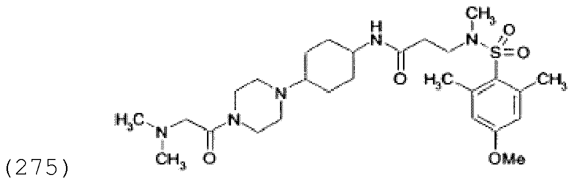 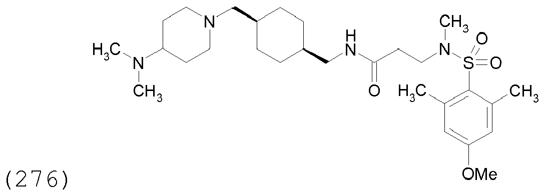 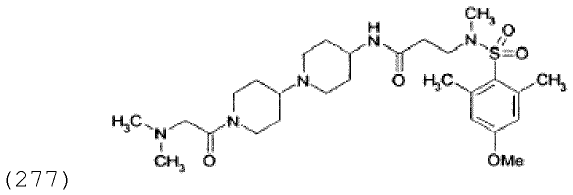 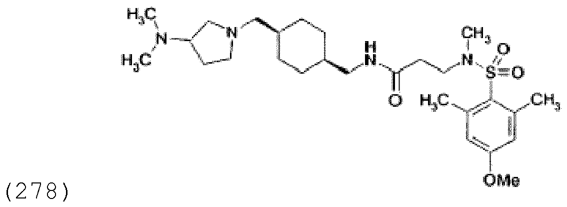 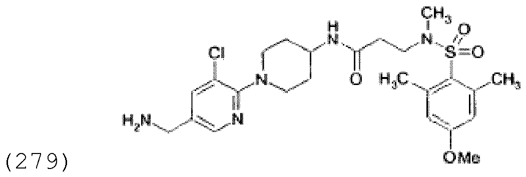 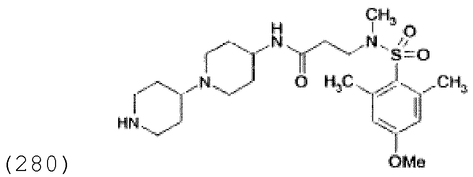 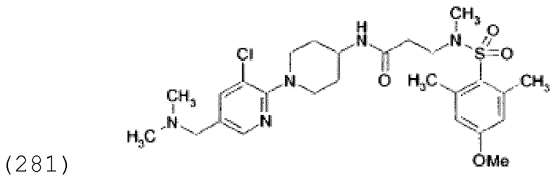 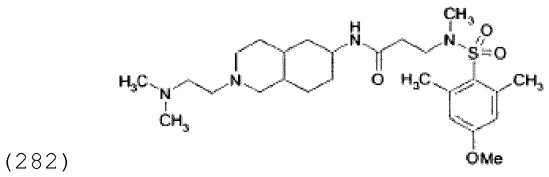 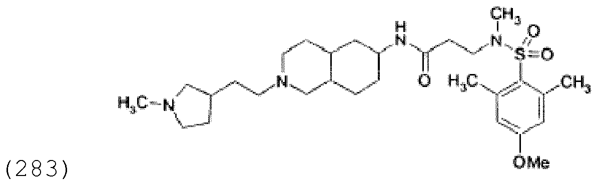 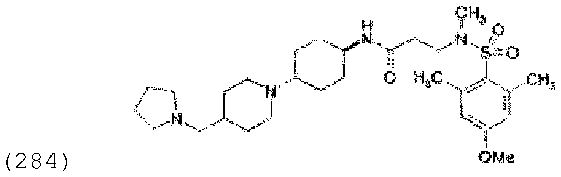 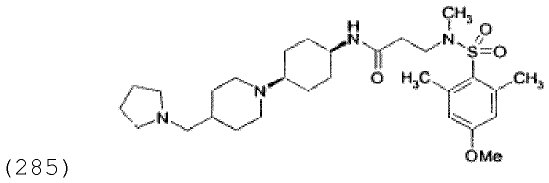 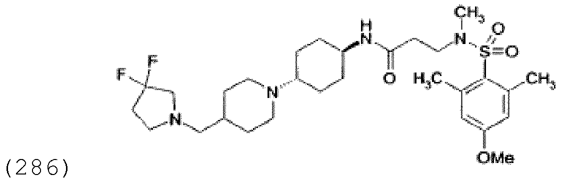 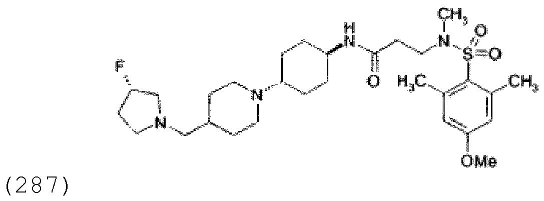 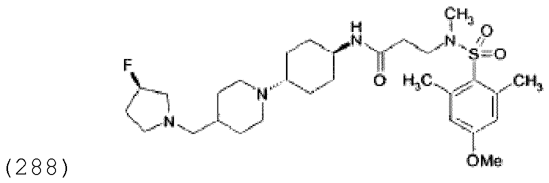 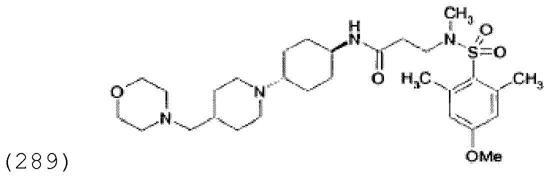 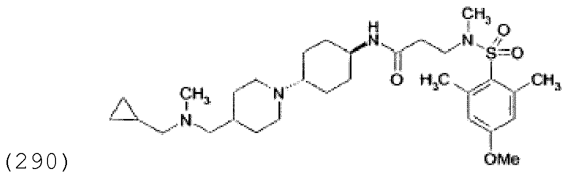 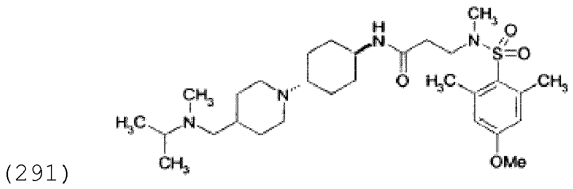 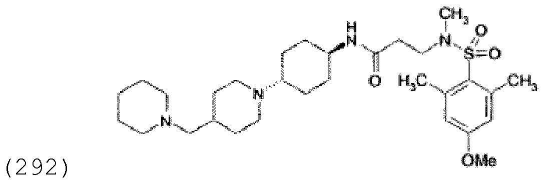 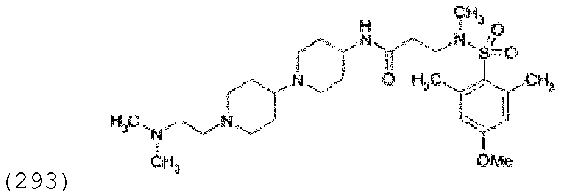 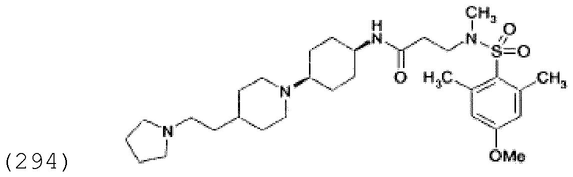 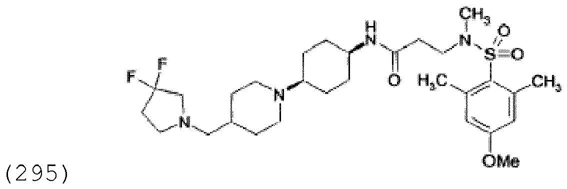 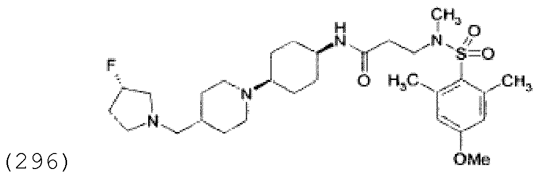 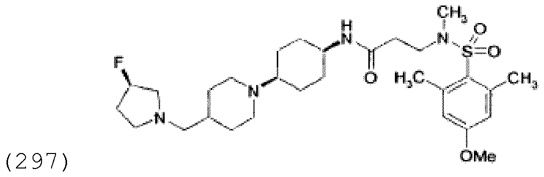 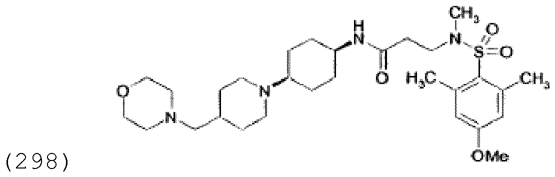 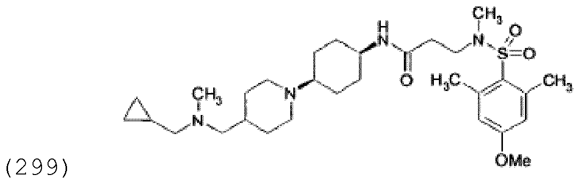 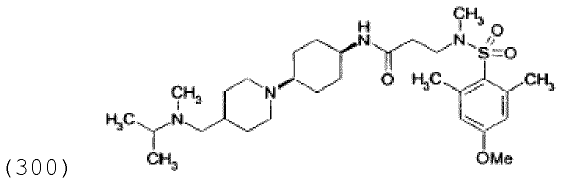 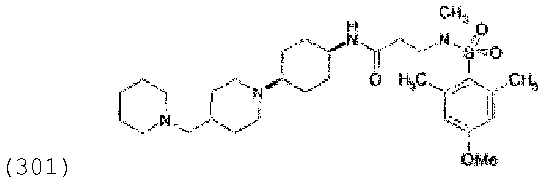 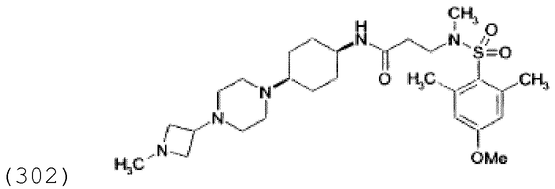 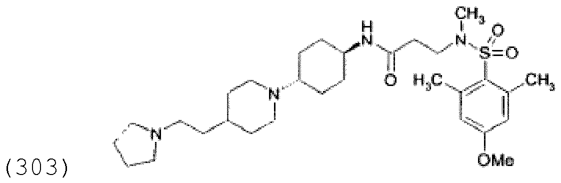 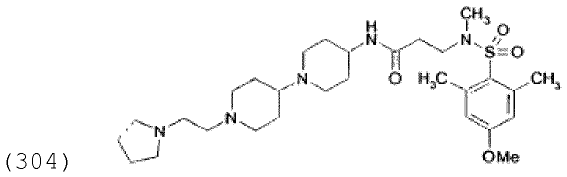 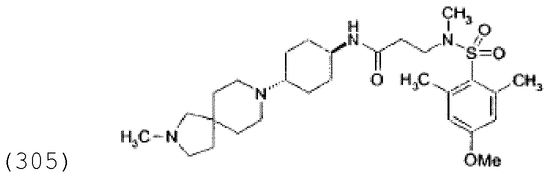 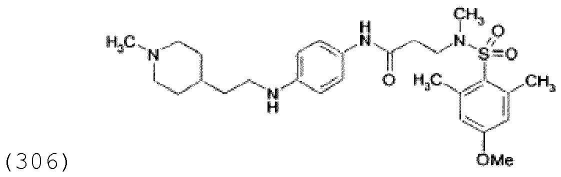 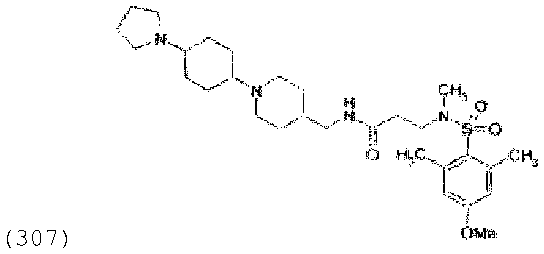 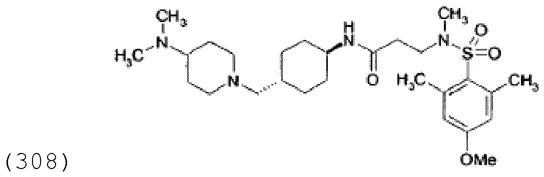 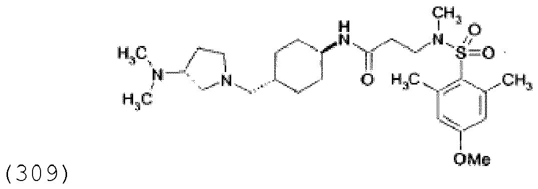 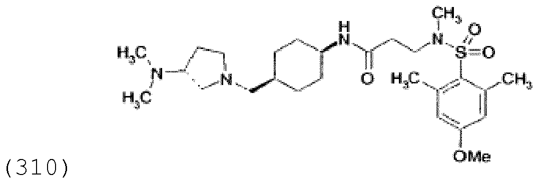 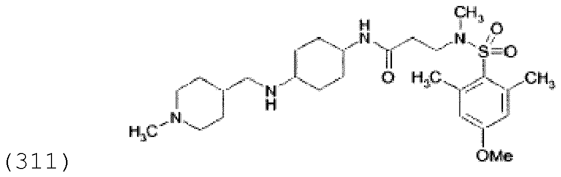 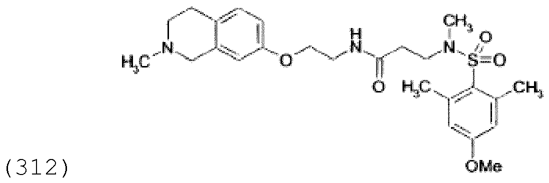 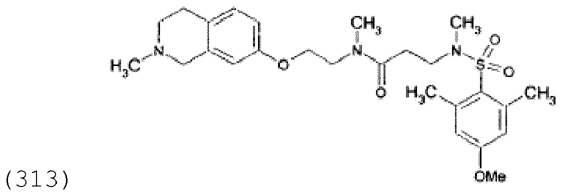 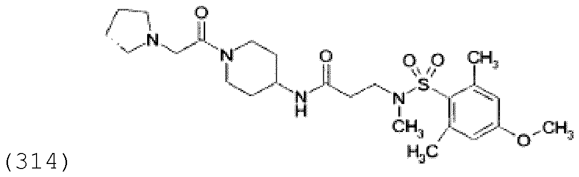 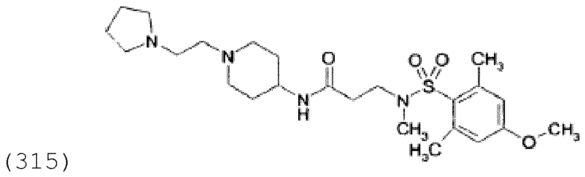 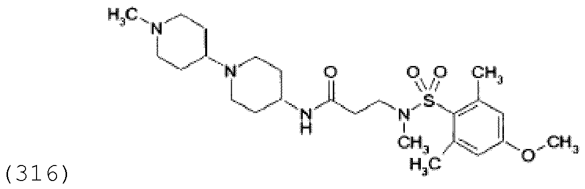 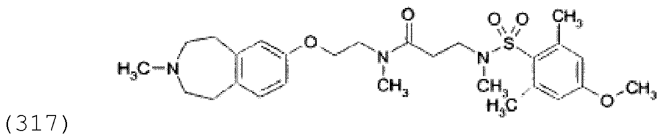 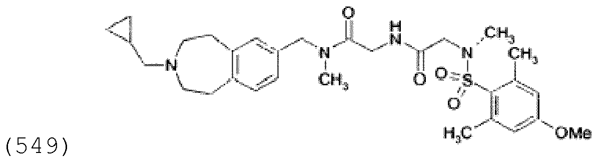 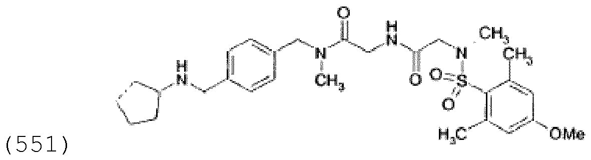 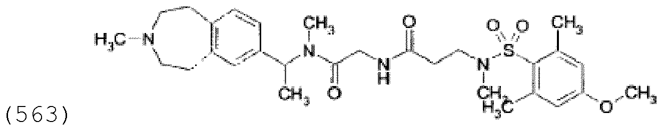 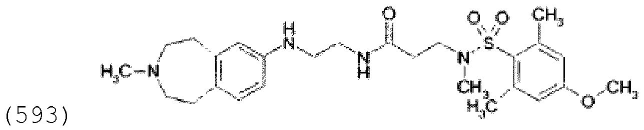 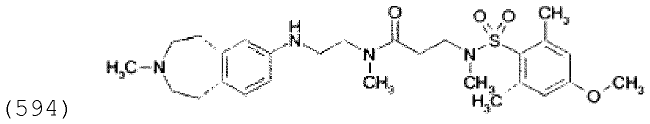 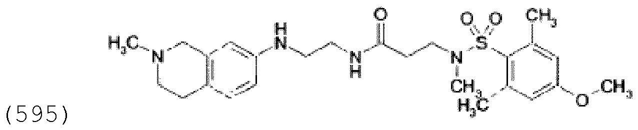 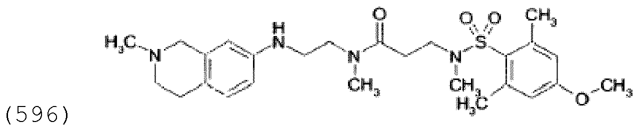 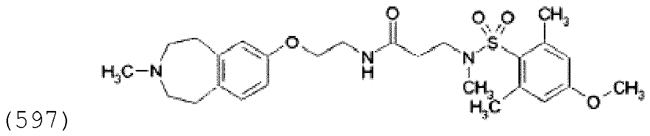 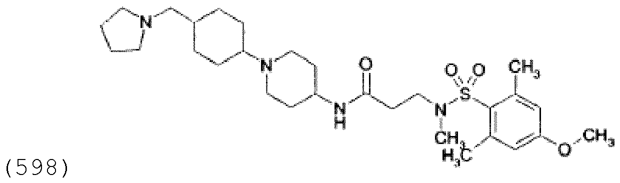 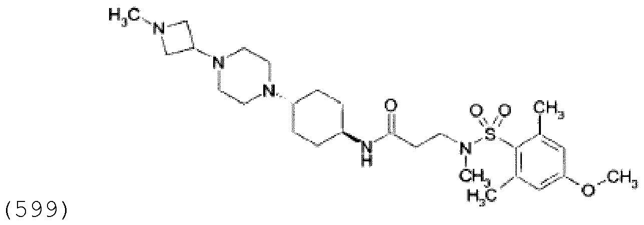 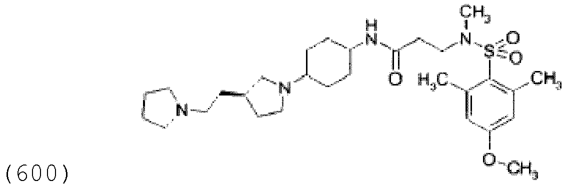 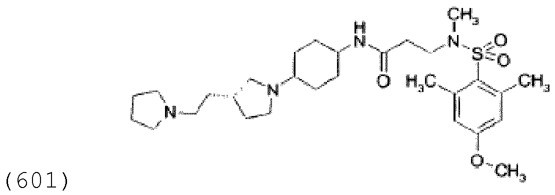 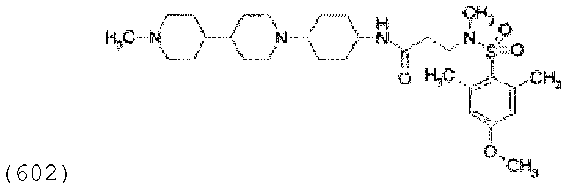 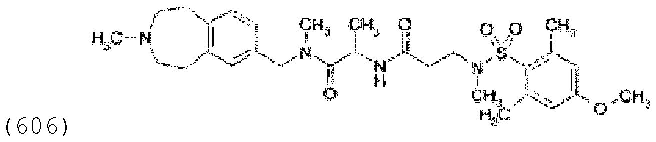 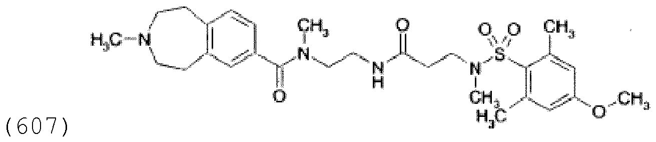 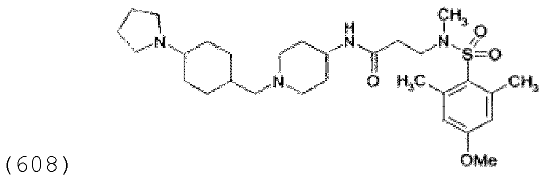 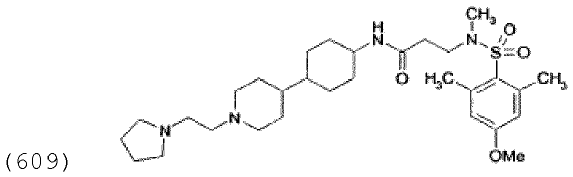 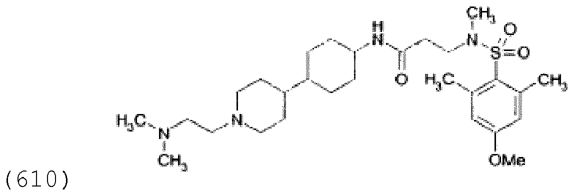 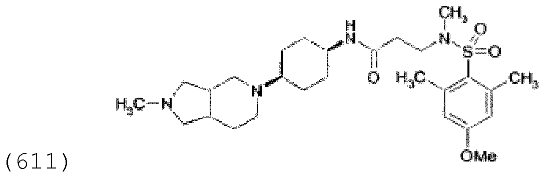 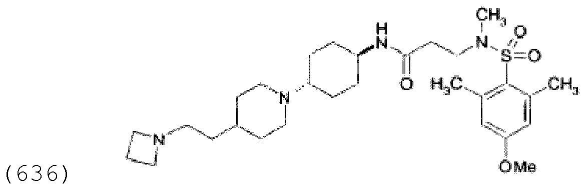 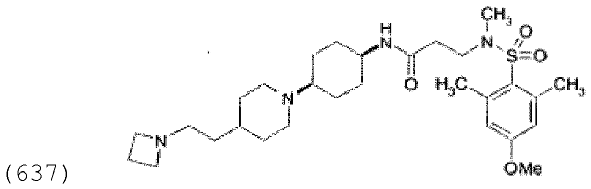 leurs énantiomères, leurs diastéréomères, leurs mélanges et leurs sels, en particulier, leurs sels physiologiquement acceptables avec des acides ou des bases organiques ou anorganiques.

2. Sels physiologiquement acceptables des composés selon la revendication 1 avec des acides ou des bases anorganiques ou organiques.

3. Médicament contenant un composé selon la revendication 1 ou un sel physiologiquement acceptable selon la revendication 2, outre éventuellement un ou plusieurs véhicules et/ou diluants inertes.

4. Utilisation selon la revendication 1 ou 2, pour le traitement aigu ou prophylactique des douleurs aiguës, des douleurs viscérales, des douleurs neuropathiques, des douleurs inflammatoires/ médiées par un récepteur nociceptif, des douleurs tumorales et des céphalées.

5. Procédé de production d'un médicament selon la revendication 3, **caractérisé en ce que**, de façon non chimique, un composé selon au moins l'une des revendications 1 à 2 est introduit dans un ou plusieurs adjuvants et/ou diluants inertes.
